(19) Europäisches Patentamt
European Patent Office
Office européen des brevets

(11) EP 4 494 657 A1

(12) EUROPEAN PATENT APPLICATION
published in accordance with Art. 153(4) EPC

(43) Date of publication:
**22.01.2025 Bulletin 2025/04**

(21) Application number: **23742950.1**

(22) Date of filing: **18.01.2023**

(51) International Patent Classification (IPC):
***A61K 47/68*** (2017.01) ***A61K 31/48*** (2006.01)
***A61K 31/4745*** (2006.01) ***C07K 16/30*** (2006.01)
***C07K 16/28*** (2006.01) ***A61P 35/00*** (2006.01)

(52) Cooperative Patent Classification (CPC):
**A61K 31/4745; A61K 31/48; A61K 47/68; A61P 35/00; C07K 16/28; C07K 16/30**

(86) International application number:
**PCT/CN2023/073014**

(87) International publication number:
**WO 2023/138635 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.01.2022 CN 202210062910**
**26.10.2022 CN 202211318840**

(71) Applicant: **GAN & LEE PHARMACEUTICALS CO., LTD.**
**Beijing 101109 (CN)**

(72) Inventors:
- **QIN, Wenfang**
  **Beijing 101109 (CN)**
- **YIN, Lei**
  **Beijing 101109 (CN)**
- **JIAO, Jiao**
  **Beijing 101109 (CN)**
- **GU, Mengjun**
  **Beijing 101109 (CN)**
- **CHEN, Yikun**
  **Beijing 101109 (CN)**
- **QIAO, Jing**
  **Beijing 101109 (CN)**

(74) Representative: **Kraus & Lederer PartGmbB**
**Thomas-Wimmer-Ring 15**
**80539 München (DE)**

Remarks:
The complete document including Reference Table(s) and the Sequence Listing(s) can be downloaded from the EPO website

# (54) EXATECAN DERIVATIVE-ANTIBODY CONJUGATE AND MEDICAL USE THEREOF

(57) Disclosed in the present invention is an antibody-drug conjugate of a novel Exatecan analogue, a pharmaceutical composition containing the conjugate, and a medical use of the conjugate and the pharmaceutical composition. The novel Exatecan analogue of the present invention has very strong tumour inhibition efficiency, and the provided TROP-2 antibody-drug conjugate and HER3 antibody-drug conjugate have good tumour inhibition effects and good prospects for clinical application.

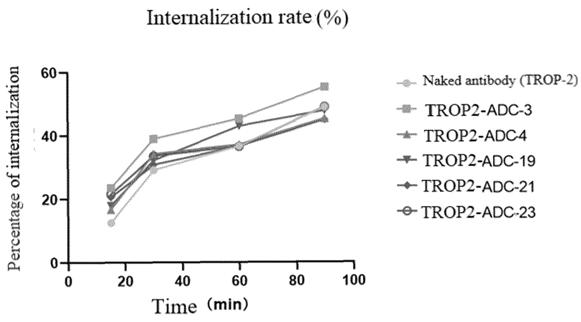


Figure 1

EP 4 494 657 A1

## Description

## TECHNICAL FIELD

The present invention relates to a class of antibody-drug conjugates of novel structural exatecan analogues. Specifically, the present disclosure relates to an antibody-drug conjugate of an exatecan analogue containing structural unit Y, a pharmaceutical composition comprising the conjugate and a use of the conjugate or pharmaceutical composition.

## BACKGROUND

Antibody-drug conjugates (ADCs) , as novel targeted drugs, generally consist of three components: an antibody or antibody-like ligand, a small molecule drug, and a linker that couples the ligand to the drug. ADCs utilize the specific recognition of antigens by antibodies to transport the drug molecules near the target cells and effectively release the drug molecules for therapeutic purposes. In 2000, Mylotarg, an ADC drug from Pfizer, was first launched on the market, since then, the promising and challenging field of ADCs has been in the public's view. In recent years, the pharmaceutical market has ushered in a new round of ADC research and development boom, with a total of 13 ADC drugs currently on the market worldwide.

As an antitumor small molecule compound, known as a camptothecin derivative that exhibits antitumor effects by inhibiting DNA topoisomerase I, exatecan was developed by Daiichi Sankyo, and was advanced to phase III clinical trials in the early stages of its use as a stand-alone chemotherapeutic agent, with the main indications for bone, prostate, breast, and pancreatic cancers. Unlike irinotecan, which is currently in clinical use, exatecan does not require activation through the use of enzymes. In addition, compared with SN-38, which is the pharmacodynamic principal of irinotecan, and topotecan, which is also used in the clinic, exatecan has stronger inhibitory activity against topoisomerase I, and has stronger cytotoxicity against a wide range of cancer cells in vitro. Exatecan has not been successfully marketed as a stand-alone chemotherapeutic agent and it is hypothesized that this is related to its higher cellular activity, resulting in a narrow therapeutic window.

Daiichi Sankyo/AstraZeneca co-developed and commercialized antibody-coupled drug DS-8201a (trade name: Enhertu) in December 2019, which combines exatecan with hydroxyacetic acid to form an amide derivative and linkage to form an ADC, as a next-generation antibody-coupled drug, Enhertu is already showing potential to become a blockbuster.

It is necessary and urgent to explore and discover exatecan derivatives with better antitumor activity to improve the safety and efficacy of antitumor small molecule compounds in ADC drug applications, so as to obtain antitumor drugs with excellent efficacy.

## SUMMARY

The present invention provides a ligand-drug conjugate or a pharmaceutically acceptable salt thereof, wherein the ligand-drug conjugate comprises a structure shown by formula ($-D_0$ ):

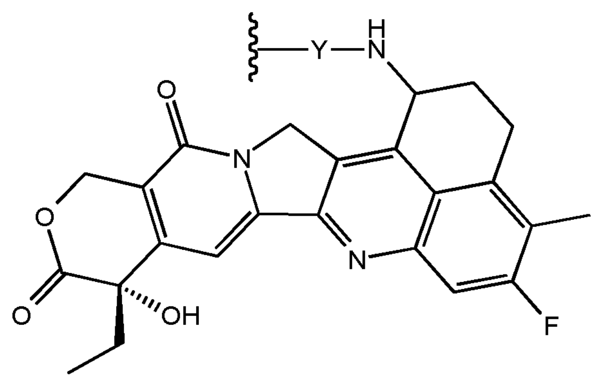

**($-D_0$)**

wherein, Y is

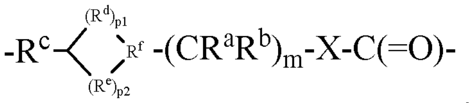 , 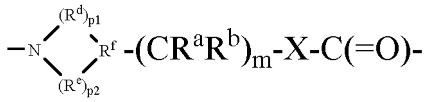 ,

$-R^c-Ar^1-(Cy)_m-(CR^aR^b)_m-X-C(=O)-$, $-N((CR^aR^b)_m\ CF_3)-C(=O)-$, $-R^c-(CR^aR^b)_m-C(=O)-R^c-Ar^1-(Cy)_m-(CR^aR^b)_m-X-C(=O)-$,

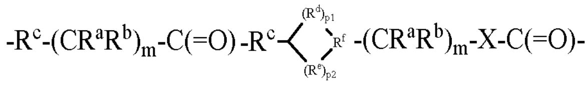 ,

or

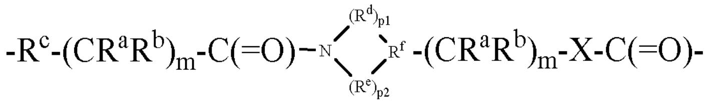 ,

wherein the C(=O)- end of Y is connected to -NH- in the structure of formula ($-D_0$); preferably, Y is

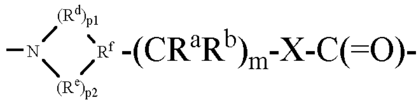 ,

, $-O-Ar^1-(Cy)_m-(CR^aR^b)_m-X-C(=O)-$, $-NH-Ar^1-(Cy)_m-(CR^aR^b)_m-X-C(=O)-$, $-N((CR^aR^b)_mCF_3)-C(=O)-$, $-O-(CR^aR^b)_m-C(=O)-NH-Ar^1-(Cy)_m-(CR^aR^b)_m-X-C(=O)-$, or

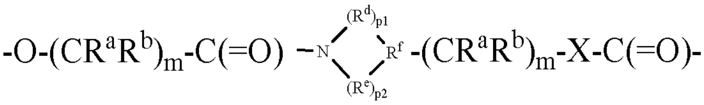 ,

wherein the C(=O)- end of Y is connected to -NH- in the structure of formula ($-D_0$);

$R^c$ at each occurrence is each independently NH, O, or S; $R^d$ and $R^e$ at each occurrence are each independently $C(R^m)_2$, $NR^m$, O, or S; $R^f$ at each occurrence is each independently $CR^m$ or N; preferably, when the ring formed by $R^d$, $R^e$, and $R^f$ is a heterocyclyl group, the heterocyclic group contains 1, 2, 3, or 4 heteroatoms, wherein the heteroatoms are each independently selected from N, O, and S;

$R^m$ at each occurrence is each independently H, halogen, haloalkyl , -OH, - CN, $-NO_2$, alkyl, alkoxy, $-NH_2$, -NH-alkyl, or $-N(alkyl)_2$, deuterium, deuterated alkyl, aminoalkylene, hydroxyalkylene, nitroalkylene, or cyanoalkylene; preferably, $R^m$ at each occurrence is each independently H, halogen, haloalkyl , -OH, -CN, $-NO_2$, alkyl, alkoxy, $-NH_2$, -NH-alkyl, or $-N(alkyl)_2$;

p1 is an integer from 0 to 17, p2 is an integer from 1 to 18, and $p1+p2\leq 18$; preferably, p1 is an integer of 0, 1, 2, 3, 4, 5, 6, or 7, p2 is an integer of 1, 2, 3, 4, 5, 6, 7, or 8, and $p1+p2\leq 8$;

Cy at each occurrence is each independently selected from cycloalkylene, heterocyclylene, arylene,or heteroarylene; preferably, Cy at each occurrence is each independently selected from 3-10 membered cycloalkylene, 3-10 membered heterocyclylene, 6-10 membered arylene, or 5-10 membered heteroarylene; preferably, the heterocyclylene and heteroarylene contain 1, 2, 3, or 4 heteroatoms, wherein the heteroatoms are independently selected from N, O, and S; the cycloalkylene, heterocyclylene, arylene, and heteroarylene are unsubstituted or optionally substituted with a substituent selected from halogen, -OH, -CN, haloalkyl, $-NO_2$, alkyl, alkoxy, $-NH_2$, -NH-alkyl, and $-N(alkyl)_2$, preferably unsubstituted or optionally substituted with a substituent selected from halogen, -OH, -CN, $-CF_3$, $-NO_2$, $C_{1-6}$ alkyl, $-OC_{1-6}$ alkyl, $-NH_2$, $-NH(C_{1-6}$ alkyl), and $-N(C_{1-6}$ alkyl$)_2$;

$Ar^1$ at each occurrence is each independently selected from arylene or heteroarylene; preferably, $Ar^1$ at each occurrence is each independently selected from 6-10 membered arylene or 5-10 membered heteroarylene; preferably, the heteroarylene contains 1, 2, 3, or 4 heteroatoms, wherein the heteroatoms are independently selected from N, O, and S; the arylene and heteroarylene are unsubstituted or optionally substituted with a substituent selected from halogen, -OH, -CN, haloalkyl, $-NO_2$, alkyl, alkoxy, $-NH_2$, -NH-alkyl, and $-N(alkyl)_2$, preferably, unsubstituted or

optionally substituted with a substituent selected from halogen, -OH, -CN, $-CF_3$, $-NO_2$, $C_{1-6}$ alkyl, $-OC_{1-6}$ alkyl, $-NH_2$, $-NH(C_{1-6}$ alkyl), and $-N(C_{1-6}$ alkyl$)_2$;

$R^a$ and $R^b$ are identical or different, and at each occurrence each independently selected from hydrogen, halogen, haloalkyl , -OH, -CN, $-NO_2$, alkyl, alkoxy, $-NH_2$, -NH-alkyl, or -N(alkyl$)_2$, deuterium, deuterated alkyl, aminoalkylene, hydroxyalkylene, nitroalkylene and cyanoalkylene; preferably, at each occurrence each independently selected from hydrogen, deuterium, halogen, alkyl, haloalkyl , deuterated alkyl, alkoxy, -OH, $-NH_2$, -CN, nitro, and hydroxyalkylene; preferably, at each occurrence each independently selected from hydrogen, deuterium, halogen, $C_{1-6}$ alkyl, halogenated $C_{1-6}$ alkyl, deuterated $C_{1-6}$ alkyl, $-OC_{1-6}$ alkyl, -OH, $-NH_2$, -CN, $-CF_3$, nitro, $-C_{1-6}$ alkylene, and -OH;

X at each occurrence is independently a single bond, -NH-, O, or S;

the wavy line in formula $(-D_0)$ represents a covalent attachment to a linker unit, an antibody or its antigen-binding fragment or a polypeptide binding to an antigen expressed by a target cell; and

m at each occurrence is each independently an integer of 0, 1, 2, 3, or 4.

The present invention provides a ligand-drug conjugate or a pharmaceutically acceptable salt thereof, wherein the ligand-drug conjugate comprises a structure shown by formula (-D):

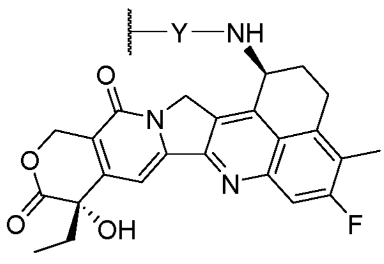

**(-D)**

wherein, Y is

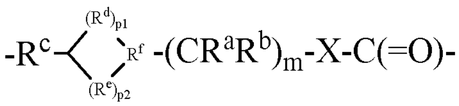,

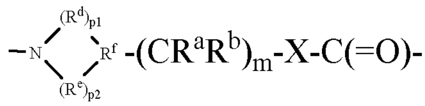,

$-R^c-Ar^1-(Cy)_m-(CR^aR^b)_m-X-C(=O)-$, $-N((CR^aR^b)_mCF_3)-C(=O)-$, $-R^c-(CR^aR^b)_m-C(=O)-R^c-Ar^1-(Cy)_m-(CR^aCR^b)_m-X-C(=O)-$,

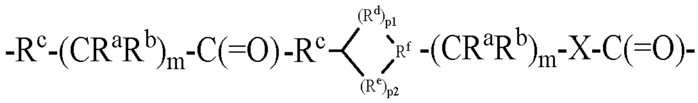,

or

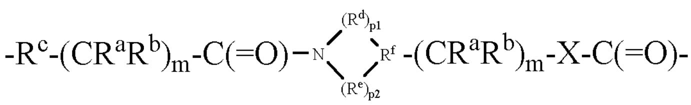,

wherein the C=O- end of Y is connected to -NH- in the structure of formula (-D); preferably, Y is

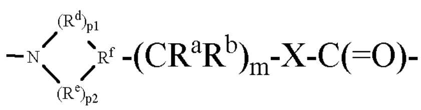,

$-O-Ar^1-(Cy)_m-(CR^aR^b)_m-X-C(=O)-$, $-NH-Ar^1-(Cy)_m-(CR^aR^b)_m-X-C(=O)-$, $-N((CR^aR^b)_mCF_3)-C(=O)-$, $-O-(CR^aR^b)_m-C(=O)-NH-Ar^1-(Cy)_m-(CR^aR^b)_m-X-C(=O)-$, or

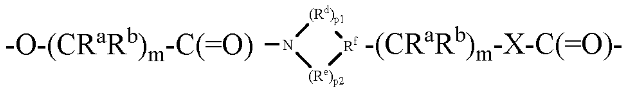

,

wherein the C(=O)- end of Y is connected to -NH- in the structure of Formula (-D);

$R^c$ at each occurrence is each independently NH, O, or S; $R^d$ and $R^e$ at each occurrence are each independently $C(R)^m{}_2$, $NR^m$, O, or S; $R^f$ at each occurrence is independently $CR^m$ or N; preferably, when the ring formed by $R^d$, $R^e$ and $R^f$ is heterocyclyl, the heterocyclyl contains 1, 2, 3, or 4 heteroatoms, and the heteroatoms are each independently selected from N, O, and S; $R^m$ at each occurrence is each independently H, halogen, haloalkyl, - OH, -CN, $-NO_2$, alkyl, alkoxy, $-NH_2$, -NH-alkyl, or $-N(alkyl)_2$, deuterium atom, deuterated alkyl, aminoalkylene, hydroxyalkylene, nitroalkylene, or cyanoalkylene, preferably, $R^m$ at each occurrence is each independently H, halogen, haloalkylene, -OH, -CN, $-NO_2$, alkyl, alkoxy, $-NH_2$, -NH-alkyl, or - $N(alkyl)_2$; p1 is an integer from 0 to 17, p2 is an integer from 1 to 18, and $p1 + p2 \leq 18$; preferably, p1 is an integer of 0, 1, 2, 3, 4, 5, 6, or 7, and p2 is an integer of 1, 2, 3, 4, 5, 6, 7, or 8, and $p1 + p2 \leq 8$; the Cy at each occurrence is each independently selected from cycloalkylene, heterocyclylene, arylene,or heteroarylene, preferably the Cy at each occurrence is each independently selected from 3-10 membered cycloalkylene, 3-10 membered heterocyclylene, 6-10 membered arylene, or 5-10 membered heteroarylene; preferably, the heterocyclylene and heteroarylene contain 1, 2, 3, or 4 heteroatoms, and the heteroatoms are independently selected from N, O and S; the cycloalkylene, heterocyclylene, arylene, andheteroarylene are unsubstituted or optionally substituted with a substituent selected from halogen, -OH, -CN, haloalkylene, $-NO_2$, alkyl, alkoxy, $-NH_2$, -NH-alkyl, and $-N(alkyl)_2$, preferably unsubstituted or optionally substituted with a substituent selected from halogen, -OH, -CN, $-CF_3$, $-NO_2$ , $C_{1-6}$ alkyl, $-OC_{1-6}$ alkyl, $-NH_2$ , $-NH(C_{1-6}$ alkyl), and $-N(C_{1-6}$ $alkyl)_2$;

the $Ar^1$ at each occurrence is each independently selected from arylene or heteroarylene, preferably, the $Ar^1$ at each occurrence is each independently selected from 6-10 membered arylene or 5-10 membered heteroarylene; preferably the heteroarylene comprises 1, 2, 3, or 4 heteroatoms, and the heteroatoms are independently selected from N, O, and S; the arylene and heteroarylene are unsubstituted or optionally substituted with a substituent selected from halogen, -OH, -CN, haloalkyl, $-NO_2$, alkyl, alkoxy, $-NH_2$, -NH-alkyl, and $-N(alkyl)_2$, preferably, unsubstituted or optionally substituted with a substituent selected from halogen, -OH, -CN, $-CF_3$, $-NO_2$ , $C_{1-6}$ alkyl, $-OC_{1-6}$ alkyl, $-NH_2$, $-NH(C_{1-6}$ alkyl), and $-N(C_{1-6}$ $alkyl)_2$;

$R^a$ and $R^b$ are identical or different, and at each occurrence is each independently selected from hydrogen atom, halogen, haloalkyl, -OH, -CN, - $NO_2$, alkyl, alkoxyl, $-NH_2$, -NH-alkyl, or $-N(alkyl)_2$, deuterium atom, deuterated alkyl, aminoalkylene, hydroxyalkylene, nitroalkylene, and cyanoalkylene, preferably, at each occurrence each independently selected from hydrogen atom, deuterium atom, halogen, alkyl, haloalkyl, deuterated alkyl, alkoxy, -OH, $-NH_2$, -CN, nitro, and hydroxyalkylene, preferably, at each occurrence each independently selected from hydrogen atom, deuterium atom, halogen, $C_{1-6}$ alkyl, $C_{1-6}$ haloalkyl, deuterated $C_{1-6}$ alkyl, $-OC_{1-6}$ alkyl, -OH, - $NH_2$, -CN, $-CF_3$, nitro, $-C_{1-6}$ alkylene and -OH-;

X at each occurrence is each independently a single bond, -NH-, O, or S;

the wavy line in formula (-D) represents a covalent attachment to a linker unit, an antibody or an antigen-binding fragment thereof, or a polypeptide binding to an antigen expressed by a target cell; preferably, the wavy line in formula $(-D_0)$ represents a covalent attachment through a linker unit to an antibody or an antigen-binding fragment thereof, or a polypeptide binding to an antigen expressed by a target cell; and

m at each occurrence is each independently an integer of 0, 1, 2, 3, or 4.

In some embodiments of the present invention, there provide a ligand-drug conjugate or a pharmaceutically acceptable salt thereof, which is a ligand-drug conjugate shown by the general formula $(Pc-L-D_0)$ or the general formula (Pc-L-D), or a pharmaceutically acceptable salt thereof:

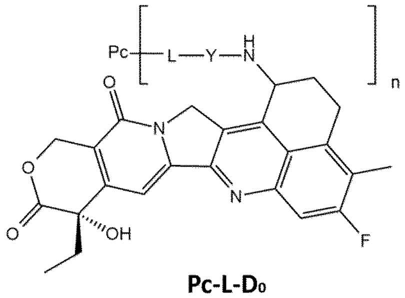

Pc-L-$D_0$

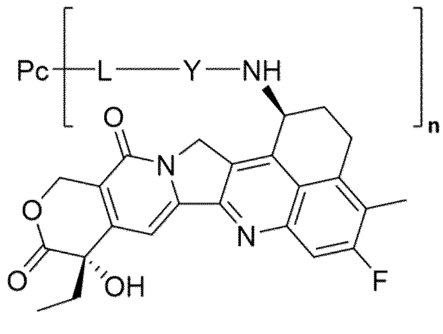

Pc-L-D

Wherein, Y is as described above, n is an integer or a decimal from 1 to 15; preferably, n is an integer or a decimal from 1 to 13; preferably, n is an integer or a decimal from 1 to 10; more preferably, n is an integer or a decimal from 3 to 8; Pc is a ligand; and L is a linker.

In some embodiments of the present invention, $R^c$ at each occurrence is each independently NH, or O; $R^d$ and $R^e$ at each occurrence are each independently $C(R^m)_2$ or $NR^m$; $R^f$ at each occurrence is $CR^m$; and when the ring formed by $R^d$, $R^e$, and $R^f$ is heterocyclyl, the heterocyclyl contains 1, 2, or 3 heteroatoms, and the heteroatoms are each independently selected from N and O;

In some embodiments, $R^m$ at each occurrence is each independently H, halogen, -OH, -CN, $-NO_2$, $-CF_3$, $-C_{1-6}$ alkyl, $-OC_{1-6}$ alkyl, $-NH_2$, $-NH(C_{1-6}$ alkyl), or $-N(C_{1-6}$ alkyl$)_2$ , preferably H, halogen, -OH, -CN, $-CF_3$, $-NO_2$ , $C_{1-6}$ alkyl, or $-OC_{1-6}$ alkyl, preferably H, halogen, -OH, -CN, $-CF_3$, $-NO_2$, $C_{1-3}$ alkyl, or $-OC_{1-3}$ alkyl, preferably -Cl, -Br, -F, -OH, -CN, $-CF_3$, $-NO_2$ , $-CH_3$ , or - $OCH_3$;

In some embodiments, p1 is an integer of 0, 1, 2, or 3, p2 is an integer of 1, 2, 3, or 4, and $2 \leq p1 + p2 \leq 4$;

In some embodiments, the Cy at each occurrence is each independently selected from 6-10 membered arylene, or 5-10 membered heteroarylene, preferably selected from phenylene, or 5-6 membered heteroarylene; preferably, the heteroarylene contains 1, 2, or 3 heteroatoms, and the heteroatoms are independently selected from N, O, and S; the heteroarylene is unsubstituted or optionally substituted with a substituent selected from halogen, -OH, -CN, $-CF_3$, $-NO_2$ , $C_{1-6}$ alkyl, $-OC_{1-6}$ alkyl, $-NH_2$, $-NH(C_{1-6}$ alkyl), and $-N(C_{1-6}$ alkyl$)_2$, preferably unsubstituted or optionally substituted with a substituent selected from halogen, -OH, -CN, $-CF_3$, $-NO_2$, $C_{1-6}$ alkyl, and $-OC_{1-6}$alkyl, preferably unsubstituted or optionally substituted with a substituent selected from halogen, -OH, -CN, $-CF_3$, $-NO_2$, $C_{1-3}$ alkyl, and - $OC_{1-3}$ alkyl, preferably unsubstituted or optionally substituted with a substituent selected from -Cl, -Br, -F, -OH, -CN, $-CH_3$, and $-OCH_3$;

In some embodiments, $Ar^1$ at each occurrence is each independently selected from 6-10 membered arylene, preferably selected from phenylene; the arylene or phenylene is unsubstituted or optionally substituted with a substituent selected from halogen, -OH, -CN, $-CF_3$, $-NO_2$, $C_{1-6}$ alkyl, $-OC_{1-6}$ alkyl, $-NH_2$ , $-NH(C_{1-6}$ alkyl), and $-N(C_{1-6}$ alkyl$)_2$ , preferably unsubstituted or optionally substituted with a substituent selected from halogen, -OH, -CN, - $CF_3$, $-NO_2$, $C_{1-6}$ alkyl, and $-OC_{1-6}$ alkyl, preferably unsubstituted or optionally substituted with a substituent selected from halogen, -OH, -CN, $-CF_3$, $-NO_2$, $C_{1-3}$ alkyl, and $-OC_{1-3}$ alkyl, preferably unsubstituted or optionally substituted with a substituent selected from -Cl, -Br, -F, -OH, -CN, $-CF_3$, $-NO_2$, $-CH_3$, and $-OCH_3$;

In some embodiments, $R^a$ and $R^b$ are identical or different and are each independently selected from hydrogen atom, deuterium atom, halogen, $C_{1-3}$ alkyl, halo $C_{1-3}$ alkyl, deuterated $C_{1-3}$alkyl, $-OC_{1-3}$ alkyl, -OH, $-NH_2$, -CN, - $CF_3$, $-NO_2$, and $C_{1-3}$ alkylene-OH; preferably, $R^a$ and $R^b$ are identical or different and are each independently selected from hydrogen atom, deuterium atom, and halogen; and/or

In some embodiments, X is independently selected from single bond, -NH-, or O; m at each occurrence is each independently an integer of 0, 1, 2, or 3.

In some embodiments of the present invention, Y is selected from:

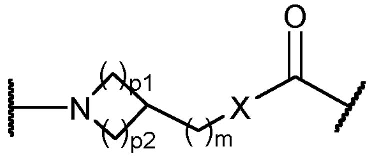

or

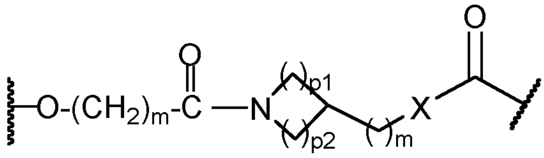

,

wherein p1 is an integer of 0, 1, 2, or 3, p2 is an integer of 1, 2, 3, or 4, and $2 \leq p1 + p2 \leq 4$; X is independently selected from single bond, -NH-, or O, and m is an integer of 0, 1, 2, or 3;

or Y is selected from:

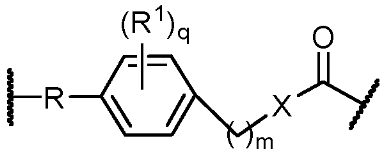, 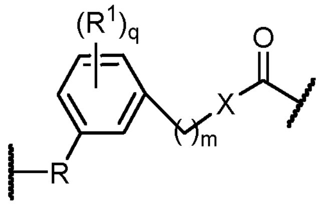,

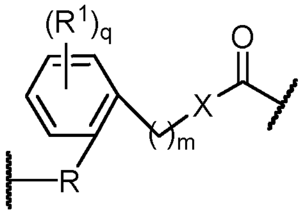, or 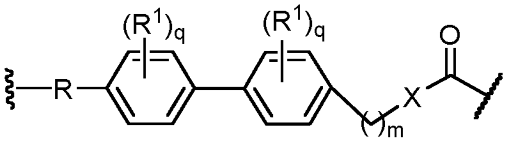,

wherein R is - NH-, O, or -O-$(CH_2)_m$-C(=O)-NH-, and the -NH- in -O-$(CH_2)_m$-C(=O)-NH- is connected to phenylene or biphenylene in Y; X is independently selected from a single bond, -NH-, or O, m is an integer of 0, 1, 2, or 3, $R^1$ at each occurrence is each independently selected from halogen, -OH, -CN , -$CF_3$ , - $NO_2$ , $C_{1-6}$ alkyl, -$OC_{1-6}$ alkyl, -$NH_2$ , -NH($C_{1-6}$ alkyl), and -N($C_{1-6}$ alkyl)$_2$, and q is an integer of 0, 1, 2, 3, or 4; preferably, X is independently selected from a single bond or -NH-, m is an integer of 0 or 1, and $R^1$ at each occurrence is each independently selected from halogen, -OH, -CN, -$CF_3$, -$NO_2$ , $C_{1-3}$ alkyl, and -$OC_{1-3}$ alkyl, preferably, independently selected from -Cl, -Br, -F, -OH, - CN, -$CF_3$, -$NO_2$ , -$CH_3$, and -$OCH_3$ , and q is an integer of 0, 1, or 2;
or Y is selected from:

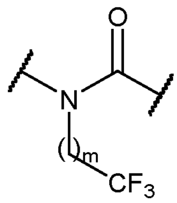,

wherein m is an integer of 0, 1, 2, or 3; preferably, m is 1 or 2; and the C(=O)- end of the Y structure is connected to -NH- in the structure of formula (-D) or formula (-$D_0$ ).

In some embodiments of the present invention, Y is selected from:

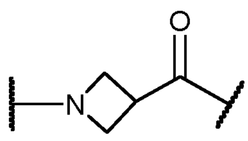, 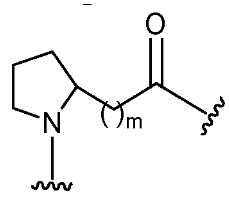, 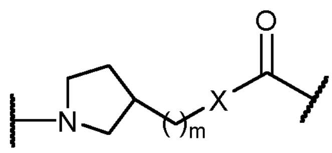,

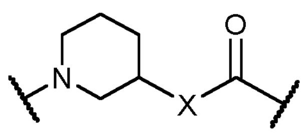, 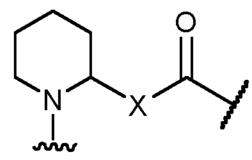, 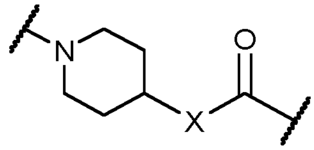,

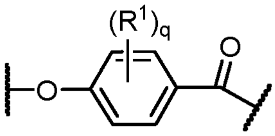 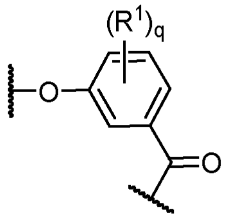 , 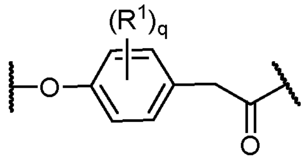 , 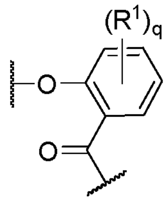 ,

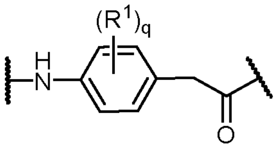 , 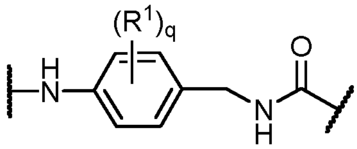 , 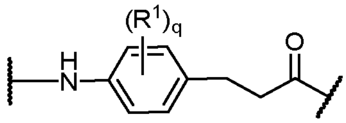 ,

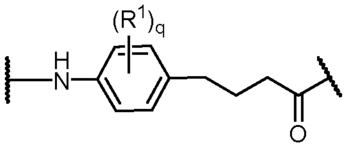 , 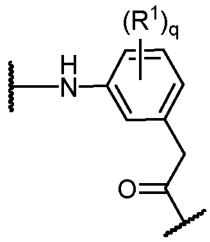 , 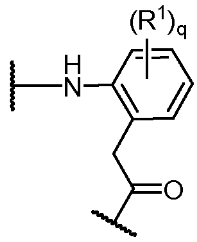 ,

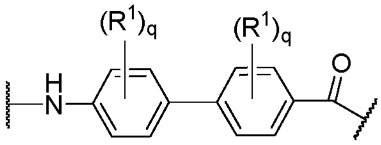 , 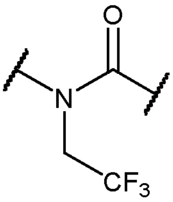 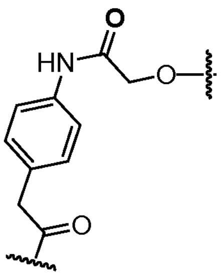 and 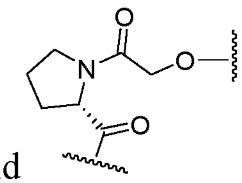 .

wherein, X is independently selected from a single bond or -NH-, m is an integer of 0 or 1;

$R^1$ is selected from halogen, -OH, -CN, $-CF_3$, $-NO_2$ , $-CH_3$, $C_{1-6}$ alkyl, $-OC_{1-6}$ alkyl, $-NH_2$, $-NH(C_{1-6}$ alkyl), and $-N(C_{1-6}$ alkyl$)_2$, preferably $R^1$ is selected from halogen, -OH, -CN, $C_{1-3}$ alkyl, and $-OC_{1-3}$ alkyl, preferably $R^1$ is selected from -Cl, -Br, -F, -OH, -CN, $-CF_3$, $-NO_2$ , $-CH_3$, and $-OCH_3$;

q is an integer of 0, 1, 2, 3 or 4; preferably, q is an integer of 0, 1 or 2; and

the C(=O)- end of the Y structure is connected to -NH- in the structure of formula (-D) or formula ($-D_0$).

In some embodiments of the present invention, the linker unit -L- is $-L^1$ $-L^2$ $-L^3$ $-L^4$ -, $L^1$ is selected from -(succinimidyl-3-yl-N)-W-C(=O)-, wherein W is $C_{1-10}$ alkylene, $C_{1-10}$ alkylene-cycloalkylene, $C_{1-10}$ heteroalkylene, $C_{1-10}$ alkylene- heterocyclylene, or $C_{1-10}$ heteroalkylene-cycloalkylene, preferably W is alkylene, alkylene-cycloalkylene, or $C_{1-8}$ heteroalkylene, the heteroalkyl contains 1-3 heteroatoms each independently selected from N, O or S, wherein the $C_{1-8}$alkyl, cycloalkyl and $C_{1-8}$heteroalkylene are unsubstituted or each independently optionally further substituted with one or more substituents selected from halogen, hydroxyl, -CN, amino, C alkyl, haloalkyl, deuterated alkyl, alkoxy and cycloalkyl, preferably unsubstituted or each independently optionally further substituted with one or more substituents selected from halogen, hydroxyl, -CN, amino, $C_{1-6}$ alkyl, halo$C_{1-6}$alkyl, deuterated $C_{1-6}$ alkyl, $C_{1-6}$ alkoxy and $C_{5-8}$ cycloalkyl, preferably unsubstituted or optionally substituted with one or more substituents selected from halogen, -OH, -CN, $C_{1-3}$ alkyl, and $-OC_{1-3}$ alkyl, preferably unsubstituted or optionally substituted with a substituent selected from Cl, Br, F, -OH, -CN, methyl, and $-OCH_3$ ;

$L^2$ is selected from $-NR^4(CH_2CH_2O)_rCH_2CH_2C(=O)-$, $-NR^4(CH_2CH_2O)_rCH_2$ $C(=O)-$, $-NR^4CH_2-Ar^2-(CH_2CH_2O)_rCH_2CH_2NR^4C(=O)CH_2OCH_2C(=O)-$, - $S(CH_2)_rC(=O)-$, or a single bond, wherein r is an integer of from 1 to 20, preferably r is an integer of 1, 2, 3, 4, 5, 6, 7, or 8;

$Ar^2$ is selected from

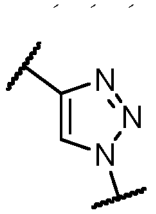 , 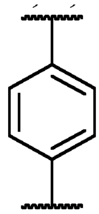 , 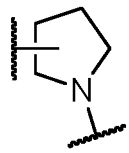 , 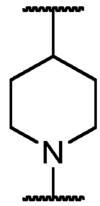 , 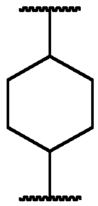 ,

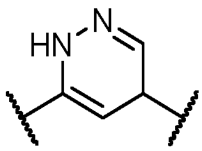 , 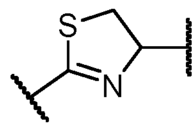 , 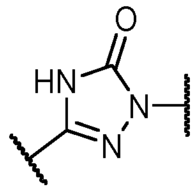 , 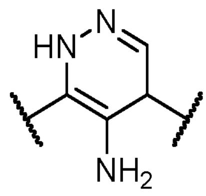

and

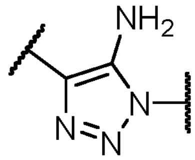 ;

$L^3$ is a peptide residue consisting of from 2 to 7 amino acids, preferably $L^3$ is a peptide residue consisting of 2, 3, 4, 5 or 6 amino acids, wherein the amino acids are unsubstituted or optionally further substituted with one or more substituents selected from halogen, hydroxyl, -CN, amino, alkyl, haloalkyl, deuterated alkyl, alkoxy, and cycloalkyl, preferably, optionally further substituted with one or more substituents selected from halogen, hydroxyl, - CN, amino, $C_{1-6}$ alkyl, halo$C_{1-6}$alkyl, deuterated $C_{1-6}$alkyl, $C_{1-6}$ alkoxy and $C_{5-8}$ cycloalkyl;

$L^4$ is selected from $-NR^5(CR^6R^7)_t$-Z-$(CR^6R^7)_t$-C(=O)-, $-NR^5$-$Ar^3$-$(CR^6R^7)_t$-Z-C(=O)-, or a single bond, wherein t at each occurrence is each independently an integer of 0, 1, 2, 3, 4, 5, or 6; Z at each occurrence is each independently a single bond, O, S, or -NH-; $Ar^3$ is a arylene or heteroarylene, preferably selected from 6-8 membered arylene or 5-8 membered heteroarylene, the heteroarylene contains 1, 2 or 3 heteroatoms, and the heteroatoms are independently selected from N, O, and S; the arylene or heteroarylene is unsubstituted or optionally substituted with a substituent selected from H, halogen, -OH, -CN, $C_{1-6}$ alkyl, $-OC_{1-6}$ alkyl, $-NH_2$ , $-NH(C_{1-6}$ alkyl), and $-N(C_{1-6}$ alkyl$)_2$;

$R^4$ and $R^5$ are identical or different and at each occurrence are each independently selected from hydrogen atom, halogen, $C_{1-6}$ alkyl, halo $C_{1-6}$ alkyl, deuterated $C_{1-6}$ alkyl, and $-C_{1-6}$ alkylene-OH;

$R^6$ and $R^7$ are identical or different and at each occurrence are each independently selected from hydrogen atom, halogen, $C_{1-6}$ alkyl, halo$C_{1-6}$alkyl, deuterated $C_{1-6}$ alkyl, and $-C_{1-6}$ alkylene-OH; and

the $L^1$ end is connected to the ligand, and the $L^4$ end is connected to Y

In some embodiments of the present invention, $L^1$ is selected from - (succinimidyl-3-yl-N)-$(CH_2)_s$-C(=O)- and -(succinimidyl-3-yl-N)-$CH_2$-cyclohexyl-C(=O)-, wherein s is an integer of 2, 3, 4, 5, 6, 7, or 8.

In some embodiments of the present invention, $L^2$ is selected from $-NR^4(CH_2CH_2O)_rCH_2CH_2C(=O)$-, and $-NR^4CH_2$-$Ar^2$-$(CH_2CH_2O)_rCH_2CH_2NR^4C(=O)CH_2OCH_2C(=O)$-, wherein r is an integer from 1 to 20; preferably r is an integer of 1, 2, 3, 4, 5, 6, 7, or 8, and $Ar^2$ is

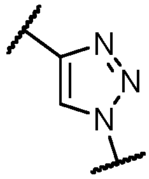 .

In some embodiments of the present invention, $L^3$ is a peptide residue consisting of 2 to 7 amino acids selected from alanine, phenylalanine, glycine, valine, lysine, citrulline, serine, glutamic acid, and aspartic acid; preferably $L^3$ is a dipeptide residue, tripeptide residue, or tetrapeptide residue consisting of amino acids selected from alanine, phenylalanine, glycine, valine, lysine, citrulline, serine, glutamic acid, and aspartic acid; more preferably $L^3$ is selected form dipeptide residue of valine-alanine, tripeptide residue of alanine-alanine-alanine, or tetrapeptide residue of glycine-glycine-phenylalanine-glycine.

In some embodiments of the present invention, $L^4$ is selected from - $NR^5(CR^6R^7)_t$-Z-$(CR^6R^7)_t$-C(=O)- or -$NR^5$-$Ar^3$-$(CR^6R^7)_t$-Z-C(=O)-, t at each occurrence is independently an integer of 1, 2 or 3;

Z is selected from a single bond, O, S or -NH-;

$Ar^3$ is selected from 6-8 membered arylene, preferably is phenylene, the arylene or phenylene is unsubstituted or optionally substituted with a substituent selected from H, halogen, -OH, -CN, and $C_{1-6}$ alkyl;

$R^5$ at each occurrence is each independently selected from hydrogen atom, $C_{1-3}$ alkyl, halo$C_{1-3}$ alkyl, and deuterated $C_{1-3}$ alkyl;

$R^6$ and $R^7$ are identical or different and at each occurrence are each independently selected from hydrogen atom, halogen, $C_{1-3}$ alkyl, $C_{1-3}$ haloalkyl, and deuterated $C_{1-3}$ alkyl.

In some embodiments of the present invention, the linker unit -L- is:

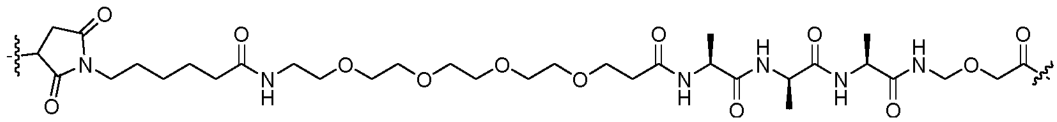

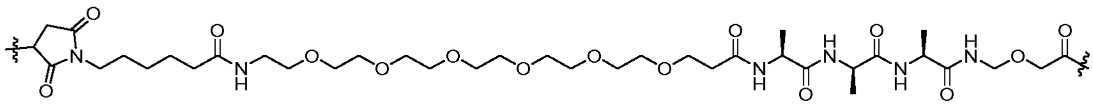

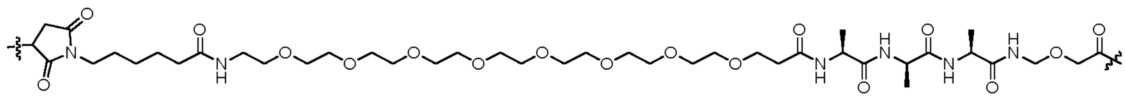

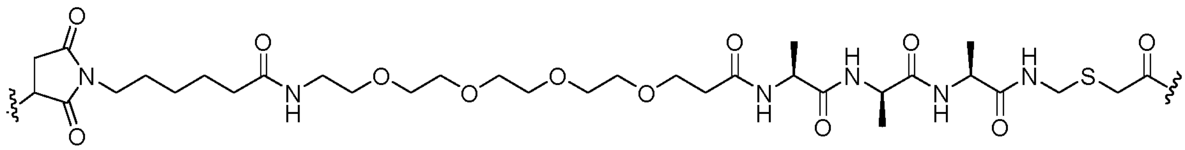

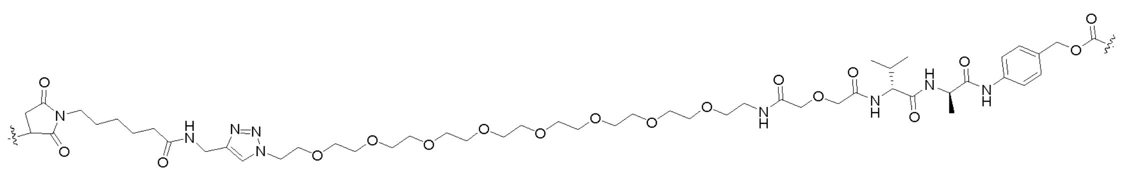

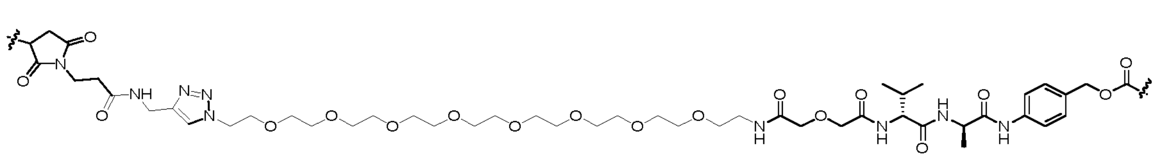

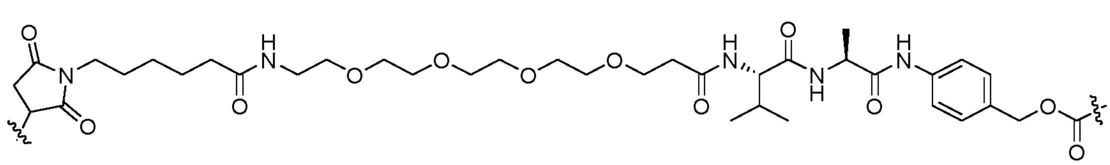

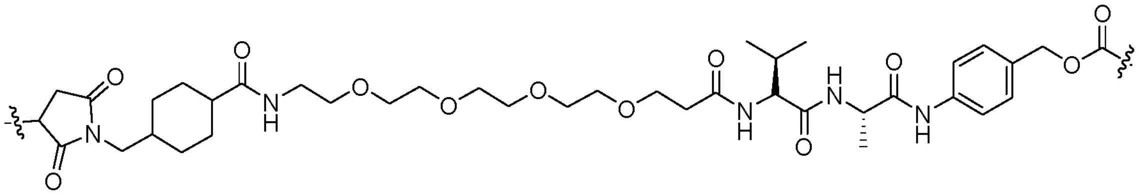

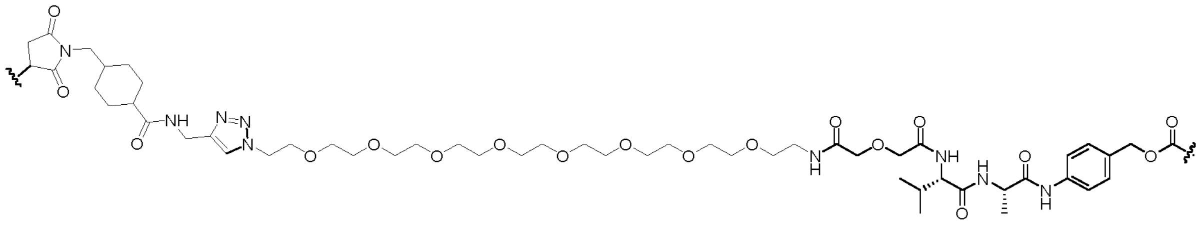

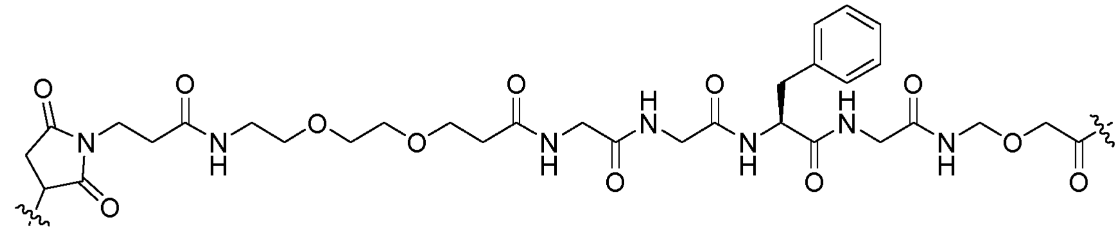

or

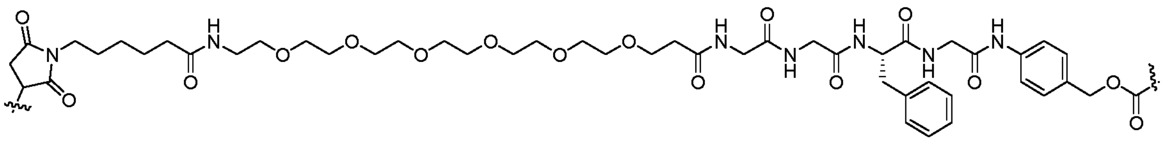

.

In some embodiments of the present invention, there provide a ligand-drug conjugate or the pharmaceutically acceptable salt thereof, selected from the following structural formulas:

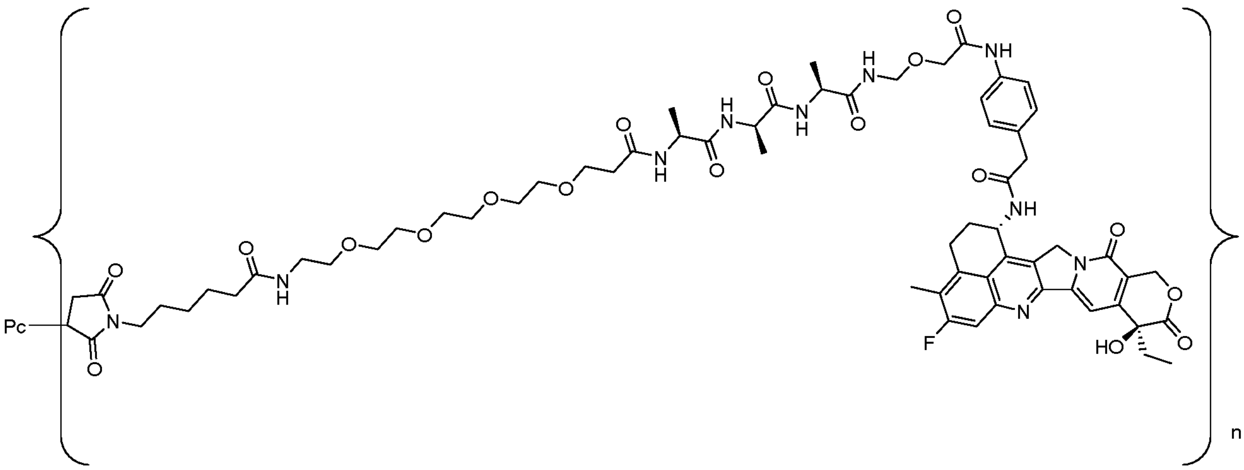

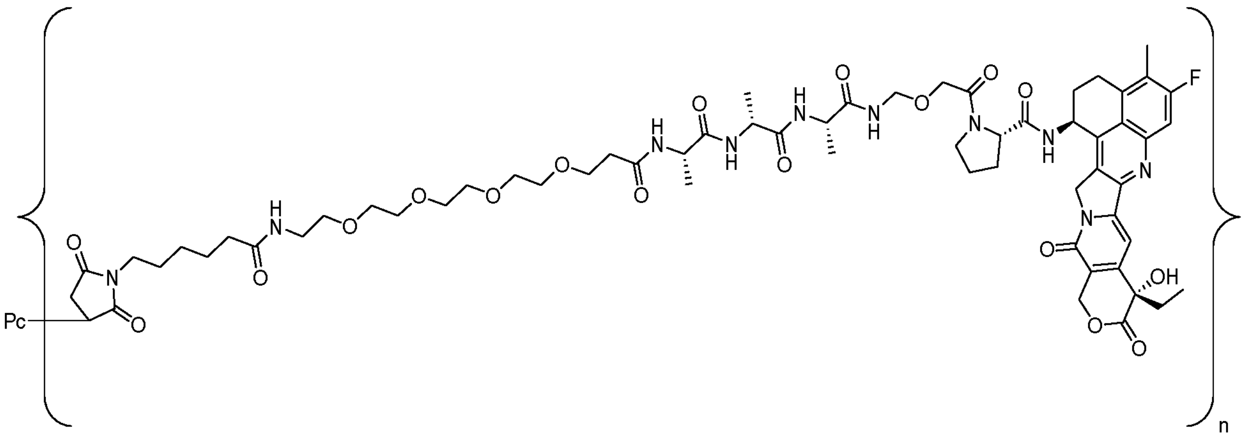

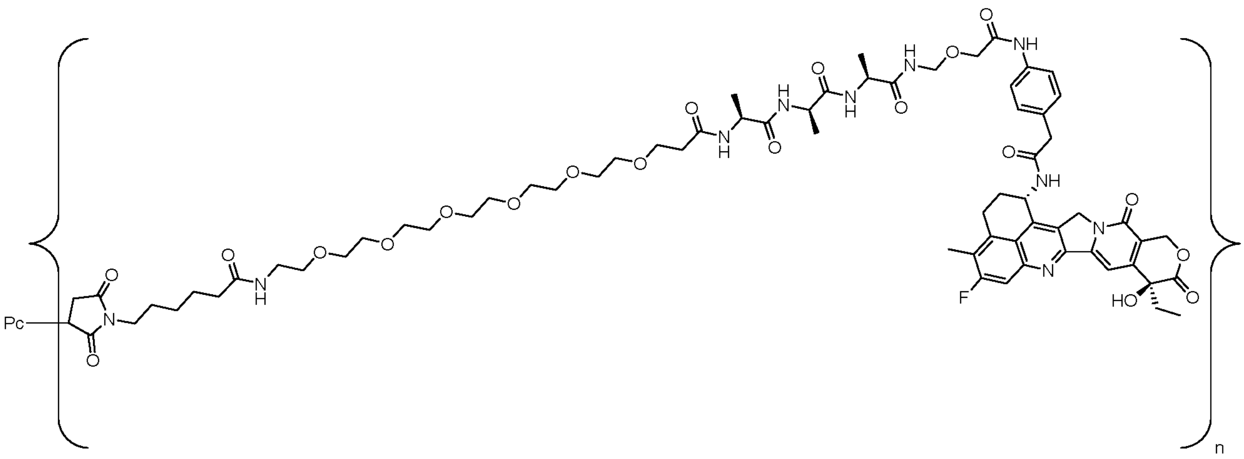

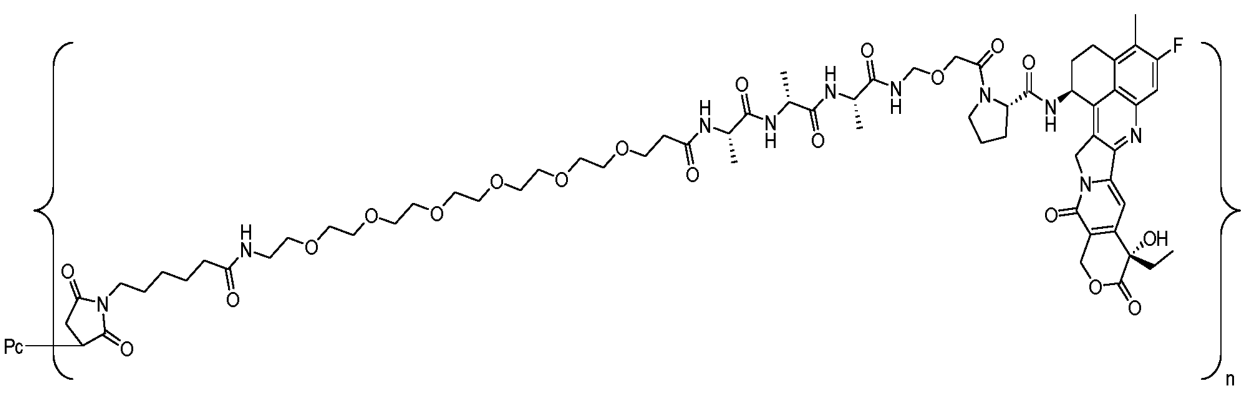

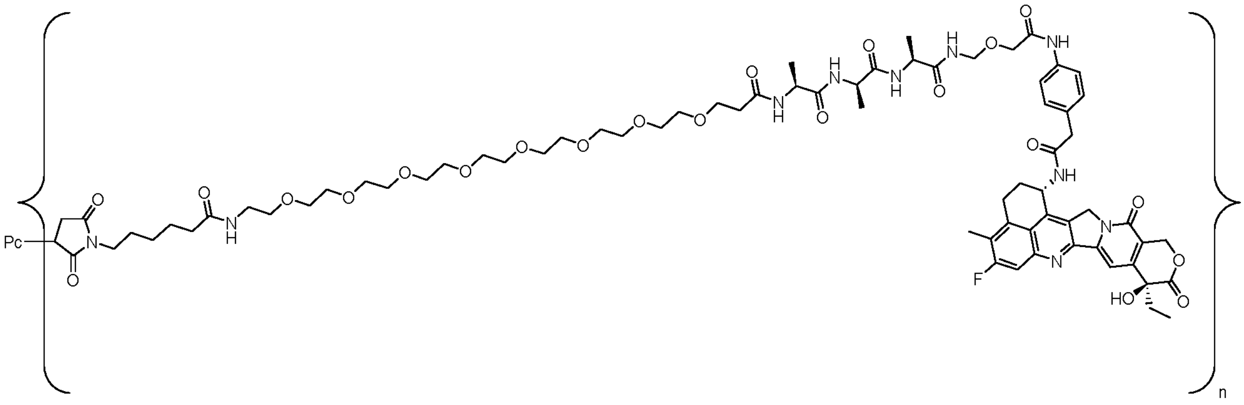

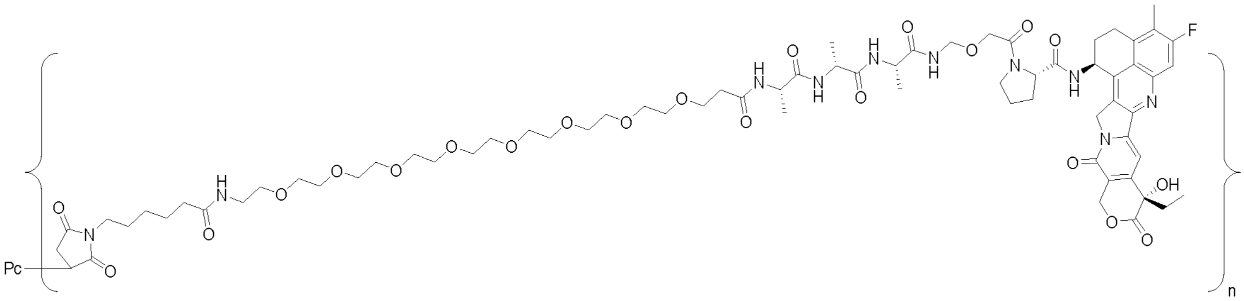

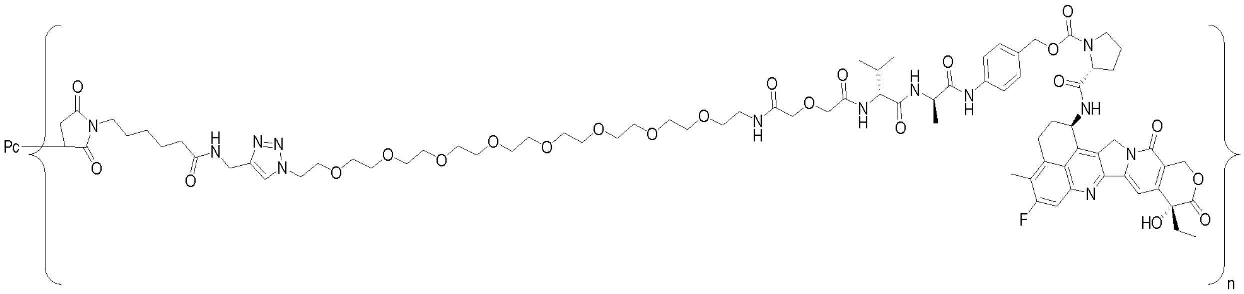

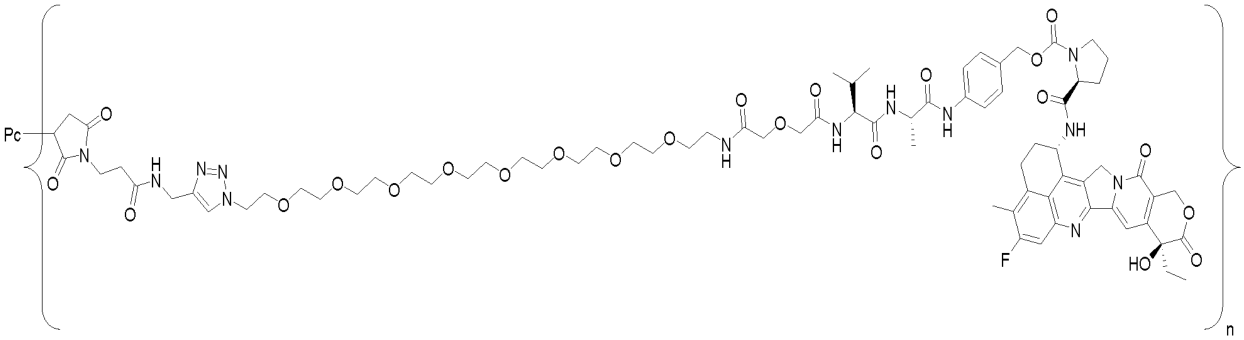

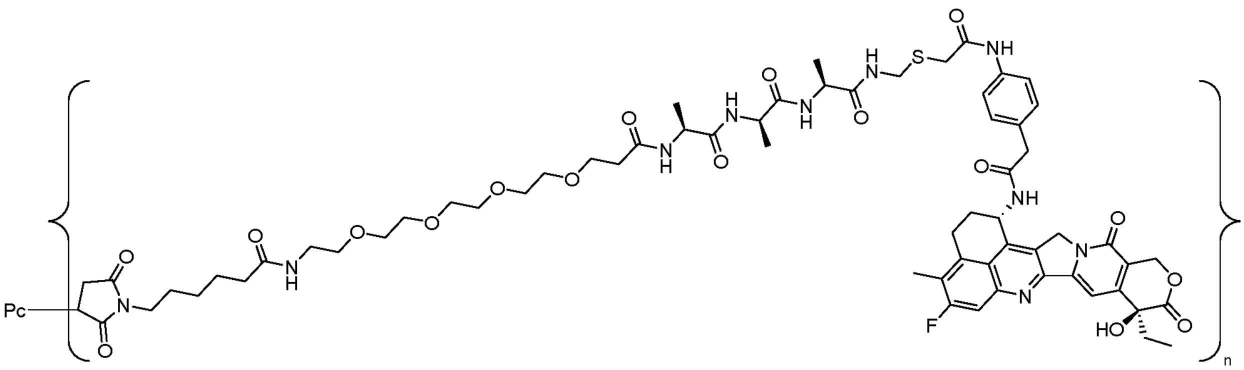

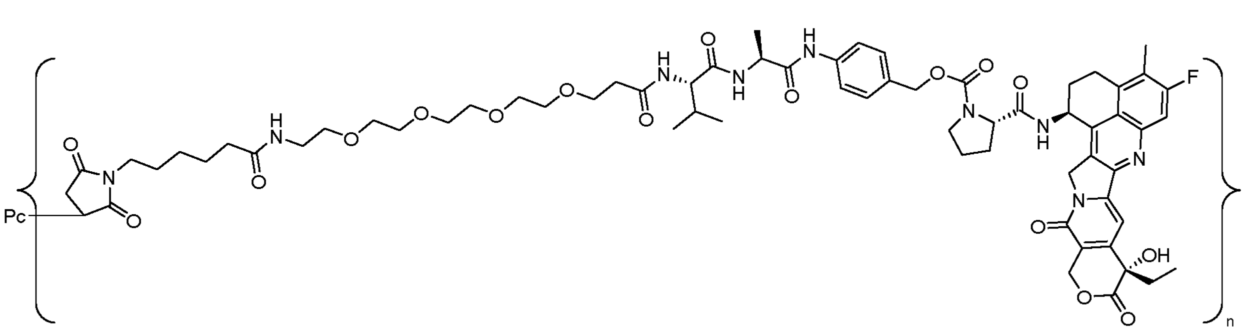

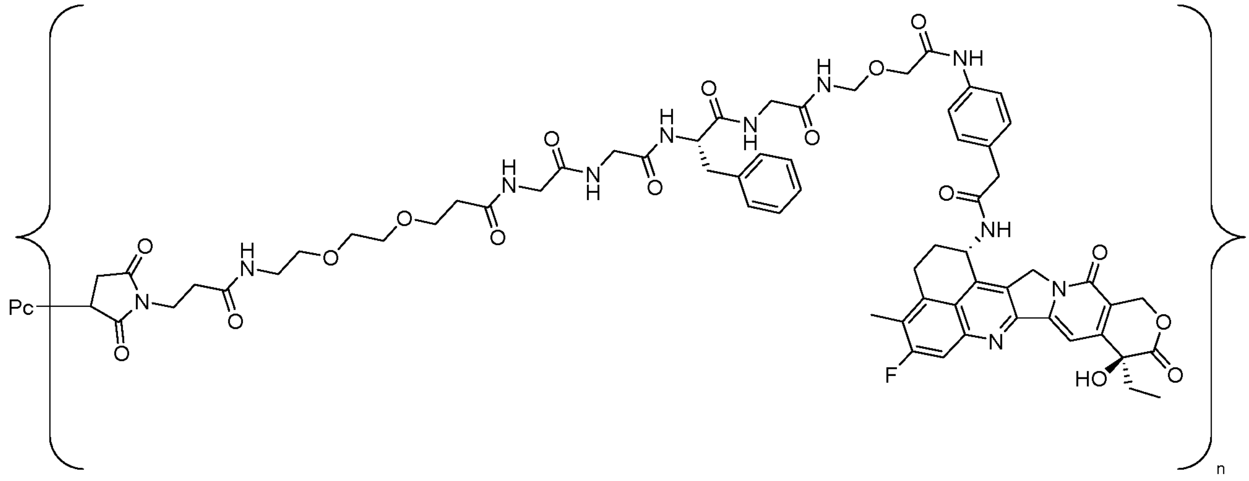

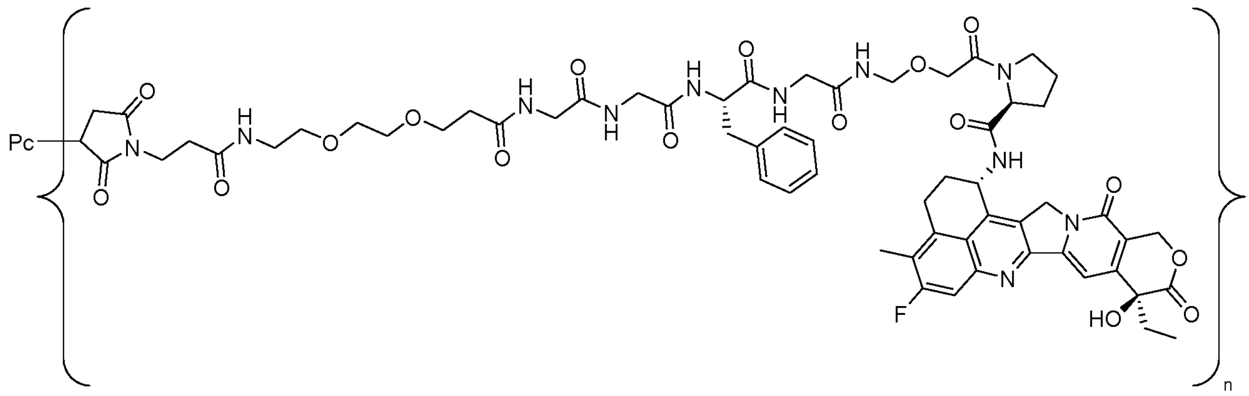

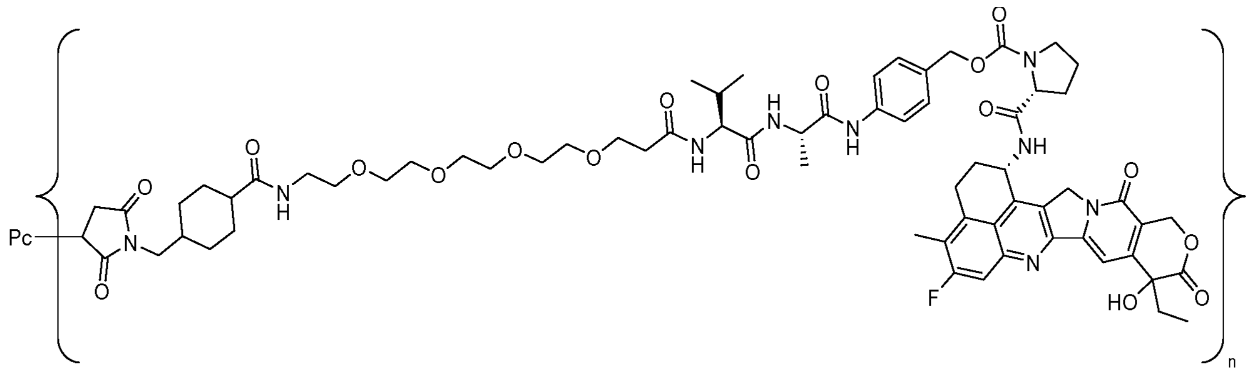

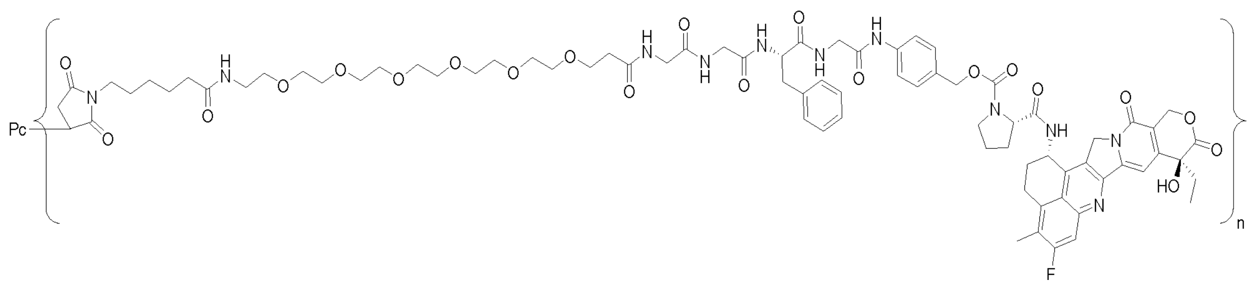

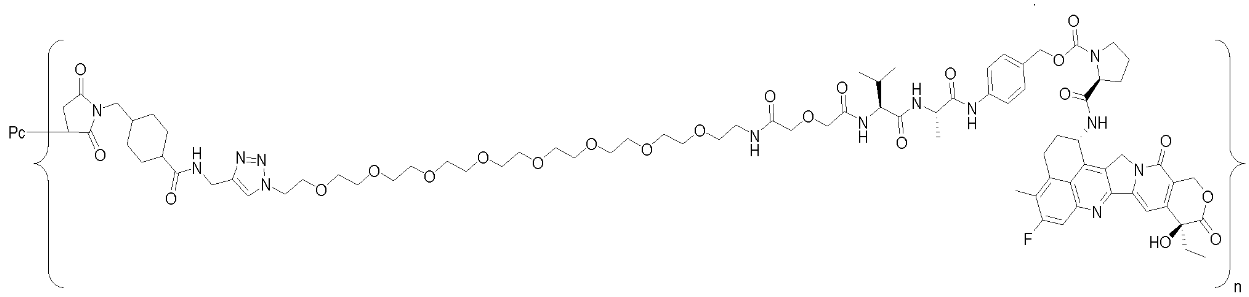

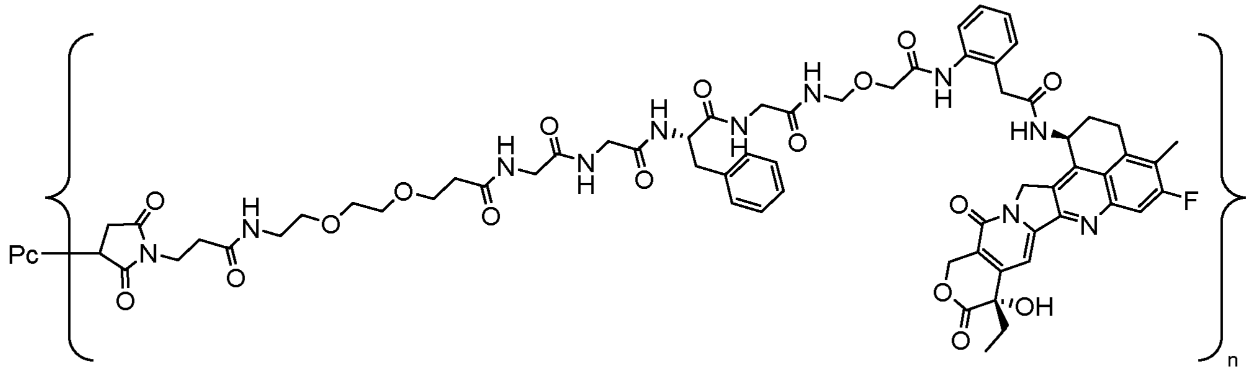

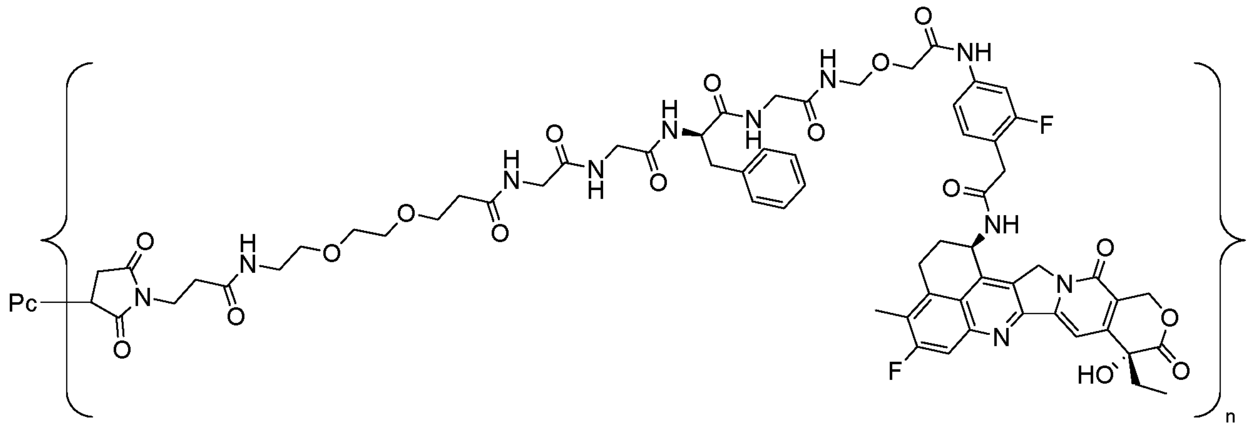

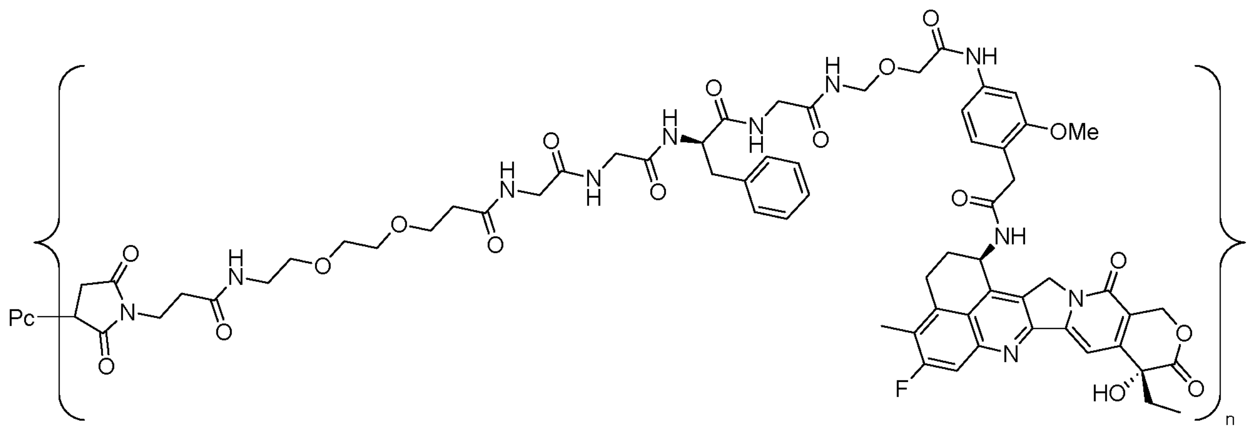

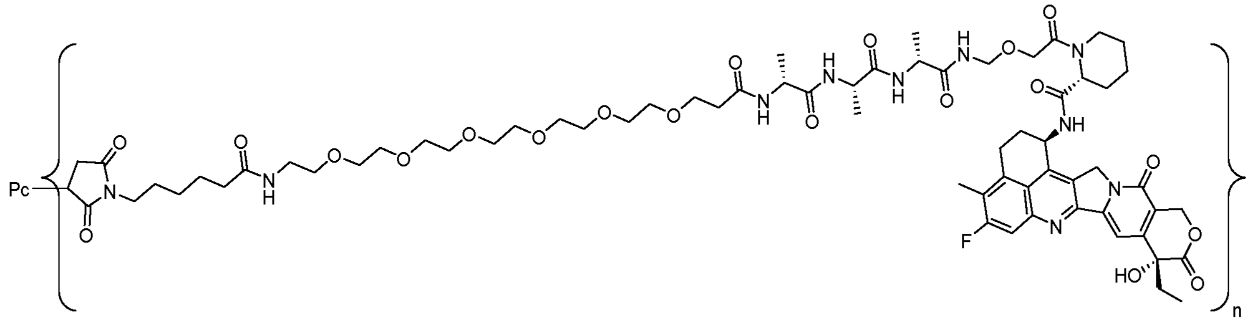

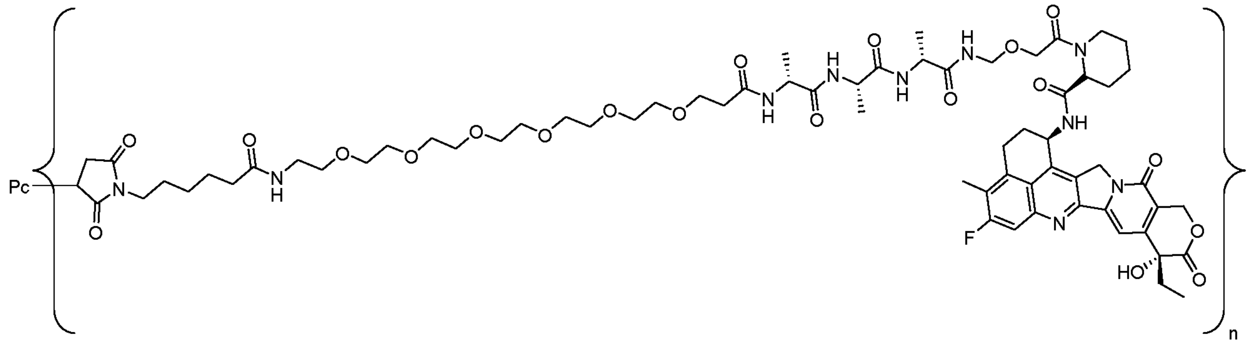

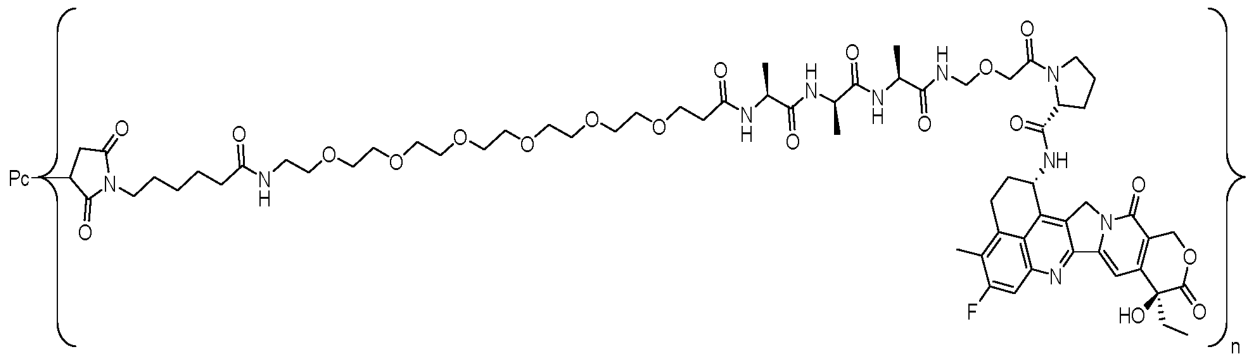

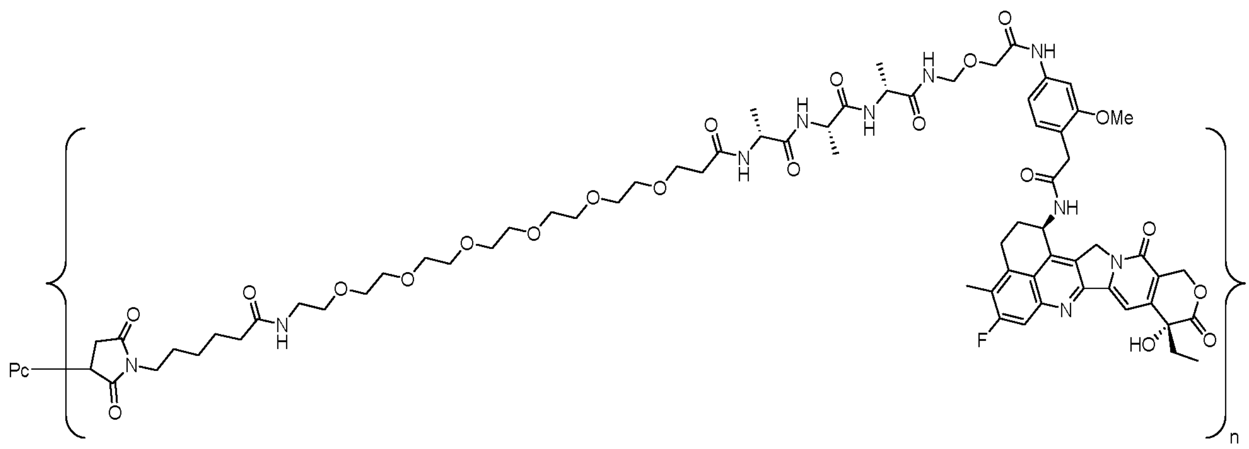

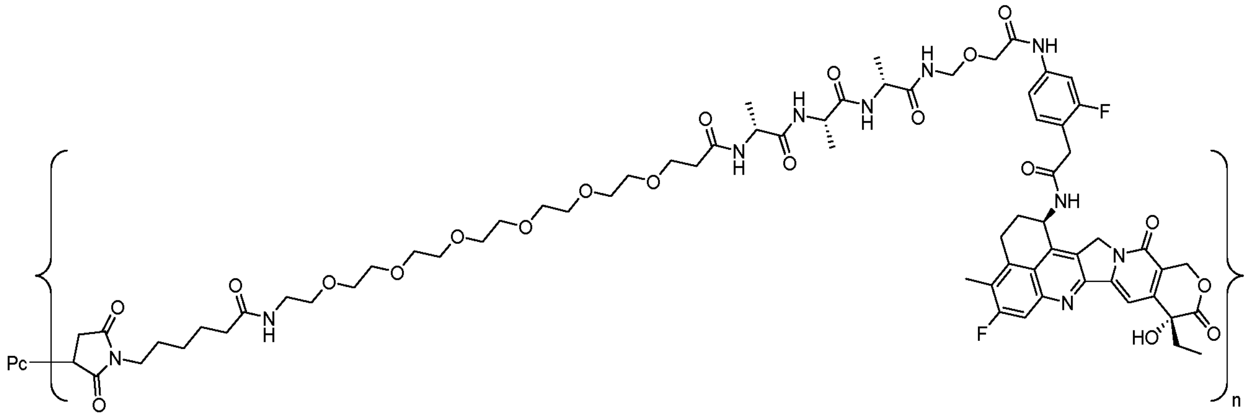

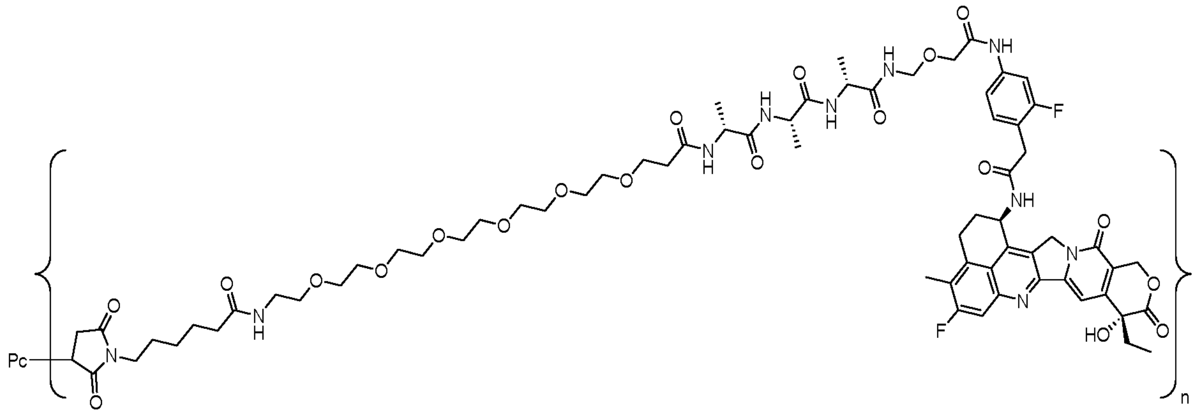

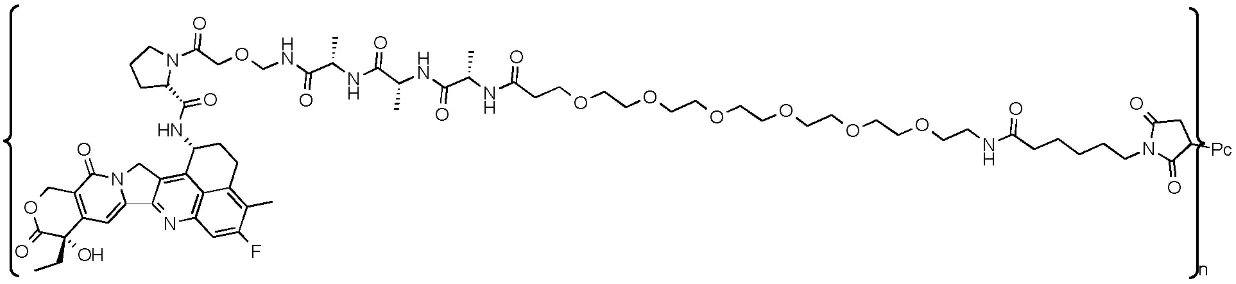

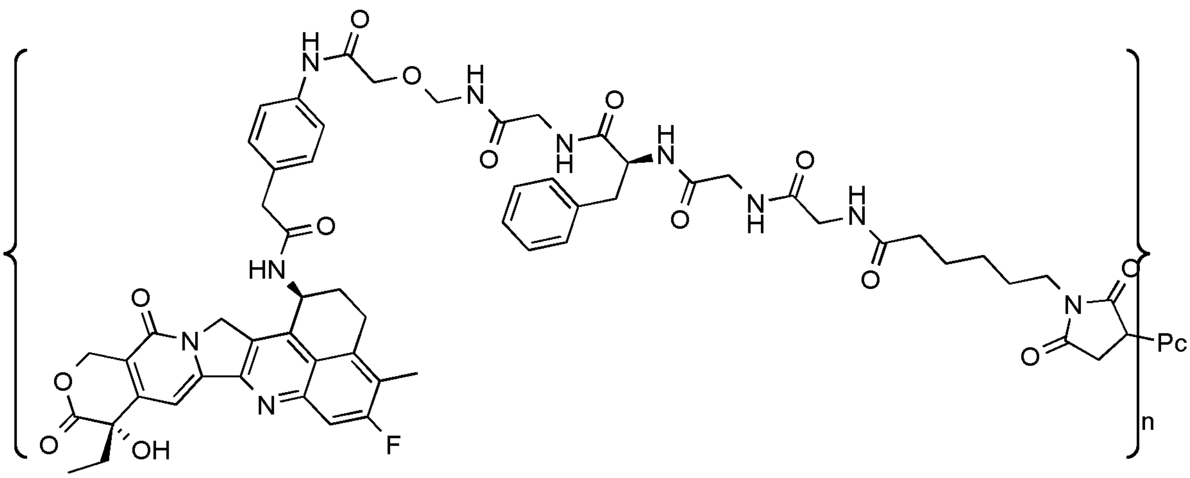

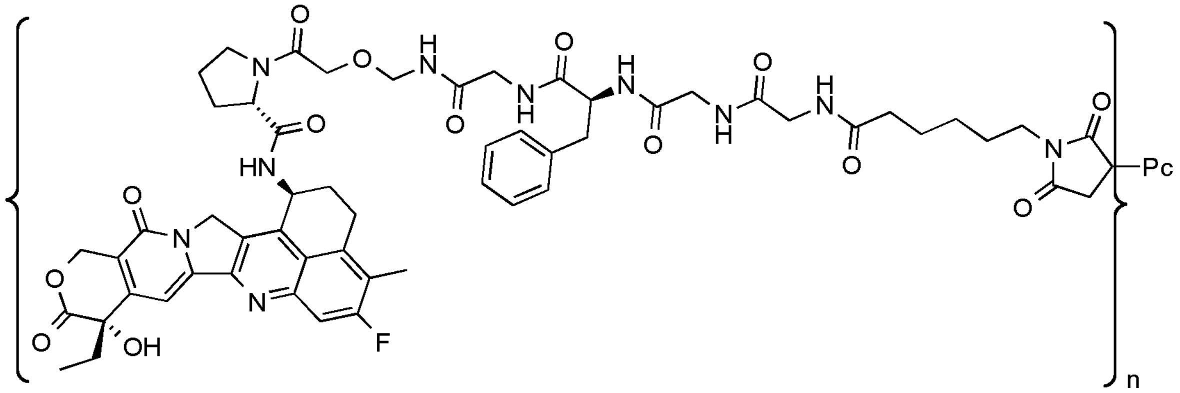

and

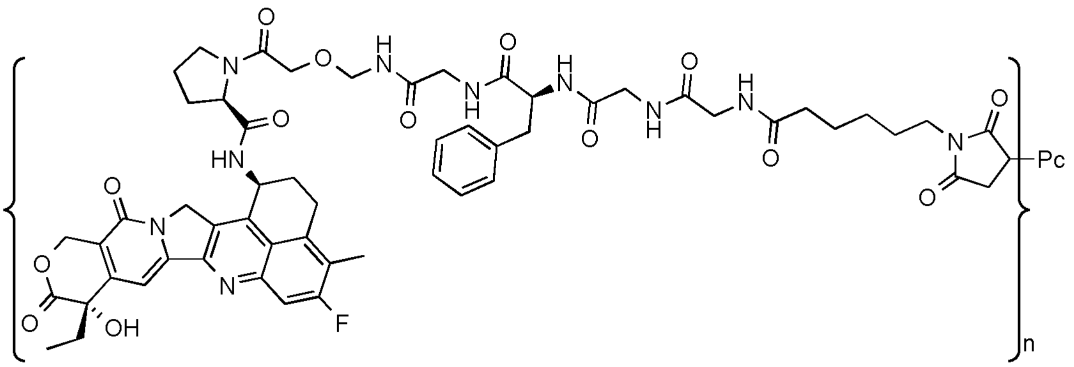

;

wherein n is an integer or a decimal from 1 to 15; preferably, n is an integer or a decimal from 1 to 13; preferably, n is an integer or a decimal from 1 to 10; more preferably, n is an integer or a decimal from 3 to 8; and Pc is a ligand.

In some embodiments of the present invention, Pc is an antibody or an antigen-binding fragment thereof or a polypeptide, wherein the antibody is selected from a chimeric antibody, a humanized antibody and a fully human antibody;

preferably, the antibody or antigen-binding fragment thereof is selected from an anti-TROP-2 antibody, an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-LIV-1 antibody, an anti-ROR1 antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-Mesothelin antibody, or an antigen-binding fragment thereof;

preferably, the antibody or antigen-binding fragment thereof is an anti-TROP-2 antibody, an anti-HER2(ErbB2) antibody, an anti-HER3(ErbB3) antibody, an anti-LIV-1 antibody, an anti-ROR1 antibody, or an antigen-binding fragment thereof;preferably, the antibody or antigen-binding fragment thereof is an anti-TROP-2 antibody or an anti-HER3 (ErbB3) antibody, or an antigen-binding fragment thereof preferably, the heavy chain of the anti-TROP-2 antibody or antigen-binding fragment thereof comprises: an HCDR1 consisting of the amino acid sequence of SEQ ID No: 1, an HCDR2 consisting of the amino acid sequence of SEQ ID No:2 and an HCDR3 consisting of the amino acid sequence of SEQ ID No:3 and/or, the light chain comprises: an LCDR1 consisting of the amino acid sequence of SEQ ID No:4, an LCDR2 consisting of the amino acid sequence of SEQ ID No:5, an LCDR3 consisting of the amino acid sequence of SEQ ID No:6; more preferably, the heavy chain of the anti-TROP-2 antibody or antigen-binding fragment thereof comprises, the heavy chain variable region shown in SEQ ID No:7, and/or, the light chain of the anti-TROP-2 antibody or antigen-binding fragment thereof comprises the light chain variable region shown in SEQ ID No:8; more preferably, the anti-TROP-2 antibody or antigen-binding fragment thereof comprises a heavy chain of the amino acid sequence shown in SEQ ID NOV, and/or, a light chain of the amino acid sequence shown in SEQ ID NO: 10;

preferably, the heavy chain of the anti-HER3 antibody or antigen-binding fragment thereof comprises, an H'CDR1 consisting of the amino acid sequence of SEQ ID No: 11, an H'CDR2 consisting of the amino acid sequence of SEQ ID No: 12 and an H'CDR3 consisting of the amino acid sequence of SEQ ID No: 13, and/or, the light chain of the anti-HER3 antibody or antigen-binding fragment thereof comprises an L'CDR1 consisting of the amino acid sequence of SEQ ID No: 14, an L'CDR2 consisting of the amino acid sequence of SEQ ID No: 15 and an L'CDR3 consisting of the amino acid sequence of SEQ ID No:16; more preferably, the anti-HER3 antibody or antigen-binding fragment thereof comprises a heavy chain variable region as shown in SEQ ID No: 17 in the heavy chain and/or a light chain variable region as shown in SEQ ID No:18 in the light chain; more preferably, the anti-HER3 antibody or antigen-binding fragment thereof comprises a heavy chain of the amino acid sequence shown by SEQ ID NO :19, and/or, a light chain of the amino acid sequence shown by SEQ ID NO:20; further preferably, the antibody or antigen-binding fragment thereof is sacituzumab, trastuzumab, patritumab, or pertuzumab.

Another aspect of the present invention provides a compound shown by the general formula ($E_0$) or formula (E), or a pharmaceutically acceptable salt thereof:

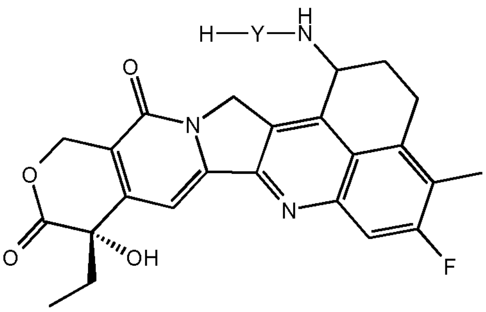

**($E_0$)**

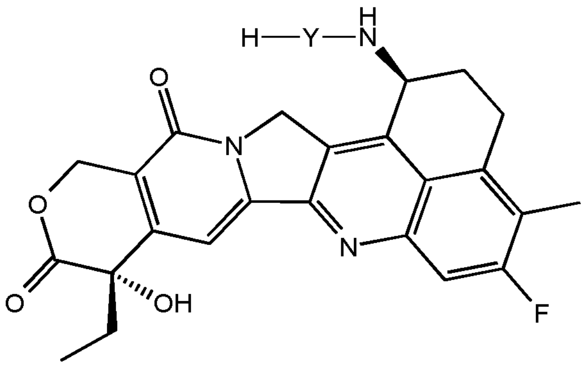

**(E)**

wherein Y is

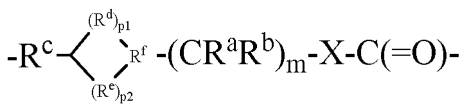,

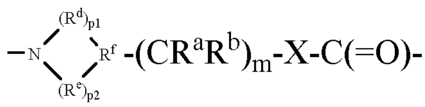,

$-R^c-Ar^1-(Cy)_m-(CR^aR^b)_m-X-C(=O)-$ , $-N((CR^aR^b)_mCF_3)-C(=O)-$ , $-R^c-(CR^aR^b)_m-C(=O)-R^c-Ar^1-(Cy)_m-(CR^aR^b)_m-X-C(=O)-$,

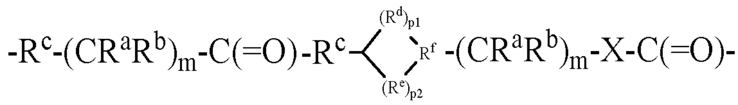,

or

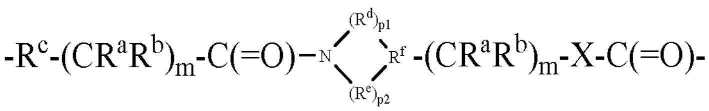,

wherein the C=O-end of Y is connected to -NH- in the structure of formula (D); preferably, Y is

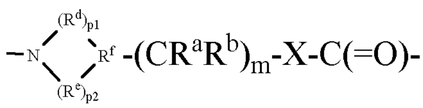,

$-O-Ar^1-(Cy)_m-(CR^aR^b)_m-X-C(=O)-$, $-NH-Ar^1-(Cy)_m-(CR^aR^b)_m-X-C(=O)-$, $-N((CR^aR^b)_mCF_3)-C(=O)-$, $-O-(CR^aR^b)_m-C(=O)-NH-Ar^1-(Cy)_m-(CR^aR^b)_m-X-C(=O)-$, or

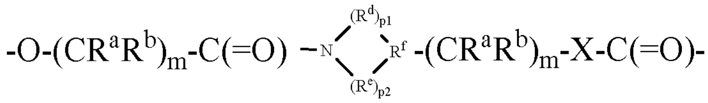

,

wherein the C(=O)-end of Y is connected to -NH- in the structure of formula (D);

$R^c$ at each occurrence is each independently NH, O, or S; $R^d$ and $R^e$ at each occurrence are each independently $C(R^m)_2$, $NR^m$, O, or S; $R^f$ at each occurrence is each independently $CR^m$ or N; preferably, when the ring formed by $R^d$, $R^e$, and $R^f$ is heterocyclyl, the heterocyclyl comprises 1, 2, 3, or 4 heteroatoms, and the heteroatoms each independently selected from N, O, and S;

$R^m$ at each occurrence is each independently H, halogen, haloalkyl, -OH, - CN, $-NO_2$, alkyl, alkoxy, $-NH_2$, -NH-alkyl, or $-N(alkyl)_2$, deuterium atom, deuterated alkyl, aminoalkylene, hydroxyalkylene, nitroalkylene, or cyanoalkylene, and preferably $R^m$ at each occurrence is each independently H, halogen, haloalkylene, -OH, -CN, $-NO_2$, alkyl, alkoxy, $-NH_2$ , -NH-alkyl, or - $N(alkyl)_2$ ;

p1 is an integer from 0 to 17, p2 is an integer from 1 to 18, and $p1+p2 \leq 18$; preferably, p1 is an integer of 0, 1, 2, 3, 4, 5, 6, or 7, and p2 is an integer of 1, 2, 3, 4, 5, 6, 7, or 8, and $p1 + p2 \leq 8$;

the Cy at each occurrence is each independently selected from cycloalkylene, heterocyclylene, arylene or heteroarylene, preferably the Cy at each occurrence is each independently selected from 3-10 membered cycloalkylene, 3-10 membered heterocyclylene, 6-10 membered arylene, or 5-10 membered heteroarylene; preferably, the heterocyclylene and heteroarylene contain 1, 2, 3, or 4 heteroatoms, and the heteroatoms are independently selected from N, O and S; the cycloalkylene, heterocyclylene, arylene, and heteroarylene are unsubstituted or optionally substituted with a substituent selected from halogen, -OH, -CN, haloalkyl, $-NO_2$ , alkyl, alkoxy, $-NH_2$ , -NH-alkyl, and $-N(alkyl)_2$, preferably unsubstituted or optionally substituted with a substituent selected from halogen, -OH, -CN, $-CF_3$ , $-NO_2$ , $C_{1-6}$ alkyl, -O $C_{1-6}$ alkyl, $-NH_2$ , $-NH(C_{1-6}$ alkyl), and $-N(C_{1-6}$ $alkyl)_2$;

$Ar^1$ at each occurrence is each independently selected from aryl or heteroaryl, preferably, the $Ar^1$ at each occurrence is each independently selected from a 6-10 membered aryl or a 5-10 membered heteroaryl; preferably the heteroaryl contains 1, 2, 3, or 4 heteroatoms, and the heteroatoms are independently selected from N, O, and S; the aryl and heteroaryl are unsubstituted or optionally substituted with substituents selected from halogen, -OH, -CN, haloalkyl, $-NO_2$, alkyl, alkoxy, $-NH_2$ , -NH-alkyl, and $-N(alkyl)_2$, preferably, unsubstituted or optionally substituted with substituents selected from halogen, -OH, -CN, $-CF_3$, $-NO_2$ , $C_{1-6}$ alkyl, $-OC_{1-6}$ alkyl, $-NH_2$ , $-NH(C_{1-6}$ alkyl), and $-N(C_{1-6}$ $alkyl)_2$ ;

$R^a$ and $R^b$ are identical or different and at each occurrence are each independently selected from hydrogen atom, halogen, haloalkyl, -OH, -CN, - $NO_2$, alkyl, alkoxyl, $-NH_2$ , -NH-alkyl, or $-N(alkyl)_2$, deuterium atom, deuterated alkyl, aminoalkylene, hydroxyalkylene, nitroalkylene, and cyanoalkylene, and preferably, at each occurrence $R^a$ and $R^b$ are each independently selected from hydrogen atom, deuterium atom, halogen, alkyl, haloalkyl, deuterated alkyl, alkoxy, -OH, $-NH_2$, -CN, nitro, and hydroxyalkylene, preferably, at each occurrence $R^a$ and $R^b$ are each independently selected from hydrogen atom, deuterium atom, halogen, $C_{1-6}$ alkyl, $C_{1-6}$ haloalkyl, deuterated $C_{1-6}$ alkyl, $-OC_{1-6}$ alkyl, -OH, $-NH_2$ , -CN, - $CF_3$ , nitro, $-C_{1-6}$ alkylene, and -OH;

X at each occurrence is each independently a single bond, -NH-, O or S; and m at each occurrence is each independently an integer of 0, 1, 2, 3, or 4;

Preferably, the compound of formula ($E_0$ ) or formula (E) is not

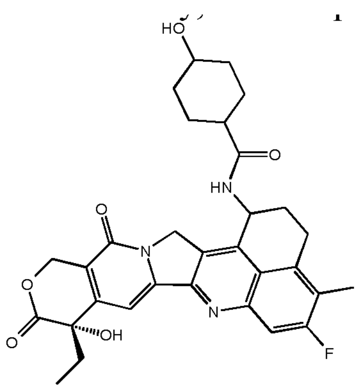

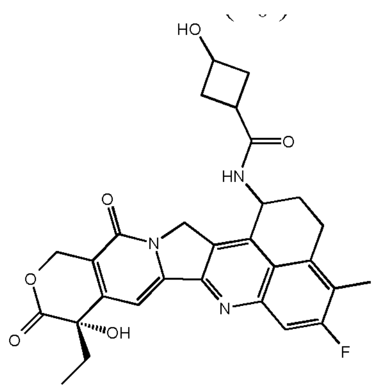

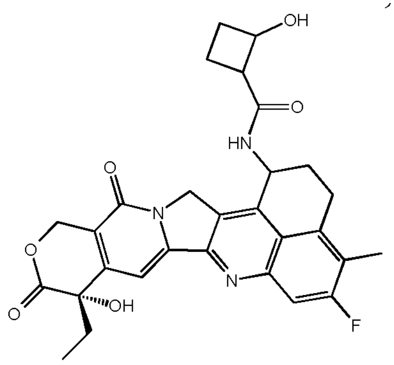

,

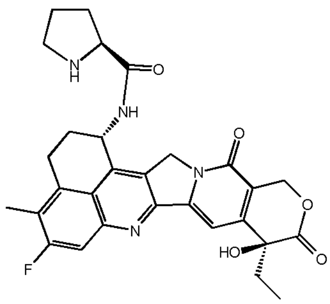

,

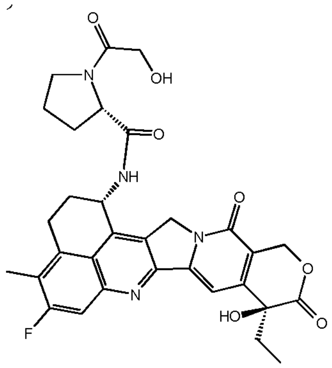

,

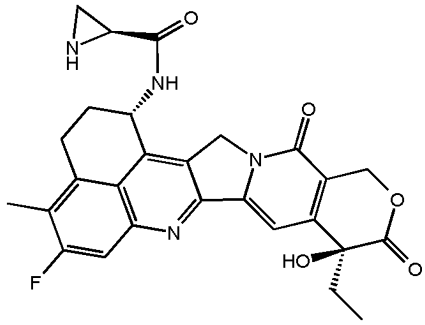

,

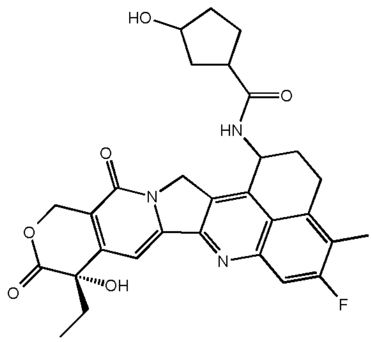

,

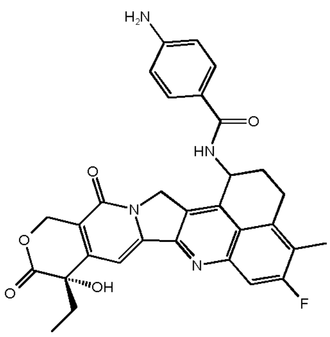

,

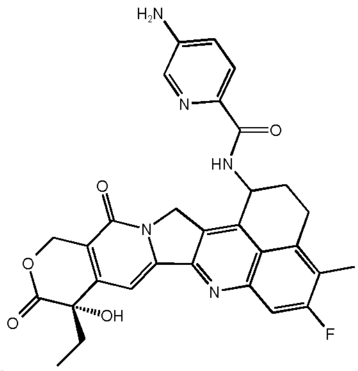

,

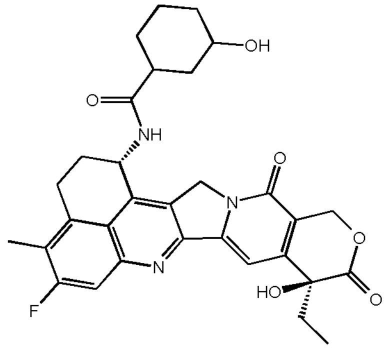

,

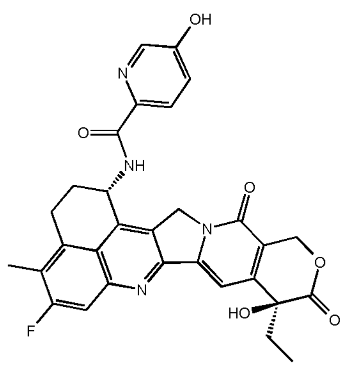

or

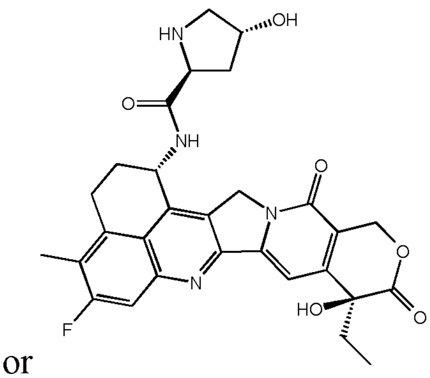

.

In some embodiments of the present invention, Y is selected from:

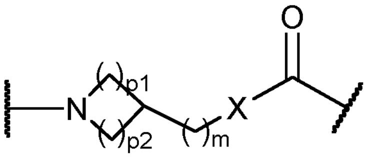 or 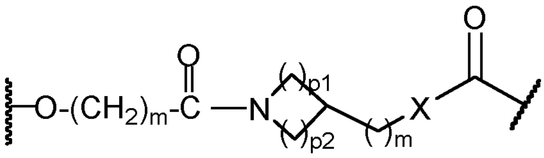 ,

wherein p1 is an integer of 0, 1, 2, or 3, p2 is an integer of 1, 2, 3, or 4, and $2 \leq p1 + p2 \leq 4$; X is independently selected from single bond, -NH- or O, and m is an integer of 0, 1, 2, or 3; or

Y is selected from:

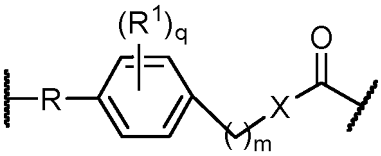 , 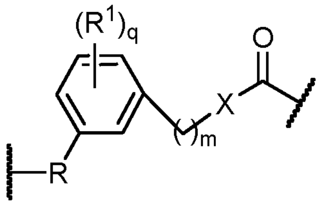 ,

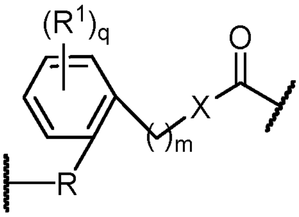 , or 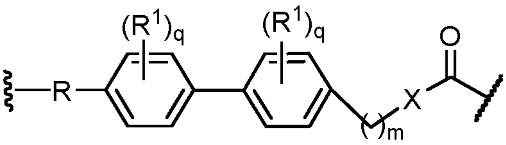 ,

wherein R is - NH-, O, or $-O-(CH_2)_m-C(=O)-NH-$ , and the -NH- in $-O-(CH_2)_m-C(=O)-NH-$is connected to the phenyl or biphenyl group in Y; X is independently selected from a single bond, -NH-, or O, m is an integer of 0, 1, 2, or 3, and $R^1$ at each occurrence is each independently selected from halogen, -OH, -CN, $-CF_3$, $-NO_2$, $C_{1-6}$ alkyl, $-OC_{1-6}$ alkyl, $-NH_2$, $-NH(C_{1-6}$ alkyl), and $-N(C_{1-6}$ alkyl$)_2$, q is an integer of 0, 1, 2, 3, or 4; preferably, X is independently selected from a single bond or -NH-, m is an integer of 0 or 1, and $R^1$ at each occurrence is each independently selected from halogen, -OH, -CN, $-CF_3$, $-NO_2$, $C_{1-3}$ alkyl, and $-OC_{1-3}$ alkyl, preferably $R^1$ at each occurrence is each independently selected from -Cl, -Br, -F, -OH, -CN, $-CF_3$ , $-NO_2$ , methyl, and $-OCH_3$ , and q is an integer of 0, 1, or 2;

or Y is selected from:

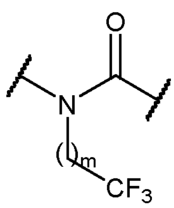 ,

wherein m is an integer of 0, 1, 2 or 3; preferably, m is 1 or 2; and the C(=O)-end of the structure Y is connected to - NH- in the structure of formula (D).

In some embodiments of the present invention, Y is selected from:

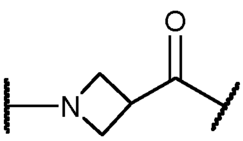 , 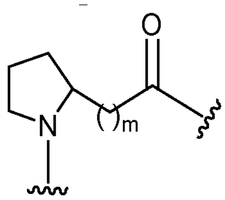 , 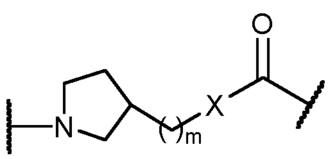 ,

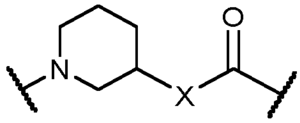, 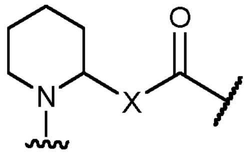, 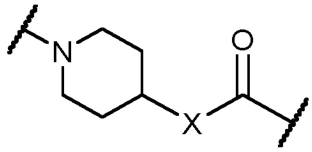, ,

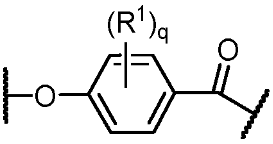 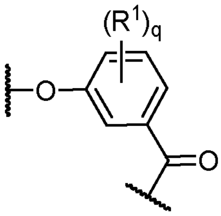, 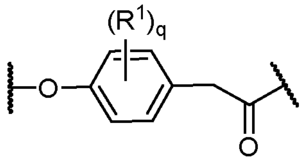, 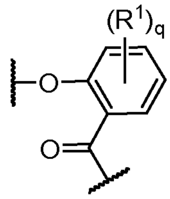,

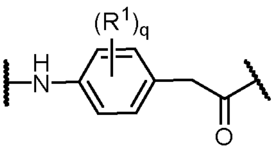, 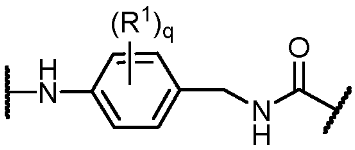, 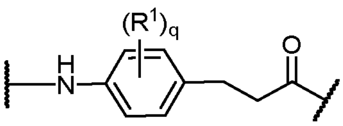,

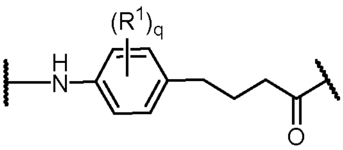, 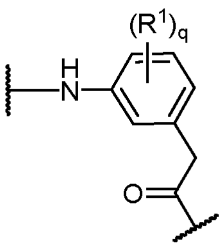, 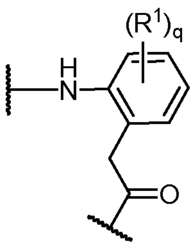,

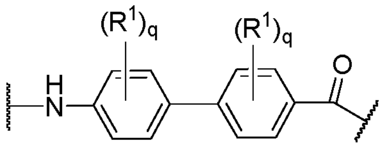, 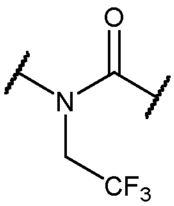, 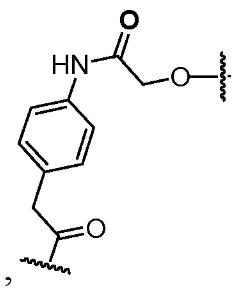 and 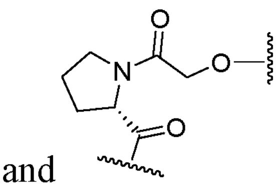.

wherein, X is independently selected from a single bond or -NH-, m is an integer of 0 or 1; $R^1$ is selected from halogen, -OH, -CN, $-CF_3$, $-NO_2$, $C_{1-6}$ alkyl, $-OC_{1-6}$ alkyl, $-NH_2$, $-NH(C_{1-6}$ alkyl), and $-N(C_{1-6}$ alkyl$)_2$, preferably $R^1$ is selected from halogen, -OH, -CN, $C_{1-3}$ alkyl, $-OC_{1-3}$ alkyl, preferably $R^1$ is selected from - Cl, -Br, -F, -OH, -CN, $-CF_3$, $-NO_2$, methyl, and $-OCH_3$;
q is an integer of 0, 1, 2, 3 or 4; preferably, q is an integer of 0, 1 or 2; and
the C(=O)-end of the Y structure is connected to -NH- in the structure of formula (E) or formula ($E_0$).

The compounds of the present invention shown in the formula (E), including but not limited to:

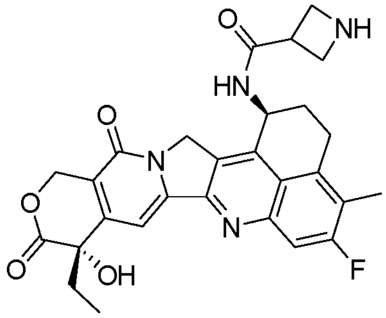
,

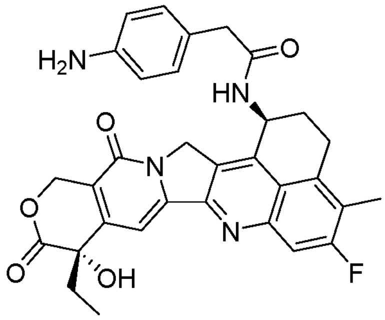
,

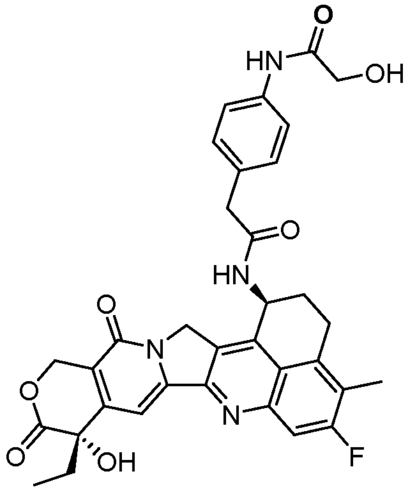
,

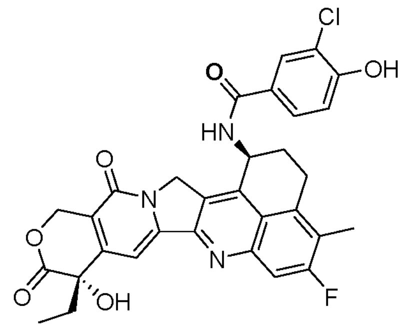
,

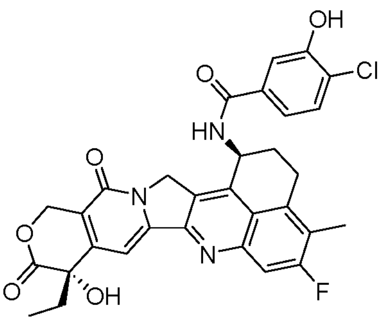
,

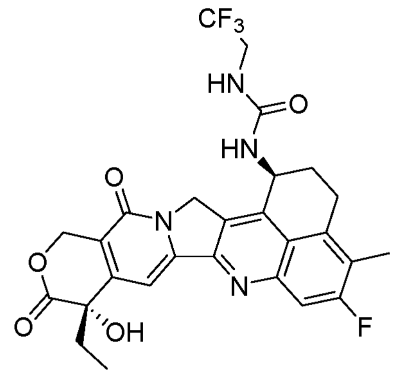
,

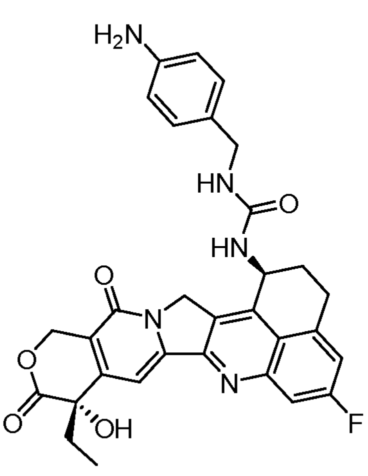
,

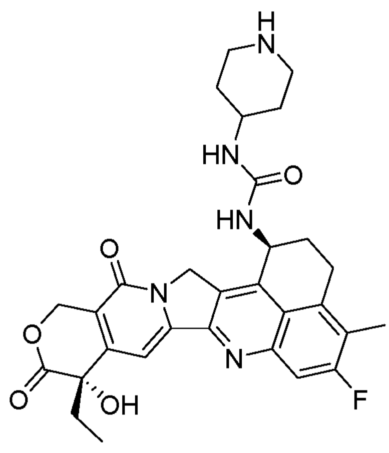
,

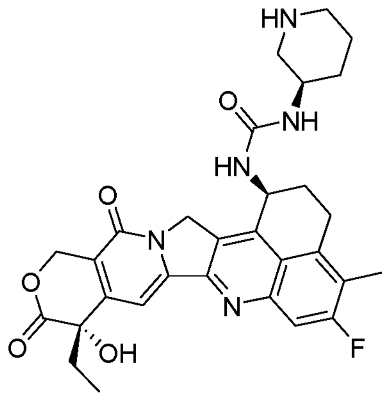

,

,

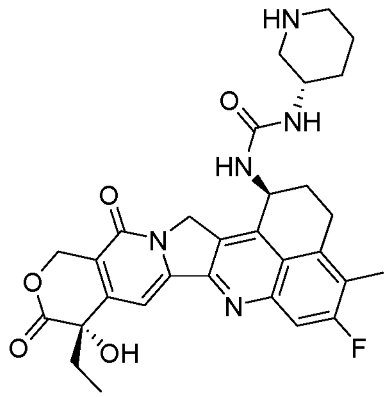

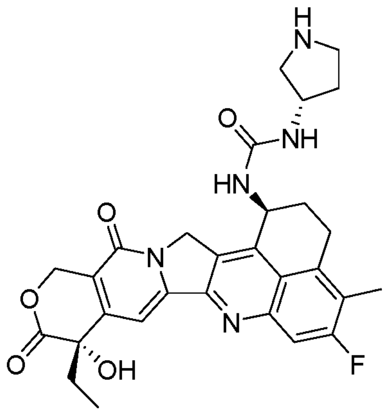

,

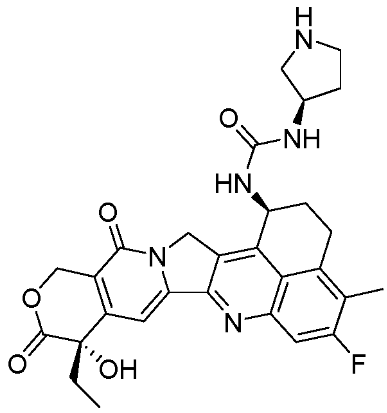

,

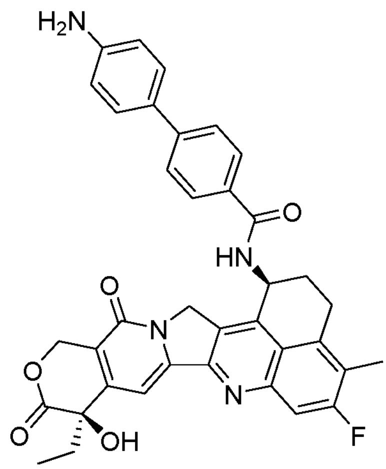

,

,

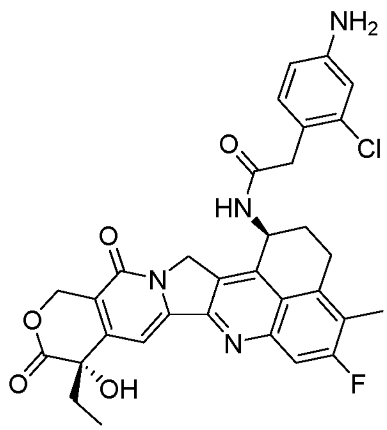

, ,

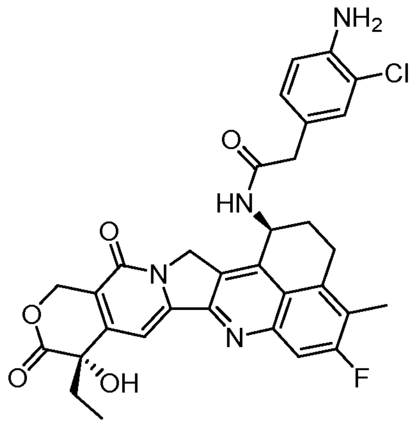
,
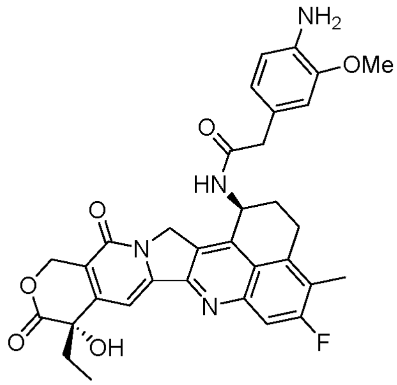
, ,

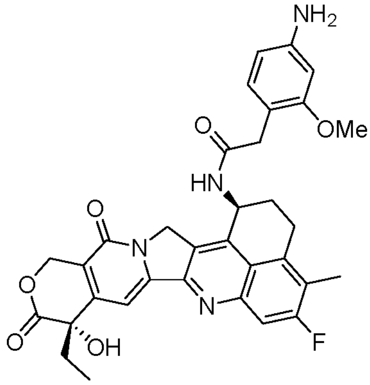
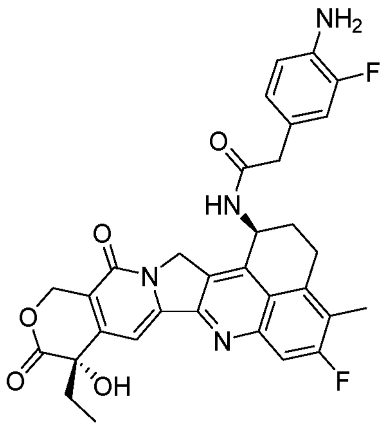
,

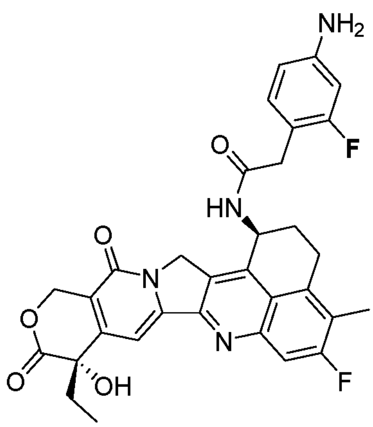
,
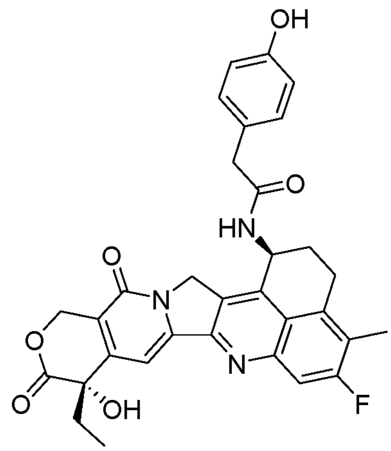
,

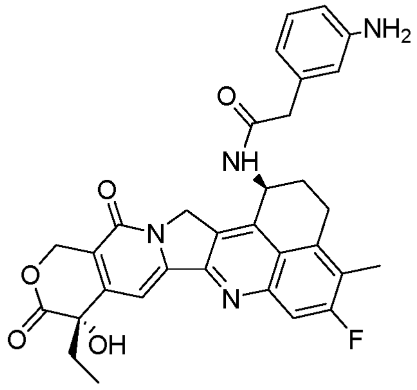
,

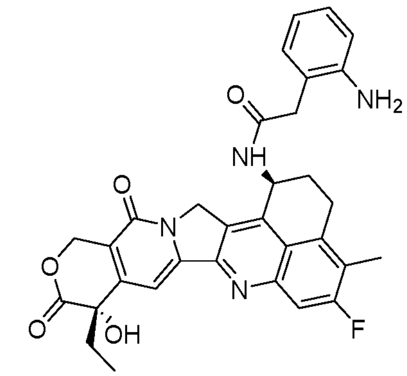
,

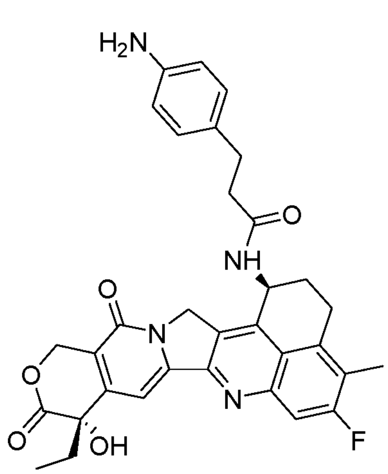
,

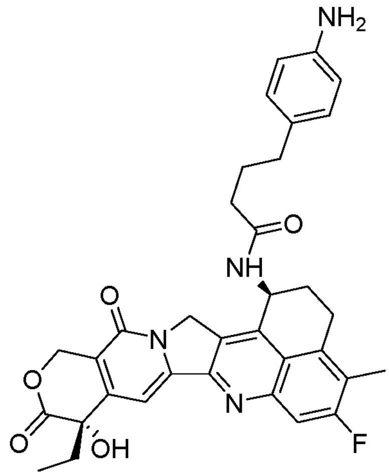
,

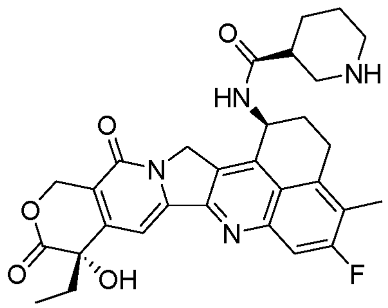
,

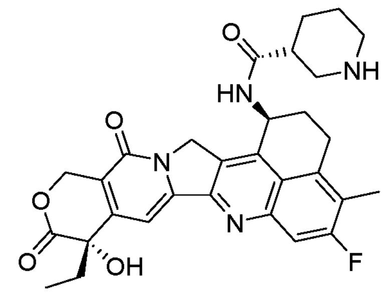
,

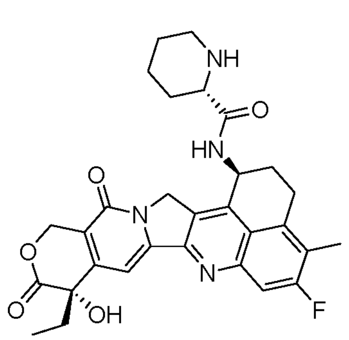
,

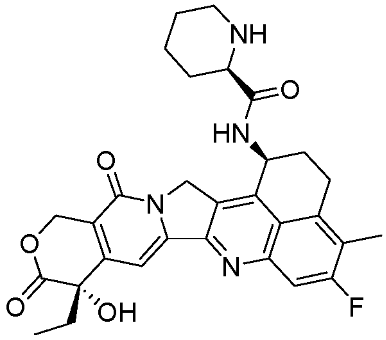
,

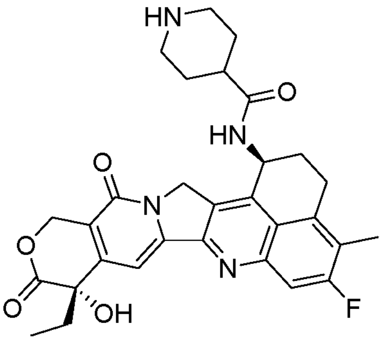,

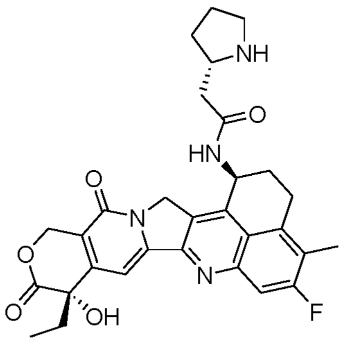,

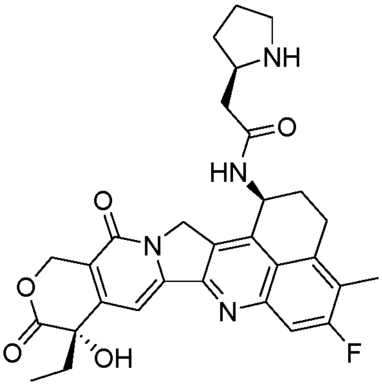,

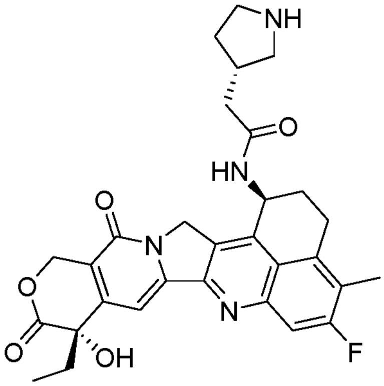,

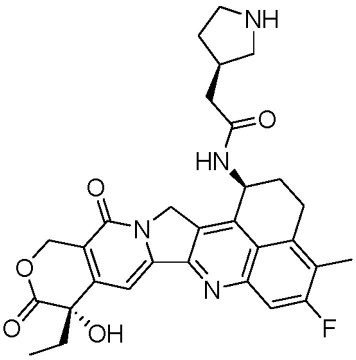

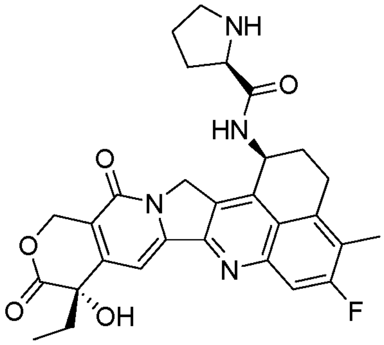

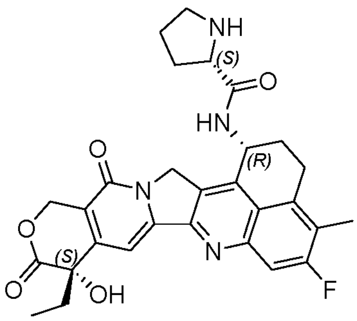

and

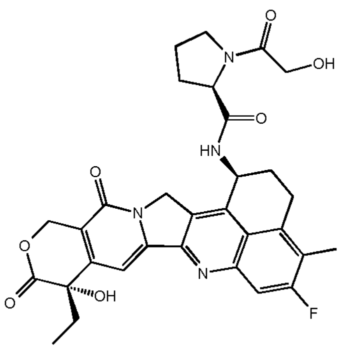.

Another aspect of the present invention provides a compound having the structure shown in the following formula (I):

$L^1$-$L^2$-$L^3$-$L^4$ (I)

wherein, $L^1$ is selected from maleimidyl-N-$(CH_2)_s$-C(=O)-, maleimidyl-N-$CH_2$-cyclohexyl-C(=O)-, wherein s is an integer from 2 to 8;

$L^2$ is selected from -$NR^4(CH_2CH_2O)_rCH_2CH_2C$(=O)- and -$NR^4CH_2$-$Ar^2$-$(CH_2CH_2O)_rCH_2CH_2NR^4C$(=O)$CH_2OCH_2C$(=O)-, wherein r is an integer from 1 to 20, and $Ar^2$ is

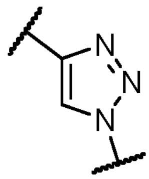

;

$L^3$ is a dipeptide residue of valine-alanine, a tripeptide residue of alanine-alanine-alanine, or a tetrapeptide residue of glycine-glycine-phenylalanine-glycine;

$L^4$ is selected from -$NR^5(CR^6R^7)_t$-Z-$(CR^6R^7)_t$-COOH or -$NR^5$-$Ar^3$-$(CR^6R^7)_t$-OH, wherein, t is an integer from 1 to 3; Z is selected from a single bond, O, S or -NH-;

$Ar^3$ is selected from phenyl, the phenyl is unsubstituted or optionally substituted with a substituent selected from H, halogen, -OH, -CN, -$CF_3$, -$NO_2$, $C_{1-6}$ alkyl;

$R^4$ and $R^5$ are identical or different, and at each occurrence are each independently selected from hydrogen atom, halogen, $C_{1-3}$alkyl, $C_{1-3}$haloalkyl and deuterated $C_{1-3}$ alkyl; and

$R^6$ and $R^7$ are identical or different, and at each occurrence are each independently selected from hydrogen atom, halogen, $C_{1-3}$ alkyl, $C_{1-3}$ haloalkyl and deuterated $C_{1-3}$ alkyl.

In some embodiments of the present invention, the compound of formula (I) is selected from:

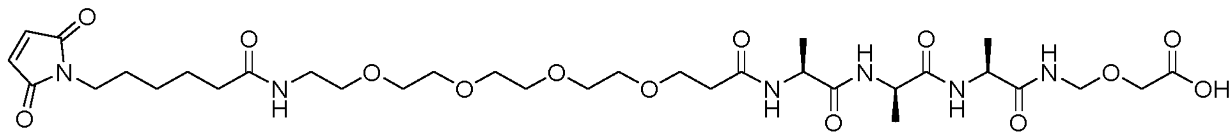

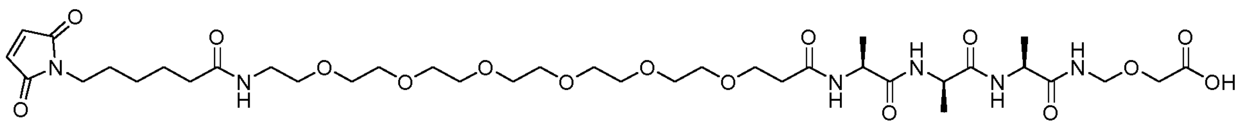

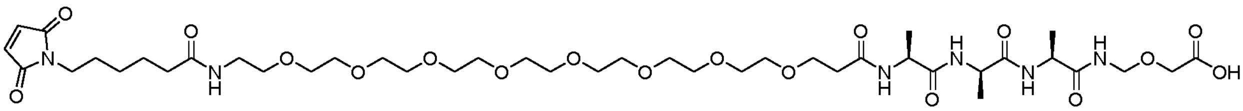

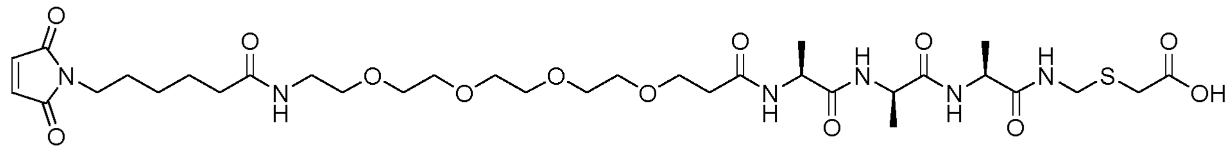

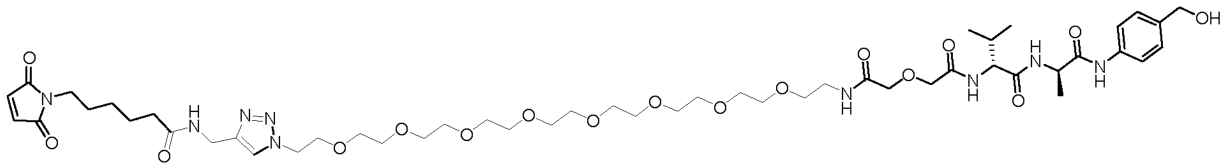

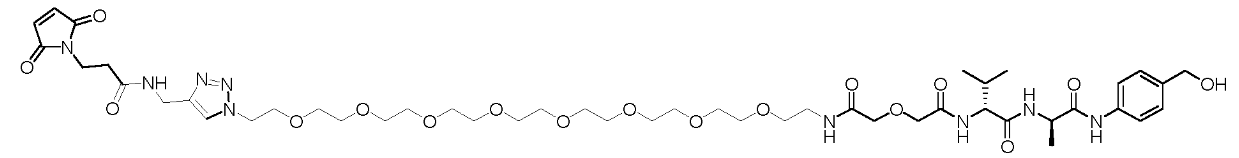

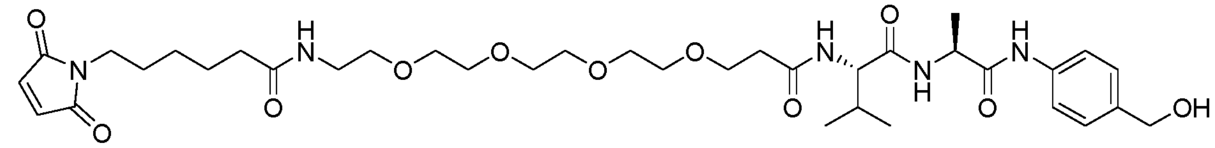

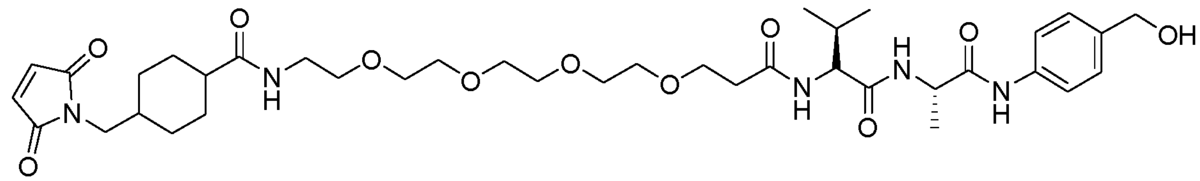

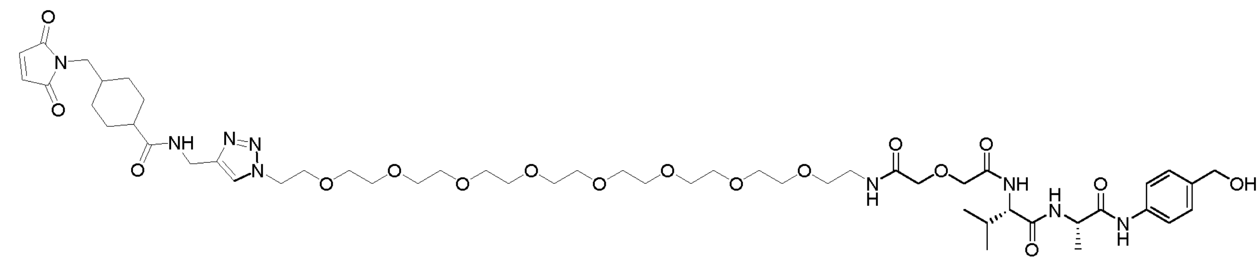

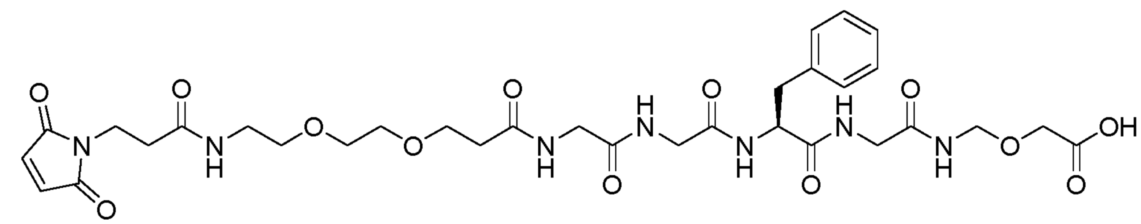

and

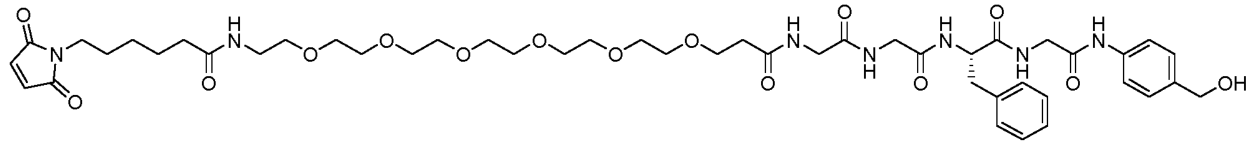

Pharmaceutical compositions, formulations and kits

Another aspect of the present invention further relates to a pharmaceutical composition comprising a therapeutically effective amount of a ligand-drug conjugate or compound as described herein, or a pharmaceutically acceptable salt or solvent compound thereof, and one or more pharmaceutically acceptable carriers.

Another aspect of the present invention also relates to a pharmaceutical formulation comprising a compound of formula I or a pharmaceutically acceptable salt thereof as an active ingredient, or a pharmaceutical composition of the present invention. In some embodiments, the formulation is in the form of a solid formulation, a semi-solid formulation, a liquid formulation or a gaseous formulation.

The pharmaceutical compositions of the present invention may be administered by a variety of routes, depending on whether topical or systemic treatment is required and the area to be treated. The drug may be administered topically (e.g., transdermally, dermally, ocularly, and mucosally including intranasal, vaginal, and rectal delivery), pulmonary (e.g., by inhalation or blowing in a powder or aerosol, including through a nebulizer; intratracheal, intranasal), orally, or

parenterally. Parenteral delivery includes intravenous, intra-arterial, subcutaneous, intraperitoneal or intramuscular injection or infusion; or intracranial, e.g., intrathecal or intracerebroventricular delivery.

Another aspect of the present invention is to provide a product, for example in the form of a kit. The product as used herein is intended to include, but is not limited to, a kit and packaging. The kit may also include instructions for use.

Treatments and uses

Another aspect of the present invention further relates to ligand-drug conjugates or compounds, or pharmaceutically acceptable salts or solvent compounds thereof, or pharmaceutical compositions thereof, as described herein, which are used as drugs,

Another aspect of the present invention further relates to the use of a ligand-drug conjugate or compound, or a pharmaceutically acceptable salt or solvates thereof, or a pharmaceutical composition thereof, as described herein in the preparation of a drug for the treatment or prevention of tumors; preferably, wherein the tumor is a cancer associated with TROP-2 expression, or the tumor is a cancer associated with HER3 expression; preferably, the HER3 expression-associated cancer is selected from non-small cell lung cancer, melanoma, breast cancer and colorectal adenocarcinoma; preferably, the TROP-2 expression-associated cancer are selected from breast cancer, triple negative breast cancer, gastric cancer and pancreatic cancer.

Another aspect of the present invention further relates to a method of treating and/or preventing a tumor, including administering a therapeutically effective amount of the ligand-drug conjugate or the pharmaceutically acceptable salt thereof, or the compound or the pharmaceutically acceptable salt thereof or the pharmaceutical composition as described herein to a subject in need thereof; preferably, wherein the tumor is a cancer associated with TROP-2 expression, or the tumor is a cancer associated with HER3 expression; preferably, the HER3 expression-associated cancer is selected from non-small cell lung cancer, melanoma, breast cancer and colorectal adenocarcinoma;preferably, the TROP-2 expression-associated cancers are selected from breast cancer, triple negative breast cancer, gastric cancer and pancreatic cancer.

Another aspect of the present invention further relates to a ligand-drug conjugate or a pharmaceutically acceptable salt thereof, or a compound or a pharmaceutically acceptable salt thereof, or a pharmaceutical composition as described herein, for use in the prevention or treatment of a tumor; preferably, wherein the tumor is a cancer associated with TROP-2 expression.

beneficial effect

The exatecan analogues provided by the present invention have strong tumor inhibition efficiency, and the TROP-2 antibody-drug conjugates and HER-3 antibody-drug conjugates provided by the present invention have good tumor inhibition effect, which is suitable for clinical drug application.

General terms and definitions

Unless otherwise defined hereinafter, all technical and scientific terms used herein are intended to have the same meaning as commonly understood by those skilled in the art.

Certain compounds of the present invention may be present in free form or, where appropriate, in the form of pharmaceutically acceptable derivatives thereof. In the present invention, pharmaceutically acceptable derivatives include, but are not limited to, pharmaceutically acceptable salts, prodrugs, stereoisomers (including, but not limited to, diastereomers and enantiomers), tautomers, solvates, polymorphs and isotopic compounds, which are capable of delivering the compounds of the present invention or metabolites thereof, either directly or indirectly, upon administration to a patient in need thereof. Accordingly, when reference is made herein to "the compounds of the present invention", it is also intended to cover the various derivative forms of the compounds described above.

The term "pharmaceutically acceptable salt" refers to a salt that retains the biological potency of the free acids and bases of a particular compound without biological adverse effects. Examples of pharmaceutically acceptable salts include, but are not limited to: (1) acid addition salts , formed with inorganic acids such as hydrochloric acid, sulfuric acid, hydrobromic acid, nitric acid, phosphoric acid, and the like; or formed with organic acids such as malic acid, fumaric acid, maleic acid, benzoic acid, phenylacetic acid, succinic acid, tartaric acid, citric acid, methanesulfonic acid, ethanesulfonic acid, hydroxyacetic acid, cinnamic acid, pyruvic acid, formic acid, acetic acid, propionic acid, oxalic acid, malonic acid, acrylic acid, mandelic acid, and the like; or (2) alkali addition salts formed with alkali metals such as lithium, sodium, potassium, and the like; with alkaline earth metals such as calcium, magnesium, and the like; and with organic bases such as ammonium, choline, diethanolamine, lysine, ethylenediamine, tertiary-butylamine, tertiary-octylamine, tris(hydroxymethyl)aminomethane, N-methylglucosamine, triethanolamine, dehydroabietic amine, and the like. Other pharmaceutically acceptable salts are known to those skilled in the art.

Prodrugs of the compounds of the present invention are included within the scope of protection of the present

invention. Typically, the prodrugs are functional derivatives that are readily converted in vivo to the desired compounds. Accordingly, the term "administer" in the therapeutic methods provided by the present invention encompasses administering a compound disclosed in the present invention, or, a compound, although not expressly disclosed in the present invention, that is capable of being converted in vivo to a compound disclosed in the present invention upon administration to a subject for the treatment of the various diseases described. Conventional methods for selecting and preparing suitable prodrugs derivatives are documented, for example, in the book Design of Prodrugs (H. Bundgaard, Elsevier, 1985).

The compounds described in the present invention may contain one or more asymmetric centers and may result in diastereomers and optical isomers. The present invention includes all possible diastereomerss and racemic mixtures thereof, substantially pure split enantiomers thereof, all possible geometric isomers and pharmaceutically acceptable salts thereof.

The compounds of the present invention do not precisely define the stereo structure of the compound at a particular position. The present invention includes all stereoisomers of the compounds and pharmaceutically acceptable salts thereof. Moreover, mixtures of stereoisomers and isolated specific stereoisomers are included in the present invention. The products produced during the synthetic process of preparing such compounds, or using racemization or differential isomerization methods well known to those of ordinary skill in the art, may be mixtures of stereoisomers.

When tautomers of the compounds of the present invention exist, the present invention includes any possible tautomers and pharmaceutically acceptable salt thereof, and mixtures thereof, unless specifically stated.

When the compounds of the invention and pharmaceutically acceptable salts thereof are present in the form of solvates or polymorphs, the invention encompasses any possible solvate and polymorph forms. The type of solvent used to form the solvates is not specifically limited, as long as the solvent is pharmaceutically acceptable. For example, water, ethanol, propanol, acetone, and similar solvents may be employed.

The present invention also includes all pharmaceutically acceptable isotope compounds which are identical to the compounds of the present invention except that one or more atoms are replaced by atoms having the same atomic number but a different atomic mass or mass number than that which predominates in nature. Examples of isotopes suitable for inclusion in the compounds of the present invention include, but are not limited to, isotopes of hydrogen (e.g., deuterium ($^{2}H$), tritium ($^{3}H$)); isotopes of carbon (e.g., $^{13}C$ and $^{14}C$); isotopes of chlorine (e.g., $^{37}Cl$); isotopes of iodine (e.g., $^{125}I$); isotopes of nitrogen (e.g., $^{13}N$ and $^{15}N$); isotopes of oxygen (e.g.,$^{17}O$ and $^{8}O$); isotopes of phosphorus (e.g., $^{32}P$); and isotopes of sulfur (e.g.,$^{34}$ S).

The term "ligand" is a macromolecular compound that recognizes and binds an antigen or receptor associated with a target cell. The role of the ligand is to present the drug to the target cell population to which the ligand is bound, in this embodiment of the invention the ligand is denoted as Pc, and the ligand may form a linkage with a linkage unit by means of a heteroatom on the ligand, preferably an antibody, or an antigen-binding fragment thereof, or a polypeptide, and the antibody is selected from chimeric antibodies, humanized antibodies, fully human or murine antibodies; preferably a monoclonal antibody.

The term "drug" refers to a cytotoxic drug, denoted as E or $E_0$, which is a chemical molecule that has a strong ability to disrupt the normal growth of tumor cells.

The term "linker unit" or "linkage fragment" or "linkage unit" refers to a fragment or bond of a chemical structure that is attached to a ligand at one end and to the drug at the other end, or can be attached to other linkers and then connected to the drug. A preferred embodiment of the present disclosure is represented by L and $L^1$ to $L^4$, wherein the $L^1$ end is attached to the ligand and the $L^4$ end is attached to the structural unit Y and then to the drug (E or $E_0$ ).

The term "ligand-drug conjugate" refers to the attachment of a ligand to a biologically active drug through a stabilized linkage unit. In the present disclosure "ligand-drug conjugate" is preferably an antibody-drug conjugate (ADC), which refers to the attachment of a monoclonal antibody or antibody fragment to a biologically active toxic drug through a stabilized linkage unit.

The three-letter codes and single-letter codes for amino acids used in this disclosure are as described in J. biol. chem, 243, p3558 (1968).

The term "antibody" refers to immunoglobulins, which are tetrapeptide chains consisting of two identical heavy chains and two identical light chains linked by interchain disulfide bonds. Immunoglobulins differ in the composition and order of amino acids in the constant region of the heavy chain, and therefore differ in their antigenicity. Accordingly, immunoglobulins can be categorized into five classes, or isoforms of immunoglobulins, i.e., IgM, IgD, IgG, IgA, and IgE, and the corresponding heavy chains are $\mu$-chain, $\delta$-chain, $\gamma$-chain, $\alpha$-chain, and $\varepsilon$-chain thereof, respectively. The same class of Ig can be divided into different subclasses according to the differences in the amino acid composition of its hinge region and the number and position of disulfide bonds in the heavy chain, e.g., IgG can be classified into IgG1, IgG2, IgG3 and IgG4. light chain is classified into $\kappa$-chain or $\lambda$-chain by the differences in the constant region. Each of the five classes of Ig may have either a $\kappa$ chain or a $\lambda$ chain. The antibodies described in the present disclosure are preferably specific antibodies against cell surface antigens on target cells, and non-limiting embodiments are the following antibodies: anti-TROP-2 antibody, anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-LIV-1 antibody, anti-ROR1 antibody, anti-CD20 antibody, anti-CD22

antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody, anti-Mesothelin antibody, or antigen-binding fragments thereof; more preferably, the antibody or antigen-binding fragment thereof is an anti-TROP-2 antibody, an anti-HER2 (ErbB2) antibody, anti-HER3 (ErbB3) antibody, anti-LIV-1 antibody, anti-ROR1 antibody, or antigen-binding fragments thereof; most preferably, the antibody or antigen-binding fragment thereof is sacituzumab, trastuzumab, or pertuzumab.

The antibodies of the present invention include murine antibodies, chimeric antibodies, humanized antibodies and fully human antibodies, preferably humanized antibodies and fully human antibodies.

The term "murine antibody" is used in the present invention to mean an antibody prepared from a mouse according to the knowledge and skill in the art. The preparation involves injecting to a test subject with a specific antigen and then isolating a hybridoma expressing an antibody having the desired sequence or functional properties.

The term "chimeric antibody" is used to describe an antibody made by fusing the variable region of a murine antibody with the constant region of a human antibody, which can reduce the immune response induced by the murine antibody. To create a chimeric antibody, a hybridoma secreting a murine-specific monoclonal antibody is established, the variable region gene is cloned from the murine hybridoma cells, the constant region gene of the human antibody is cloned according to the need, and the chimeric gene is inserted into an expression vector by linking the murine variable region gene and the human constant region gene, and then the chimeric antibody molecule is expressed in a eukaryotic system or a prokaryotic system.

The term "humanized antibody", also known as CDR-grafted antibody, refers to antibodies produced by transplanting murine CDR sequences into the variable region framework of human antibodies, i.e., the framework sequences of different types of human germline antibodies. It can overcome the heterologous reaction induced by chimeric antibodies due to carrying a large number of murine protein components.

The term "fully human antibody", also known as "fully human monoclonal antibodies", is used to describe antibodies that are of human origin in both the variable and constant regions, removing immunogenicity and toxicity.

The term "antigen-binding fragment" refers to one or more fragments of an antibody that maintain the ability to specifically bind antigen. It has been shown that fragments of a full-length antibody can be utilized for the antigen-binding function of the antibody. Examples of binding fragments included in "antigen-binding fragment" include (i) a Fab fragment, a monovalent fragment comprising the structural domains of VL, VH, CL, and CH1, (ii) a $F(ab')_2$ fragment, a bivalent fragment comprising two Fab fragments linked by a disulfide bridge over a hinge region, (iii) a F'd fragment comprising the structural domains of VH and CH1, (iv) an Fv fragment comprising the VH and VL structural domains of the single arm of the antibody, (v) a single structural domain or dAb fragment (Ward et al, (1989) Nature 341:544-546) consisting of the VH structural domain, and (vi) an isolated complementarity determining region (CDR) or (vii) a combination of two or more isolated CDRs optionally connected by a synthesized linker.In addition, although the two structural domains VL and VH of the Fv fragment are encoded by separate genes, recombinant methods may be used to connect them by synthetic linker, thereby enabling them to produce a single protein chain for which the VL and VH regions pair to form a monovalent molecule (referred to as single chain Fv (scFv); see, e.g., Bird et al. (1988) Science242:423- 426; and Huston et al. (1988) Proc. Natl. Acad. Sci USA85:5879-5883). Such single-chain antibodies are also intended to be included in the term "antigen-binding fragment" of an antibody. Such antibody fragments are obtained using conventional techniques known to those skilled in the art, and the fragments are screened for utility in the same manner as for intact antibodies. The antigen-binding portion may be produced by recombinant DNA techniques or by enzymatic or chemical cleavage of intact immunoglobulins.

Fab is an antibody fragment having a molecular weight of about 50,000 and antigen-binding activity from a fragment obtained by treatment of an IgG antibody molecule with the protease papain (cleaving amino acid residue at position 224 of the H-chain), wherein about one-half of the N-terminal side of the H-chain and the whole of the L-chain are bound together by disulfide bonds.

F(ab')2 is an antibody fragment having a molecular weight of about 100,000 and antigen-binding activity and containing two Fab regions linked at the hinge position, obtained by digesting the lower portion of the two disulfide bonds in the hinge region of IgG with the enzyme pepsin.

Fab' is an antibody fragment having a molecular weight of about 50,000 and antigen-binding activity obtained by cleaving a disulfide bond in the hinge region of F(ab')2 described above.

In addition, the Fab' can be produced by inserting DNA encoding a Fab' fragment of an antibody into a prokaryotic expression vector or a eukaryotic expression vector and introducing the vector into a prokaryotic or eukaryotic organism to express Fab'.

The terms "single-chain antibody", "single-chain Fv" or "scFv" mean a molecule comprising an antibody heavy chain variable structure domain (or region; VH) and an antibody light chain variable structure domain (or region; VL) linked by a linker. Such scFv molecules may have the general structure: $NH_2$-VL-linker-VH-COOH or $NH_2$ -VH-linker-VL-COOH.

Suitable prior art linkers comprise repeated GGGGS amino acid sequences or variants thereof, e.g., using variants of 1-4 repeats (Holliger et al. (1993), Proc. Natl. Acad. Sci. USA 90:6444-6448). Other linkers that can be used in the present disclosure are as described by Alfthan et al. (1995), Protein Eng. 8:725-731, Choi et al. (2001), Eur. J. Immuno l. 31:94-106, Hu et al. (1996), Cancer Res. 56:3055-3061, Kipriyanov et al. (1999), J. Mol. Biol. 293:41-56 and Roovers et al. (2001), Cancer Immunol.

The term "CDR" refers to one of the six highly variable regions within the variable structural domain of an antibody that primarily contribute to antigen binding. One of the most commonly used definitions of the 6 CDRs is provided by Kabat E.A. et al, (1991) Sequences of proteins of immunological interest. (NIH Publication 91-3242). As used herein, the Kabat definition of CDR should only be applied to CDR1, CDR2, and CDR3 (CDR L1, CDR L2, CDR L3 or L1, L2, L3) of light chain variable structural domains, and CDR2 and CDR3 (CDR H2, CDR H3 or H2, H3) of heavy chain variable structural domains.

The term "vector" refers to a nucleic acid molecule capable of transporting another nucleic acid to which it has been attached. In one embodiment, the vector is a "plasmid", which refers to a circular double-stranded DNA loop to which an additional DNA segments can be ligated. In another embodiment, the vector is a viral vector in which additional DNA segments can be attached to a viral genome. The vectors disclosed herein are capable of replicating autonomously in a host cell into which they have been introduced (e.g., bacterial vectors and additive mammalian vectors having the replication start point of a bacterium) or may be integrated into the genome of the host cell after introduction into the host cell, thereby replicating along with the host genome (e.g., non-episomal mammalian vectors).

The term "alkyl" refers to a saturated aliphatic hydrocarbon group, which is a straight or branched chain group comprising 1 to 20 carbon atoms, preferably an alkyl comprising 1 to 12 carbon atoms, more preferably an alkyl comprising 1 to 10 carbon atoms, and most preferably an alkyl comprising 1 to 6 carbon atoms. The term "$C_{1-6}$ alkyl" refers to a saturated straight or branched hydrocarbon group having 1 to 6 carbon atoms (e.g. 1, 2, 3, 4, 5 or 6 carbon atoms). For example, "$C_{1-6}$ alkyl" may be methyl, ethyl, n-propyl, isopropyl, n-butyl, isobutyl, sec-butyl, tert-butyl, n-pentyl, iso-pentyl, neo-pentyl, or n-hexyl.

The term "cycloalkyl" refers to a saturated or partially unsaturated monocyclic or polycyclic cyclic hydrocarbon substituent, wherein the cycloalkyl ring comprises 3 to 20 carbon atoms, preferably 3 to 12 carbon atoms, more preferably 3 to 10 carbon atoms, and most preferably 3 to 8 carbon atoms. Non-limiting examples of monocyclic cycloalkyls include cyclopropyl, cyclobutyl, cyclopentyl, cyclopentenyl, cyclohexyl, cyclohexenyl, cyclohexadienyl, cycloheptenyl, cycloheptatrienyl, cyclooctyl, and the like; polycyclic cycloalkyls include spiro, fused and bridged cycloalkyls.

The term "heterocyclyl" refers to a saturated or partially unsaturated monocyclic or polycyclic cyclic hydrocarbon substituent comprising from 3 to 20 ring atoms, wherein one or more (e.g., 1, 2, or 3) of the ring atoms are selected from nitrogen, oxygen, or sulfur and the remaining ring atoms are carbon. Preferably, it contains from 3 to 12 ring atoms, wherein 1 to 4 of which are heteroatoms; more preferably the cycloalkyl ring contains from 3 to 10 ring atoms. Non-limiting examples of monocyclic heterocyclyls include pyrrolidinyl, piperidinyl, piperazinyl, morpholinyl, thiomorpholinyl, homopiperazinyl, and the like. Polycyclic heterocyclyls include spiro, fused and bridged heterocyclyls.

The term "aryl" refers to 6 to 14 membered all-carbon monocyclic or densely fused polycyclic (i.e., rings sharing adjacent pairs of carbon atoms) groups having a conjugated $\pi$-electronic system, preferably 6 to 10 membered, e.g., phenyl and naphthyl, preferably phenyl. The aryl ring may be densely bonded to a heteroaryl, heterocyclyl or cycloalkyl ring, wherein the ring attached to the parent structure is an aryl ring.

The term "heteroaryl" refers to a heteroaromatic system comprising 1 to 4 heteroatoms and 5 to 14 ring atoms, wherein the heteroatoms are selected from oxygen, sulfur and nitrogen. Heteroaryl groups are preferably from 5 to 10 membered, more preferably 5 or 6 membered, such as furanyl, thiophenyl, pyridyl, pyrrolyl, N-alkylpyrrolyl, pyrimidyl, pyrazinyl, imidazolyl, tetrazolyl and the like. The heteroaryl ring may be fused on an aryl, heterocyclic or cycloalkyl ring, wherein the ring attached to the parent structure is a heteroaryl ring.

The term "haloalkyl" means an alkyl substituted with one or more halogens.

The term "deuterated alkyl" means an alkyl substituted with one or more deuterium atoms.

The term "halogen" means fluorine, chlorine, bromine or iodine.

The terms "substitut" and "substituted" mean that one or more (e.g., one, two, three, or four) hydrogens on the designated atom are substituted with a selection from the indicated moiety, provided that the normal atomic valence of the designated atom is not exceeded in the present context,and the substitution forms a stable compound. Combinations of substituents and/or variables are permitted only if such combinations form stable compounds.

If the substituent is described as "optionally... substituted", the substituent may be (1) unsubstituted or (2) substituted. If an atom or group is described as being optionally substituted with one or more of a list of substituents, one or more hydrogens on the atom or group may be replaced with an independently selected, optional substituent. If the substituents are described as "independently selected from" or "each independently are", the substituents are each selected independently. Thus, each substituent may be the same as or different from another (other) substituent. For example, when a substituent or substituent position or a different substituent or substituent position has an R group (e.g., but not limited to, $R^2$ , $R^3$ , $R^h$ , $R^i$ , $R^x$ and/or $R^y$ ) that may have identical or different symbols, the individual R are each

selected independently, i.e., they may be identical or different. The same applies to the selection of numerical values such as d, g, m, and n.

Unless indicated, as used herein, the linkages of substituent may be derived from any suitable position of the substituent.

When the bond of the substituent is shown to be through a bond connecting two atoms in the ring, then such a substituent may be bonded to any of the ring-forming atoms in the substituted ring.

The terms "include", "comprise", "have", "containg" or "involve" and other variations thereof are inclusive or open-ended herein and do not exclude other elements or method steps not enumerated. It should be understood by those skilled in the art that terms such as "comprising" cover the meaning of "consisting of".

In the present invention, "pharmaceutically acceptable carrier" means a diluent, excipient, or medium that is administered with the active ingredient and that is suitable, within the bounds of reasonable medical judgment, for exposure to the tissues of human beings and/or other animals without undue toxicity, irritation, allergic reaction, or other problems or complications commensurate with a reasonable benefit/risk ratio. The term "active ingredient", "therapeutic agent", "active substance" or "active agent" refers to a chemical entity that is effective in treating one or more symptoms of a target disease or condition.

As used herein, the term "effective amount" (e.g., "therapeutically effective amount" or " preventively effective amount") refers to an amount of an active ingredient that, when administered, achieves the desired effect to a certain extent, such as relieving one or more symptoms of the condition being treated or preventing the occurrence of the condition or its symptoms.

Unless otherwise indicated, as used herein, the term "treating" means reversing, mitigating, inhibiting the progression of, the disease or condition which to the term applies, or one or more symptoms of the disease or condition, or preventing the disease or condition, or one or more symptoms of the disease or condition.

## BRIEF DESCRIPTION OF THE DRAWINGS

FIG. 1 shows a line graph of the internalization levels of the TROP-2-ADC compounds provided by the present invention for MDA-MB-468 cells;

FIG. 2 shows a line graph of the inhibition of tumor volume growth for the MDA-MB-468 cell line by the TROP-2-ADC compounds provided by the present invention;

FIG. 3 shows a line graph of the results of tumor volume growth inhibition experiments with the TROP-2-ADC compounds provided by the present invention;

FIG. 4 shows a growth curve graph of the inhibition of gastric cancer tumor with the TROP-2-ADC compounds provided by the present invention;

FIG. 5 shows a growth curve graph of inhibiting MDA-MB-453 cell line tumors in the presence of HER3-ADC compounds provided by the present invention;

FIG. 6 shows a growth curve graph of inhibiting SW620 cell line tumors in the presence of the HER3-ADC compounds provided by the present invention, which is an in vivo efficacy evaluation of multiple dose repeated administration;

FIG. 7 shows a graph of the results of assaying the cellular internalization levels of the antibody drug conjugates (ADCs) provided by the present invention; wherein, a is a line graph of the internalization levels of the HER3-ADC compounds provided by the present invention for MAD-MB-453 cells, b is a line graph of the internalization levels of the HER3-ADC compounds provided by the present invention for HCC1569 cells, and c is a line graph of the internalization levels of the HER3-ADC compounds provided by the present invention for SW620 cells;

FIG. 8 shows a line graph of the level of melanoma inhibition by the HER3-ADC compounds provided by the present invention in mice;

FIG. 9 shows a line graph of the level of tumor inhibition of non-small cell lung cancer by the HER3-ADC compounds provided by the present invention in mice;

FIG. 10 shows a graph of stability assay results of HER3-ADC compounds provided by the present invention in animal plasma; wherein, A is a graph of stability assay results of HER3-ADC compounds provided by the present invention in human plasma; B is a graph of stability assay results of HER3-ADC compounds provided by the present invention in monkey plasma; C is a graph of stability assay results of HER3-ADC compounds provided by the present invention in rat plasma; D is a graph of the stability assay results of the HER3-ADC compounds provided by the present invention in mouse plasma;

FIG. 11 shows a line graph of the change of HER3 antibody levels in rat plasma after administration of the HER3-ADC compounds provided by the present invention and the control drug U3-1402;

FIG. 12 shows a line graph of the change of HER3 antibody levels in rat plasma after administration of the HER3-ADC compounds provided by the present invention and the control drug U3-1402;

FIG. 13 shows a schematic diagram of the content of small molecule components produced in vivo in rats by the HER3-ADC drugs provided by the present invention;
Figure 14 shows a line graph of the content of small molecule components produced in vivo in rats by the control drug U3-1402;
FIG. 15 shows a graph of growth curve of inhibiting SW620 cell line tumors in the presence of HER3-ADC compounds provided by the present invention, which is an in vivo efficacy evaluation of a single dose repeated administration.

## DETAILED DESCRIPTION

The experimental methods in the following embodiments are conventional methods unless otherwise specified. The chemical raw materials, reagents and the like used in the following embodiments are commercially purchased products, unless otherwise specified. Abbreviations and their meanings appearing herein are shown below:

Table 1 Abbreviations and their meanings

| abbreviation | paraphrase |
|---|---|
| EXD | Exatecan mesylate |
| DMF | N,N-Dimethylformamide |
| HATU | 2-(7-Azobenzotriazole)-N,N,N',N'-tetramethyluronium hexafluorophosphate |
| DIEA | N,N-Diisopropylethylamine |
| EDCI·HCl | 1-(3-Dimethylaminopropyl)-3-ethylcarbodiimide hydrochloride |
| DCC | N,N'-Dicyclohexylcarbodiimide |
| HOSu | N-Hydroxysuccinimide |
| DMAP | 4-Dimethylaminopyridine |
| TFA | trifluoroacetic acid |
| HOBt | 1 -Hydroxybenzotriazole |
| EEDQ | 2-Ethoxy-1 -ethoxycarbonyl-1,2-dihydroquinoline |
| NPC | Bis(p-nitrophenyl) carbonate |
| EMCA | 6-Maleimidohexanoic acid |

Cell name and origin

Unless otherwise stated, the cell strains or cell lines used in the embodiments of the present invention are available commercially. Wherein,

HEK293f cell line , purchased from Nanjing Cobioer Biosciences Co., LTD;
SW620 cell line, purchased from Nanjing Cobioer Biosciences Co., LTD;
Bxpc-3 cell line , purchased from Nanjing Cobioer Biosciences Co., LTD;
NCI-N87 cell line, purchased from Nanjing Cobioer Biosciences Co., LTD;
A375 cell line, purchased from ATCC (American type culture collection);
NCI-H358 cell line, purchased from ATCC;
293F cell line, purchased from Zhuhai Kairui Biotechnology Co., LTD;
HCC1569 cell line, purchased from ATCC;
MDA-MB-453 cell line, purchased from Nanjing Cobioer Biosciences Co., LTD;
MDA-MB-468 cell line, purchased from Nanjing Cobioer Biosciences Co., LTD;
Nalm6-GFP cells , purchased from Creative Biogene.

## EXAMPLES

### Example 1

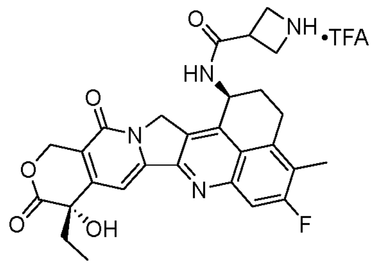

**1**

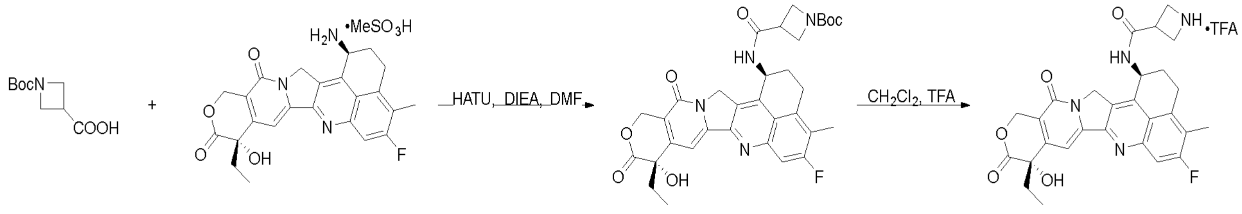


**1a** **EXD** **1b** **1**

Synthesis of compound 1b:

EXD (25mg, 47 μ mol, 1.0 eq, purchased from MedChemExpress, item no. HY-13631A) and compound 1a (9.5 mg, 47μmol, 1.0 eq) were dissolved in DMF (1 mL). HATU (26.8 mg, 70 μmol, 1.5 eq) and DIEA (19 μL, 118 μmol, 2.5 eq) were add and stirred for 2 h at room temperature. Water was added, extracted with ethyl acetate, and the organic phase was washed with saturated saline, dried, concentrated and then purified by column chromatography (9% MeOH in $CH_2Cl_2$) to give a white solid (29 mg, 100%). MS (ESI): *m/z* found $[M-99]^+$ = 519.2.

Synthesis of compound 1:

Compound 1b (28 mg, 45 μmol) was dissolved in trifluoroacetic acid (0.4mL) and dichloromethane (2 mL) and stirred for 3h at room temperature. Concentrated and then purified by reversed-phase column chromatography (32% $CH_3CN$ in $H_2O$, 0.1% TFA) to obtain a yellow solid (28.6 mg, 100%).MS (ESI): *m/z* found $[M+1]^+$ =519.2.

$^1H$ NMR (400 MHz, DMSO-$d_6$): $\delta$ 8.76 (br, 2H), 8.69 (d, *J* = 8.4 Hz, 1H), 7.82 (d, *J* = 11.2 Hz, 1H), 7.32 (s, 1H), 6.52 (br, 1H), 5.62 (t, *J* = 4.0 Hz, 1H), 5.43 (s, 2 H), 5.23 ( q, *J* = 19.8 Hz, 2H), 4.10-4.02 (m, 4H), 3.18-3.12 (m, 2H), 2.41 (s, 3 H), 2.19-2.16 (m 2H), 0.87 (t, *J* = 7.2 Hz, 3H).

Example 2

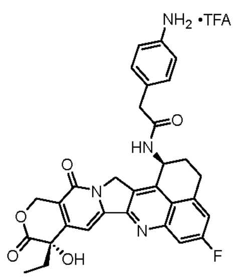

**2**

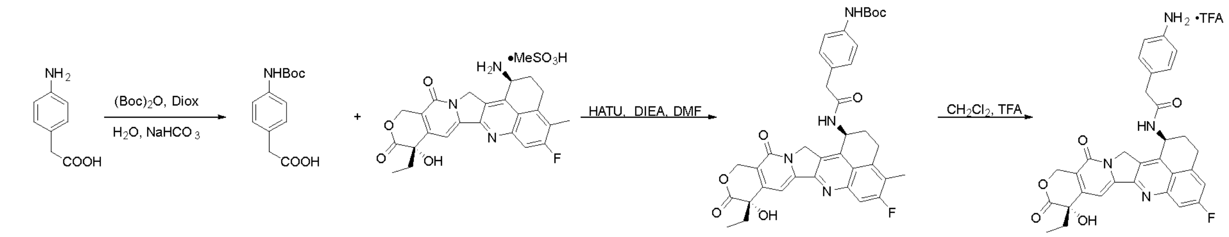


2a 2b EXD 2c 2

Synthesis of compound 2b:

p-Aminophenylacetic acid 2a (617mg, 4.08mmol, 1.0 eq) was dissolved in dioxane (8 mL) and water (4 mL), and 1N sodium bicarbonate (4 mL) and $(Boc)_2O$ (980 mg, 4.49 mmol, 1.1 eq) were added, and stirred for 24 h at room temperature. The reaction solution was neutralized with 2N hydrochloric acid, diluted with 50 mL of water, extracted with ethyl acetate, washed with saturated brine, dried and concentrated and purified by column chromatography (25% EA in PE) to obtain a white solid (700 mg, 68%), MS (ESI): *m/z* found $[M-1]^-$= 250.0.

Synthesis of compound 2c:

EXD (25 mg, 47 μmol, 1.0 eq) and compound 2a (11.8 mg, 47 μmol, 1.0 eq) were dissolved in DMF (1 mL). HATU (26.8 mg, 70 μmol, 1.5 eq) and DIEA (19 μL, 118 μmol, 2.5 eq) were added, and stirred for 2 h at room temperature. Water was added, extracted with ethyl acetate, and the organic phase was washed with saturated saline, dried and concentrated and purified by column chromatography (4% MeOH in $CH_2Cl_2$) to obtain a white solid (27 mg, 86%), MS (ESI): *m/z* found $[M+1]^+$ = 669.2.

Synthesis of compound 2: Compound 2c (27 mg, 40 μmol) was dissolved in trifluoroacetic acid (0.4 mL) and dichloromethane (2 mL) and stirred for 2.5 h at room temperature. Concentrated and purified by reversed-phase column chromatography (31% $CH_3CN$ in $H_2O$, 0.1% TFA) to obtain a yellow solid (19.3 mg, 70%), MS (ESI): *m/z* found $[M+1]^+$ =569.2.

$^1H$ NMR (400 MHz, DMSO-$d_6$ ): $\delta$ 8.71 (d, *J* = 8.4 Hz, 1H), 7.82 (d, *J* = 10.8 Hz, 1H), 7.32-7.30 (m, 3H), 7.11 (d, *J* = 8.0 Hz, 2H), 6.55 (br, 1H), 5.56 - 5.50 (m, 1H), 5.44 (s, 2H), 5.21 (dd, *J* = 18.4, 32.8 Hz, 2H), 3.50 - 3.18 (m, 4H), 2.42 (s, 3H), 2.14 - 2.12 (m, 2H), 1.93-1.82 (m, 2H), 0.88 (t, *J* =7.2 Hz, 3H).

## Example 3

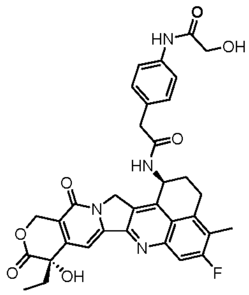

3

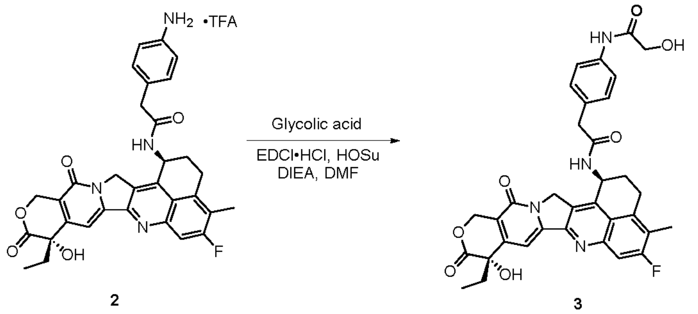


2 3

Synthesis of compound 3:

Hydroxyacetic acid (2.0 mg, 26 μmol, 1.0 eq) was dissolved in DMF (1 mL). EDCI•HCl (6.1 mg, 32 μmol, 1.2 eq) and HOSu (3.7 mg, 32 μmol, 1.2 eq) were added, and stirred for 2h at room temperature. Compound 2 (18 mg, 26 μmol, 1.0 eq) and DIEA (11μL, 66 μmol, 2.5 eq) were added and stirring was continued for 10 hours. The reaction solution was purified by HPLC to obtain a pale yellow solid (10.0 mg, 61%), MS (ESI): *m/z* found $[M+1]^+$ = 627.2.

$^1$H NMR (400 MHz, DMSO-$d_6$ ): $\delta$ 9.59 (s, 1H), 8.66 (t, *J* = 9.0 Hz, 1H), 7.79 (d, *J* = 11.0 Hz, 1H), 7.59 (d, *J* = 8.0 Hz,, 2H), 7.29 (d, *J* = 1.9 Hz, 1H), 7.20 (dd, *J* = 8.4, 3.5 Hz 2H), 6.52 (s, 1H), 5.65 (s, 1H), 5.51 (dt, *J* = 9.1, 4.9 Hz, 1H), 5.42 (d, *J* = 3.3 Hz, 2H), 5.24 (dd, *J* = 18.9, 2.9 Hz, 1H), 5.12 (dd,*J* = 19.0, 4.6 Hz, 1H), 3.95 (d, *J* = 4.2 Hz, 2H), 3.43 (d, *J* = 4.0 Hz, 2H), 3.16 (d, *J* = 6.0 Hz, 2H), 2.39 (s, 3H), 2.15-2.06 (m, 2H), 1.90-1.79 (m, 2H), 0.85 (td, *J* = 7.4, 2.8 Hz, 3H).

## Example 4

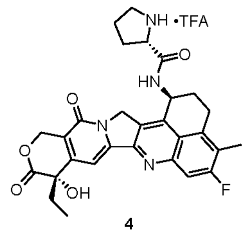

4

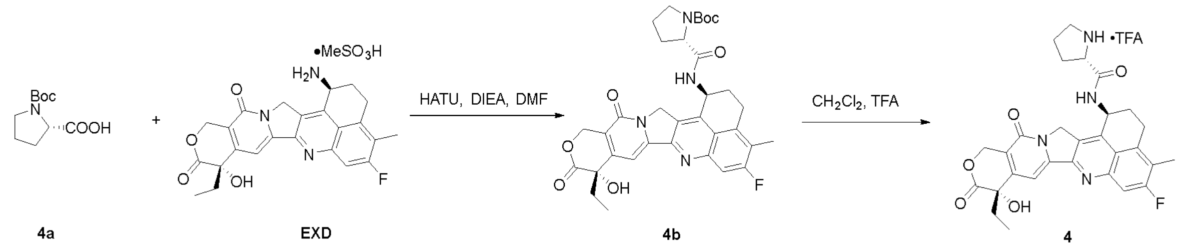


4a EXD 4b 4

Synthesis of compound 4b:

EXD (25 mg, 47 μmol, 1.0 eq) and N-Boc-L-proline 4a (10 mg, 47 μmol, 1.0 eq) were dissolved in DMF (1 mL), and HATU (26.8 mg, 70 μmol, 1.5 eq) and DIEA (19 μL, 118 μmol, 2.5 eq) were added, and stirred for 2 h at room temperature. The reaction solution changed from turbid to clear. Water was added, and the reaction solution was extracted with ethyl acetate, washed with saturated saline, dried, concentrated and purified by column chromatography (4% MeOH in $CH_2Cl_2$) to obtain a white solid (26 mg, 88%), MS (ESI): m/z found [M+1]+ = 633.2.

Synthesis of compound 4:

Compound 4b (27 mg, 43 μmol) was dissolved in trifluoroacetic acid (0.4 mL) and dichloromethane (2 mL) and stirred for 2.5 h at room temperature. Concentrated and purified by reversed-phase column chromatography (31% $CH_3CN$ in $H_2O$, 0.1% TFA) to obtain a yellow solid (18.5 mg, 67%), MS (ESI): *m/z* found $[M+1]^+$ =533.2.

$^1$H NMR (400 MHz, DMSO-$d_6$ ): $\delta$ 9.11 (d, *J* = 8.4 Hz, 1H), 7.84 (d, *J* = 10.8 Hz, 1H), 7.32 (s, 1H), 6.55 (s, 1H),

5.65-5.61 (m, 1H), 5.43 (s, 2 H), 5.30 (d, *J* = 18.8 Hz, 1H), 5.09 (d, *J* = 18.8 Hz, 1H), 4.17 (s, 1H), 3.38-3.21 (m, 4 H), 2.42 (s, 3 H), 2.25-2.20 (m, 3 H), 1.94-1.83 (m, 5 H), 0.88 (t, *J* =7.2 Hz, 3 H).

**Example 5**

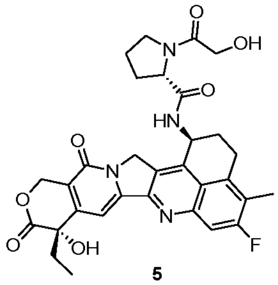

5

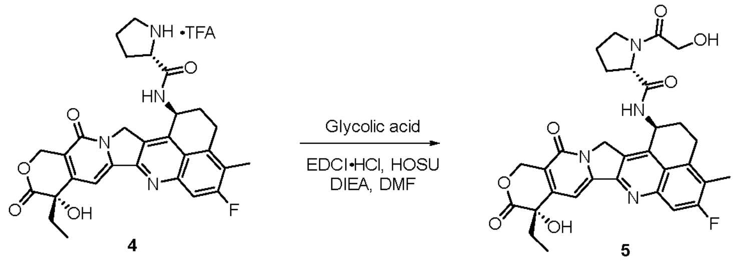


4 5

Synthesis of compound 5:

Hydroxyacetic acid (3.5 mg, 46 μmol, 1.0 eq) was dissolved in DMF (1 mL). EDCI-HCl (10.7 mg, 56 μmol, 1.2 eq) and HOSu (6.4 mg, 56 μmol, 1.2 eq) were added, and stirred for 3 h at room temperature. Compound 4 (30 mg, 46 μmol, 1.0 eq) and DIEA (15 μL, 93 μmol, 2.0 eq) were added and stirring was continued for 4 hours. The reaction solution was purified by HPLC to obtain a white solid (7.2 mg, 26%).MS (ESI): *m/z* found $[M+1]^+$ = 591.4.

$^1H$ NMR (400 MHz, DMSO-d6): δ 8.69-8.44 (m, 1H), 7.79 (d, J = 10.9 Hz, 1H), 7.30 (s, 1H), 6.52 (s, 1H), 5.52 (dt, J = 9.1, 4.7 Hz, 1H), 5.41 (d, J = 4.2 Hz, 2H), 5.20 (d, J = 2.5 Hz, 2H), 4.40 (t, J = 5.8 Hz, 1H), 4.25 (dd, J = 8.1, 4.0 Hz, 1H), 4.07 (dd, J = 20.7, 5.3 Hz, 1H), 4.01 (d, J = 5.1 Hz, 1H), 3.50-3.44 (m, 2H), 3.15 (d, J = 4.0 Hz, 2H), 2.38 (s, 3H), 2.16-1.96 (m, 4H), 1.93-1.76 (m, 4H), 0.84 (t, J = 7.4 Hz, 3H) ppm.

**Example 6**

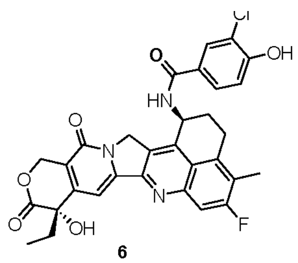

6

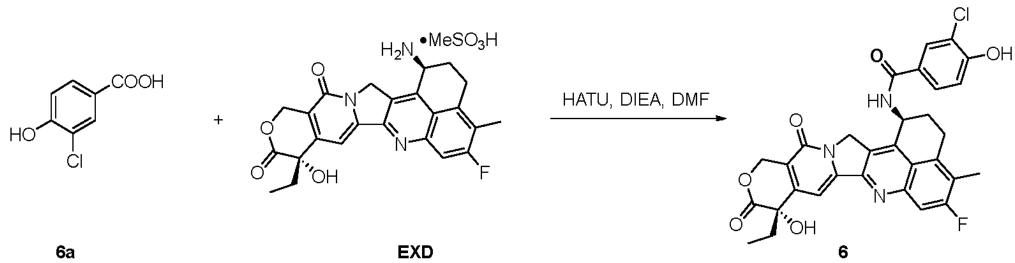


6a EXD 6

Synthesis of compound 6:

EXD (25 mg, 47μmol, 1.0 eq) and compound 6a (8.1 mg, 47 μmol, 1.0 eq) were dissolved in DMF (1 mL), and HATU (20 mg, 52 μmol, 1.1 eq) and DIEA (16 μL, 0.1 mmol, 2.1 eq) were added, and stirred for 2 h at room temperature. The reaction solution was purified by reversed-phase column chromatography (55% $CH_3CN$ in $H_2O$, 0.05% HCOOH) to obtain a white solid (10.4 mg, 38%), MS (ESI): *m/z* found $[M+1]^+$ = 590.0.

1H NMR (400 MHz, DMSO-$d_6$): $\delta$ 8.86 (d, *J* = 8.4 Hz, 1H), 7.95 (d, *J* = 2.2 Hz, 1H), 7.88-7.74 (m, 2H), 7.30 (s, 1H), 7.01 (d, *J* = 8.5 Hz, 1H), 6.52 (s, 1H), 5.76 (q, J = 6.4 Hz, 1H), 5.38 (s, 2H), 5.25-5.05 (m, 2H), 3.26-3.12 (m, 2H), 3.26-3.12 (m, 2H) 5.76 (q, *J* = 6.4 Hz, 1H), 5.38 (s, 2H), 5.25-5.05 (m, 2H), 3.26-3.12 (m, 2H), 2.42 (s, 3H), 2.31-2.14 (m, H), 1.85 ( hept, *J* = 7.1 Hz, 2H), 0.86 (t, *J* = 7.3 Hz, 3H).

## Example 7

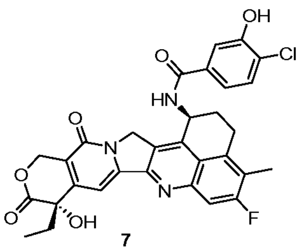

7

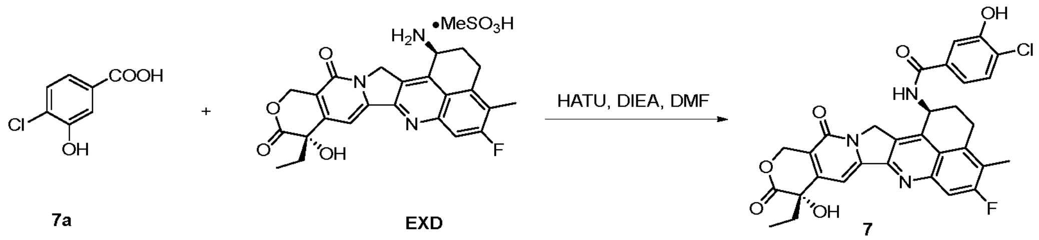


7a EXD 7

Synthesis of compound 7:

EXD (25 mg, 47 μmol, 1.0 eq) and compound 7a (8.1 mg, 47 μmol, 1.0 eq) were dissolved in DMF (1 mL). HATU (26.8 mg, 70 μmol, 1.5 eq) and DIEA (16 μL, 118 μmol, 2.1 eq) were added, and stirred for 2 h at room temperature. The reaction solution was purified by reversed-phase column chromatography (55% $CH_3CN$ in $H_2O$, 0.05% HCOOH) to obtain a white solid (5.2 mg, 19%), MS (ESI): *m/z* found $[M+1]^+$ = 590.0.

$^1H$ NMR (400 MHz, DMSO-$d_6$): $\delta$ 10.59 (s, 1H), 9.01 (d, *J* = 8.4 Hz, 1H), 7.81 (d, *J* = 11.0 Hz, 1H), 7.54 (d, *J* = 2.0 Hz, 1H), 7.41 (d, *J* = 8.3 Hz, 1H), 7.34 (dd, *J* = 8.3, 1.9 Hz, 1H), 7.30 (s, 1H), 6.54 (s, 1H), 5.75 (q, *J* = 6.9, 6.2 Hz, 1H), 5.38 (s, 2 H), 5.22 (d, *J* = 18.8 Hz, 1H), 5.08 (d, *J* = 18.9 Hz, 1H), 3.26-3.12 (m, 2 H), 2.41 (s, 3 H 2.41 (s, 3 H), 2.31-2.20 (m, 2 H), 1.90-1.78 (m, *J* = 7.2 Hz, 2 H), 0.86 (t, *J* = 7.3 Hz, 3 H).

## Example 8

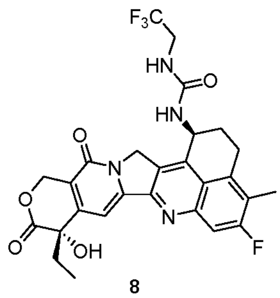

**8**

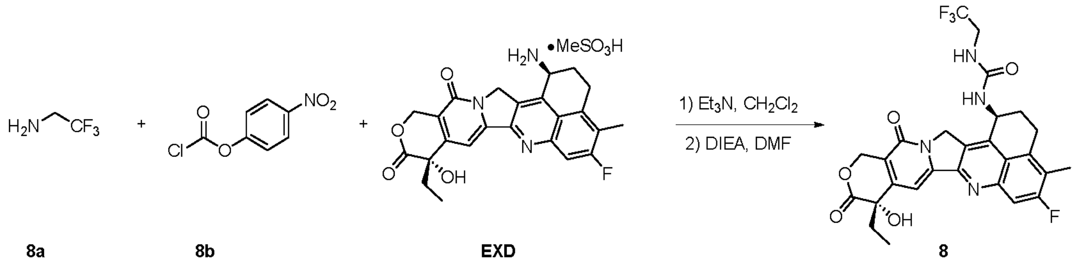


**8a** **8b** **EXD** **8**

Synthesis of compound 8:

p-Nitrophenyl chloroformate 8b (141 mg, 0.7 mmol, 1.0 eq) was dissolved in dichloromethane (2.0 mL), and pyridine (56 μL, 0.7 mmol, 1.0 eq) and trifluoroethylamine (50 μL, 0.7 mmol, 1.0 eq) were added in an ice bath and stirred for 2 h at room temperature to obtain the reaction intermediate, MS (ESI): *m*/*z* found $[M+1]^+$ = 265.0.

EXD (30 mg, 56 μmol, 1 eq) was dissolved in DMF (2.0 mL) and DIEA (19 μL, 0.11 mmol, 2.0 eq) was added. After the reaction solution was clarified, 0.2 mL of the reaction solution intermediate was added and stirred for 1 h at room temperature. The reaction solution was quenched with water, extracted with ethyl acetate, dried and concentrated and then purified by column chromatography (8% MeOH in EA) to obtain a white solid (20.5 mg, 65%), MS (ESI): *m*/*z* found $[M+1]^+$ = 561.2.

$^1$H NMR (400 MHz, DMSO-$d_6$): $\delta$ 8.67 (d, *J* = 10.8 Hz, 1H), 7.30 (s, 1H), 7.12 (d, *J* = 8.8 Hz, 1H), 6.68 (t, *J* = 6.0 Hz, 1H), 6.52 (br, 1H), 5.42 (s, 2H), 5.40- 5.35 (m, 1H), 5.27 (s, 2H), 3.94-3.85 (m, 2H), 3.17 (t, *J* = 6.0 Hz, 2H), 2.40 (s, 3H), 2.18 (t, *J* = 6.0 Hz, 2H), 1.92-1.83 (m, 2H), 0.87 (t, J = 6.0 Hz, 2H), 1.92-1.83 (m, 2H), 1.92-1.83 (m, 2H), 1.92-1.83 (m, 2H) 0.87 (t, *J* = 4.0 Hz, 3H).

## Example 9

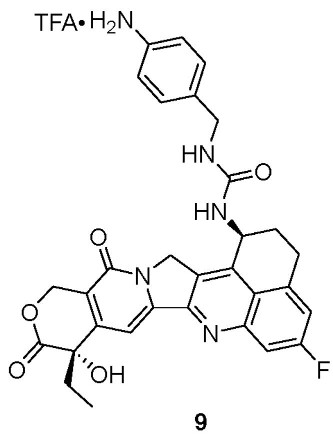

**9**

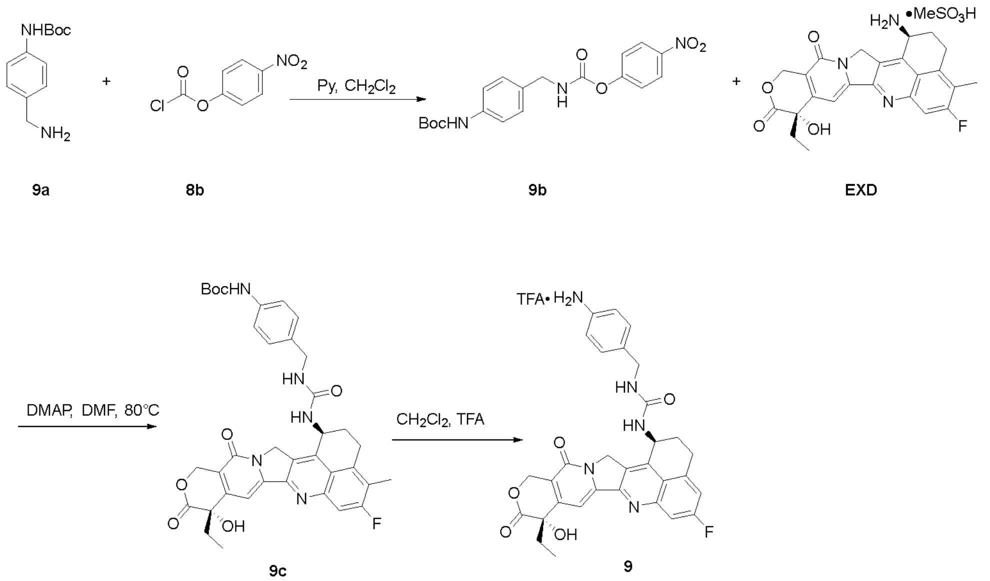


9a 8b 9b EXD

9c 9

Synthesis of compound 9b:

Compound 9a (100 mg, 0.45mmol, 1.0 eq) was dissolved in dichloromethane (2mL). Pyridine (40μL, 0.49mmol, 1.1 eq) and compound 8b (100 mg, 0.49 mmol, 1.1 eq) were added in an ice bath and stirred for 2 h at room temperature. Water was added, extracted with dichloromethane, dried and concentrated and then purified by column chromatography (25% EA in PE) to obtain a yellow solid (120 mg, 69%).MS (ESI): *m/z* found $[M+1]^+$ = 388.1

Synthesis of compound 9c:

EXD (30 mg, 56 μmol, 1.0 eq) was dissolved in DMF (1.0 mL) and DMAP (13.8 mg, 0.11 mmol, 2.0 eq) was added. After the reaction solution was clarified, the compound 9b was added (22 mg, 56 μmol, 1.0 eq) and stirred at 80°C for 5 hours. The reaction solution was quenched with water, extracted with ethyl acetate, dried and concentrated and then purified by column chromatography (5% MeOH in $CH_2Cl_2$) to give a white solid (26 mg, 67%), MS (ESI): *m/z* found $[M-55]^+$ =628.3.

Synthesis of compound 9:

Compound 9b (25 mg, 37 μmol) was dissolved in dichloromethane (2 mL), and trifluoroacetic acid (0.5 mL) was added, and stirred for 2.5 h at room temperature. Concentrated and then purified by reversed-phase column chromatography (33% $CH_3CN$ in $H_2O$, 0.1% TFA) to obtain a yellow solid (15 mg, 59%) with MS (ESI): *m/z* found $[M+1]^+$ =584.2.

$^1H$ NMR (400 MHz, DMSO-$d_6$): δ 7.67 (d, *J* = 10.9 Hz, 1H), 7.20 (s, 1H), 7.06 (d, *J* = 7.9 Hz, 2H), 6.76 (d, *J* = 7.9 Hz, 2H), 6.64 (d, *J* = 8.9 Hz, 1H), 6.42 (s, 1H), 6.25 (t, J= 5.9 Hz, 1H), 5.34 (s, 2H), 5.30-5.23 (m, 2H), 5.15 (d, *J* = 19.2 Hz, 1H), 4.14-4.02 (m, 2H), 3.05 (q, *J* = 6.2 Hz, 3H), 2.28 (s, 3H), 2.04 (tt, J = 6.9 Hz, 1H) 2.04 (tt, *J* = 12.0, 6.5 Hz, 2H), 1.84-1.69 (m, 2H), 0.77 (t, *J*= 7.3 Hz, 3H).

**Example 10**

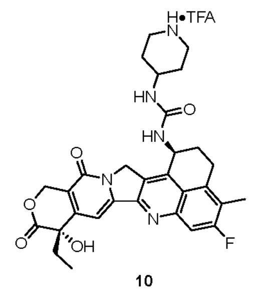

10

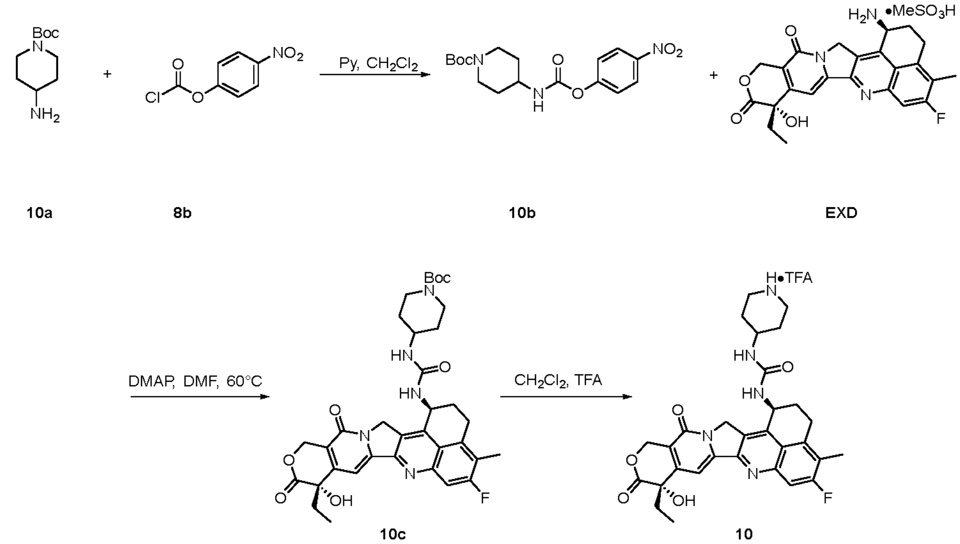


10a 8b 10b EXD

10c 10

Synthesis of compound 10b:

Compound 10a (100 mg, 0.50 mmol, 1.0 eq) was dissolved in dichloromethane (2 mL). Pyridine (44 μL, 0.55 mmol, 1.1 eq) and compound 8b (111 mg, 0.55 mmol, 1.1 eq) were added in an ice bath and stirred for 2 h at room temperature. Water was added, extracted with dichloromethane, dried and concentrated and then purified by column chromatography (33% EA in PE) to obtain a yellow solid (108 mg, 59%).

MS (ESI): *m/z* found $[M-99]^+$ = 266.2

Synthesis of compound 10c:

EXD (20 mg, 38 μmol, 1 eq) was dissolved in DMF (0.8 mL), and DMAP (9.2 mg, 75 μmol, 2.0 eq) was added. After the reaction solution was clarified, compound 10a (14 mg, 38 μmol, 1.0 eq) was added and stirred at 60°C for 3 hours. The reaction solution was quenched with water, extracted with ethyl acetate, dried and concentrated and then purified by column chromatography (6% MeOH in $CH_2Cl_2$) to obtain a white solid (16 mg, 67%), MS (ESI): *m/z* found $[M-55]^+$ =606.3.

Synthesis of compound 10:

Compound 10c (15 mg, 23 μmol) was dissolved in dichloromethane (2 mL), and trifluoroacetic acid (0.5 mL) was added, and stirred for 3 h at room temperature. Concentrated and then purified by reversed-phase column chromatography (33% $CH_3CN$ in $H_2O$, 0.1% TFA) to obtain a yellow solid (9.1 mg, 59%) with MS (ESI): *m/z* found $[M+1]^+$ =562.2.

$^1H$ NMR (400 MHz, DMSO-$d_6$ ): δ 8.43 (s, 1H), 8.23 (s, 1H), 7.73 (d, *J* = 10.9 Hz, 1H), 7.26 (s, 1H), 6.54 (d, *J* = 8.9 Hz, 1H), 6.49 (s, 1H), 6.26 (d,*J* = 7.6 Hz, 1H), 5.37 (s, 2H), 5.30 (d, J = 19.0 Hz, 1H), 3.76-3.68 (m, 1H), 5.12 (d, J = 19.2 Hz, 1H), 3.76-3.68 (m, 19.2 Hz, 1H). 2H), 5.30 (d, *J* = 19.0 Hz, 1H), 5.12 (d, *J* = 19.2 Hz, 1H), 3.76-3.68 (m, 1H), 3.26-3.09 (m, 4H), 3.02-2.91 (m, 2H), 2.33 (s, 3H), 2.18-2.15 (m, 3H), 2.18-2.15 (m, 3H), 2.33 (s, 3H), 2.33 (s, 3H), 2.33 (s, 3H) 2.18-2.15 (m, 1H), 2.07 (dd, *J* = 9.1, 4.4 Hz, 1H), 2.01-1.90 (m, 2H), 1.81 (dq, *J* = 14.1, 7.0 Hz, 2H), 1.59-1.46 (m 2H), 0.83 (t, *J* = 7.3 Hz, 3H).

## Example 11

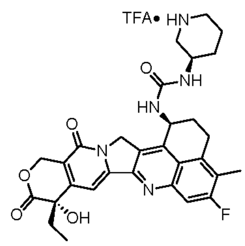

11

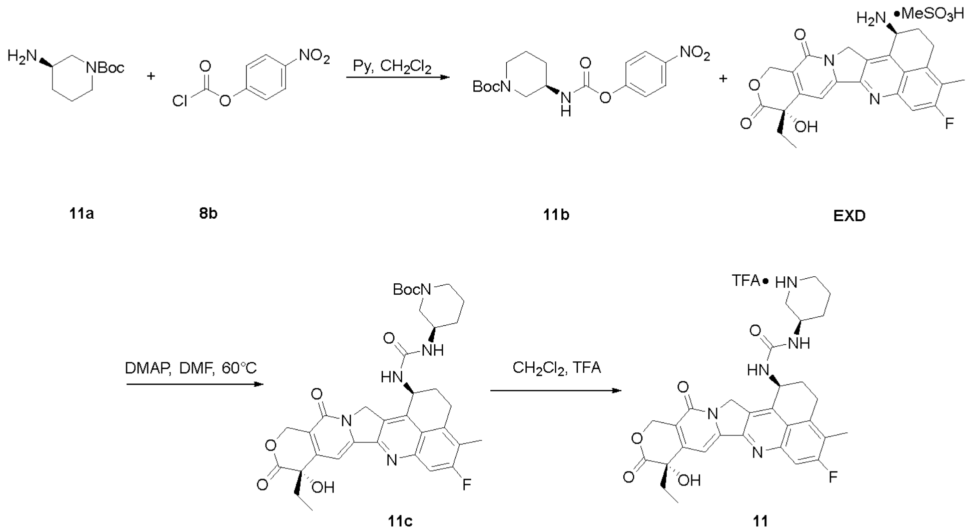


11a 8b 11b EXD

11c 11

Synthesis of compound 11b:

Compound 11a (100mg, 0.5mmol, 1.0eq) was dissolved in dichloromethane (2 mL). Pyridine (44 μL, 0.55mmol, 1.1eq) and compound 8b (111mg, 0.55mmol, 1.1eq) were added to the mixture in an ice bath and stirred for 2 h at room temperature. Water was added, extracted with dichloromethane, dried and concentrated and then purified by column chromatography (33% EA in PE) to obtain a yellow solid (110 mg, 60%). MS (ESI): *m/z* found $[M-99]^+$ = 266.2

Synthesis of compound 11c:

EXD (20 mg, 38 μmol, 1 eq) was dissolved in DMF (0.8 mL), and DMAP (9.2 mg, 75 μmol, 2.0 eq) was added. After the reaction solution was clarified, compound 11b (14 mg, 38 μmol, 1.0 eq) was added and stirred at 60°C for 3 h. The reaction solution was quenched with water, extracted with ethyl acetate, dried and concentrated and purified by column chromatography (6% MeOH in $CH_2Cl_2$) to obtain a white solid (20 mg, 80%), MS (ESI): *m/z* found $[M-55]^+$ =606.3.

Synthesis of compound 11:

Compound 11c (20 mg, 30 μmol) was dissolved in dichloromethane (2 mL), and trifluoroacetic acid (0.5 mL) was added and stirred for 5 h at room temperature. Concentrated and then purified by reversed-phase column chromatography (33% $CH_3CN$ in $H_2O$, 0.1% TFA) to obtain a yellow solid (12 mg, 59%) with MS (ESI): *m/z* found $[M+1]^+$ =562.2.

$^1H$ NMR (400 MHz, DMSO-$d_6$ ): $\delta$ 8.55 (s, 1H), 8.46 (d, $J$ = 9.8 Hz, 1H), 7.69 (d, $J$ = 10.9 Hz, 1H), 7.23 (s, 1H), 6.68 (d, $J$ = 8.9 Hz, 1H), 6.46 (s, 1H), 6.16 (d, $J$ = 7.5 Hz, 1H), 5.33 (s, 2H), 5.31-5.21 (m, 2H), 5.09 (d, $J$ = 19.0 Hz, 1H), 3.84-3.75 (m, 1H), 3.16-3.01 (m, 4H), 2.77 -2.61 (m, 2H), 2.30 (s, 3H), 2.16-2.09 (m, 1H), 2.06-1.97 (m, 1H), 1.85-1.70 (m, 4H), 1.62 -1.51 (m, 1H), 1.42-1.31 (m, 1H), 0.78 (t, $J$ = 7.3 Hz, 3H).

## Example 12

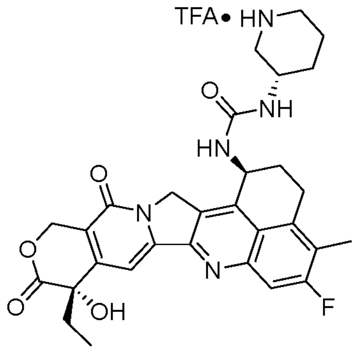

12

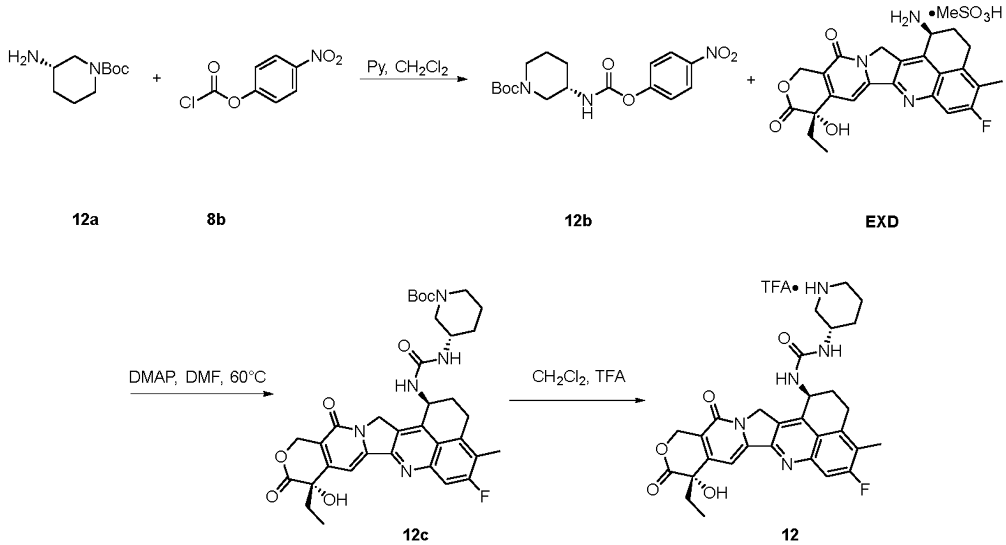


12a 8b 12b EXD

12c 12

Synthesis of compound 12b:

Compound 12a (100mg, 0.5mmol, 1.0eq) was dissolved in dichloromethane (2mL). Pyridine (44 μL, 0.55mmol, 1.1eq) and compound 8b (111 mg, 0.55 mmol, 1.1 eq) were added under an ice bath and stirred for 2 h at room temperature. Water was added, extracted with dichloromethane, dried and concentrated and then purified by column chromatography (33% EA in PE) to obtain a yellow solid (115 mg, 63%). MS (ESI): *m/z* found $[M-99]^+$ = 266.2

Synthesis of compound 12c:

EXD (20 mg, 38 μmol, 1 eq) was dissolved in DMF (0.8 mL), and DMAP (9.2 mg, 75 μmol, 2.0 eq) was added. After the reaction solution was clarified, compound 12b (14 mg, 38 μmol, 1.0 eq) was added and stirred at 60°C for 3h. The reaction solution was quenched with water, extracted with ethyl acetate, dried and concentrated and purified by column chromatography (6% MeOH in $CH_2Cl_2$) to obtain a white solid (15 mg, 64%), MS (ESI): *m/z* found $[M-55]^+$ =606.3.

Synthesis of compound 12:

Compound 12c (15 mg, 23 μmol) was dissolved in tetrahydrofuran (0.5 mL), and added to dissolve in dichloromethane (2 mL). Trifluoroacetic acid (0.5 mL) was added and stirred at room temperature for 5 hours. Concentrated and then purified by reversed-phase column chromatography (33% $CH_3CN$ in $H_2O$, 0.1% TFA) to obtain a yellow solid (10 mg, 65%), MS (ESI): *m/z* found $[M+1]^+$ =562.2.

$^1H$ NMR (400 MHz, DMSO-$d_6$): $\delta$ 8.58 (s, 1H), 8.49 (s, 1H), 7.77 (d, *J* = 10.9 Hz, 1H), 7.28 (s, 1H), 6.72 (d, *J* = 8.8 Hz, 1H), 6.51 (s, 1H), 6.09 (d, *J* = 7.4 Hz, 1H), 5.39 (s 2H), 5.36-5.20 (m, 2H), 5.20 (d, *J* = 19.2 Hz, 1H), 3.89-3.74 (m, 1H), 3.25-3.10 (m, 4H), 2.80-2.62 (m, 2H), 2.37 (s, 3H), 2.20-2.06 (m, 2H), 1.90-1.78 (m, 4H), 1.67-1.56 (m, 1H), 1.44-1.36 (m, 1H), 0.84 (t, J, 1H), 1.44-1.36 (m, 1H), 1.44-1.36 (m, 1H), 0.84 (t, J, 1H) 0.84 (t, *J* = 7.3 Hz, 3H).

## Example 13

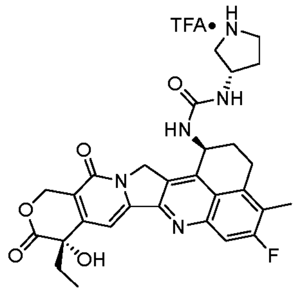

13

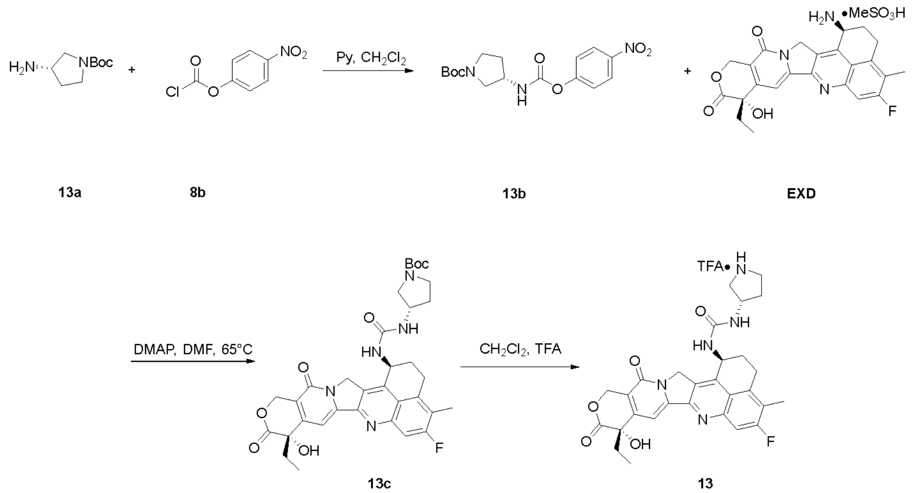


Synthesis of compound 13b:

Compound 13a (100 mg, 0.54 mmol, 1.0 eq) was dissolved in dichloromethane (2 mL). Pyridine (48μL, 0.59 mmol, 1.1 eq) and compound 8b (119 mg, 0.59 mmol, 1.1 eq) were added in an ice bath and stirred for 2h at room temperature. Water was added, extracted with dichloromethane, dried and concentrated and then purified by column chromatography (30% EA in PE) to obtain a yellow solid (120 mg, 64%). MS (ESI): *m*/*z* found $[M-55]^+$ = 296.2

Synthesis of compound 13c:

EXD (30 mg, 56 μmol, 1.0 eq) was dissolved in DMF (1.0 mL) and DMAP (14 mg, 0.11 mmol, 2.0 eq) was added. After the reaction solution was clarified, compound 13b (20 mg, 56 μmol, 1.0 eq) was added and stirred at 65°C for 6 hours. The reaction solution was quenched with water, extracted with ethyl acetate, dried and concentrated and purified by column chromatography (10% MeOH in $CH_2Cl_2$) to obtain a yellow solid (25.6 mg, 70%), MS (ESI): *m*/*z* found $[M-55]^+$ =592.3.

Synthesis of compound 13:

Compound 13c (25 mg, 39 μmol) was dissolved in dichloromethane (2 mL). Trifluoroacetic acid (0.5 mL) was added, and stirred for 5 h at room temperature. Concentrated and purified by reversed-phase column chromatography (30% $CH_3CN$ in $H_2O$, 0.1% TFA) to obtain a yellow solid (15 mg, 60%), MS (ESI): *m*/*z* found $[M+1]^+$ = 548.2.

$^1H$ NMR (400 MHz, DMSO-$d_6$): δ 8.90 (s, 1H), 8.81 (s, 1H), 7.69 (d, *J* = 10.9 Hz, 1H), 7.23 (s, 1H), 6.91 (d, J= 8.9 Hz, 1H), 6.55 (d, *J* = 6.4 Hz, 1H), 6.46 (s, 1H), 5.34 (s 2H), 5.31-5.23 (m, 2H), 5.10 (d, *J* = 19.1 Hz, 1H), 4.21 (m, 1H), 3.25-3.20 (m, 1H), 3.18-3.03 (m, 4H), 3.18 -3.03 (m, 4H), 3.03-2.93 (m, 1H), 2.30 (s, 3H), 2.14-2.01 (m, 3H), 1.83-1.72 (m, 3H), 0.79 (t, *J* = 7.3 Hz, 3H).

**Example 14**

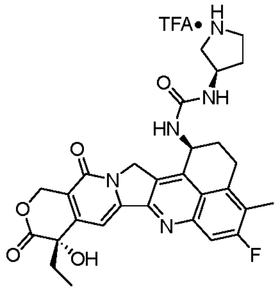

**14**

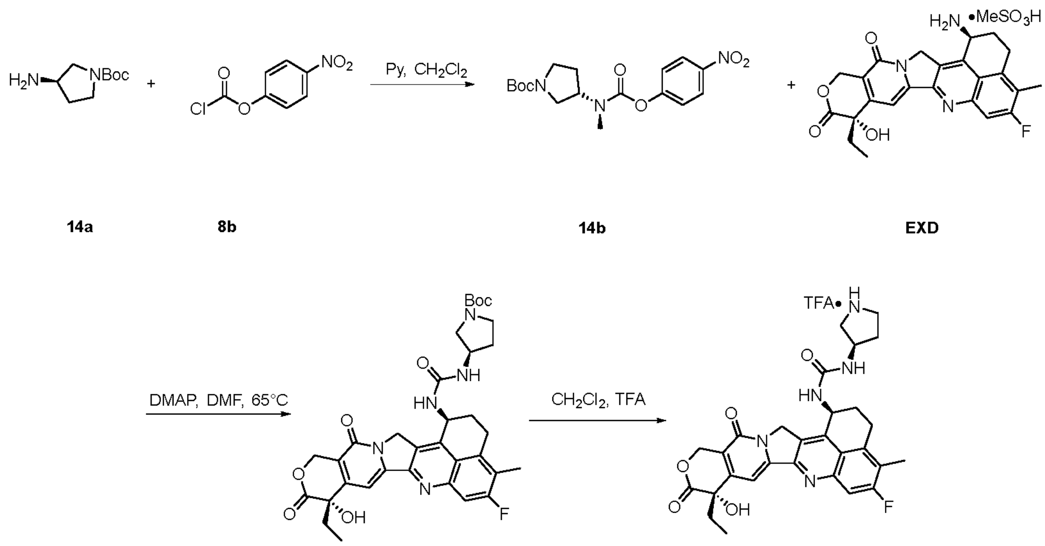


**14a** **8b** **14b** **EXD**

**14c** **14**

Synthesis of compound 14b:

Compound 14a (100 mg, 0.54 mmol, 1.0 eq) was dissolved in dichloromethane (2 mL). Pyridine (48 μL, 0.59 mmol, 1.1 eq) and compound 8b (119 mg, 0.59 mmol, 1.1 eq) were added in an ice bath and stirred for 2h at room temperature. Water was added, extracted with dichloromethane, dried and concentrated and then purified by column chromatography (30% EA in PE) to obtain a yellow solid (107 mg, 57%). MS (ESI): *m/z* found $[M-55]^+$ = 296.2

Synthesis of compound 14c:

EXD (30 mg, 56 μmol, 1.0 eq) was dissolved in DMF (1.0 mL) and DMAP (14 mg, 0.11 mmol, 2.0 eq) was added. After the reaction solution was clarified, compound 14b (20 mg, 56 μmol, 1.0 eq) was added and stirred at 65°C for 6 hours. The reaction solution was quenched with water, extracted with ethyl acetate, dried and concentrated and then purified by column chromatography (6% MeOH in $CH_2Cl_2$) to obtain a yellow solid (25 mg, 69%), MS (ESI): *m/z* found $[M-55]^+$ =592.3.

Synthesis of compound 14:

Compound 14c (25 mg, 39 μmol) was dissolved in dichloromethane (2 mL). trifluoroacetic acid (0.5 mL) was added, and stirred for 3 h at room temperature. Concentrated and then purified by reversed-phase column chromatography (30% $CH_3CN$ in $H_2O$, 0.1% TFA) to obtain a yellow solid (14 mg, 56%), MS (ESI): *m/z* found $[M+1]^+$ = 548.2.

$^1H$ NMR (400 MHz, DMSO-$d_6$ ): δ 8.76 (s, 1H), 8.69 (s, 1H), 7.72 (d, *J* = 10.9 Hz, 1H), 7.27 (s, 1H), 6.82 (d, *J* = 9.0 Hz, 1H), 6.50 (s, 1H), 6.44 (d, *J* = 6.2 Hz, 1H), 5.37 (s, 2H), 5.35-5.24 (m, 2H), 5.11 (d, J = 19.0 Hz, 1H), 4.24 (h, J = 6.2 Hz, 1H), 4.24 (h, J = 6.2 Hz, 1H) 2H), 5.35-5.24 (m, 2H), 5.11 (d, *J* = 19.0 Hz, 1H), 4.24 (h, *J* = 6.2 Hz, 1H), 3.28-3.22 (m, 2H), 3.14 (ddt, *J* = 19.7, 14.8, 7.2 Hz, 4H), 2.33 (s, 3H) 2.33 (s, 3H), 2.20-2.09 (m, 2H), 2.06 (tdt, *J* = 8.1, 3.8 Hz, 1H), 1.89-1.73

(m, 3H), 0.82 (t, *J* = 7.3 Hz, 3H).

**Example 15**

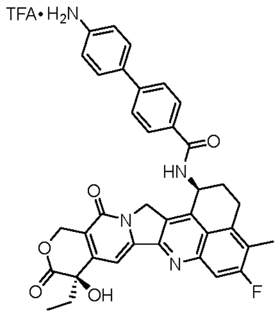

**15**

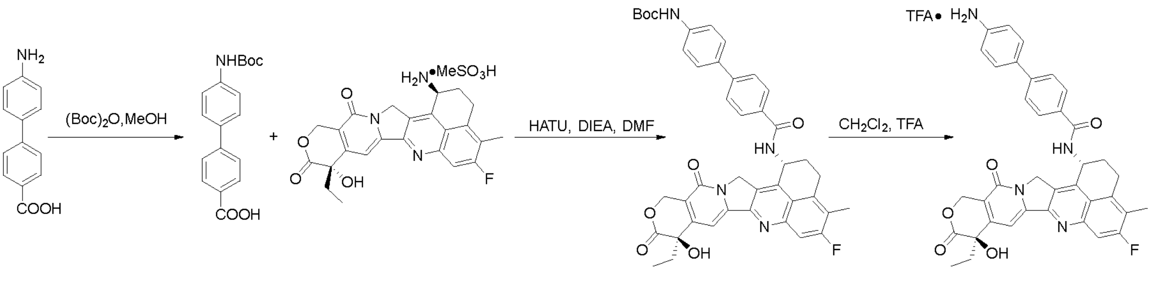


**15a** **15b** **EXD** **15c** **15**

Synthesis of compound 15b:

Compound 15a (50 mg, 0.23 mmol, 1.0 eq) was dissolved in methanol (1 mL). $(Boc)_2O$ (0.5 mL) was added and stirred for 6 h at room temperature. The reaction solution was concentrated and then purified by column chromatography (40% EA in PE) to obtain a white solid (65 mg, 88%). MS (ESI): *m*/*z* found $[M-1]^-$= 312.1

Synthesis of compound 15c:

EXD (25 mg, 47 μmol, 1 eq) and compound 15b (15 mg, 47 μ mol, 1 eq) were dissolved in DMF (1 mL). HATU (26.8 mg, 70 μ mol, 1.5 eq) and DIEA (19 μ L, 118 μmol, 2.5 eq) were added, and stirred for 2 h at room temperature. The reaction solution was directly purified by reversed-phase column chromatography (60% $CH_3CN$ in $H_2O$, 0.05% HCOOH) to obtain a white solid (29 mg, 85%). MS (ESI): *m*/*z* found $[M-55]^+$ = 675.3

Synthesis of compound 15:

Compound 15c (29 mg, 40 μmol) was dissolved in trifluoroacetic acid (0.4 mL) and dichloromethane (2 mL) and stirred for 2.5 h at room temperature. Concentrated and then purified by reversed-phase column chromatography (34% $CH_3CN$ in $H_2O$, 0.1% TFA) to obtain a yellow solid (18 mg, 70 %). MS (ESI): *m*/*z* found $[M+1]^+$ =631.2.

$^1H$ NMR (400 MHz, *DMSO-d*$_6$) : δ 9.01 (d, *J* = 8.4 Hz, 1H), 7.96 (d, *J* = 8.5 Hz, 2H), 7.82 (d, *J* = 11.0 Hz, 1H), 7.66 (d, *J* = 8.5 Hz, 2H), 7.50 (d,*J* = 8.5 Hz, 2H), 7.31 (s, 1H), 7.25-6.96 (m, 2H), 6.77 (d, *J* = 8.2 Hz, 2H), 6.55 (s, 1H), 5.81 (d, *J* = 8.2 Hz, 1H) 7.25-6.96 (m, 2H), 6.77 (d, *J* = 8.2 Hz, 2H), 6.55 (s, 1H), 5.81 (dd, *J*= 13.2, 6.4 Hz, 1H), 5.37 (s, 2H), 5.24 (d, *J* = 18.8 Hz, 1H), 5.13 (d, *J* = 18.9 Hz 1H), 2.42 (s, 3H), 2.34-2.23 (m, 2H), 1.83 (td, *J*= 14.4, 7.0 Hz, 2H), 1.52 (d, *J* = 7.0 Hz, 1H), 0.86 (t, *J* = 7.3 Hz, 3H).

**Example 16**

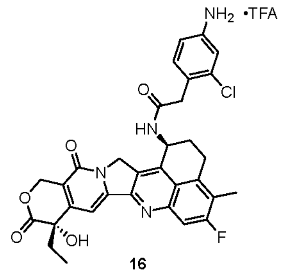

16

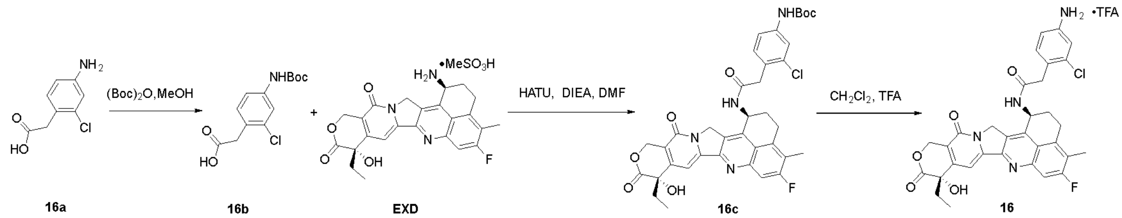


16a 16b EXD 16c 16

Synthesis of compound 16b:

Compound 16a (50 mg, 0.27 mmol, 1.0 eq) was dissolved in methanol (1 mL), and $(Boc)_2O$ (0.5 mL) was added and stirred for 6h at room temperature. The reaction solution was concentrated and then purified by column chromatography (50% EA in PE) to obtain a white solid (70 mg, 91%). MS (ESI): *m/z* found $[M-1]^-$= 284.1

Synthesis of compound 16c:

EXD (25 mg, 47 $\mu$mol, 1 eq) and compound 16b (13.5 mg, 47 $\mu$mol, 1 eq) were dissolved in DMF (1 mL). HATU (26.8 mg, 70 $\mu$mol, 1.5 eq) and DIEA (19 $\mu$L, 118 $\mu$mol, 2.5 eq) were added, and stirred for 2 h at room temperature. The reaction solution was directly purified by reversed-phase column chromatography (65% $CH_3CN$ in $H_2O$, 0.05% HCOOH) to obtain a white solid (28 mg, 85%). MS (ESI): *m/z* found $[M-55]^+$ = 647.2

Synthesis of compound 16:

Compound 16c (28 mg, 40 $\mu$mol) was dissolved in trifluoroacetic acid (0.4 mL) and dichloromethane (2 mL) and stirred for 2.5 h at room temperature. Concentrated and then purified by reversed-phase column chromatography (35% $CH_3CN$ in $H_2O$, 0.1% TFA) to obtain a yellow solid (18.6 mg, 65%).MS (ESI): *m/z* found $[M+1]^+$ =603.2.

$^1H$ NMR (400 MHz, *DMSO-$d_6$*): $\delta$ 8.61 (d, *J* = 8.6 Hz, 1H), 7.81 (d, *J* = 11.0 Hz, 1H), 7.32 (s, 1H), 7.09 (d,J= 8.2 Hz, 1H), 6.70 (d, *J* = 2.0 Hz, 1H), 6.57 (dd, *J* = 8.2, 2.0 Hz, 1H), 5.59-5.52 (m, 1H), 5.44 (s, 2H), 5.28 (d, J = 18.9 Hz, 1H), 5.20 (d, 2H), 5.20 (d, J = 18.9 Hz, 1H) 1H), 5.59-5.52 (m, 1H), 5.44 (s, 2H), 5.28 (d, *J* = 18.9 Hz, 1H), 5.20 (d, *J* = 19.0 Hz, 1H), 3.49 (s, 2H), 3.22-3.15 (m, 2H), 2.41 (s 3H), 2.22-2.08 (m, 2H), 1.94-1.82 (m, 2H), 0.88 (t, *J* = 7.3 Hz, 3H).

## Example 17

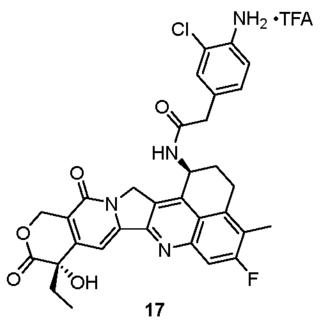

17

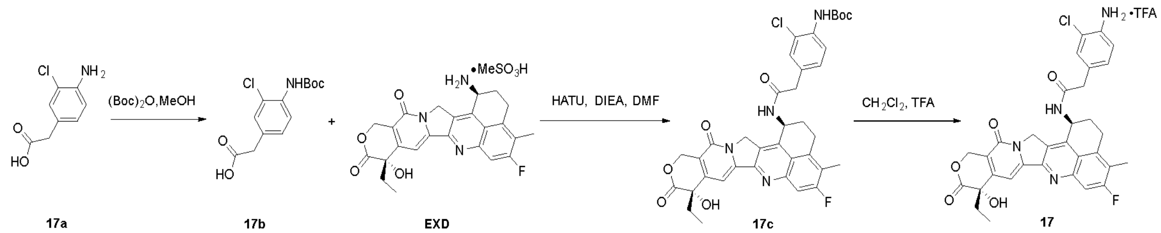


17a 17b EXD 17c 17

Synthesis of compound 17b:

Compound 17a (50 mg, 0.27 mmol, 1.0 eq) was dissolved in methanol (1 mL), and $(Boc)_2O$ (0.5 mL) was added and stirred for 6 h at room temperature. The reaction solution was concentrated and then purified by column chromatography (40% EA in PE) to obtain a white solid (68 mg, 88%). MS (ESI): *m/z* found $[M-1]^-$= 284.1.

Synthesis of compound 17c:

EXD (25 mg, 47 μ mol, 1 eq) and compound 17b (13.5 mg, 47 μ mol, 1 eq) were dissolved in DMF (1 mL), and HATU (26.8 mg, 70 μ mol, 1.5 eq) and DIEA (19 μL, 118 μ mol, 2.5 eq) were added, and stirred for 2 h at room temperature. The reaction solution was directly purified by reversed-phase column chromatography (65% $CH_3CN$ in $H_2O$, 0.05% HCOOH) to obtain a white solid (23 mg, 70%). MS (ESI): *m/z* found $[M-55]^+$ = 647.2

Synthesis of compound 17:

Compound 17c (23 mg, 33 μmol) was dissolved in trifluoroacetic acid (0.4 mL) and dichloromethane (2 mL) and stirred for 2.5 h at room temperature. Concentrated and then purified by reversed-phase column chromatography (35% $CH_3CN$ in $H_2O$, 0.1% TFA) to obtain a yellow solid (15.5 mg, 66%). MS (ESI): *m/z* found $[M+1]^+$ =603.2.

$^1H$ NMR (400 MHz, *DMSO-$d_6$*): $\delta$ 8.58 (d, *J* = 8.6 Hz, 1H), 7.81 (d, *J* = 11.0 Hz, 1H), 7.31 (s, 1H), 7.05 (d, *J* = 1.8 Hz, 1H), 6.93 (dd, *J* = 8.2, 1.9 Hz, 1H), 6.71 (d, *J* = 8.2 Hz, 1H), 6.53 (s, 1H), 5.55-5.49 (m, 1H), 5.44 (s, 2H), 5.25 (d, J = 18.9 Hz 1H), 6.53 (s, 1H), 5.55-5.49 (m, 1H), 5.44 (s, 2H), 5.25 (d, *J* = 18.9 Hz, 1H), 5.05 (d, *J* = 19.0 Hz, 1H), 3.31 (s, 2H), 3.17 (s, 2H), 2.41 (s, 3H). 2.13 (ddd, *J* = 16.6, 9.5, 5.7 Hz, 2H), 1.93-1.81 (m, 2H), 0.88 (t, *J* = 7.3 Hz, 3H).

## Example 18

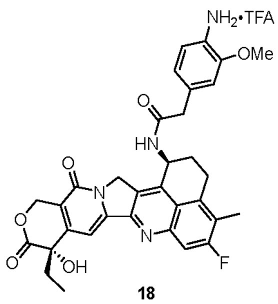

18

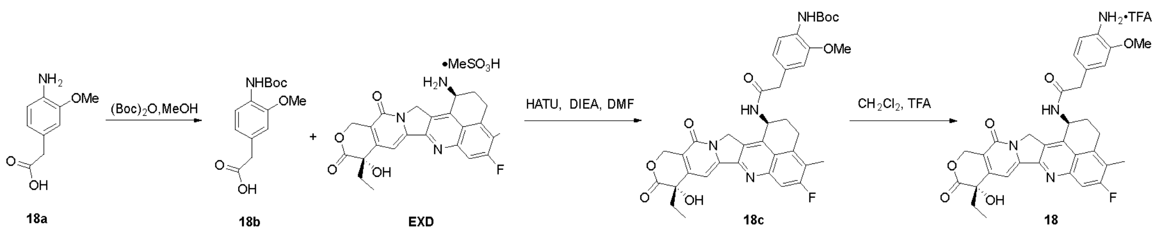


18a 18b EXD 18c 18

Synthesis of compound 18b:

Compound 18a (50 mg, 0.28 mmol, 1.0 eq) was dissolved in methanol (1 mL), and $(Boc)_2O$ (0.5 mL) was added

and stirred for 6 h at room temperature. The reaction solution was concentrated and then purified by column chromatography (25% EA in PE) to obtain a white solid (65 mg, 84%). MS (ESI): *m/z* found $[M-1]^-$= 280.1

Synthesis of compound 18c:

EXD (25 mg, 47 μmol, 1 eq) and compound 18b (13.2 mg, 47 μmol, 1 eq) were dissolved in DMF (1 mL). HATU (26.8 mg, 70 μ mol, 1.5 eq) and DIEA (19 μL, 118 μmol, 2.5 eq) were added and stirred for 2 h at room temperature. The reaction solution was directly purified by reversed-phase column chromatography (64% $CH_3CN$ in $H_2O$, 0.05% HCOOH) to obtain a white solid (21 mg, 65%). MS (ESI): *m/z* found $[M-55]^+$ = 643.4

Synthesis of compound 18:

Compound 18c (21 mg, 30 μmol) was dissolved in trifluoroacetic acid (0.4 mL) and dichloromethane (2 mL) and stirred for 2.5h at room temperature. Concentrated and then purified by reversed-phase column chromatography (37% $CH_3CN$ in $H_2O$, 0.1% TFA) to obtain a yellow solid (13.2 mg, 62%). MS (ESI): *m/z* found $[M+1]^+$ =598.3.

<u>Example 19</u>

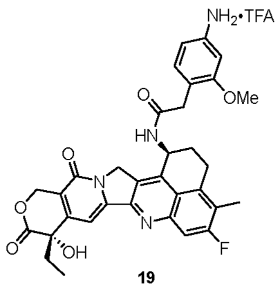

**19**

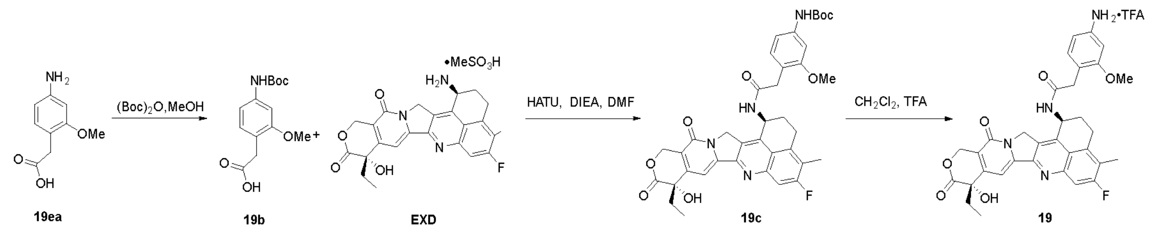


Synthesis of compound 19b:

Compound 19a (50 mg, 0.28 mmol, 1.0 eq) was dissolved in methanol (1 mL). $(Boc)_2O$ (0.5 mL) was added and stirred for 6 h at room temperature. The reaction solution was concentrated and then purified by column chromatography (25% EA in PE) to obtain a white solid (70 mg, 90%). MS (ESI): *m/z* found $[M-1]^-$= 280.1

Synthesis of compound 19c:

EXD (25 mg, 47 μmol, 1 eq) and compound 19b (13.2 mg, 47 μmol, 1 eq) were dissolved in DMF (1 mL). HATU (26.8 mg, 70 μmol, 1.5 eq) and DIEA (19 μL, 118 μmol, 2.5 eq) were added and stirred for 2 h at room temperature. The reaction solution was directly purified by reversed-phase column chromatography (64% $CH_3CN$ in $H_2O$, 0.05% HCOOH) to obtain a white solid (20 mg, 61%). MS (ESI): *m/z* found $[M-55]^+$ = 643.4

Synthesis of compound 19:

Compound 19c (20 mg, 29 μmol) was dissolved in trifluoroacetic acid (0.4 mL) and dichloromethane (2 mL) and stirred for 2.5 h at room temperature. Concentrated and then purified by reversed-phase column chromatography (37% $CH_3CN$ in $H_2O$, 0.1% TFA) to obtain a yellow solid (10.8 mg, 53%). MS (ESI): *m/z* found $[M+1]^+$ =598.3.
$^1H$ NMR (400 MHz, *DMSO-d$_6$*): $\delta$ 8.50 (d, *J* = 8.6 Hz, 1H), 7.81 (d, *J* = 11.0 Hz, 1H), 7.32 (s, 1H), 7.06 (d, *J* = 8.0 Hz,

1H), 6.53 (s, 1H), 6.48 (d, *J* = 7.8 Hz, 1H), 5.59 -5.49 (m, 1H), 5.44 (s, 2H), 5.22 (dd, *J* = 34.2, 19.0 Hz, 2H), 3.65 (s, 3H), 3.38 (s, 2H), 3.22-3.15 (m, 2H), 2.41 (s, 3H), 2.22 -2.08 (m, 2H), 1.88 (ddq, *J* = 28.9, 14.3, 7.2 Hz, 2H), 0.88 (t, *J* = 7.3 Hz, 3H).

**Example 20**

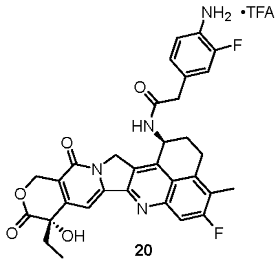

20

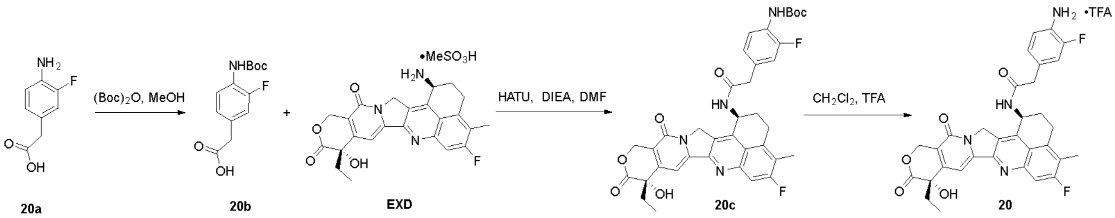


20a 20b EXD 20c 20

Synthesis of compound 20b:

Compound 20a (50 mg, 0.3 mmol, 1.0 eq) was dissolved in methanol (1 mL) and tetrahydrofuran (1 mL). $(Boc)_2O$ (82 μL, 0.35 mmol, 1.2 eq) and DIEA (60 μL, 0.35 mmol, 1.2 eq) were added, and stirred for 12 h at room temperature. The reaction solution was diluted with water, extracted with ethyl acetate, dried and concentrated and then purified by column chromatography (20% EA in PE) to obtain a white solid (70 mg, 88%). MS (ESI): *m/z* found $[M-1]^-$= 268.1

Synthesis of compound 20c:

EXD (25 mg, 47 μmol, 1 eq) and compound 20b (12.7 mg, 47 μ mol, 1 eq) were dissolved in DMF (1 mL). HATU (26.8 mg, 70 μmol, 1.5 eq) and DIEA (19 μL, 118 μmol, 2.5 eq) were added, and stirred for 2 h at room temperature. The reaction solution was directly purified by reversed-phase column chromatography (60% $CH_3CN$ in $H_2O$, 0.05% HCOOH) to obtain a white solid (20 mg, 67%). MS (ESI): *m/z* found $[M-55]^+$ = 631.3

Synthesis of compound 20:

Compound 20c (20 mg, 29 μmol) was dissolved in trifluoroacetic acid (0.4 mL) and dichloromethane (2 mL) and stirred for 2.5h at room temperature. Concentrated and then purified by reversed-phase column chromatography (37% $CH_3CN$ in $H_2O$, 0.1% TFA) to obtain a yellow solid (14.5 mg, 71%). MS (ESI): *m/z* found $[M+1]^+$ =587.3.

**Example 21**

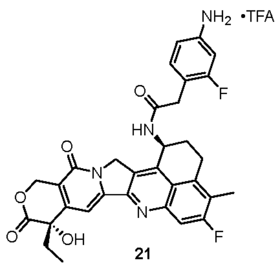

21

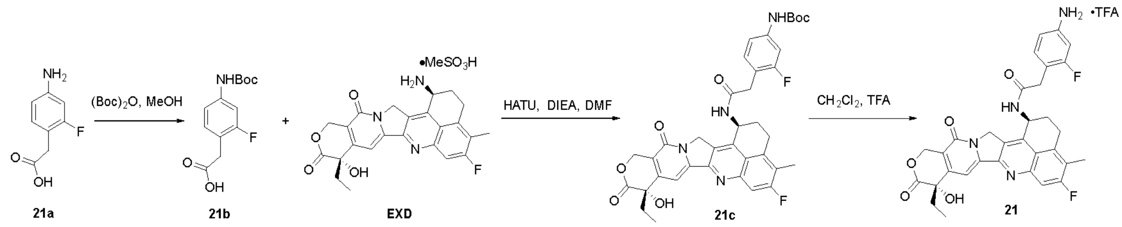


Synthesis of compound 21b:

Compound 21a (50 mg, 0.3 mmol, 1.0 eq) was dissolved in methanol (1 mL) and tetrahydrofuran (1 mL). $(Boc)_2O$ (82 μL, 0.35 mmol, 1.2 eq) and DIEA (60 μL, 0.35 mmol, 1.2 eq) were added, and stirred for 12 h at room temperature. The reaction solution was diluted with water, extracted with ethyl acetate, dried and concentrated, and then purified by column chromatography (20% EA in PE) to obtain a white solid (75 mg, 94%). MS (ESI): *m/z* found $[M-1]^-$= 268.1

Synthesis of compound 21c:

EXD (25 mg, 47 μmol, 1 eq) and compound 21b (12.7 mg, 47 μmol, 1 eq) were dissolved in DMF (1 mL). HATU (26.8 mg, 70 μ mol, 1.5 eq) and DIEA (19 μL, 118 μmol, 2.5 eq) were added, and stirred for 2 h at room temperature. The reaction solution was directly purified by reversed-phase column chromatography (60% $CH_3CN$ in $H_2O$, 0.05% HCOOH) to obtain a white solid (22 mg, 74%). MS (ESI): *m/z* found $[M-55]^+$ = 631.3

Synthesis of compound 21:

Compound 21c (22 mg, 29 μmol) was dissolved in trifluoroacetic acid (0.4 mL) and dichloromethane (2 mL) and stirred for 2.5 h at room temperature. Concentrated and then purified by reversed-phase column chromatography (37% $CH_3CN$ in $H_2O$, 0.1% TFA) to obtain a yellow solid (15.5 mg, 69%). MS (ESI): m/z found [M+1]+ =587.3.

$^1H$ NMR (400 MHz, *DMSO-d*$_6$): $\delta$ 8.62 (d, *J* = 8.5 Hz, 1H), 7.81 (d, *J* = 11.0 Hz, 1H), 7.31 (s, 1H), 7.23-6.98 (m, 1H), 7.03 (t, *J* = 8.6 Hz, 1H), 6.42 (t, *J* = 9.2 Hz, 2H), 5.59-5.50 (m, 1H), 5.44 (s, 2H), 5.27 (d, *J* = 18.9 Hz, 1H), 5.15 (d, *J* = 19.0 Hz, 1H), 3.37 (d, *J* = 5.0 Hz, 2H), 3.23-3.14 (m 2H), 2.41 (s, 3H), 2.22-2.05 (m, 2H), 1.87 (tt, *J* = 14.0, 7.0 Hz, 2H), 0.88 (t, *J* = 7.3 Hz, 3H).

## Example 22

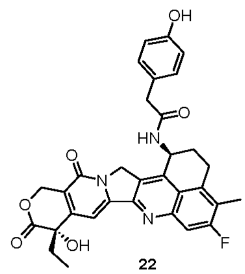

22

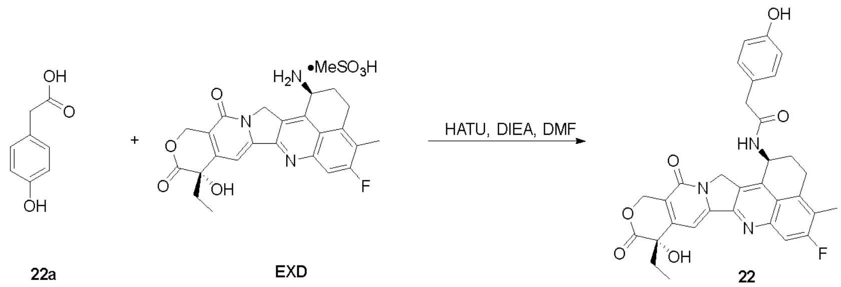


22a EXD 22

Synthesis of compound 22:

EXD (25 mg, 47 μmol, 1.0 eq) and compound 22a (7.2 mg, 47 μmol, 1.0 eq) were dissolved in DMF (1 mL). HATU (26.8 mg, 70 μmol, 1.5 eq) and DIEA (19 μL, 118 μmol, 2.5 eq) were added, and stirred for 2h at room temperature. The reaction solution was directly purified by reversed-phase column chromatography (34% $CH_3CN$ in $H_2O$, 0.05% HCOOH) to obtain a white solid (20 mg, 75%).MS (ESI): *m/z* found $[M+1]^+$ = 570.0

$^1$HNMR (400 MHz, *DMSO-d$_6$*) δ 9.22 (s, 1H), 8.59 (d, *J* = 8.6 Hz, 1H), 7.81 (d, *J* = 11.0 Hz, 1H), 7.31 (s, 1H), 7.07 (d, *J* = 8.5 Hz, 2 H), 6.72-6.64 (m, 2 H), 6.53 (s, 1H), 5.52 (dt, *J* = 9.0, 4.8 Hz, 1H), 5.44 (s, 2 H), 5.25 (d, *J* = 18.9 Hz, 1H), 5.11 (d, *J* = 18.9 Hz, 1H), 3.17 (t, *J* = 6.3 Hz, 2 H), 2.41 (d, *J* = 1.8 Hz, 2 H), 2.16 -2.09 (m, 2 H), 1.95-1.80 (m, 3 H), 0.88 (t, *J* = 7.3 Hz, 3 H).

## Example 23

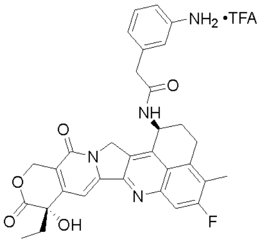

23

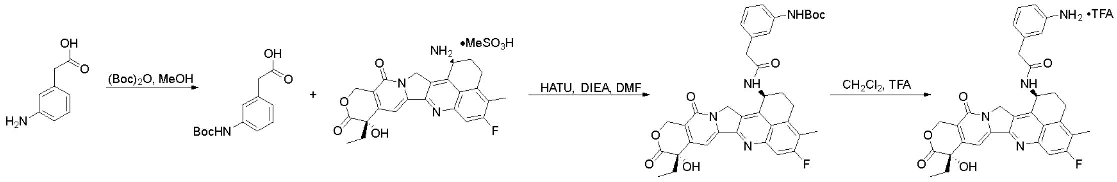


23a 23b EXD 23c 23

Synthesis of compound 23b:

Compound 23a (50 mg, 0.33 mmol, 1.0 eq) was dissolved in methanol (1 mL). $(Boc)_2O$ (0.5 mL) was added and stirred for 6h at room temperature. Then then reaction solution was purified by column chromatography (25% EA in PE) to obtain a white solid (75 mg, 90%). MS (ESI): *m/z* found $[M-1]^-$= 250.2

Synthesis of compound 23c:

EXD (25 mg, 47 μmol, 1 eq) and compound 23b (11.8 mg, 47 μmol, 1 eq) were dissolved in DMF (1 mL). HATU (26.8 mg, 70 μmol, 1.5 eq) and DIEA (19 μL, 118 μmol, 2.5 eq) were added, and stirred for 2 h at room temperature. The reaction solution was directly purified by reversed-phase column chromatography (63% $CH_3CN$ in $H_2O$, 0.05% HCOOH) to obtain a white solid (20 mg, 64%). MS (ESI): *m/z* found $[M-55]^+$ = 613.2

Synthesis of compound 23:

Compound 23c (20 mg, 30 μmol) was dissolved in trifluoroacetic acid (0.4 mL) and dichloromethane (2 mL) and stirred for 2.5 h at room temperature. Concentrated and then purified by reversed-phase column chromatography (35% $CH_3CN$ in $H_2O$, 0.1% TFA) to obtain a yellow solid (14.3 mg, 70%). MS (ESI): *m/z* found $[M+1]^+$ =569.2.

$^1$H NMR (400 MHz, *DMSO-d$_6$*): δ 8.72 (d, *J* = 8.5 Hz, 1H), 7.82 (d, *J* = 10.9 Hz, 1H), 7.33 (d, *J* = 1.9 Hz, 1H), 7.18 (t, *J* = 7.7 Hz, 1H), 6.93 (s, 1H), 6.85 (dd, *J* = 20.0, 6.9 Hz 2H), 6.56 (s, 1H), 5.58-5.50 (m, 1H), 5.45 (d, *J* = 4.1 Hz, 2H), 5.21 (dd, *J* = 16.4, 6.1 Hz, 2H), 3.46 (s, 2H), 3.18 (s, 2H), 2.41 (s, 3H), 2.14 (dd, *J* = 16.0, 11.2 Hz, 2H), 1.93-1.81 (m, 2H), 0.88 (t, *J* = 7.3 Hz, 3H).

**Example 24**

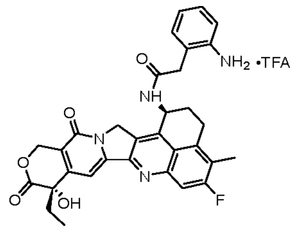

24

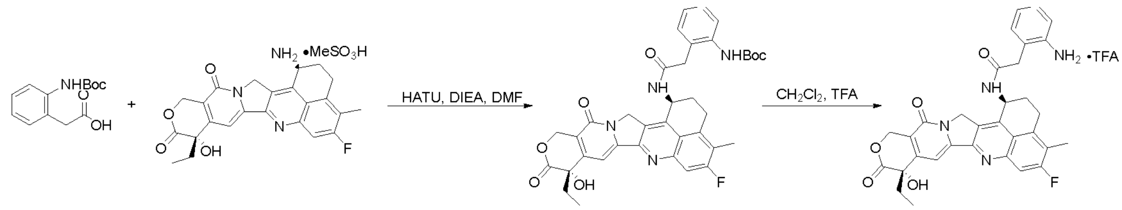


24a EXD 24b 24

Synthesis of compound 24b:

EXD (25 mg, 47 μmol, 1 eq) and compound 24a (11.8 mg, 47 μmol, 1 eq) were dissolved in DMF (1 mL). HATU (26.8 mg, 70 μmol, 1.5 eq) and DIEA (19 μL, 118 μmol, 2.5 eq) were added, and stirred for 2 h at room temperature. The reaction solution was directly purified by reversed-phase column chromatography (63% $CH_3CN$ in $H_2O$, 0.05% HCOOH) to obtain a white solid (22 mg, 70%). MS (ESI): *m*/*z* found $[M-55]^+$ = 613.2

Synthesis of compound 24:

Compound 24b (22 mg, 33 μmol) was dissolved in trifluoroacetic acid (0.4 mL) and dichloromethane (2 mL) and stirred for 2.5 h at room temperature. Concentration was followed by reversed-phase column chromatography purification (35% $CH_3CN$ in $H_2O$, 0.1% TFA) to obtain a yellow solid (14.4 mg, 64%).MS (ESI): *m*/*z* found $[M+1]^+$ =569.2.

$^1$H NMR (400 MHz, *DMSO-d$_6$*): $\delta$ 8.78 (d, *J* = 8.5 Hz, 1H), 7.83 (d, *J* = 11.0 Hz, 1H), 7.32 (s, 1H), 7.10 (t, *J* = 7.7 Hz, 2H), 6.90 (d, *J* = 7.3 Hz, 1H), 6.81 (s, 1H), 6.55 (s, 1H), 5.54 (dt, *J* = 8.5, 4.2 Hz, 1H), 5.43 (s, 2H), 5.30 (d, *J* = 18.9 Hz, 1H), 5.15 (d, *J* = 18.9 Hz, 1H), 3.48 (s, 2H), 3.18 (s, 2H) , 2.41 (s, 3H), 2.22-2.02 (m, 2H), 1.87 (tt, *J* = 14.0, 7.2 Hz, 2H), 0.88 (t, *J* = 7.3 Hz, 3H).

**Example 25**

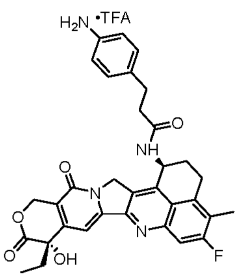

25

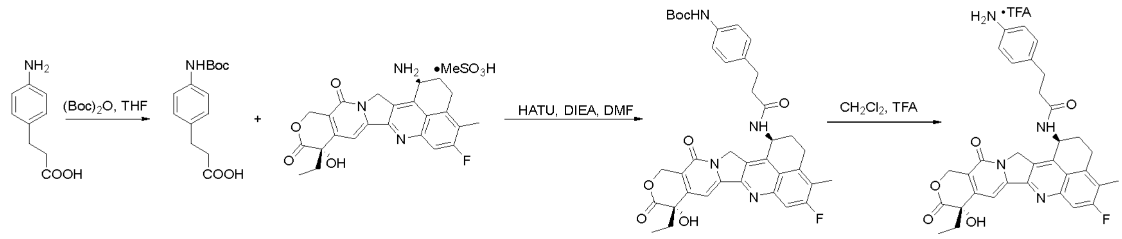


25a 25b EXD 25c 25

Synthesis of compound 25b:

Compound 25a (50 mg, 0.3 mmol, 1.0 eq) was dissolved in tetrahydrofuran (1 mL). $(Boc)_2O$ (83 μL, 0.36 mmol, 1.2 eq) was added, and stirred for 16 h at room temperature. The reaction solution was concentrated and then purified by column chromatography (20% EA in PE) to obtain a white solid (60 mg, 75%). MS (ESI): *m*/*z* found $[M-1]^-$= 264.1

Synthesis of compound 25c:

EXD (25 mg, 47 μmol, 1 eq) and compound 25b (12.5 mg, 47 μmol, 1 eq) were dissolved in DMF (1 mL). HATU (26.8 mg, 70 μmol, 1.5 eq) and DIEA (19 μL, 118 μmol, 2.5 eq) were added, and stirred for 2 h at room temperature. The reaction solution was directly purified by reversed-phase column chromatography (57% $CH_3CN$ in $H_2O$, 0.05% HCOOH) to obtain a white solid (20 mg, 63%). MS (ESI): *m*/*z* found $[M-55]^+$ = 627.3

Synthesis of compound 25:

Compound 25c (20 mg, 29 μmol) was dissolved in trifluoroacetic acid (0.4mL) and dichloromethane (2mL) and stirred for 2.5h at room temperature. Concentrated and then purified by reversed-phase column chromatography (25% $CH_3CN$ in $H_2O$, 0.1% TFA) to obtain a yellow solid (12 mg, 59%). MS (ESI): *m*/*z* found $[M+1]^+$ =583.3.

$^1H$ NMR (400 MHz, *DMSO-d$_6$*) : $\delta$ 8.45 (d, *J* = 8.7 Hz, 1H), 7.80 (d, *J* = 10.9 Hz, 1H), 7.31 (s, 1H), 7.17 (d, *J* = 8.0 Hz, 2 H), 6.96 (d,J= 7.8 Hz, 2 H), 6.54 (s, 1H), 5.54 (dt, J = 9.1, 4.8 Hz, 1H), 5.44 (s, 2 H), 5.23 (d, *J* = 18.9 Hz, 1H), 5.10 (d, *J* = 19.0 Hz, 1H), 3.16-3.07 (m, 2 H), 2.97-2.89 (m, 2 H), 2.89 - 2.82 (m, 2 H), 2.40 (d, J = 1.9 Hz, 3 H), 2.07 (dt, *J* = 13.8, 8.1 Hz, 2 H), 1.92-1.81 (m, 2 H), 1.16 (t, *J* = 7.3 Hz, 2 H), 0.88 (t, *J* = 7.3 Hz, 3 H).

## Example 26

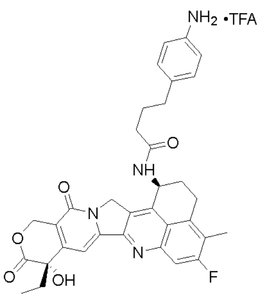

26

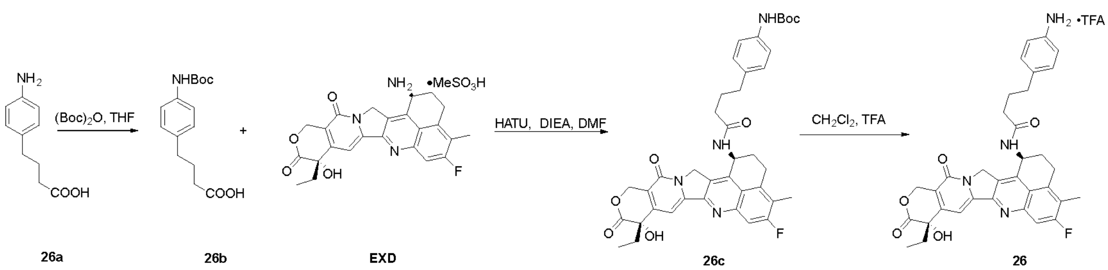


26a 26b EXD 26c 26

Synthesis of compound 26b:

Compound 26a (50 mg, 0.28 mmol, 1.0 eq) was dissolved in tetrahydrofuran (1 mL). $(Boc)_2O$ (77μL, 0.33 mmol, 1.2 eq) was added, and stirred for 18 h at room temperature. The reaction solution was concentrated and then purified by column chromatography (25% EA in PE) to obtain a white solid (65 mg, 83%). MS (ESI): *m/z* found $[M-1]^-$ = 278.1

Synthesis of compound 26c:

EXD (25 mg, 47 μmol, 1 eq) and compound 26b (13.1 mg, 47 μmol, 1 eq) were dissolved in DMF (1 mL). HATU (26.8 mg, 70 μmol, 1.5 eq) and DIEA (19 μL, 118 μmol, 2.5 eq) were added, and stirred for 2 h at room temperature. The reaction solution was directly purified by reversed-phase column chromatography (57% $CH_3CN$ in $H_2O$, 0.05% HCOOH) to obtain a white solid (18 mg, 55%). MS (ESI): *m/z* found $[M-55]^+$ = 641.3

Synthesis of compound 26:

Compound 26c (18 mg, 26 μmol) was dissolved in trifluoroacetic acid (0.4 mL) and dichloromethane (2 mL) and stirred for 2.5 h at room temperature. Concentrated and then purified by reversed-phase column chromatography (25% $CH_3CN$ in $H_2O$, 0.1% TFA) to obtain a yellow solid (12 mg, 65%). MS (ESI): *m/z* found $[M+1]^+$ =597.3.

$^1H$ NMR (400 MHz, *DMSO-d$_6$* ): $\delta$ 8.48 (t, *J* = 7.6 Hz, 1H), 7.81 (d, *J* = 11.0 Hz, 1H), 7.31 (d, *J* = 2.3 Hz, 1H), 7.28 (dd, *J* = 8.3, 3.9 Hz, 2 H), 7.16 (d, *J* = 8.3 Hz, 2 H), 5.58 (dt *J* = 9.5, 5.1 Hz, 1H), 5.42 (s, 2 H), 5.25 (d, *J* = 19.0 Hz, 1H), 5.16 (d, *J* = 18.9 Hz, 1H), 3.17 (s, 2H), 2.60 (t, *J* = 7.7 Hz, 2 H), 2.40 (d, *J* = 1.8 Hz, 3 H), 2.19 (t, *J* = 7.5 Hz , 2 H), 2.13 (dd, *J* = 11.9, 5.9 Hz, 2 H), 1.95-1.78 (m, 4 H), 1.54-1.38 (m, 1H), 1.28-1.23 (m, 1H), 1.07 (dt, *J* = 17.7, 5.7 Hz, 1H), 0.87 (t, *J* = 7.3 Hz, 3 H).

<u>**Example 27**</u>

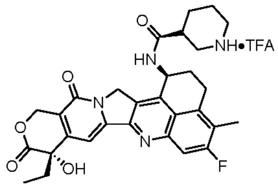

27

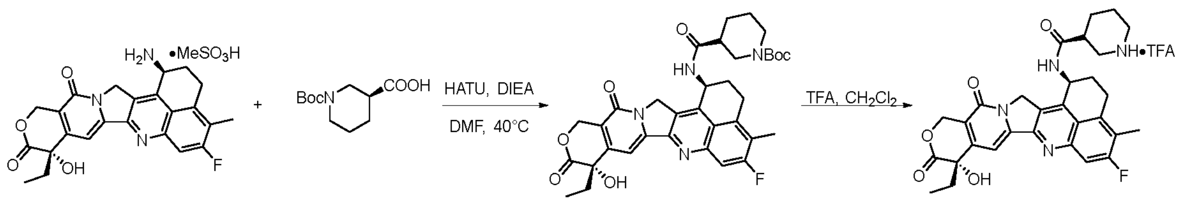


EXD 27a 27 b 27

Synthesis of compound 27b:

EXD (25 mg, 47 μmol, 1.0 eq) and compound 27a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40 °C for 1.5 h. The reaction solution was purified by reverse column chromatography (65% $CH_3CN$ in $H_2O$, 0.05% HCOOH). The reaction solution was directly purified by reversed-phase column chromatography (65% $CH_3CN$ in $H_2O$, 0.05% HCOOH) to obtain a yellow solid (24 mg, 79%). MS (ESI): *m/z* found $[M+1]^+$ = 647.2.

Synthesis of compound 27:

Compound 27b (24 mg, 37 μmol) was dissolved in trifluoroacetic acid (0.2 mL) and dichloromethane (2 mL) and stirred for 2.5 h at room temperature. Concentration was followed by reversed-phase column chromatography purification (35% $CH_3CN$ in $H_2O$, 0.1% TFA) to obtain a yellow solid (16 mg, 65%). MS (ESI): *m/z* found $[M+1]^+$ = 547.2.

$^1$H NMR (400 MHz, *DMSO-$d_6$*): $\delta$ 8.71 (d, *J* = 8.6 Hz, 1H), 8.57 (s, 2H), 7.82 (d, *J* = 10.9 Hz, 1H), 7.32 (s, 1H), 6.56 (s, 1H), 5.56 (dt, *J* = 9.0, 4.7 Hz, 1H), 5.43 (s, 2H), 5.26, 5.16 (ABq, 2H, *J* = 18.8 Hz), 3.35-3.28 (m, 1H), 3.22-3.07 (m, 4H), 2.94 (s, 1H), 2.65 (dq, *J* = 10.0, 5.9, 5.0 Hz, 1H), 2.41 (s 3H), 2.14 (tt, *J* = 8.4, 5.8, 5.4 Hz, 2H), 1.98-1.78 (m, 4H), 1.64 (td, *J* = 17.9, 15.7, 8.4 Hz, 2H), 0.87 (t, *J* = 7.3 Hz, 3H).

**Example 28**

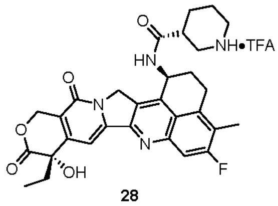

28

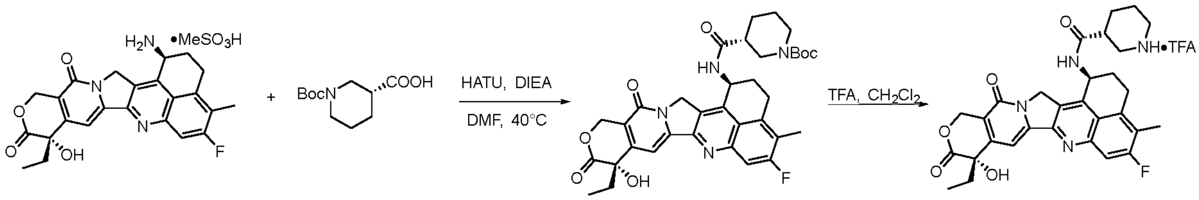


EXD 28a 28b 28

Synthesis of compound 28b:

EXD (25 mg, 47 μmol, 1.0 eq) and compound 28a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40°C for 1.5 h. The reaction solution was directly purified by reversed-phase column chromatography (62% $CH_3CN$ in $H_2O$, 0.05% HCOOH) to obtain a yellow solid (26 mg, 86%). MS (ESI): *m/z* found $[M-55]^+$ = 591.2.

Synthesis of compound 28:

Compound 28b (26 mg, 40 μmol) was dissolved in trifluoroacetic acid (0.2 mL) and dichloromethane (2 mL) and stirred for 2.5 h at room temperature. Concentrated and then purified by reversed-phase column chromatography (33% $CH_3CN$ in $H_2O$, 0.1% TFA) to obtain a yellow solid (18 mg, 73%). MS (ESI): *m/z* found $[M+1]^+$ =547.4.

$^1$H NMR (400 MHz, DMSO-$d_6$): $\delta$ 8.75 (d, *J* = 8.7 Hz, 1H), 8.63-8.46 (m, 2H), 7.81 (d, *J* = 11.0 Hz, 1H), 7.31 (s, 1H), 6.54 (s, 1H), 5.57 (dt, *J* = 9.2, 4.9 Hz. 1H), 5.42 (s, 2H), 5.23 (d, *J* = 18.8 Hz, 1H), 5.06 (d, *J* = 18.8 Hz, 1H), 3.29 (d, *J* = 12.3 Hz, 1H), 3.20-3.04 (m, 4H), 2.92 (q, *J* = 10.7 Hz, 1H), 2.65 (tt, *J* = 10.1, 4.1 Hz, 1H), 2.40 (d, *J* = 1.9 Hz, 3H), 2.15 (t, *J* = 6.0 Hz, 2H), 1.97-1.74 (m, 4H), 1.69-1.50 (m, 2H), 0.88 (t, *J* = 7.3 Hz, 3H) .

**Example 29**

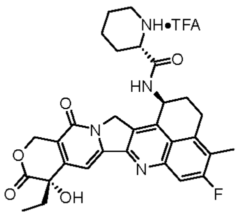

29

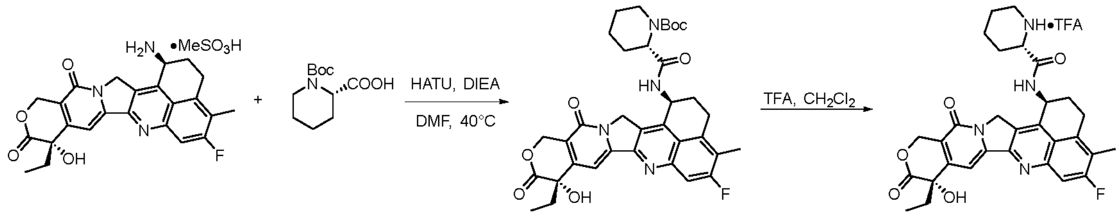


EXD 29a 29b 29

Synthesis of compound 29b:

EXD (25 mg, 47 μmol, 1.0 eq) and compound 29a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40°C for 1.5 h. The reaction solution was directly purified by reversed-phase column chromatography (62% $CH_3CN$ in $H_2O$, 0.05% HCOOH) to obtain a yellow solid (26 mg, 86%). MS (ESI): *m/z* found $[M-55]^+$ = 591.2.

Synthesis of compound 29:

Compound 29b (26 mg, 40 μmol) was dissolved in trifluoroacetic acid (0.2 mL) and dichloromethane (2 mL) and stirred for 2.5 h at room temperature. Concentrated and then purified by reversed-phase column chromatography (31% $CH_3CN$ in $H_2O$, 0.1% TFA) to obtain a yellow solid (22 mg, 83%). MS (ESI): *m/z* found $[M+1]^+$ =547.4.

$^1H$ NMR (400 MHz, *DMSO-d$_6$*): $\delta$ 9.06 (d, *J* = 8.7 Hz, 1H), 9.00 (d, *J* = 10.9 Hz, 1H), 8.88 (q, *J* = 10.7 Hz, 1H), 7.84 (d, *J* = 11.0 Hz, 1H), 7.32 (s, 1H), 6.56 (s, 1H), 5.65 (dt *J* = 8.8, 4.4 Hz, 1H), 5.43 (s, 2H), 5.29 (s, 1H), 5.05 (s, 1H), 3.78-3.71 (m, 1H), 3.30-3.20 (m, 2H), 3.19-3.09 (m 1H), 2.99-2.86 (m, 1H), 2.42 (s, 3H), 2.17 (tq, *J* = 13.8, 8.8, 6.6 Hz, 2H), 2.04 (d, *J* = 13.0 Hz, 1H), 1.96-1.77 (m, 2H), 1.76 -1.65 (m, 2H), 1.57 (qd, *J* = 13.1, 3.6 Hz, 2H), 1.38 (d, *J* = 12.3 Hz, 1H), 0.88 (t, *J* = 7.3 Hz, 3H).

**<u>Example 30</u>**

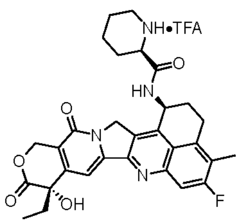

30

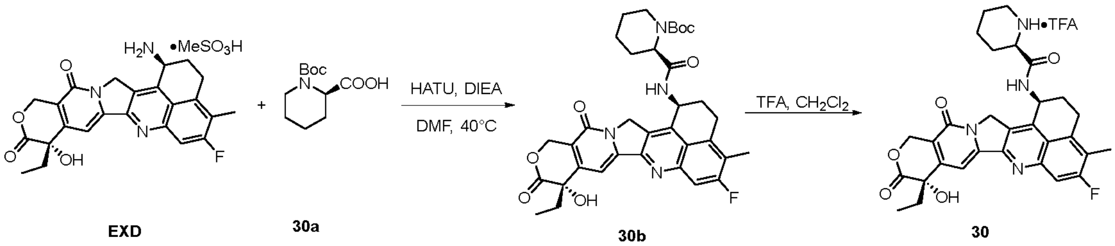


EXD 30a 30b 30

Synthesis of compound 30b:

EXD (25 mg, 47 μmol, 1.0 eq) and compound 30a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40 °C for 1.5h. The reaction solution was directly purified by reversed-phase column chromatography (62% $CH_3CN$ in $H_2O$, 0.05% HCOOH) to obtain a yellow solid (28 mg, 92%). MS(ESI): *m/z* found $[M+1]^+$ = 647.2.

Synthesis of compound 30:

Compound 30a (28 mg, 43 μmol) was dissolved in trifluoroacetic acid (0.2 mL) and dichloromethane (2 mL) and stirred for 2.5h at room temperature. Concentrated and then purified by reversed-phase column chromatography (31% $CH_3CN$ in $H_2O$, 0.1% TFA) to obtain a yellow solid (24 mg, 85%). MS (ESI): *m/z* found $[M+1]^+$ =547.4.

$^1H$ NMR (400 MHz, *DMSO-$d_6$*): $\delta$ 8.98-8.93 (m, 2H), 8.77 (q, *J* = 10.9 Hz, 1H), 7.85 (d, *J* = 10.9 Hz, 1H), 7.33 (s, 1H), 6.57 (s, 1H), 5.65 (dt, *J* = 8.0, 3.5 Hz , 1H), 5.44 (s, 2H), 5.41 (d, *J* = 19.0 Hz, 1H), 5.23 (d, *J* = 19.0 Hz, 1H), 3.68-3.61 (m, 1H), 3.31-3.18 (m, 2H), 3.16- 3.04 (m, 1H), 2.84 (q, *J* = 11.5 Hz, 1H), 2.42 (s, 3H), 2.26-2.18 (m, 1H), 2.11 (td, *J* = 13.2, 6.5 Hz, 2H), 1.92-1.76 (m, 3H), 1.73 -1.52 (m, 3H), 1.43-1.36 (m, 1H), 0.86 (t, *J*= 7.4 Hz, 3H).

## Example 31

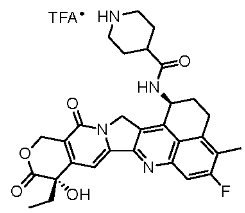

31

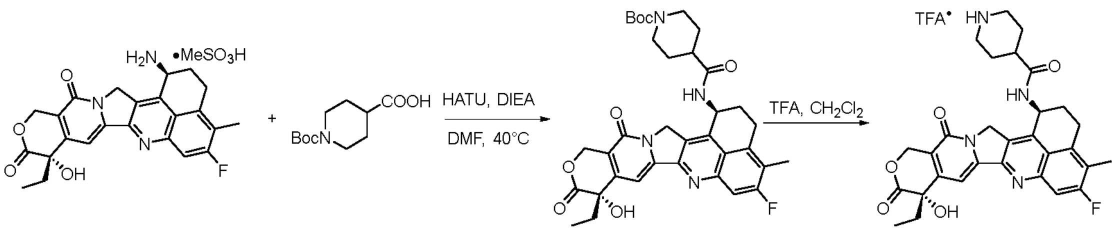


EXD 31a 31b 31

Synthesis of compound 31b:

EXD (25 mg, 47 μmol, 1.0 eq) and compound 30a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40 °C for 1.5 h. The reaction solution was directly purified by reversed-phase column chromatography (58% $CH_3CN$ in $H_2O$, 0.05% HCOOH) to obtain a yellow solid (25 mg, 82%).MS (ESI): *m/z* found $[M-99]^+$ = 547.2.

Synthesis of compound 31:

Compound 31b (25 mg, 39 μmol) was dissolved in trifluoroacetic acid (0.2 mL) and dichloromethane (2 mL) and stirred for 2.5h at room temperature. Concentrated and then purified by reversed-phase column chromatography (30% $CH_3CN$ in $H_2O$, 0.1% TFA) to obtain a yellow solid (20 mg, 66%). MS (ESI): *m/z* found $[M+1]^+$ = 547.4.

$^1H$ NMR (400 MHz, *DMSO-$d_6$*): $\delta$ 8.62-8.55 (m, 2H), 8.31 (d, *J* = 10.5 Hz, 1H), 7.81 (d, *J* = 11.0 Hz, 1H), 7.31 (s, 1H), 6.55 (s, 1H), 5.57 (dt, *J* = 9.3, 5.1 Hz , 1H), 5.42 (s, 2H), 5.22, 5.09 (ABq, *J* = 18.8 Hz, 2H), 3.38-3.28 (m, 2H), 3.17 (t, *J* = 6.3 Hz, 2H), 2.94-2.79 (m, 2H), 2.49 -2.44 (m, 1H), 2.40 (s, 3H), 2.14 (q, *J* = 6.0 Hz, 2H), 1.98-1.76 (m, 6H), 0.87 (t, *J* = 7.3 Hz, 3H).

## Example 32

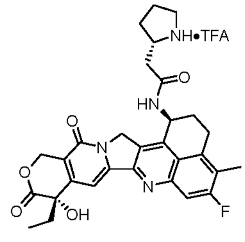

32

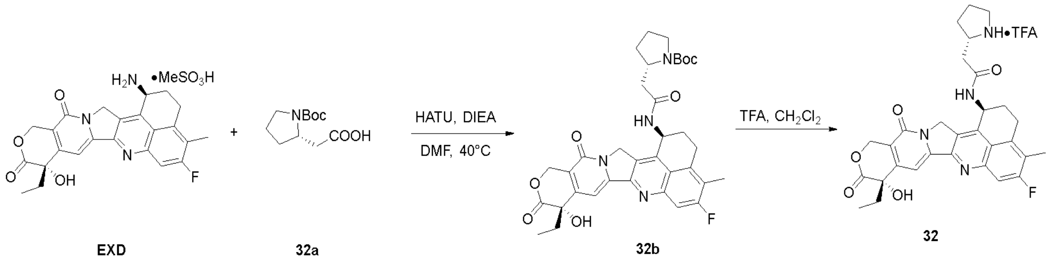


EXD 32a 32b 32

Synthesis of compound 32b:

EXD (25 mg, 47 μmol, 1.0 eq) and compound 30a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40 °C for 1.5 h. The reaction solution was directly purified by reversed-phase column chromatography (58% $CH_3CN$ in $H_2O$, 0.05% HCOOH) to obtain a yellow solid (26 mg, 86%).MS (ESI): *m*/*z* found $[M-99]^+$ = 547.2.

Synthesis of compound 32:

Compound 32b (26 mg, 40 μmol) was dissolved in trifluoroacetic acid (0.2 mL) and dichloromethane (2 mL) and stirred for 2.5 h at room temperature. Concentrated and then purified by reversed-phase column chromatography (30% $CH_3CN$ in $H_2O$, 0.1% TFA) to obtain a yellow solid (19 mg, 71%). MS (ESI): *m*/*z* found $[M+1]^+$ = 547.4.

$^1$H NMR (400 MHz, *DMSO-d$_6$* ): δ 9.09-8.98 (m, 1H), 8.72 (d, *J* = 8.6 Hz, 1H), 8.55-8.47 (m, 1H), 7.83 (d, *J* = 10.9 Hz, 1H), 7.33 (s, 1H), 6.56 (s, 1H), 5.60 (dt, *J* = 8.6, 4.3 Hz, 1H), 5.44 (s, 2H), 5.33 (d, *J* = 8.6, 4.3 Hz, 1H), 5.44 (s, 2H), 5.44 (s, 2H) 6.56 (s, 1H), 5.60 (dt, *J* = 8.6, 4.3 Hz, 1H), 5.44 (s, 2H), 5.33 (d, *J* = 18.9 Hz, 1H), 5.23 (d, *J* = 18.9 Hz, 1H), 3.79-3.71 (m, 1H), 3.21 -3.13 (m, 4H), 2.62 (qd, *J* = 16.1, 6.9 Hz, 2H), 2.42 (s, 3H), 2.25-2.06 (m, 3H), 1.96-1.77 (m, 4H), 1.54 (dq, *J* = 13.0 , 8.6 Hz, 1H), 0.87 (t, *J* = 7.3 Hz, 3H).

## Example 33

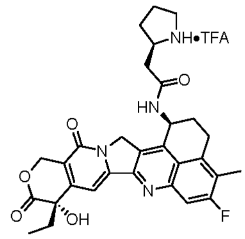

33

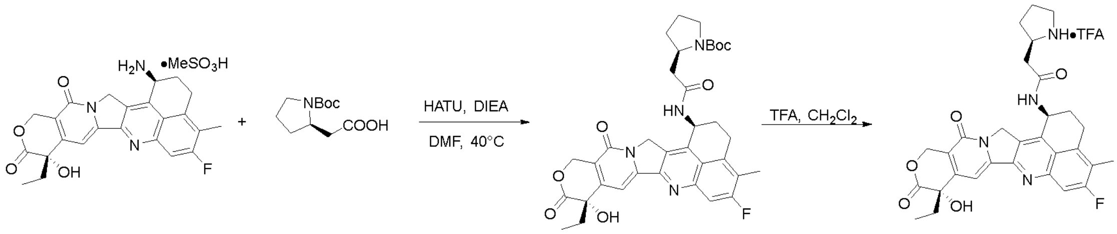


EXD 33a 33b 33

Synthesis of compound 33b:

EXD (25 mg, 47 μmol, 1.0 eq) and compound 30a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40 °C for 1.5h. The reaction solution was directly purified by reversed-phase column chromatography (56% $CH_3CN$ in $H_2O$, 0.05% HCOOH) to obtain a yellow solid (24 mg, 79%). MS (ESI): *m/z* found $[M-99]^+$ = 547.2.

Synthesis of compound 33:

Compound 33b (24 mg, 37 μmol) was dissolved in trifluoroacetic acid (0.2 mL) and dichloromethane (2 mL) and stirred for 2.5 h at room temperature. Concentrated and then purified by reversed-phase column chromatography (28% $CH_3CN$ in $H_2O$, 0.1% TFA) to obtain a yellow solid (19 mg, 78%). MS (ESI): *m/z* found $[M+1]^+$ =547.4.

$^1H$ NMR (400 MHz, *DMSO-d$_6$*): δ 9.05 (s, 1H), 8.73 (d, *J* = 8.7 Hz, 1H), 8.55 (s, 1H), 7.82 (d, *J* = 11.0 Hz, 1H), 7.32 (s, 1H), 6.56 (s, 1H), 5.61 (dt,*J* = 8.9, 4.6 Hz, 1H), 5.43 (s, 2H), 5.35-5.18 (m, 2H), 3.82-3.74 (m, 1H), 3.21-3.14 (m, 4H), 2.64 (d, *J* = 6.9 Hz, 2H), 2.41 (s, 3H), 2.21 -2.05 (m, 3H), 1.95-1.77 (m, 4H), 1.61-1.49 (m, 1H), 0.87 (t, *J*= 7.3 Hz, 3H).

## Example 34

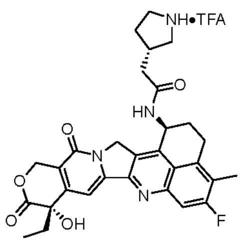

34

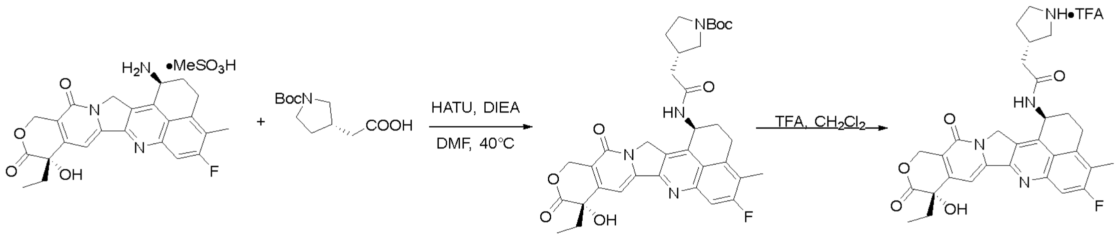


EXD 34a 34b 34

Synthesis of compound 34b:

EXD (25 mg, 47 μmol, 1.0 eq) and compound 30a (11 mg, 47 μmol, 1.0 eq) were dissolved in DMF (1 mL). HATU (21 mg, 56 μmol, 1.2 eq) and DIEA (17 μL, 103 μmol, 2.2 eq) were added, and stirred at 40 °C for 1.5 h. The reaction solution was directly purified by reversed-phase column chromatography (57% $CH_3CN$ in $H_2O$, 0.05% HCOOH) to obtain a yellow solid (26 mg, 86%). MS (ESI): *m/z* found $[M-99]^+$ = 547.2.

Synthesis of compound 34:

Compound 34b (24 mg, 40 μmol) was dissolved in trifluoroacetic acid (0.2 mL) and dichloromethane (2 mL) and stirred for 2.5 h at room temperature. Concentrated and then purified by reversed-phase column chromatography (28% $CH_3CN$ in $H_2O$, 0.1% TFA) to obtain a yellow solid (20 mg, 82%). MS (ESI): *m/z* found $[M+1]^+$ = 547.4.

$^1H$ NMR (400 MHz, *DMSO-d₆*): δ 8.72 (s, 2H), 8.57 (d, *J* = 8.7 Hz, 1H), 7.81 (d, *J* = 11.0 Hz, 1H), 7.32 (s, 1H), 6.56 (s, 1H), 5.58 (dt, *J* = 9.3, 5.1 Hz, 1H), 5.43 (s, 2H), 5.27-5.12 (m, 2H), 3.43-3.36 (m, 1H), 3.27-3.09 (m, 4H), 2.87-2.78 (m, 4H), 3.27-3.09 (m, 4H), 2.87-2.78 (m, 4H) 5.27-5.12 (m, 2H), 3.43-3.36 (m, 1H), 3.27-3.09 (m, 4H), 2.87-2.78 (m, 1H), 2.64-2.55 (m, 1H), 2.43-2.29 (m, 5H), 2.16-2.08 (m, 3H), 1.87 (p, *J* = 7.0 Hz, 2H), 1.61-1.51 (m, 1H), 0.87 (t, *J* = 7.3 Hz 3H).

## Example 35

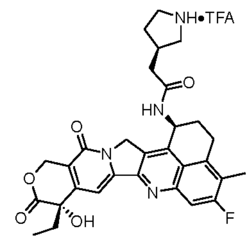

35

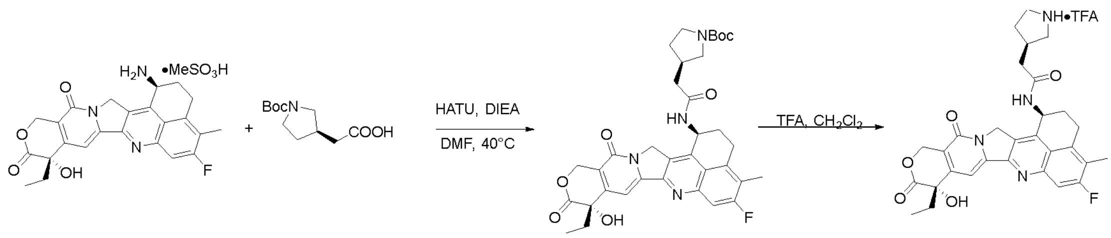


EXD 35a 35b 35

Synthesis of compound 35b:

EXD (25 mg, 47 μmol, 1.0 eq) and compound 30a (11 mg, 47 μmol, 1.0 eq) were dissolved in DMF (1 mL), and HATU (21 mg, 56 μmol, 1.2 eq) and DIEA (17 μL, 103 μmol, 2.2 eq) were added, and stirred at 40 °C for 1.5 h. The reaction solution was directly purified by reversed-phase column chromatography (57% $CH_3CN$ in $H_2O$, 0.05% HCOOH) to obtain a yellow solid (22 mg, 72%).MS (ESI): *m/z* found $[M+1]^+$ = 647.2.

Synthesis of compound 35:

Compound 35b (22 mg, 34 μmol) was dissolved in trifluoroacetic acid (0.2 mL) and dichloromethane (2 mL) and stirred for 2.5 h at room temperature. Concentrated and then purified by reversed-phase column chromatography (28% $CH_3CN$ in $H_2O$, 0.1% TFA) to obtain a yellow solid (17 mg, 76%). MS (ESI): *m/z* found $[M+1]^+$ =547.4.

$^1H$ NMR (400 MHz, *DMSO-d₆*): δ 8.75 (s, 1H), 8.67 (s, 1H), 8.58 (d, *J* = 8.7 Hz, 1H), 7.81 (d, *J* = 10.9 Hz, 1H), 7.31 (s, 1H), 6.55 (s, 1H), 5.58 (dt, *J* = 9.3, 5.1 Hz, 1H), 5.43 (s, 2H), 5.29-5.10 (m, 2H), 3.40 (dtd, J = 11.0, 6.8, 3.3 Hz, 1H) 5.43 (s, 2H), 5.29-5.10 (m, 2H), 3.40 (dtd, *J* = 11.0, 6.8, 3.3 Hz, 1H), 3.27 - 3.07 (m, 4H), 2.85 (ddd, *J* = 15.7, 12.0, 6.6 Hz, 1H), 2.60 (p, *J* = 7.8 Hz, 1H), 2.40 (s, 3H), 2.36 (d, *J* = 7.2 Hz, 2H), 2.18-2.04 (m, 3H), 1.86 (hept, *J* = 7.2 Hz, 2H), 1.56 (dq, *J* = 13.0, 8.8 Hz, 1H), 0.87 (d, *J* = 7.3 Hz, 3H).

## Example 36

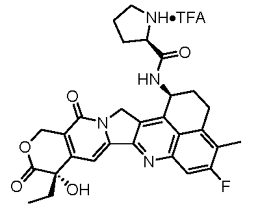

36

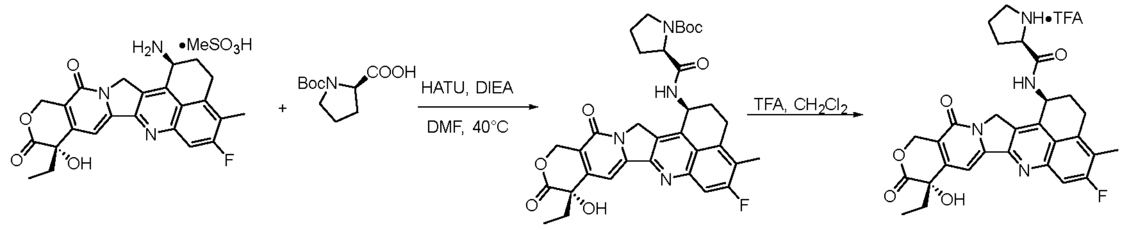


EXD 36a 36b 36

Synthesis of compound 36b:

EXD (25 mg, 47 μmol, 1.0 eq) and compound 30a (10 mg, 47 μ mol, 1.0 eq) were dissolved in DMF (1 mL). HATU (21 mg, 56 μmol, 1.2 eq) and DIEA (17 μL, 103 μmol, 2.2 eq) were added, and stirred at 40°C for 1.5 h. Water was added, extracted with ethyl acetate, washed with saturated saline, dried and concentrated and then purified by column chromatography (4% MeOH in $CH_2Cl_2$) to obtain a white solid (29 mg, 98%). MS (ESI): *m*/*z* found $[M+1]^+$ = 633.4.

Synthesis of compound 36:

Compound 36b (29 mg, 46 μmol) was dissolved in trifluoroacetic acid (0.2 mL) and dichloromethane (2 mL) and stirred for 2.5 h at room temperature. Concentrated and then purified by reversed-phase column chromatography (31% $CH_3CN$ in $H_2O$, 0.1% TFA) to obtain a yellow solid (22 mg, 74%). MS (ESI): *m*/*z* found $[M+1]^+$ =533.4.

$^1H$ NMR (400 MHz, *DMSO-$d_6$*): $\delta$ 9.18 (d, *J* = 8.0 Hz, 1H), 9.00 (d, *J* = 8.4 Hz, 1H), 8.72 (d, *J* = 5.2 Hz, 1H), 7.85 (d, *J* = 11.0 Hz, 1H), 7.33 (s, 1H), 6.57 (s, 1H), 5.62 (dt, *J* = 8.1, 3.7 Hz, 1H), 5.44 (s, 2H), 5.39 (d, *J* = 19.0 Hz, 1H), 5.27 (d, *J* = 19.0 Hz, 1H), 4.14-4.07 (m, 1H), 3.33-3.19 (m, 3H), 3.17 -3.06 (m, 1H), 2.43 (s, 3H), 2.29-2.20 (m, 2H), 2.11 (ddt, *J* = 12.4, 8.1, 4.3 Hz, 1H), 1.96-1.79 (m, 5H), 0.86 (t, *J* = 7.3 Hz, 3H).

## Example 37

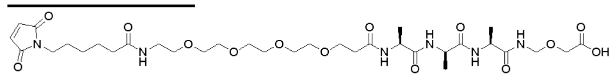

37

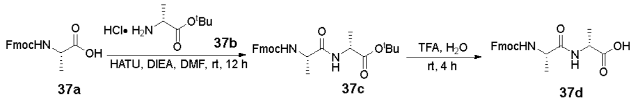


37a 37c 37d

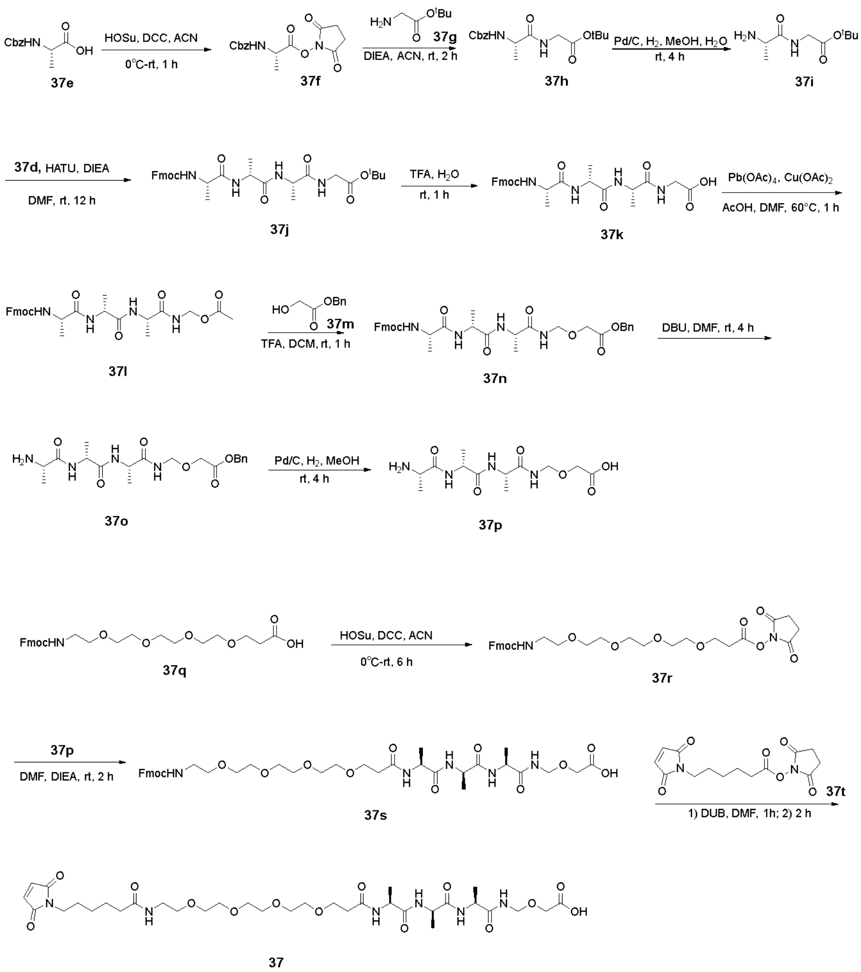


37e 37f 37h 37i 37j 37k 37l 37n 37o 37p 37q 37r 37s 37

Synthesis of compound 37c

To a solution of compound 37a (30 g, 96.5 mmol) and compound 37b (17.5 g, 96.5 mmol, 1.0 eq) in DMF (400 mL) were added HATU (36.7 g, 96.5 mmol, 1.0 eq) and DIEA (32 mL, 193 mmol, 2.0 eq). After stirring the reaction for 12 h at room temperature, the reaction solution was poured into water (1500 mL), extracted with ethyl acetate (500 mL× 3), washed with saturated saline (500 mL), dried with anhydrous sodium sulfate, concentrated and then purified by column chromatography (25% EA in PE) to obtain a white solid (30 g, 70.9%). MS (ESI): m/z found $[M-55]^+$ = 383.2.

Synthesis of compound 37d

A solution of TFA (150 mL) and $H_2O$ (10 mL) of compound 37c (30 g, 68.4 mmol) was reacted with stirring at room temperature for 4h. The reaction solution was then concentrated and pulped with petroleum ether (500 mL) and ethyl acetate (50 mL) to obtain a white solid (34 g, 100%). MS (ESI): m/z found $[M+1]^+$ = 383.2.

Synthesis of compound 37f

To a solution of compound 37e (30 g, 135 mmol) and HOSu (16.2 g, 141 mmol, 1.05 eq) in acetonitrile (300 mL) was added DCC (29 g, 141 mmol, 1.05 eq) in batches under an ice bath, and the reaction was stirred at room temperature for 2 h after the ice bath was withdrawn. At the end of the reaction the insoluble material was filtered out and used directly in the next step without concentration.

Synthesis of compound 37h

To a solution of compound 37f (solution above) and compound 37g (17.6 g, 141 mmol, 1.05 eq) in acetonitrile (400 mL) was added DIEA (44 mL, 282 mmol, 2.1 eq). After stirring the reaction for 2h at room temperature, the reaction solution was concentrated, redissolved in $CH_2Cl_2$ (500 mL), washed with sodium bicarbonate solution (100 mL) and

saturated saline (100 mL), dried with anhydrous sodium sulfate, concentrated and purified by normal-phase flash column chromatography (50% EA in PE) to obtain a white solid (27 g, 59.5%). MS (ESI): m/z found $[M-55]^+$ = 281.0.

Synthesis of compound 37i

To a solution of MeOH (300 mL) and $H_2O$ (10 mL) of compound 37h (18 g, 53.6 mmol) was added 10% Pd/C (1.0 g, 55% $H_2O$), evacuated and reacted under $H_2$ atmosphere for 4h. The insoluble material was filtered off and concentrated to obtain the colorless oily compound (10.8 g, 100%). MS (ESI): m/z found $[2M+1]^+$ = 405.

Synthesis of compound 37j

To a solution of compound 37d (37.5 g, 98 mmol) and compound 37i (19.8 g, 98 mmol, 1.0 eq) in DMF (200 mL) were added HATU (37.3 g, 98 mmol, 1.0 eq) and DIEA (16 mL, 98 mmol, 1.0 eq). After stirring the reaction at room temperature for 4h, the reaction solution was poured into water (1 L) to precipitate the white solid, filtered, washed with water, and pulped by methyl tert-butyl ether to obtain the white solid (40.0 g, 72%). MS (ESI): m/z found $[M-55]^+$ = 511.2.

Synthesis of compound 37k

A solution of TFA (100 mL) and dichloromethane (300 mL) of compound 37i (40.0 g, 71 mmol) was reacted with stirring at room temperature for 3 h. It was concentrated to obtain the yellow oil, and recrystallized with ethyl acetate (500 mL) to obtain a white solid (34.9 g, 97%). MS (ESI): m/z found $[M-1]^-$ = 509.2.

Synthesis of compound 37l

$Pb(OAc)_4$ (31.3 g, 72 mmol, 1.15 eq) was added to a solution of compound 37i (32.1 g, 63 mmol) and compound $Cu(OAc)_2$ (4.33 g, 24 mmol, 0.38 eq) in DMF (300 mL) under nitrogen protection. The reaction solution was warmed up to 60 °C and after stirring the reaction at this temperature for 1 h, the reaction solution was poured into water (1.5 L) and a white solid was precipitated, which was filtered and dried to obtain a white solid (33.2 g, 94%).MS (ESI): m/z found $[M+1]^+$ = 525.2.

Synthesis of compound 37n

To a solution of $CH_2Cl_2$ (500 mL) of compound 37l (32 g, 61 mmol) and compound 37m (17.3 mL, 122 mmol, 2.0 eq) was added TFA (120 mL). After stirring the reaction at room temperature for 1h, the reaction was concentrated, dissolved in DMF, and purified by reversed-phase flash column chromatography (52% ACN in $H_2O$, 0.05% HCOOH) to obtain compound 37n (16.0 g, 40%) as a white solid. MS (ESI): m/z found $[M+23]^+$ = 653.2.

Synthesis of compound 37o

To a solution of compound 37l (15.2 g, 24 mmol) in DMF (80 mL) was added DBU (3.6 mL, 24 mmol, 1.0 eq). After stirring the reaction at room temperature for 1.5 h, the reaction solution was purified by reversed-phase flash column chromatography (26% ACN in $H_2O$, 0.05% HCOOH) to obtain a white solid (7.8 g, 76%).MS (ESI): m/z found $[M+1]^+$ = 409.2.

Synthesis of compound 37p

To a solution of compound 37o (4.0 g, 9.8 mmol) in MeOH (60 mL) was added 10% Pd/C (0.5 g), vacuumized, and reacted under a hydrogen atmosphere for 4h. The insoluble material was filtered off and concentrated to obtain white (2.8 g, 90%). MS (ESI): m/z found $[M+1]^+$ = 319.0.

Synthesis of compound 37r

To a solution of compound 37q (650 mg, 1.33 mmol) in ACN (10 mL) was added HOSu (161 mg, 1.40 mmol, 1.05 eq) and DCC (288 mg, 1.40 mmol, 1.05 eq) under an ice bath and the reaction was stirred at room temperature for 4h. At the end of the reaction, the insoluble material was filtered out and concentrated to obtain the yellow oil compound (770 mg,100%). MS (ESI): m/z found $[M+1]^+$ = 585.0.

Synthesis of compound 37s

To a solution of compound 37r (777 mg, 1.33 mmol) and compound 37p (423 mg, 1.33 mmol, 1.0 eq) in DMF (8 mL) was added DIEA (219 μ uL, 1.33 mmol, 1.0 eq). After stirring the reaction at room temperature for 2h, the compound was purified by reversed-phase flash column chromatography (40% ACN in $H_2O$, 0.05% HCOOH) to obtain the white solid compound (702 mg, 67.0%). MS (ESI): m/z found $[M+1]^+$ = 788.2.

Synthesis of compound 37

To a solution of compound 37s (702 mg, 0.99 mmol) in DMF (6 mL) was added DBU (149 μL, 0.99 mmol, 1.0 eq). After 2 h of stirring at room temperature, compound 37t (315 mg, 0.99 mmol, 1.0 eq) was added. After stirring the reaction at room temperature for 1 h, the reaction solution was purified by reversed-phase flash column chromatography (23% ACN in $H_2O$, 0.05% HCOOH) to obtain the colorless oily compound.

MC-$PEG_4$-AAA-$OCH_2COOH$ (600 mg, 79.8%). MS (ESI): m/z found $[M+1]^+$ = 759.2.

## Example 38

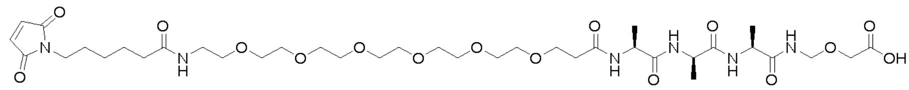

38

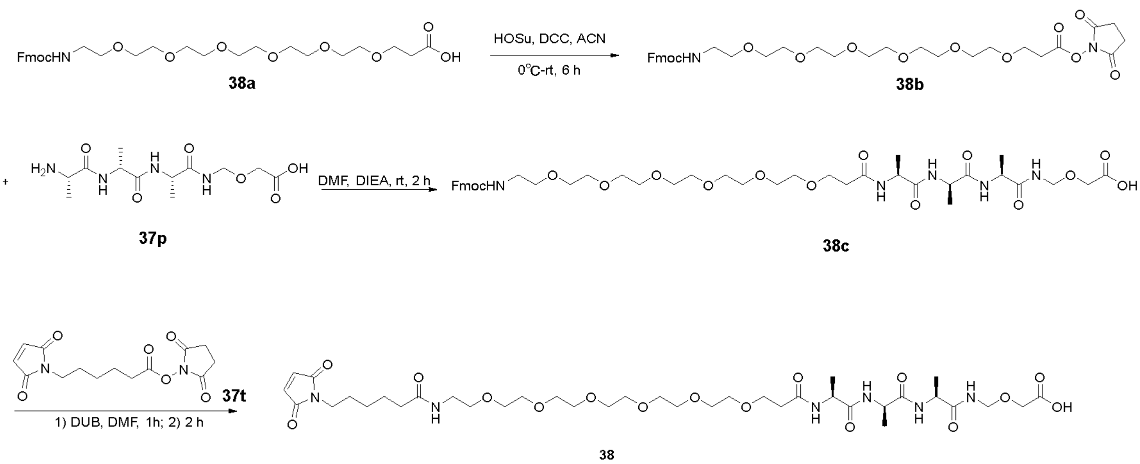


38a 38b 37p 38c 38

Synthesis of compound 38b

To a solution of compound 38a (500 mg, 0.87 mmol) in ACN (10 mL) was added HOSu (110 mg, 0.96 mmol, 1.1 eq) and DCC (198 mg, 0.96 mmol, 1.1 eq) in an ice bath, and the reaction was stirred at room temperature for 4 h. At the end of the reaction, the insoluble material was filtered out, and the reaction mixture was concentrated to obtain the yellow oily compound (assumed 100% yield). MS (ESI): m/z found $[M+1]^+$ = 673.0.

Synthesis of compound 38c

To a solution of compound 38b (585 mg, 0.87 mmol) and compound 37p (277 mg, 0.87 mmol, 1.0 eq) in DMF (8 mL) was added DIEA (144 μ L, 0.87 mmol, 1.0 eq). After stirring the reaction at room temperature for 2 h, the reaction mixture was purified by reversed-phase fast column chromatography (38% ACN in $H_2O$, 0.05% HCOOH) to obtain the colorless oily compound (580 mg, 72%).MS (ESI): m/z found $[M-1]^-$ = 874.2.

Synthesis of compound 38

To a solution of compound 38c (550 mg, 0.63 mmol) in DMF (6 mL) was added DBU (95 μ L, 0.63 mmol, 1.0 eq). After 2 h of stirring at room temperature, 37t (194 mg, 0.63 mmol, 1.0 eq) was added. After 1 h of stirring at room

temperature, the reaction solution was purified by reversed-phase flash column chromatography (24% ACN in $H_2O$, 0.05% HCOOH) to obtain the colorless oily compound MC-PEG6-AAA-$OCH_2$ COOH (200 mg, 37%).MS (ESI): m/z found $[M-1]^-$ = 845.4 .

## Example 39

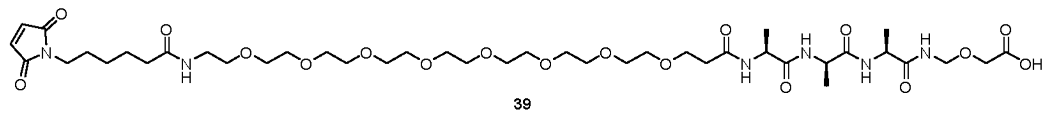

39

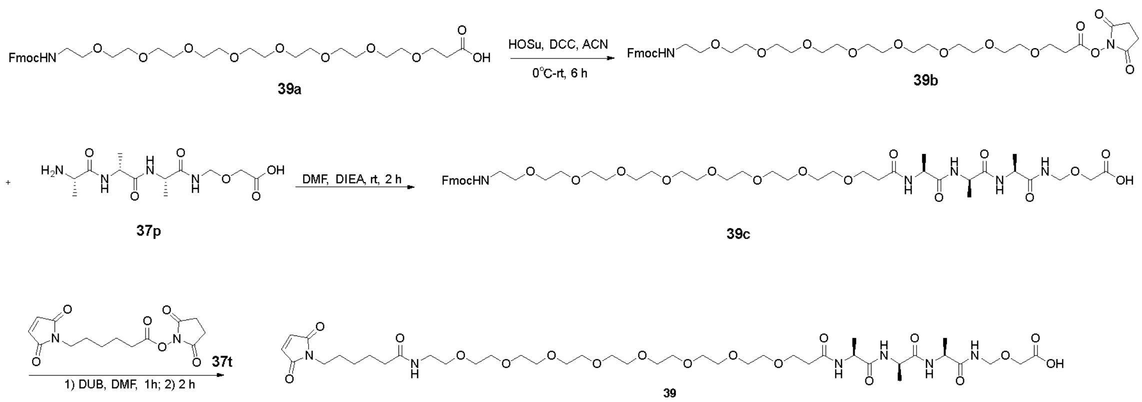


39a, 39b, 37p, 39c, 37t, 39

Synthesis of compound 39b

To a solution of ACN (10 mL) of compound 39a (650 mg, 0.98 mmol) was added HOSu(124 mg, 1.1 mmol, 1.1 eq) and DCC (222 mg, 1.1 mmol, 1.1 eq) under an ice bath, the ice bath was withdrawn, and the reaction was stirred at room temperature for 4h. At the end of the reaction, the insoluble material was filtered out, and the reaction was concentrated to obtain the colorless oily compound 39b (746 mg, 100%). MS (ESI): *m/z* found $[M+1]^+$ = 761.3.

Synthesis of compound 39c

To a solution of compound 39b (945 mg, 0.98 mmol) and compound 37p (312 mg, 0.98 mmol, 1.0 eq) in DMF (8 mL) was added DIEA (162 μ L, 0.98 mmol, 1.0 eq). After the reaction was stirred at room temperature for 2 h, the reaction mixture was purified by reversed-phase flash column chromatography (40% ACN in $H_2O$, 0.05% HCOOH) to obtain the colorless oily compound (815 mg, 86%). MS (ESI): *m/z* found $[M-1]^-$ = 962.5.

Synthesis of compound 39

To a solution of compound 39c (815 mg, 0.85 mmol) in DMF (6 mL) was added DBU (129μL, 0.85 mmol, 1.0 eq). After 2 h of stirring at room temperature, compound 6 (262 mg, 0.85 mmol, 1.0 eq) was added. After 1h of stirring reaction at room temperature, the reaction solution was purified by reversed-phase flash column chromatography (23% ACN in $H_2O$, 0.05% HCOOH) to obtain the colorless oily compound 39 (MC-$PEG_8$ -AAA-$OCH_2COOH$) (330 mg, 41%). MS (ESI): *m/z* found $[M-1]^-$ = 933.5.

## Example 40

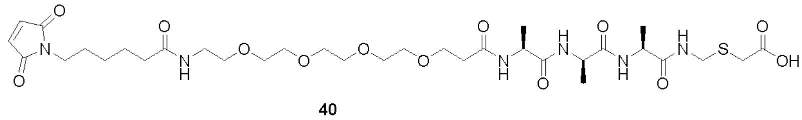

40

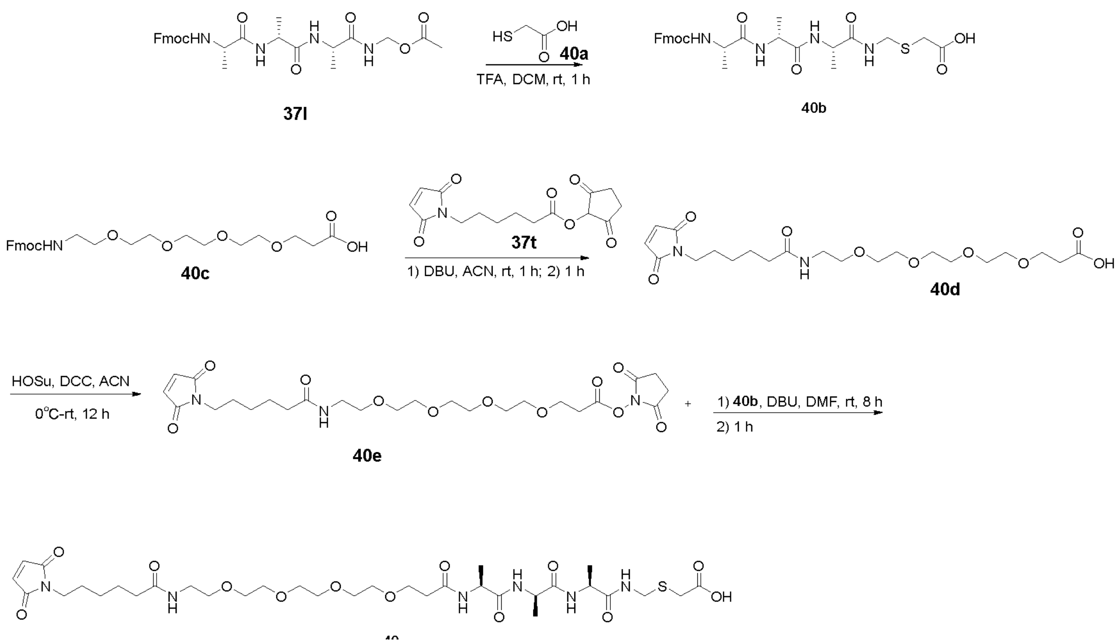


37l 40b 40c 40d 40e

Synthesis of compound 40b

To a solution of $CH_2Cl_2$ (90 mL) of compound 37l (7.7 g, 14.7 mmol) and compound 40a (5.1 mL, 73 mmol, 5.0 eq) was added TFA (16 mL). The reaction was stirred at room temperature for 1 h. The reaction was concentrated, then dissolved in DMF and purified by reversed-phase flash column chromatography (40% ACN in $H_2O$, 0.05% HCOOH) to obtain the white solid compound (5.5 g, 67%). MS (ESI): m/z found $[M+23]^+$ = 653.2.

Synthesis of compound 40d

To a solution of compound 40c (1.2 g, 2.46 mmol) in ACN (10 mL) was added DBU (367 μL, 2.46 mmol, 1.0 eq). After 2 h of stirring at room temperature, compound 37t (909 mg, 2.95 mmol, 1.2 eq) was added. After stirring the reaction at room temperature for 1 h, the reaction solution was concentrated and purified by reversed-phase flash column chromatography (28% ACN in $H_2O$, HCOOH) to obtain the colorless oily compound (460 mg, 41%). MS (ESI): m/z found $[M-1]^-$ = 457.2.

Synthesis of compound 40e

To a solution of compound 40d (460 mg, 1.0 mmol) in ACN (6 mL) was added (138 mg, 1.2 mmol, 1.2 eq) and DCC (248 mg, 1.40 mmol, 1.2 eq) under an ice bath, the ice bath was withdrawn, and the reaction was stirred at room temperature for 12h. At the end of the reaction, the insoluble material was filtered out, and the reaction was concentrated to obtain the compound 40e as a colorless oil (550 mg, 99%). MS (ESI): m/z found $[M+1]^+$ = 556.2.

Synthesis of compound 40

To a solution of compound 40e (1.2 g, 2.46 mmol) in DMF (4 mL) was added DBU (71 μL, 0.47 mmol, 1.0 eq). After 1 h of stirring at room temperature, compound 40b (260 mg, 0.47 mmol, 1.0 eq) was added. After stirring the reaction at room temperature for 1 h, the reaction solution was purified by reversed-phase flash column chromatography (24% ACN in $H_2O$, 0.05% HCOOH) to obtain the colorless oily compound 40 (MC-PEG4-AAA-$SCH_2COOH$) (100 mg, 27.5%). MS (ESI): m/z found $[M-1]^-$ = 773.2.

<u>**Example 41**</u>

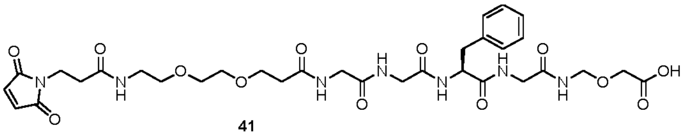

41

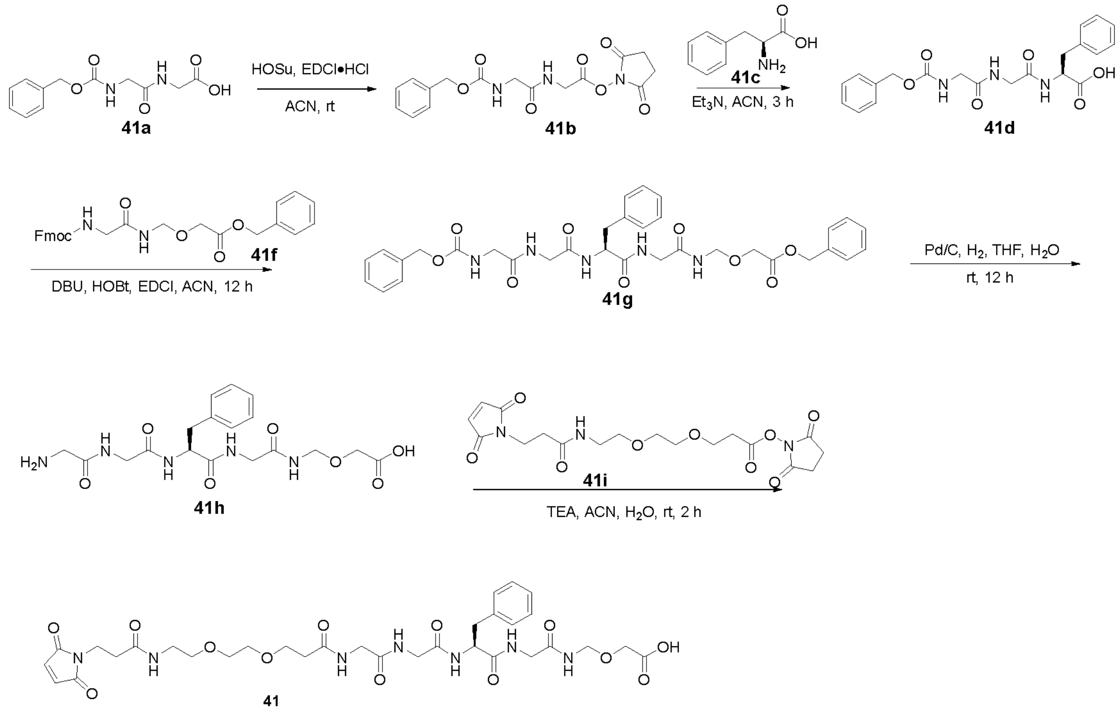


41

Synthesis of compound 41b

Compound 41a (10 g, 37.6 mmol) was dissolved in acetonitrile (100 mL). HOSu (4.76 g, 41.3 mmol, 1.1 eq) and EDCI-HCl (8.28 g, 43.2 mmol, 1.15 eq) were added and stirred at room temperature for 5 h. Approximately 10% of the feedstock remained for LCMS monitoring. Filtered and the filtrate was stirred at 0°C for 5h. A large amount of white solid was precipitated. The filtrate was filtered and the solid was dried to obtain a white solid (9.06 g, 87%). MS (ESI): m/z found $[M+1]^+$ = 364.2.

Synthesis of compound 41d

Compound 41b (9.06 g, 25 mmol) and L-phenylalanine (4.54 g, 27.4 mmol, 1.1 eq) were dissolved in acetonitrile (50 mL) and water (50 mL), and triethylamine (3.8 mL, 27.4 mmol, 1.1 eq) was added and stirred at room temperature for 2 h. The reaction solution had turbidity changed to clarification. The pH was adjusted to 2-3 with hydrochloric acid. Then the reaction solution was continued stirring at room temperature for 6 hours, and precipitated white solid. Filtered, washed with water and the solid was dried to obtain a white solid (10.69 g, 100%).MS (ESI): *m*/*z* found $[M+1]^+$ = 414.2.

Synthesis of compound 41g

DBU (951 μ L, 6.1 mmol, 0.5 eq) was added to a solution of compound 41f (5.8 g, 12.2 mmol) in ACN (150 mL). After stirring the reaction for 2 h at room temperature, HOBt (2.1 g, 15.1 mmol, 1.2 eq) and compound 41d (5.8 g, 12.8 mmol, 1.05 eq) were added, the reaction solution was cooled down to 0 °C, and then EDCI-HCl ((2.9 g, 15.1 mmol, 1.2 eq) was added in batches, and stirred at room temperature overnight. The acetonitrile was removed under reduced pressure, and the residue was extracted by EA (100 mL×3), washed with saturated saline (100 mL), dried with anhydrous sodium sulfate, and then concentrated and purified by normal-phase flash column chromatography (5% MeOH in $CH_2Cl_2$ ) to give white solid compound (6.6 g, 84.9%). MS (ESI): *m*/*z* found $[M+1]^+$ = 648.3.

Synthesis of compound 41h

To a solution of compound 41 g (3.3 g, 5.1 mmol) in THF (30 mL) and $H_2O$ (20 mL) was added 10% Pd/C (1.0 g) and. After evacuation, the reaction was carried out under a hydrogen atmosphere for 20 h. The insoluble material was filtered off and concentrated to obtain the compound 41h (3.0 g, 68.6%) as a white solid. MS (ESI): *m/z* found $[M+1]^+$= 424.2.

Synthesis of compound 41

To a solution of compound 41h (0.21 g, 0.5 mmol, 1.05 eq) in $H_2O$ (2.5 mL), a solution of compound 41i (0.41 g, 1.3 mmol, 1.1 eq) in ACN (2.5 mL) was added, followed by the addition of triethylamine (65 μ L, 0.47 mmol, 1.0 eq), and the reaction was carried out with stirring at room temperature for 2h. The reaction was purified by reversed-phase flash column chromatography (28% ACN in $H_2O$, 0.5% HCOOH) to obtain the white solid compound 41 (MAL-$PEG_2$-GGFG-$OCH_2COOH$) (0.3 g, 87.0%). MS (ESI): *m/z* found $[M-1]^-$ = 732.3.

## Example 42

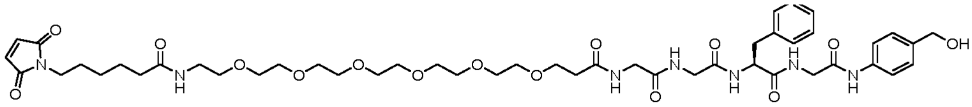

42

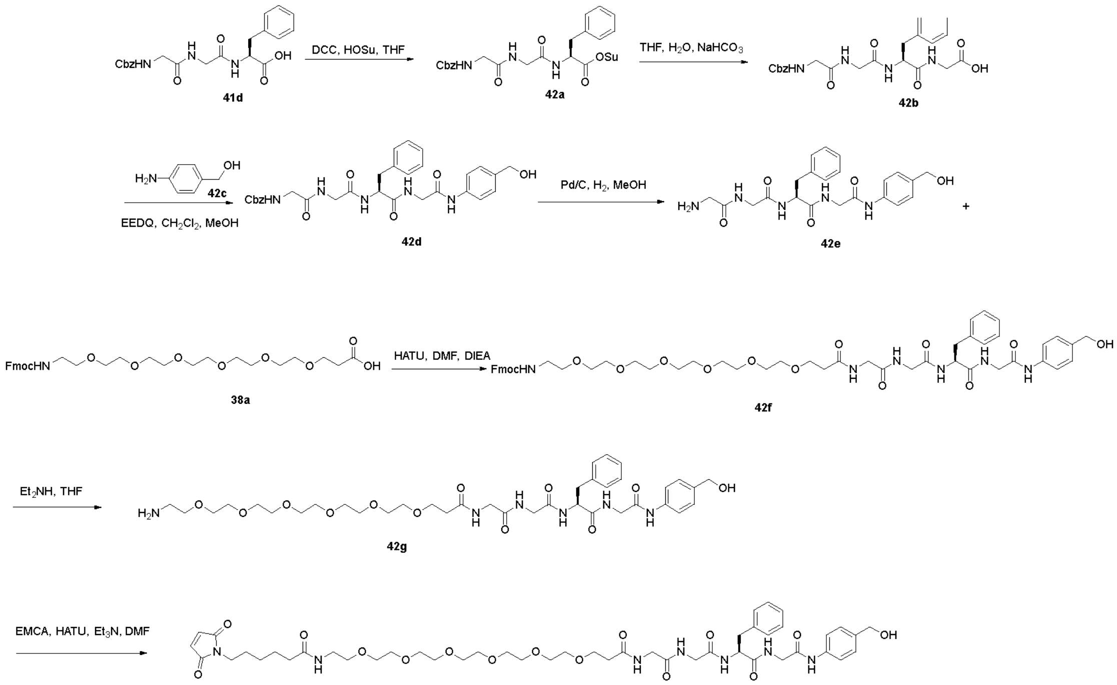


42

Synthesis of compound 42a

Compound 41d (10.5 g, 25.4 mmol, 1.0 eq) was dissolved in tetrahydrofuran (100 mL). HOSu (3.22 g, 27.9 mmol, 1.1 eq) and DCC (5.76 g, 27.9 mmol, 1.1 eq) were added, and stirred for 2 h at room temperature. The filtrate was filtered and concentrated to give a semi-solid product which was used directly in the next step without purification.MS (ESI): *m/z* found $[M+1]^+$ = 511.2.

Synthesis of compound 42b

Compound 42a (12.9 g, 25.4 mmol) and glycine (2.04 g, 27.9 mmol, 1.1 eq) were dissolved in acetonitrile (55 mL)

and water (55 mL) with the addition of triethylamine (4.0 mL, 27.9 mmol, 1.1 eq), and stirred for 2 h at room temperature. The pH was adjusted to 2-3 with hydrochloric acid, and the reaction solution was extracted with ethyl acetate, washed with saturated brine, dried and concentrated to obtain a white foamy solid (11.9 g, 100%). MS (ESI): *m/z* found $[M+1]^+$ = 471.2.

Synthesis of compound 42d

Compound 42b (11.5 g, 24.5 mmol), p-aminobenzyl alcohol (4.51 g, 36.7 mmol, 1.5 eq) and EEDQ (9.07 g, 36.7 mmol, 1.5 eq) were dissolved in dichloromethane (160 mL) and methanol (80 mL) and stirred at room temperature overnight. The solvent was drained, and the resulting mixture was dissolved with ethyl acetate, washed with saturated saline, dried and concentrated to obtain a yellow solid (5.93 g, 42%).MS (ESI): *m/z* found $[M-17]^+$ = 558.2.

Synthesis of compound 42e

Compound 42d (5.93 g, 10.3 mmol) was dissolved in methanol (100 mL), and palladium carbon (3.0 g) was added, and stirred under hydrogen atmosphere for 2.5 h. TLC (EA) monitored the disappearance of the raw material. Filtered with diatomaceous earth and concentrated to obtain a white semi-solid (3.67 g, 81%). MS (ESI): *m/z* found $[M+1]^+$ = 442.2 .

$^1$H NMR (400 MHz, $CDCl_3$ ): $\delta$ 9.77 (s, 1H), 8.47 (t, *J* = 6.0 Hz, 1H), 8.30 (d, *J* = 8.0 Hz, 1H), 8.07 (s, 1H), 7.57 (d, *J* = 8.4 Hz, 2H), 7.27-7.18 (m, 7H), 5.76 (br, 1H), 4.53-4.49 (m, 1H), 4.43 (s, 2H), 3.94-3.77 (m, 3H), 3.66 (d, *J* = 16.4 Hz, 1H), 3.10-3.06 (m, 3H), 2.82 (dd , *J* = 10.0 Hz, 13.8 Hz, 1H), 2.06 (br, 2H).

Synthesis of compound 42f

Compound 42e (1.64 g, 3.7 mmol, 1.0 eq) and compound 38a (2.35 g, 4.1 mmol, 1.1 eq) were dissolved in DMF (16 mL). HATU (2.35 g, 4.1 mmol, 1.1 eq) and DIEA (0.72 mL, 4.5 mmol, 1.2 eq) were added, and mixed at room temperature overnight. Purification by reversed-phase column chromatography (45% ACN in $H_2O$) to obtain a white solid (3.0 g, 81%).MS (ESI): *m/z* found $[M-17]^+$ = 984.1.

Synthesis of compound 42g

Compound 42f (1.0 g) was dissolved in diethylamine (20 mL) and tetrahydrofuran (40 mL) and stirred at room temperature for 4 h. The reaction solution turned into a gelatinous liquid. The solvent was drained to give a viscous solid which was used directly in the next step. MS (ESI): *m/z* found $[M+1]^+$ =777.4.

Synthesis of compound 42

Compound 42f and EMCA (233 mg, 1.1 eq) were dissolved in DMF (5 mL). HATU (419 mg, 1.1 eq) and DIEA (179 μL, 1.1 eq) were added and stirred at room temperature overnight. Purification by reversed-phase column chromatography (36% ACN in $H_2O$) to obtain a pink solid (607 mg, 58%).MS (ESI): *m/z* found $[M-1]^+$ =968.3.

## Example 43

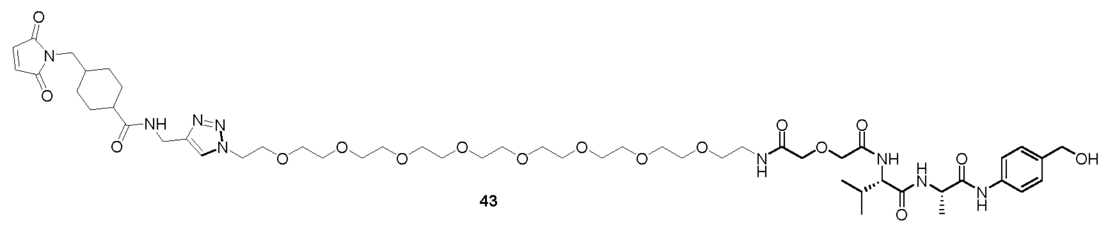

43

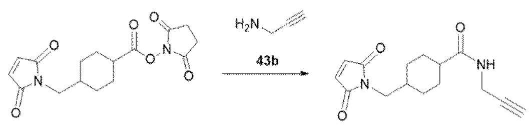


43a 43c

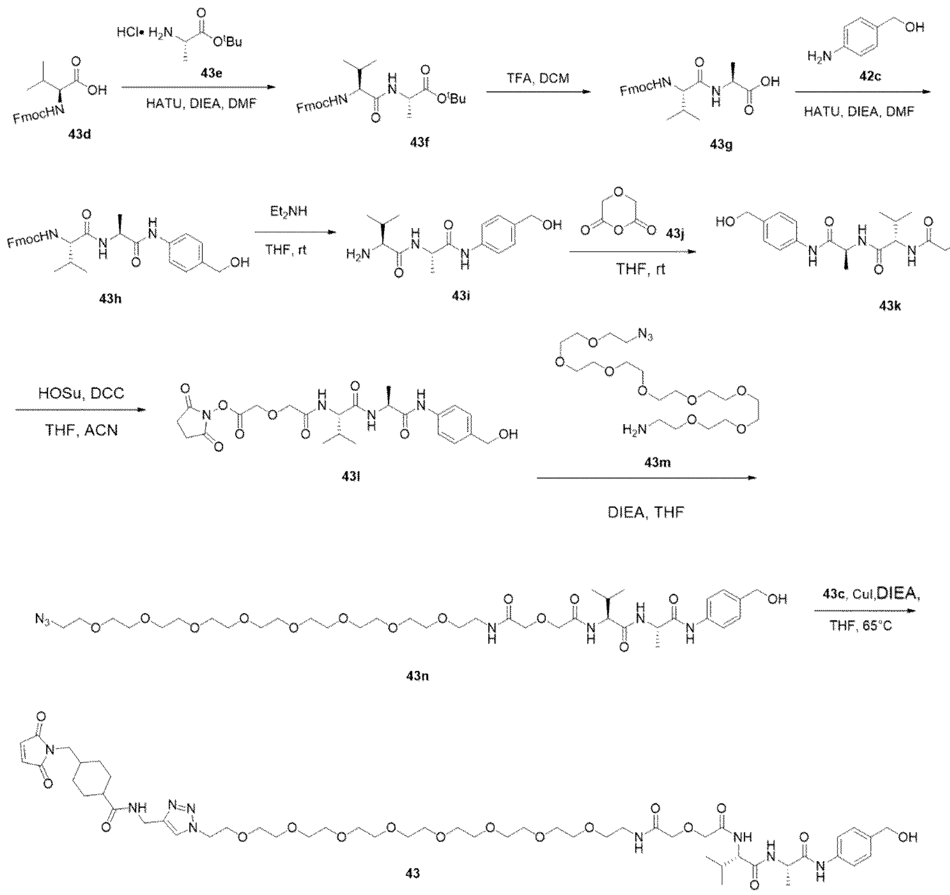


Synthesis of compound 43c

Compound 43a (1.00 g, 2.99 mmol, 1.0 eq) was dissolved in and tetrahydrofuran (20 mL). Compound 43b (0.20 g, 3.64 mmol, 1.2 eq) and DIEA (0.74 mL, 1.5 eq) were added, and stirred for 3h at room temperature. The reaction solution was purified by reversed-phase column chromatography to obtain the compound 43c. MS (ESI): m/z found $[M+1]^+$ =275.1.

Synthesis of compound 43f

Fmoc-L-valine (30.0 g, 88.5 mmol, 1.0 eq), L-alanine tert-butyl ester hydrochloride (17.68 g, 97.3 mmol, 1.1 eq), and HATU (40.35 g, 106.2 mmol, 1.2 eq) were dissolved in DMF (300 mL), and DIEA (32 mL, 0.19 mol , 2.2 eq) was added and stirred at room temperature for 5 h. The reaction solution was poured into water to precipitate a white solid, filtered and dried to obtain the compound 43f (39.0 g, 99%). MS (ESI): m/z found $[M-55]^+$ =411.2.

Synthesis of compound 43g

Compound 43f (39.0 g, 83.7 mmol) was dissolved in $CH_2Cl_2D$ (300 mL), and then TFA (100 mL) was added, after the reaction was reacted for 3 h, LC-MS monitoring showed that the reaction was complete. The reaction solution was poured into water, pulped 2 times, filtered, and the filter residue was the product. The product was placed in drying oven and dried to obtain the compound 43 g (30.9 g, 90%). MS (ESI): m/z found$[M+1]^+$ =411.2.

Synthesis of compound 43h

Compound 43g (5.0 g, 12.2 mmol, 1.0 eq), compound 42c (2.3 g, 18.7 mmol, 1.5 eq), HATU (5.1 g, 13.4 mmol, 1.1 eq), and DIEA (4.73 g, 36.6 mmol, 3.0 eq) were dissolved in DMF (30 mL). After the reaction was carried out for 3 h, LC-MS monitoring showed that the reaction was complete. The reaction solution was poured into water, pulped twice, filtered, and the filter residue was the product. The product was placed in an oven and dried to yield compound 43h (5.3 g, 84%). MS (ESI): m/z found $[M+1]^+$ =516.2.

Synthesis of compound 43i

$Et_2NH$ (36 mL) was added to a solution of compound 43h (2.6 g, 5 mmol) in THF (140 mL). After the reaction was carried out for 3 h, LC-MS monitoring showed that the reaction was complete. The reaction solution was purified by column chromatography to obtain compound 7 (1.1 g, 73%). MS (ESI): m/z found $[M+1]^+$ =294.2.

$^1H$ NMR (400 MHz, DMSO): $\delta$ 10.03 (s, 1H), 8.28 (s, 1H), 7.53 (d, *J* = 4Hz, 2H), 7.25 (d, *J* = 8.4Hz, 2H), 5.12 (s, 1H), 4.48-4.43 (m, 1H), 4.42 (s, 2H), 3.12 (d, J = 5.2Hz, 1H), 1.97-1.92 (m, 3H), 1.31 (d, J = 6.8Hz, 3H), 1.31 (d, J = 6.8Hz, 3H) 3.12 (d, *J* = 5.2 Hz, 1H), 1.97-1.92 (m, 1H), 1.31 (d, *J* = 6.8 Hz, 3H), 0.89 (dd, *J* = 33.6, 6.8 Hz, 2H) ppm.

Synthesis of compound 43k

Compound 43i (1.00 g, 3.41 mmol, 1.0 eq) was dissolved in tetrahydrofuran (10 mL), and diglycolic anhydride (0.44 g, 3.79 mmol, 1.1 eq) was added and stirred at room temperature for 2h. The reaction solution was concentrated to obtain compound 43k, which was used directly in the next step.MS (ESI): *m*/*z* found $[M-1]^-$ =409.2.

Synthesis of compound 43l

Compound 43k (1.4g, 3.42 mmol, 1.0 eq) was dissolved in tetrahydrofuran (20 mL). HOSu (0.47 g, 4.09 mmol, 1.2 eq) and DCC ((0.85 g, 4.13 mmol, 1.2 eq) were added, and stirred for 12 h at room temperature. The filtrate was filtered, concentrated and then purified by column chromatography (5% MeOH in $CH_2Cl_2$) to obtain compound 43l. MS (ESI): *m*/*z* found $[M+1]^+$ =507.2.

$^1H$ NMR (400 MHz, $CDCl_3$ ): $\delta$ 9.02 (s, 1H), 8.90 (s, 1H), 7.79 (s, 1H), 7.69 (t, *J* = 6.8 Hz, 3H), 7.58 (d, *J* = 8.4 Hz, 2H), 7.28 (s, 1H), 6.69 (s, 2H), 5.29 (s, 1H), 4.69 -4.63 (m, 1H), 4.61 (s, 2H), 4.51-4.41 (m, 4H), 4.37-4.31 (m, 1H), 4.15 (d, *J* = 14.0 Hz, 2H), 4.05- 3.93 (m, 2H), 3.82 (t, *J* = 4.9 Hz, 2H), 3.67-3.49 (m, 30H), 3.49-3.42 (m, 2H), 3.34 (d, *J* = 6.8 Hz, 2H), 3.10 (dd, *J* = 14.7, 7.1 Hz, 1H) , 2.68 (s, 1H), 2.29-2.20 (m, 1H), 1.85 (d, *J* = 11.7 Hz, 2H), 1.70 (d, *J* = 12.1 Hz, 2H), 1.56-1.39 (m, 8H), 0.98 (t, *J* = 7.4 Hz, 6H).

Synthesis of compound 43n

Compound 43l (1.00 g, 1.98 mmol, 1.2 eq) and compound 43m (0.72 g, 1.64 mmol, 1.0 eq) were dissolved in tetrahydrofuran (20 mL), and DIEA (0.3 mL, 1.98 mmol, 1.0 eq) was added, and stirred for 3 h at room temperature. The reaction solution was concentrated and purified by silica gel column chromatography (5% MeOH in $CH_2Cl_2$) to obtain the compound 43n (11.9 g, 100%). MS (ESI): *m*/*z* found $[M-1]^-$ = 828.4.

Synthesis of compound 43

Compound 43c (223 mg, 0.81 mmol, 1.2 eq) and compound 43n (562 mg, 0.68 mmol, 1.0 eq) were dissolved in tetrahydrofuran (15 mL) with the addition of cuprous iodide (155 mg, 0.82 mmol, 1.2 eq) and DIEA (0.35 mL, 3.0 eq), and stirred at 65°C for 3 hours. The reaction solution was filtered and concentrated and then purified by silica gel column chromatography (7% MeOH in $CH_2Cl_2$) to obtain the compound 43 (500 mg, 66%). MS (ESI): m/z found $[(M-18)/2+1]^+$ =543

$^1H$ NMR (400 MHz, $CDCl_3$ ): $\delta$ 9.02 (s, 1H), 8.90 (s, 1H), 7.79 (s, 1H), 7.69 (t, *J* = 6.8 Hz, 3H), 7.58 (d, *J* = 8.4 Hz, 2H), 7.28 (s, 1H), 6.69 (s, 2H), 5.29 (s, 1H), 4.69 -4.63 (m, 1H), 4.61 (s, 2H), 4.51-4.41 (m, 4H), 4.37-4.31 (m, 1H), 4.15 (d, *J* = 14.0 Hz, 2H), 4.05- 3.93 (m, 2H), 3.82 (t, *J* = 4.9 Hz, 2H), 3.67-3.49 (m, 30H), 3.49-3.42 (m, 2H), 3.34 (d, *J* = 6.8 Hz, 2H), 3.10 (dd, *J* = 14.7, 7.1 Hz, 1H) , 2.68 (s, 1H), 2.29-2.20 (m, 1H), 1.85 (d, *J* = 11.7 Hz, 2H), 1.70 (d, *J* = 12.1 Hz, 2H), 1.56-1.39 (m, 8H), 0.98 (t, *J* = 7.4 Hz, 6H).

**Example 44**

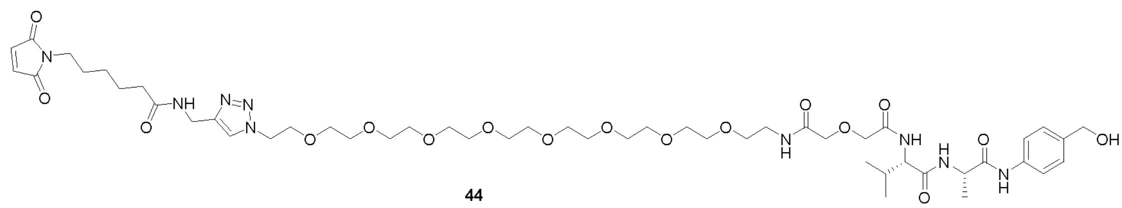

44

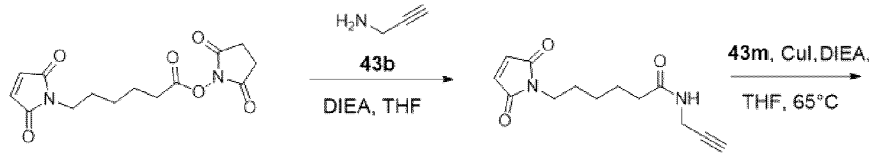


37t 44a

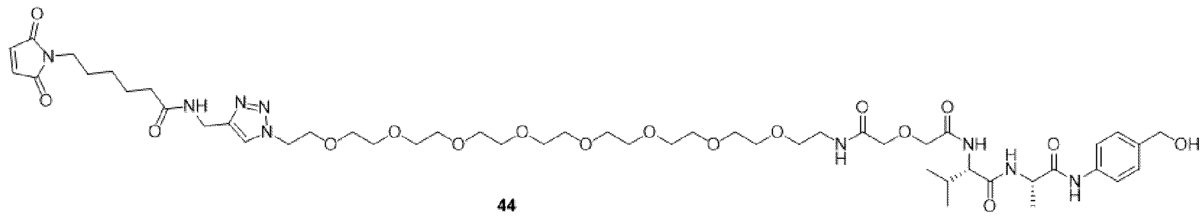

44

Synthesis of compound 44a

Compound 37t (1.33 g, 4.32 mmol, 1.0 eq) was dissolved in and tetrahydrofuran (20 mL), and compound 43b (0.28 g, 5.09 mmol, 1.2 eq) and DIEA (1.4 mL, 3.0 eq) were added, and stirred for 3 h at room temperature. The reaction solution was concentrated and purified by column chromatography (5% MeOH in $CH_2Cl_2$) to obtain the compound 44a. MS (ESI): m/z found $[M+1]^+$ =249.1.

Synthesis of compound 44

Compound 44a (120 mg, 0.48 mmol, 1.2 eq) and compound 43m (335 mg, 0.4 mmol, 1.0 eq) were dissolved in tetrahydrofuran (10 mL) with the addition of cuprous iodide (92 mg, 0.48 mmol, 1.2 eq) and DIEA (0.2 mL, 3.0 eq), and stirred at 65 °C for 3 hours. The reaction solution was filtered, concentrated and then purified by silica gel column chromatography (7% MeOH in $CH_2Cl_2$) to obtain compound 44 (500 mg, 66%). MS (ESI): m/z found $[(M-18)/2+1]^+$ =530.

**Example 45**

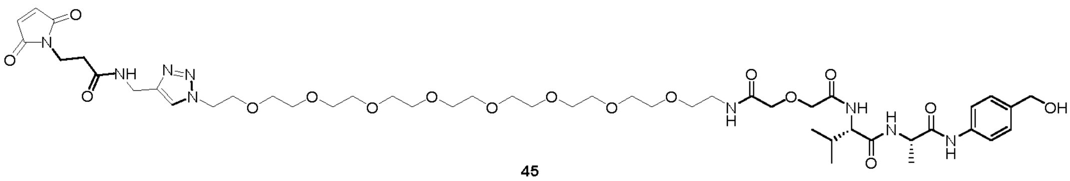

45

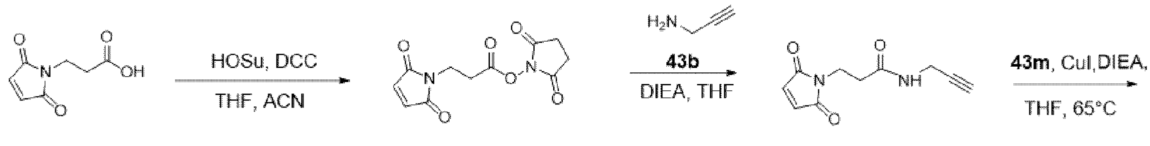


45a 45b 45c

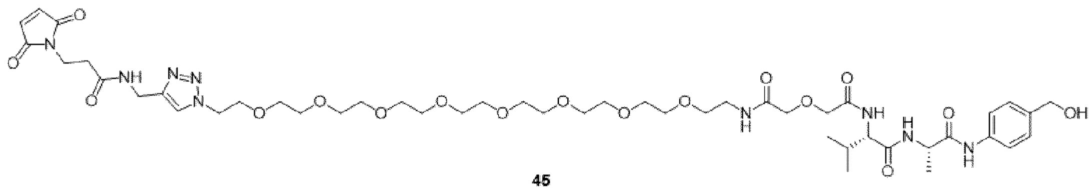

45

Synthesis of compound 45b

To a solution of compound 45a (1.0 g, 5.92 mmol, 1.0 eq) in ACN (5 mL) was added HOSu (0.82 g, 7.13 mmol, 1.2 eq) and DCC (1.46 g, 7.09 mmol, 1.2 eq) in an ice bath, and the reaction was stirred at room temperature for 12 h. At the end of the reaction, the insoluble material was filtered out, and the reaction was concentrated to obtain the yellow oily compound (1.5 g, 96%).MS (ESI): m/z found $[M+1]^+$ = 267.1.

Synthesis of compound 45c

Compound 45b (1.0 g, 3.76 mmol, 1.0 eq) was dissolved in and tetrahydrofuran (20 mL), compound 43b (0.25 g, 4.55 mmol, 1.2 eq) and DIEA (1.2 mL, 2.0 eq) were added, and the reaction was stirred for 3 h at room temperature. The reaction solution was concentrated and then purified by column chromatography (4% MeOH in $CH_2Cl_2$) to obtain the compound 45c. MS (ESI): m/z found $[M+1]^+$ =207.1.

Synthesis of compound 45

Compound 45c (152 mg, 0.74 mmol, 1.2 eq) and compound 43m (510 mg, 0.62 mmol, 1.0 eq) were dissolved in tetrahydrofuran (10 mL) with the addition of cuprous iodide (140 mg, 0.74 mmol, 1.2 eq) and DIEA (0.30 mL, 3.0 eq), and stirred at 65°C for 3 hours. The reaction solution was filtered and concentrated and then purified by silica gel column chromatography (7% MeOH in $CH_2Cl_2$) to obtain the compound 45 (325 mg, 51%). MS (ESI): m/z found $[(M-18)/2+1]^+$ =509.

## Example 46

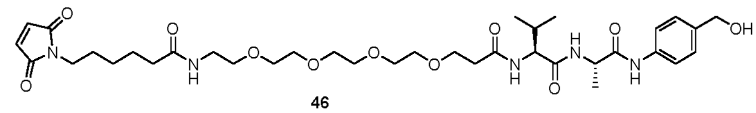

46

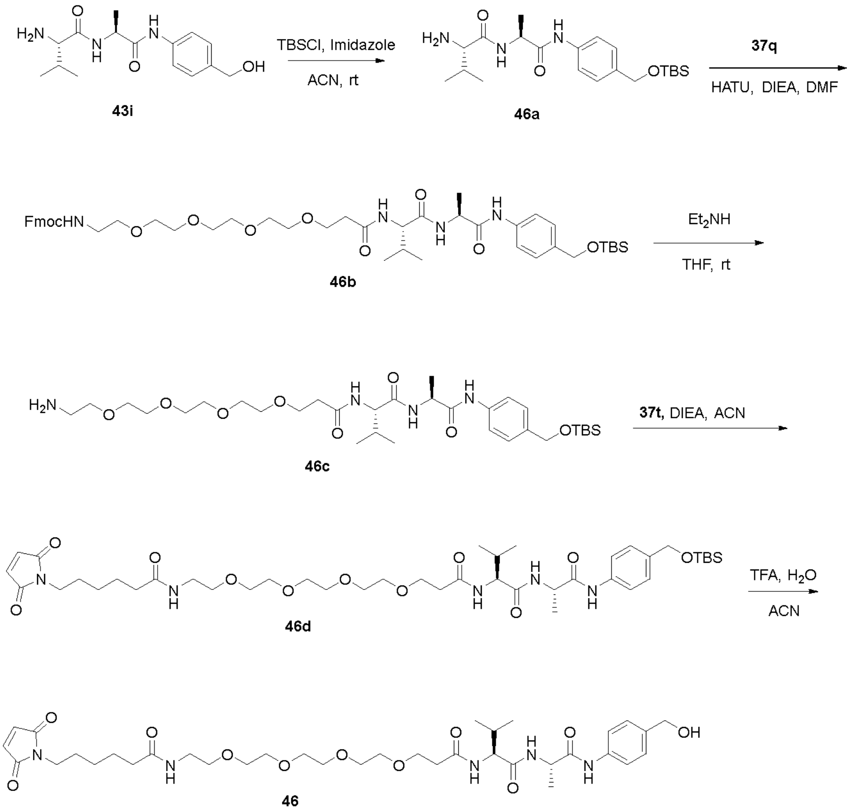


43i 46a 46b 46c 46d 46

Synthesis of compound 46a

Compound 43i (500 mg, 1.7 mmol, 1.0 eq), imidazole (174 mg, 2.6 mmol, 1.5 eq) and TBSCl (334 mg, 2.2 mmol, 1.3 eq) were dissolved in ACN (20 mL), and the reaction was completed as shown by LC-MS monitoring after 16 h of reaction. The reaction solution was purified by column chromatography to obtain the compound 46a (625 mg, 90%). MS (ESI): m/z found $[M+1]^+$ =408.3.

Synthesis of compound 46b

Compound 46a (757 mg, 1.9 mmol, 1.0 eq), compound 37q (996 mg, 2.0 mmol, 1.1 eq), HATU (1.06 g, 2.8 mmol,1.5 eq) andDIEA(920 μL, 5.6 mmol, 3.0 eq) were dissolved in DMF (20 mL), and the reaction was carried out for 5 h. The LC-MS monitoring showed that the reaction was completed. The reaction solution was extracted with EA and saturated saline, and the organic phase was dried and concentrated to obtain the crude product. The crude product was purified by column chromatography to obtain the compound 46b (615 mg, 50%). MS (ESI): m/z found $[M\text{-}TBS\text{-}17]^+$ =745

Synthesis of compound 46c

$Et_2NH$ (4 mL) was added to a solution of compound 46b (600 mg, 0.7 mmol) in THF (20 mL). After the reaction was carried out for 3h, LC-MS monitoring showed that the reaction was completed. The reaction solution was drained and purified by reversed-phase column chromatography to obtain the compound 46c (403 mg, 90%). MS (ESI): m/z found $[M+H]^+$ =655

Synthesis of compound 46d

Compound 37t (392 mg, 1.9 mmol, 1.5 eq), compound 46c (811 mg, 1.2 mmol, 1.0 eq), HATU (942 mg, 2.5 mmol, 2.0 eq), and DIEA (480 mg, 3.7 mmol, 3.0 eq) were dissolved in DMF (20 mL), and the reaction was carried out for 3 h. After 3 h, LC-MS monitoring showed that the reaction was completed. The reaction solution was extracted with EA and saturated saline, and the organic phase was dried and concentrated to obtain the crude product. The crude product was purified by column chromatography to obtain the compound 46d (260 mg, 25%). MS (ESI): m/z found $[M\text{-}TBS\text{-}17]^+$ =716

Synthesis of compound 46

TFA (0.3 mL) and $H_2O$ (0.2 mL) were added to a solution of compound 46d (260 mg, 0.3 mmol) in ACN (6 mL), and the reaction was completed by LC-MS monitoring after 10 min. The reaction solution was spun dry, dissolved in DMF and purified to obtain the compound 46 (82 mg, 36%) on a $C_{18}$ column. MS (ESI): m/z found $[M+H]^+$ =734.

**Example 47**

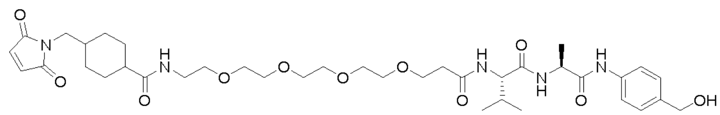

47

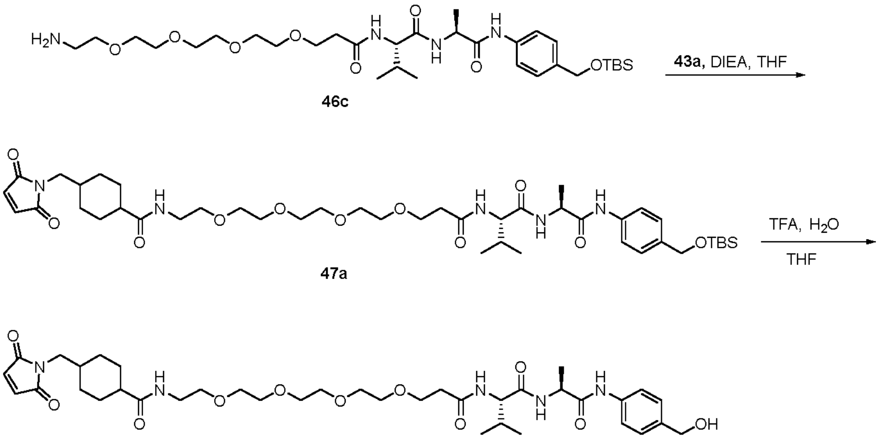


46c

47a

47

Synthesis of compound 47a

Compound 43a (120 mg, 0.4 mmol, 1.5 eq), compound 46c (157 mg, 0.2 mmol, 1.0 eq) and DIEA(40 μL, 0.2 mmol, 1.0 eq) were dissolved in ACN (2 mL) for 1 h. After 1 h of the reaction, the reaction was completed by LC-MS monitoring. The reaction solution was purified by column chromatography to obtain the compound 47a (188 mg, 90%). MS (ESI): m/z found $[M-TBS-17]^+$ =742

Synthesis of compound 47

TFA (0.1 mL) and $H_2O$ (0.1 mL) were added to ACN (2 mL) solution of compound 47a (188 mg, 0.2 mmol), and the reaction was completed by LC-MS monitoring after 10 min. The reaction solution was spun dry and dissolved in DMF and purified by $C_{18}$ column to obtain the compound 47 (130 mg, 80%). MS (ESI): m/z found $[M+H]^+$ =760.

## Example 48

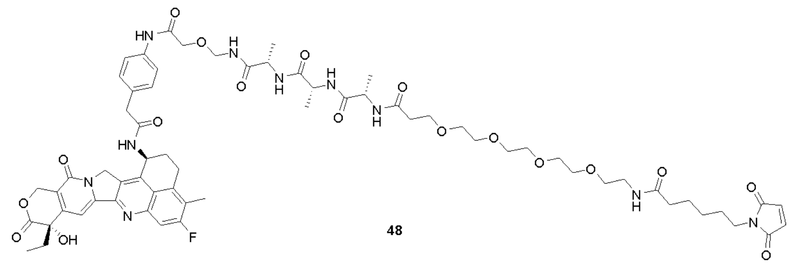

48

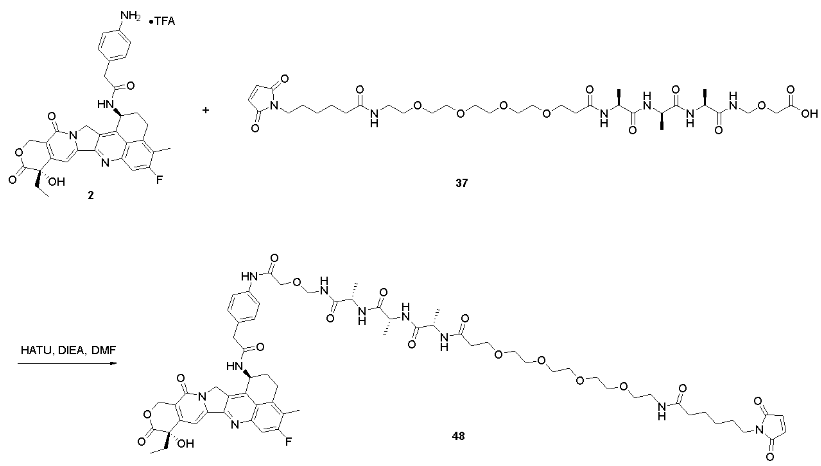


2

37

48

Synthesis of Compound 48:

Compound 2 (30mg, 44μmol, 1.0eq) and compound 37 (33mg, 47μmol, 1.0eq) were dissolved in DMF (0.5mL). HATU (25mg, 66μmol, 1.5eq) and DIEA (18μL, 110μmol, 2.5eq ) were added, and stirred at room temperature for 2 hours. The reaction solution was directly purified by reverse phase column chromatography (38% $CH_3CN$ in $H_2O$, 0.05% HCOOH) to obtain a white solid (35mg, 61%). MS (ESI): m/z found $[M/2+1]^+$=655.2.

$^1H$ NMR (400 MHz, $CDCl_3$): $\delta$ 8.79 (s, 1H), 8.55 (t, *J* = 6.4 Hz, 1H), 7.65 (d, *J* = 6.4 Hz, 1H), 7.62-7.58 (m, 4H), 7.44 (d, *J* = 8.0 Hz, 1H), 7.36 (d, *J* = 8.4 Hz, 2H), 7.32-7.30 (m, 2H), 6.98 (d, *J* = 8.2 Hz, 1H), 6.70 (d, *J* = 5.2 Hz, 1H), 6.69 (s, 1H), 6.61 (t, *J* = 5.3 Hz, 1H), 5.73 (d, *J* = 16.2 Hz, 1H), 5.55-5.53 (m, 1H), 5.38 (d, *J* = 16.2 Hz, 1H), 5.29 (d, *J* = 19.1 Hz, 1H), 5.17 (d, *J* = 19.0 Hz, 1H), 4.80 (q, *J* = 10.0 Hz, 1H), 4.69 (q, *J* = 10.0 Hz, 1H), 4.27 (t, *J*=6.7 Hz, 1H), 4.13-3.92 (m, 6H), 3.81-3.69 (m, 2H), 3.68-3.59 (m, 12H), 3.55 (t, *J* = 5.2 Hz, 2H), 3.49 (t, *J* = 7.2 Hz, 2H), 3.43 (q, *J* = 5.2 Hz, 2H), 3.21-3.15 (m, 1H), 3.10-3.05 (m, 1H), 2.53 (q, *J* = 6.0 Hz, 2H), 2.39 (s, 3H), 2.35-2.30 (m, 2H), 2.18 (t, *J* = 7.6 Hz, 2H), 1.95-1.87 (m, 2H), 1.67-1.54 (m, 2H), 1.38 (d, *J* = 7.1 Hz, 3H), 1.33-1.26 (m, 4H), 1.20 (d, *J* = 7.1 Hz, 6H), 1.05 (t, *J* = 7.4 Hz, 3H).

## Example 49

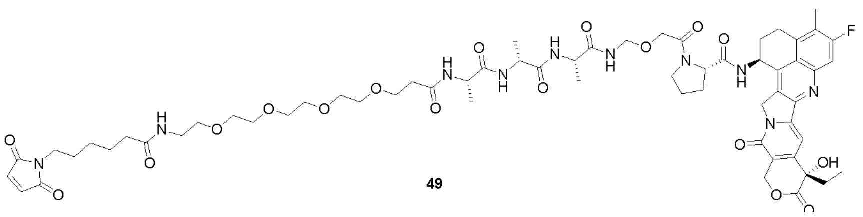

49

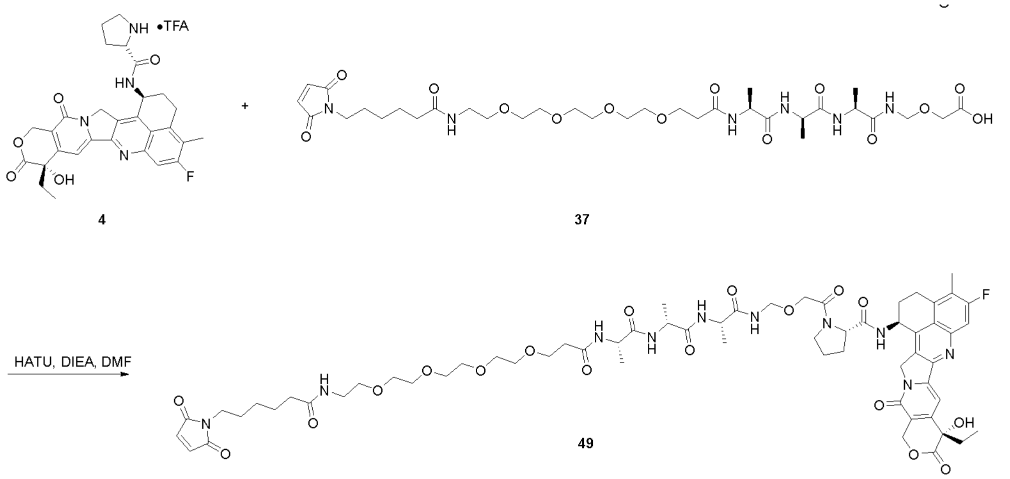


4

37

49

Synthesis of Compound 49:

Compound 4 (19mg, 29μmol, 1.0eq) and compound 37 (22mg, 29μmol, 1.0eq) were dissolved in DMF (0.5mL). HATU (17mg, 44μmol, 1.5eq) and DIEA (12μL, 73μmol, 2.5eq) were added and stirred at room temperature for 3 hours. The reaction solution was directly purified by reverse phase column chromatography (33% $CH_3CN$ in $H_2O$, 0.05% HCOOH) to obtain a pale yellow solid (18mg, 48%). MS(ESI): m/z found Fragment 1, Positive = 591.2, Fragment 2, Positive = 683.2

$^1H$ NMR (400 MHz, $CDCl_3$): $\delta$ 8.15 (d, *J* = 8.4 Hz, 1H), 7.74 (t, *J* = 6.2 Hz, 1H), 7.6 (s, 1H), 7.56-7.52 (m, 2H), 7.46 (d, *J* = 5.6 Hz, 1H), 7.36 (d, *J* = 6.6 Hz, 1H), 6.68 (s, 2H), 6.50 (t, *J* = 5.7 Hz, 1H), 5.71-5.56 (m, 3H), 5.26 (d, *J* = 16.4 Hz, 1H), 5.08 (d, *J* = 19.2 Hz, 1H), 4.67 (dd, *J* = 8.6, 2.8 Hz, 1H), 4.35 (t, *J* = 9.8 Hz, 1H), 4.24 (t, *J* = 7.0 Hz, 1H), 4.19-4.12 (m, 5H), 3.93 (d, *J* = 15.7 Hz, 1H), 3.73-3.66 (m, 3H), 3.63-3.57 (m, 12H), 3.53 (t, *J* = 5.2 Hz, 2H), 3.48 (t, *J* = 7.2 Hz, 2H), 3.41 (t, *J* = 5.4 Hz, 2H), 3.37-3.32 (m, 1H), 3.23-3.15 (m, 1H), 3.12-3.04 (m, 1H), 2.47-2.25 (m, 8H), 2.15 (t, *J* = 7.5 Hz, 2H), 2.03-1.96 (m, 3H), 1.66-1.54 (m, 4H), 1.34 (d, *J* = 3.2 Hz, 3H), 1.33 (d, *J* = 3.1 Hz, 3H), 1.31-1.24 (m, 3H), 1.18 (dd, *J* = 25.2, 7.3 Hz, 1H), 1.05-0.99 (m, 6H).

**Example 50**

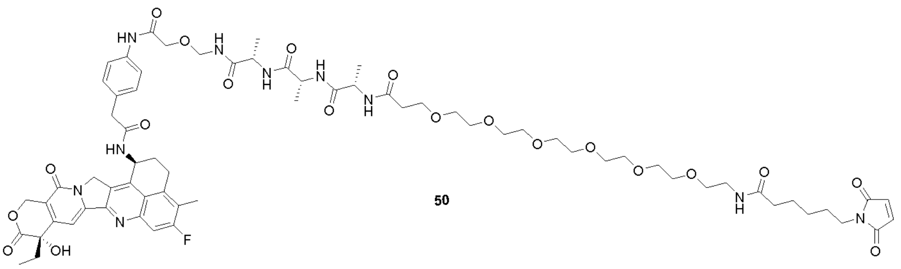

50

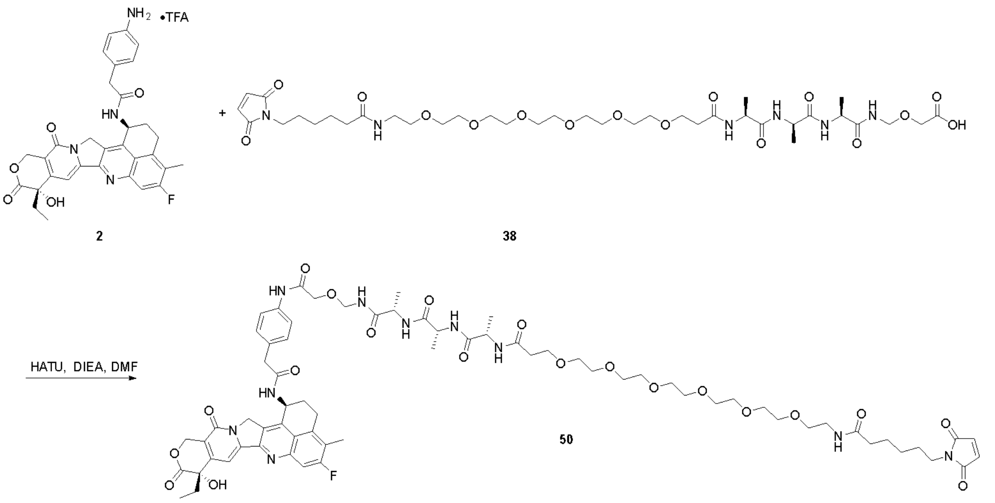


2

38

50

Synthesis of Compound 50:

Compound 2 (30mg, 44μmol, 1.0eq) and compound 38 (37mg, 44μmol, 1.0eq) were dissolved in DMF (0.5mL). HATU (25mg, 66μmol, 1.5eq) and DIEA (18μL, 110μmol, 2.5eq ) were added, and stirred at room temperature for 2 hours. The reaction solution was directly purified by reverse phase column chromatography (37% $CH_3CN$ in $H_2O$, 0.05% HCOOH) to obtain a pale yellow solid (27mg, 43%). MS(ESI): m/z found Fragment 1, Positive=627.2, Fragment 2, Positive=771.2

$^1H$ NMR (400 MHz, $CDCl_3$): $\delta$ 8.79 (s, 1H), 8.53 (t, *J* = 6.3 Hz, 1H), 7.67 (d, *J* = 5.8 Hz, 1H), 7.60 (s, 1H), 7.58 (s, 1H), 7.56 (s, 1H), 7.55 (s, 1H), 7.52 (t, *J* = 5.9 Hz, 1H), 7.41 (d, *J* = 7.7 Hz, 1H), 7.34 (s, 1H), 7.32 (s, 1H), 7.28 (s, 1H), 6.90 (d, *J* = 5.5 Hz, 1H), 6.67 (s, 2H), 6.57 (t, *J* = 5.3 Hz, 1H), 5.71 (d, *J* = 16.3 Hz, 1H), 5.53 (q, *J* = 7.6 Hz, 1H), 5.36 (d, *J* = 20.2 Hz, 1H), 5.26 (d, *J* = 19.2 Hz, 1H), 5.15 (d, *J* = 20.1 Hz, 1H), 4.77 (dd, *J* = 6.7, 10.2 Hz, 1H), 4.63 (dd, *J* = 6.2, 10.2 Hz, 1H), 4.25-4.18 (m, 1H), 4.09 (d, *J* = 16.2 Hz, 1H), 4.05-3.99 (m, 2H), 3.90 (d, *J* = 16.0 Hz, 1H), 3.80-3.73 (m, 2H), 3.70-3.57 (m, 22H), 3.53 (t, *J* = 5.1 Hz, 2H), 3.47 (t, *J* = 7.0 Hz, 2H), 3.41 (q, *J* = 5.2 Hz, 2H), 3.15 (dt, *J* = 5.3, 17.0 Hz, 1H), 3.05 (t, *J* = 7.9 Hz, 1H), 2.56-2.41 (m, 2H), 2.37 (s, 3H), 2.33 (s, 1H), 2.30-2.24 (m, 1H), 2.15 (t, *J* = 7.4 Hz, 2H), 1.65-1.52 (m, 4H), 1.37 (d, *J* = 7.1 Hz, 3H), 1.33-1.23 (m, 4H), 1.19 (d, *J* = 7.0 Hz, 3H), 1.16 (d, *J* = 7.2 Hz, 3H), 1.02 (t, *J* = 7.4 Hz, 3H).

**Example 51**

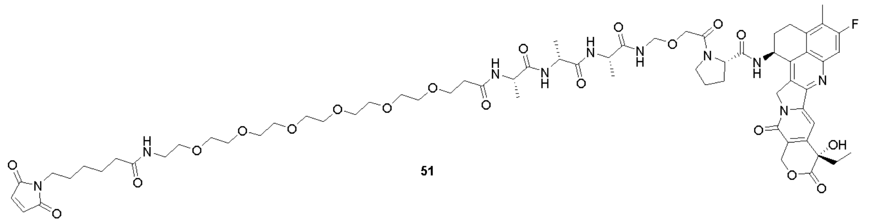

51

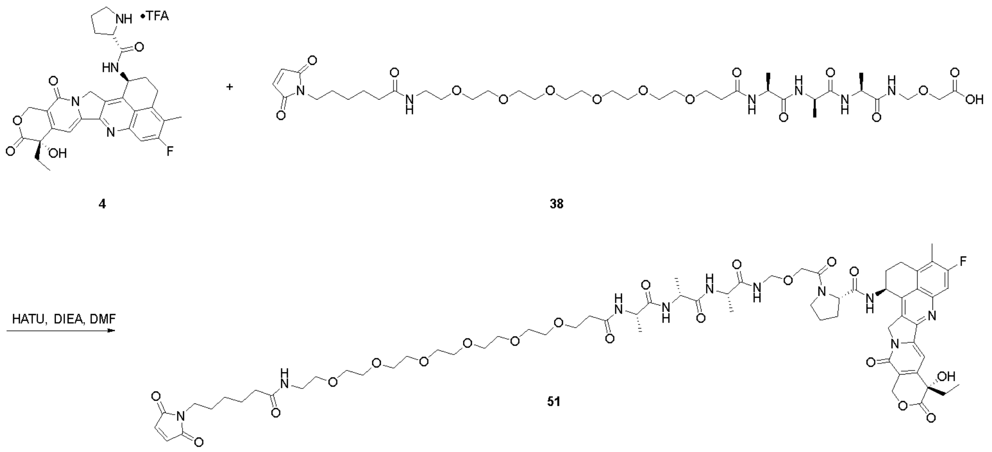


4

38

51

Synthesis of compound 51:

Compound 4 (30mg, 46μmol, 1.0eq) and compound 38 (39mg, 46μmol, 1.0eq) were dissolved in DMF (0.5mL). HATU (26mg, 70μmol, 1.5eq) and DIEA (19μL, 116μmol, 2.5eq ) were added, stirred at room temperature for 2 hours. The reaction solution was directly purified by reversed-phase column chromatography (34% $CH_3CN$ in $H_2O$, 0.05% HCOOH) to obtain a pale yellow solid (28 mg, 44%). MS(ESI): m/z found Fragment 1, Positive=591.2, Fragment 2, Positive=771.2

$^1H$ NMR (400 MHz, $CDCl_3$): $\delta$ 8.17 (d, *J* = 8. 4 Hz, 1H), 7.68 (t, *J* = 7.8 Hz, 1H), 7.61-7.50 (m, 4H), 7.36 (d, *J* = 6.6 Hz, 1H), 6.68 (s, 2H), 6.52 (t, *J* = 5.7 Hz, 1H), 5.68 (t, *J* = 16.2 Hz, 2H), 5.58 (q, *J* = 6.4 Hz, 1H), 5.27 (d, *J* = 16.4 Hz, 1H), 5.10 (d, *J* = 19.4 Hz, 1H), 4.68 (dd, *J* = 8.4, 3.0 Hz, 1H), 4.31 (t, *J* = 10.0 Hz, 1H), 4.22 (t, *J* = 7.1 Hz, 1H), 4.17-4.04 (m, 5H), 3.89 (d, *J* = 15.6 Hz, 1H), 3.75 - 3.67 (m, 3H), 3.66 - 3.56 (m, 20H), 3.53 (t, *J* = 5.2 Hz, 2H), 3.49 (t, *J* = 7.2 Hz, 2H), 3.41 (t, *J* = 5.3 Hz, 2H), 3.33 (q, *J* = 8.8 Hz, 1H), 3.24-3.16 (m, 1H), 3.12-3.04 (m, 1H), 2.49-2.23 (m, 8H), 2.16 (t, *J* = 7.5 Hz, 2H), 1.92-1.86 (m, 3H), 1.66-1.54 (m, 4H), 1.35 (t, *J* = 7.7 Hz, 6H), 1.31-1.25 (m, 3H), 1.18 (dd,*J* = 25.2, 7.3 Hz, 1H), 1.03 (t, *J* = 7.2 Hz, 3H), 1.01 (t, *J* = 5.1 Hz, 3H).

**Example 52**

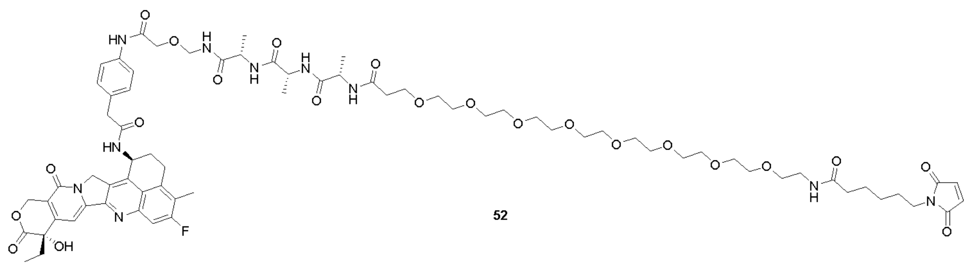

52

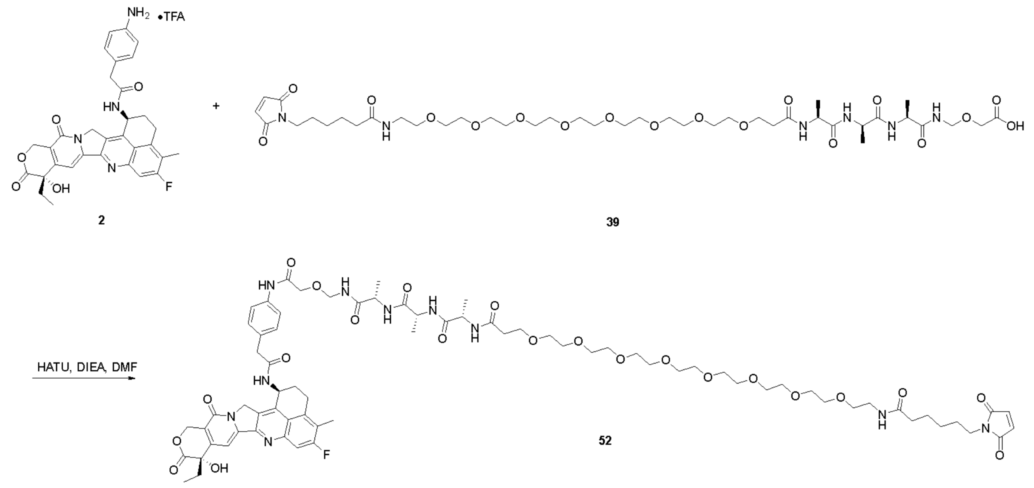


2

39

52

Synthesis of compound 52:

Compound 2 (28mg, 41μmol, 1.0eq) and compound 39 (38mg, 41μmol, 1.0eq) were dissolved in DMF (0.5mL). HATU (23mg, 62μmol, 1.5eq) and DIEA (17μL, 103μmol, 2.5eq) were added, and stirred at room temperature for 2 hours. The reaction solution was directly purified by reverse phase column chromatography (37% $CH_3CN$ in $H_2O$, 0.05% HCOOH) to obtain a yellow solid (32 mg, 52%). MS(ESI): m/z found Fragment 1, Positive = 627.2, Fragment 2, Positive = 859.4

$^1$H NMR (400 MHz, $CDCl_3$): $\delta$ 8.79 (s, 1H), 8.53 (t, *J* = 6.3 Hz, 1H), 7.68 (d, *J* = 5.7 Hz, 1H), 7.60 (s, 1H), 7.58 (s, 2H), 7.55-7.51 (m, 2H), 7.43 (d, *J* = 8.0 Hz, 1H), 7.34 (s, 1H), 7.31 (s, 2H), 6.87 (d, *J* = 8.6 Hz, 1H), 6.67 s, 2H), 6.47 (t, *J* = 5.5 Hz, 1H), 5.72 (d, *J* = 16.3 Hz, 1H), 5.52 (q, *J* = 7.2 Hz, 1H), 5.34 (d, *J* = 16.2 Hz, 1H), 5.26 (d, *J* = 19.2 Hz, 1H), 5.11 (d, *J* = 18.9 Hz, 1H), 4.77 (dd, *J* = 6.5, 11.8Hz, 1H), 4.62 (dd, *J* = 6.2, 9.9Hz, 1H), 4.20 (t, *J* = 6.6 Hz, 1H), 4.08 (d, *J* = 16.1 Hz, 1H), 4.04-3.96 (m, 2H), 3.89 (d, *J* = 16.0 Hz, 1H), 3.78-3.71 (m, 2H), 3.68-3.56 (m, 30H), 3.52 (t, *J* = 5.0 Hz, 2H), 3.47 (t, *J* = 7.3 Hz, 2H), 3.41 (q, *J* = 5.1 Hz, 2H), 3.17-3.10 (m, 1H), 3.06-2.98 (m, 1H), 2.56-2.49 (m, 1H), 2.46-2.40 (m, 1H), 2.36 (s, 3H), 2.28-2.25 (m, 2H), 2.15 (t, *J* = 7.6 Hz, 2H), 1.66-1.53 (m, 4H), 1.36 (d, *J* = 7.1 Hz, 3H), 1.33-1.24 (m, 4H), 1.20 (d, *J* = 7.1 Hz, 3H), 1.15 (d, *J* = 7.2 Hz, 3H), 1.01 (t, *J* = 7.3 Hz, 3H).

**Example 53**

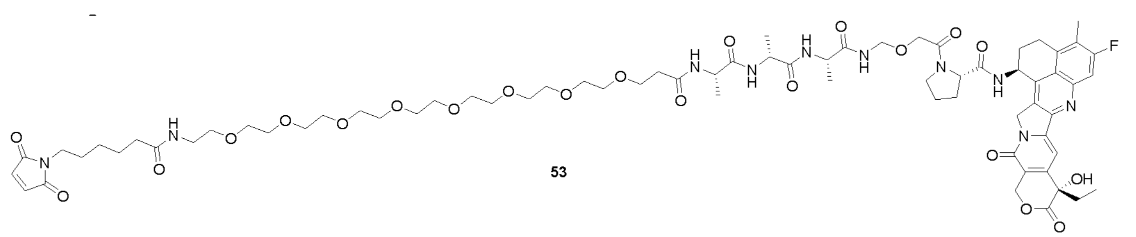

53

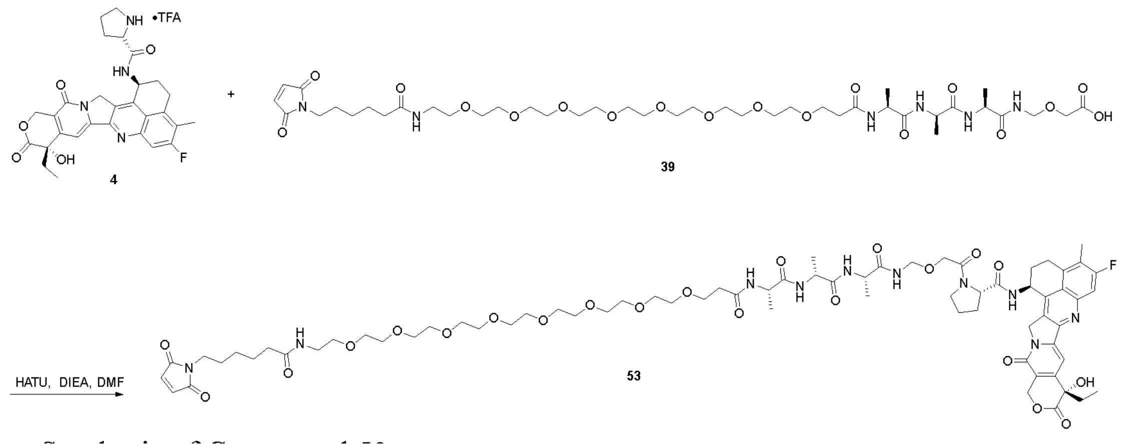


4 39 53

Synthesis of Compound 53:

Compound 4 (25mg, 39μmol, 1.0eq) and compound 39 (36mg, 39μmol, 1.0eq) were dissolved in DMF (0.5mL). HATU (22mg, 58μmol, 1.5eq) and DIEA (16μL, 97μmol, 2.5eq) were added, and stirred at room temperature for 2 hours. The reaction solution was directly purified by reversed-phase column chromatography (34% $CH_3CN$ in $H_2O$, 0.05% HCOOH) to obtain a yellow solid (17 mg, 30%). MS(ESI): m/z found Fragment 1, Positive = 591.2, Fragment 2, Positive = 859.4

$^1H$ NMR (400 MHz, $CDCl_3$): $\delta$ 8.17 (d, $J$ = 8.6 Hz, 1H), 7.67 - 7.62 (m, 2H), 7.62 - 7.50 (m, 4H), 7.36 (d, $J$ = 6.6 Hz, 1H), 6.68 (s, 2H), 6.52 (t, $J$ = 5.8 Hz, 1H), 5.71 (dd, $J$ = 15.1, 7.7 Hz, 2H), 5.58 (q, $J$ = 7.4 Hz, 1H), 5.27 (d, $J$ = 16.2 Hz, 1H), 5.12 (d, $J$ = 19.4 Hz, 1H), 4.68 (dd, $J$ = 8.3, 2.9 Hz, 1H), 4.28 (t, $J$ = 9.6 Hz, 1H), 4.23-4.15 (m, 2H), 4.13-4.04 (m, 4H), 3.88 (d, $J$ = 15.7 Hz, 1H), 3.74-3.57 (m, 30H), 3.53 (t, $J$ = 4.7 Hz, 2H), 3.49 (t, $J$ = 7.2 Hz, 2H), 3.42 (t, $J$ = 5.2 Hz, 2H), 3.33 (q, $J$ = 8.9 Hz, 1H), 3.25-3.17 (m, 1H), 3.12-3.05 (m, 1H), 2.47-2.31 (m, 8H), 2.16 (t, $J$ = 7.5 Hz, 2H), 1.91-1.86 (m, 3H), 1.67-1.54 (m, 4H), 1.35 (t, $J$ = 8.0 Hz, 6H), 1.32-1.25 (m, 3H), 1.19 (dd, $J$ = 7.0, 18.8 Hz, 1H), 1.05-0.99 (m, 6H).

## Example 54

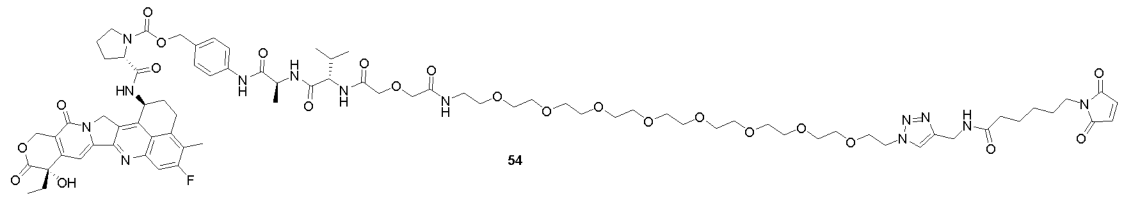

54

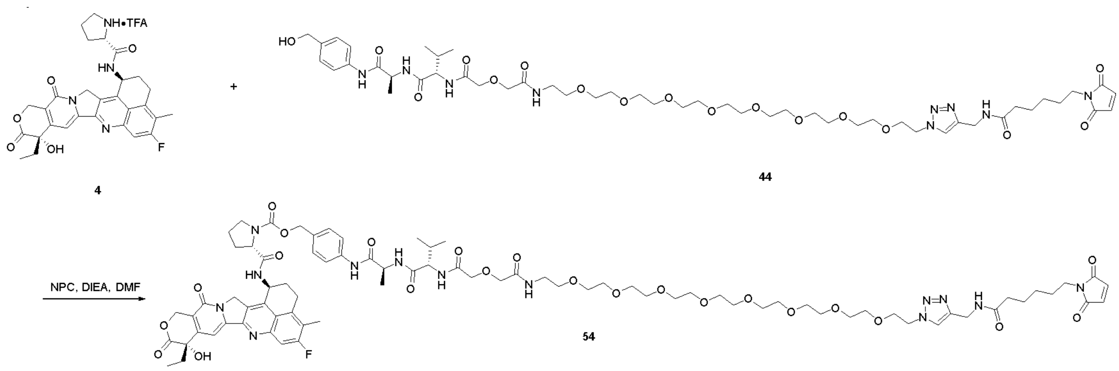


4 44 54

Synthesis of Compound 54

Compound 44 (27mg, 25μmol, 1.0eq) was dissolved in DMF (0.5mL). NPC (9.1mg, 30μmol, 1.2eq) and DIEA (8μL, 50μmol, 2.0eq) were added, and stirred at room temperature for 5 hours. Compound 4 (16.0 mg, 25μmol, 1.0eq) was

added to the reaction solution, and stirring was continued at room temperature for 2 hours. The reaction solution was directly purified by reversed-phase column chromatography (43% $CH_3CN$ in $H_2O$, 0.05% HCOOH) to obtain a white solid (15 mg, 37%). MS(ESI):m/z found$[M/2+1]^+$ = 819.0

**Example 55**

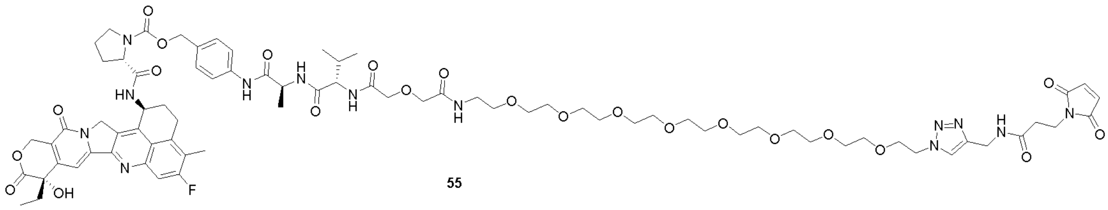

55

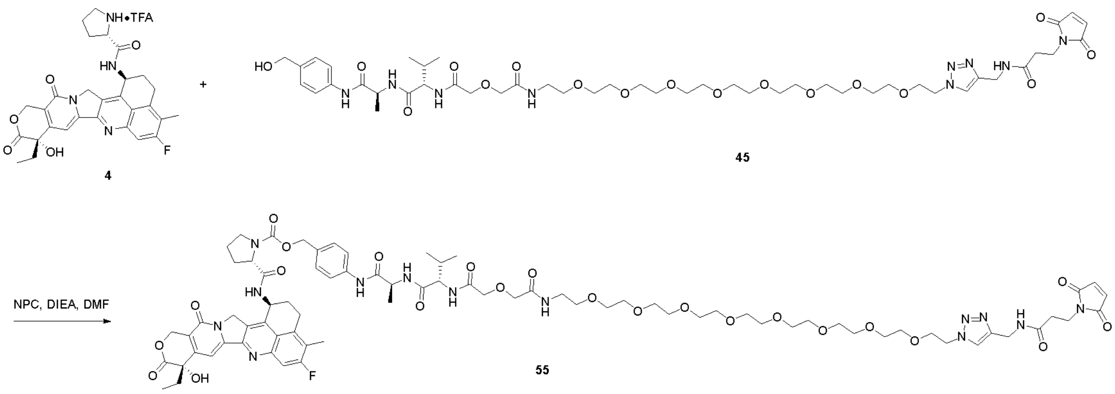


4

45

55

Synthesis of compound 55

Compound 45 (33mg, 32μmol, 1.0eq) was dissolved in DMF (0.5mL). NPC (11.6mg, 38μmol, 1.2eq) and DIEA (10.5μL, 64μmol, 2.0eq) were added, and stirred at room temperature for 5 hours. Compound 4 (20.6 mg, 32μmol, 1.0eq) was added to the reaction solution, and stirring was continued at room temperature for 2 hours. The reaction solution was directly purified by reverse phase column chromatography (38% $CH_3CN$ in $H_2O$, 0.05% HCOOH) to obtain a white solid (14 mg, 28%). MS(ESI):m/z found$[M/2+1]^+$ = 798.0

**Example 56**

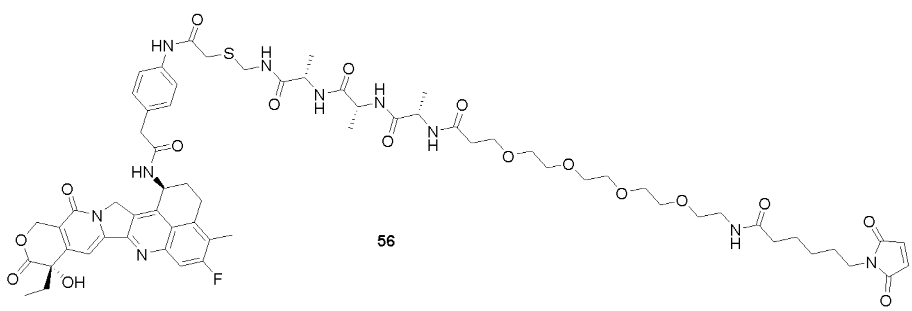

56

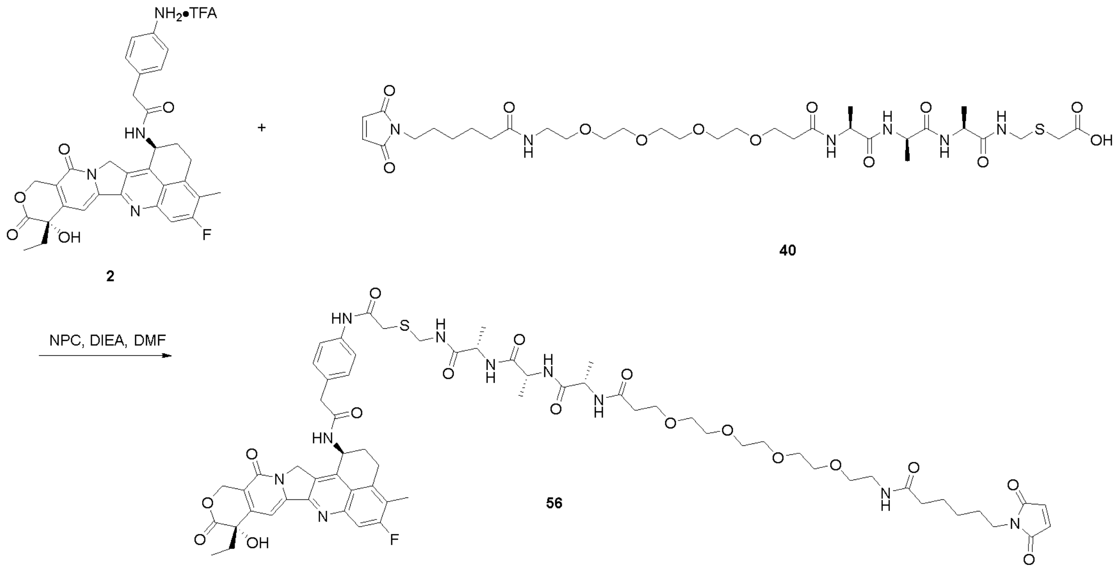


2 40 56

Synthesis of compound 56

Compound 2 (38mg, 56μmol, 1.0eq) and compound 40 (43mg, 56μmol, 1.0eq) were dissolved in DMF (1mL), and HATU (32mg, 83μmol, 1.5eq) and DIEA (23μL, 139μmol, 2.5eq) were added, and stirred at room temperature for 3 hours. Purified by HPLC to obtain a white solid (14 mg, 19%). MS(ESI):m/z found$[M/2+1]^+$ = 663.6.

$^1H$ NMR (400 MHz, $CDCl_3$): $\delta$ 9.44 (s, 1H), 8.15 (t, *J* = 5.2 Hz, 1H), 7.64 (d, *J* = 7.7 Hz, 1H), 7.60 (s, 2H), 7.56-7.54 (m, 2H), 7.53 (s, 1H), 7.48 (d, *J* = 5.2 Hz, 1H), 7.35 (s, 1H), 7.33 (s, 1H), 7.19 (d, *J* = 7.7 Hz, 1H), 6.79 (s, 1H), 6.67 (s, 2H), 6.44 (t, *J* = 6.0 Hz, 1H), 5.72 (d, *J* = 16.2 Hz, 1H), 5.67-5.61 (m, 1H), 5.33 (d, *J* = 16.2 Hz, 1H), 5.31 (d, *J* = 19.0 Hz, 1H), 5.12 (d, *J* = 19.0 Hz, 1H), 4.42 (dd, *J* = 6.6, 13.5Hz, 1H), 4.26 (dd, *J* = 6.0, 13.6Hz, 1H), 4.15 (t, *J* = 6.3 Hz, 2H), 4.05 (t, *J* = 6.3 Hz, 2H), 3.81-3.73 (m, 2H), 3.71-3.57 (m, 15H), 3.52 (t, *J* = 5.1 Hz, 2H), 3.46 (t, *J* = 7.2 Hz, 2H), 3.39 (q, *J* = 7.2 Hz, 2H), 3.23-3.18 (m, 1H), 3.10-3.05 (m, 1H), 3.44 (q, *J* = 5.4 Hz, 2H), 3.36 (s, 3H), 2.29-2.22 (m, 1H), 2.13 (t, *J* = 7.5 Hz, 2H), 1.91-1.86 (m, 3H), 1.62-1.53 (m, 4H), 1.33 (s, 3H), 1.31 (s, 3H), 1.29-1.25 (m, 2H), 1.11 (d, *J* = 7.2 Hz, 3H), 1.01 (t, *J* = 7.4 Hz, 3H)

**Example 57**

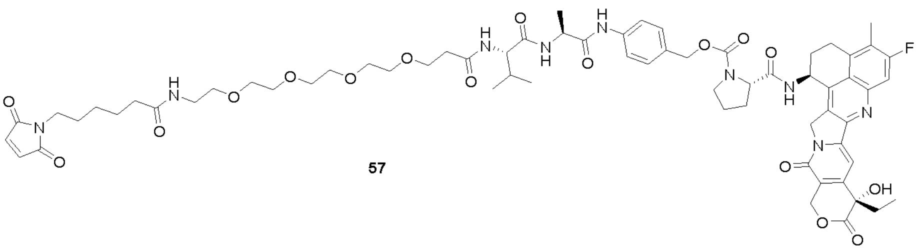

57

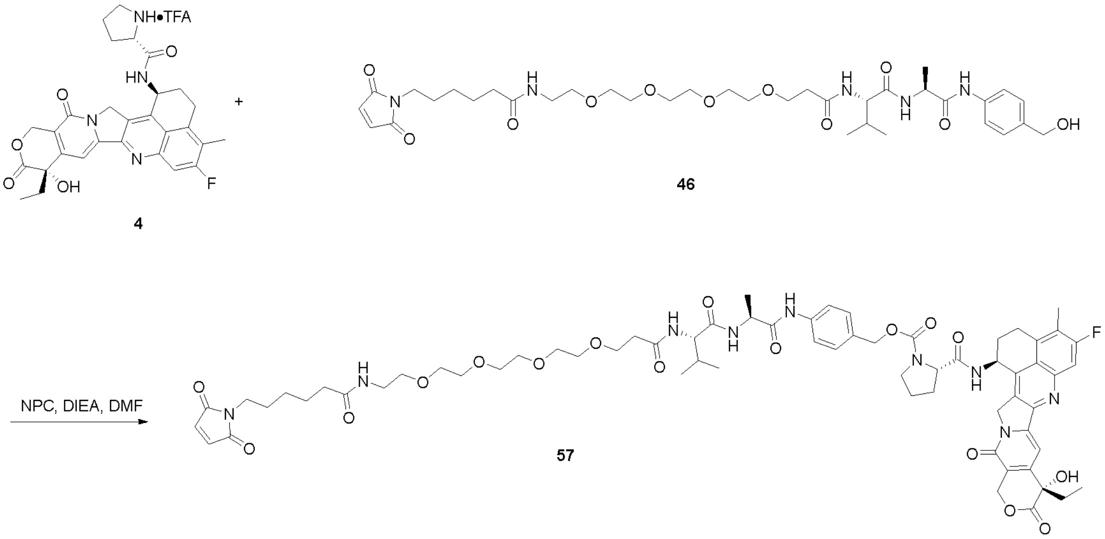


Synthesis of compound 57

Compound 46 (31mg, 42μmol, 1.0eq) was dissolved in DMF (0.5mL),.NPC (15.2mg, 50μmol, 1.2eq) and DIEA (14μL, 84μmol, 2.0eq) were added, and stirred at room temperature for 5 hours. Compound 4 (27 mg, 42μmol, 1.0eq) was added to the reaction solution, and stirring was continued at room temperature for 2 hours. The reaction solution was directly purified by reversed-phase column chromatography (41% $CH_3CN$ in $H_2O$, 0.05% HCOOH) to obtain a yellow solid (27 mg, 50%). MS(ESI):m/z found$[M/2+1]^+$ = 646.8

$^1H$ NMR (400 MHz, *DMSO-d$_6$*): $\delta$ 9.86 (d, *J* = 11.3 Hz, 1H), 8.60 (t, *J* = 9.2 Hz, 1H), 8.41 (d, *J* = 7.5 Hz, 1H), 7.99 (d, *J* = 8.0 Hz, 1H), 7.79 (m, 2H), 7.64 (dd, *J* = 17.7, 8.3 Hz, 2H), 7.28 m, 4H), 6.51 (s, 1H), 5.47-5.37 (m, 2H), 5.19 (d, *J* = 4.6 Hz, 1H), 5.07-4.91 (m, 3H), 4.48-4.36 (m, 1H), 4.20 (q, *J* = 7.3, 5.4 Hz, 1H), 4.12 (t, *J* = 7.7 Hz, 1H), 4.04 (dd, *J* = 8.9, 5.1 Hz, 1H), 3.62-3.53 (m, 4H), 3.45 (m, 14H), 3.15 (q, *J* = 6.0 Hz, 4H), 2.92 (d, *J* = 9.8 Hz, 1H), 2.80-2.71 (m, 1H), 2.45 (d, *J* = 5.9 Hz, 2H), 2.41-2.30 (m, 4H), 2.18-2.09 (m, 1H), 2.01 (t, *J* = 7.4 Hz, 2H), 1.96-1.89 (m, 3H), 1.82 (dt, *J* = 14.1, 7.5 Hz, 4H), 1.48-1.39 (m, 4H), 1.30 (t, *J* = 7.0 Hz, 3H), 1.20-1.11 (m, 2H), 0.96 (t, *J* = 7.1 Hz, 3H), 0.85 (t, *J* = 7.0 Hz, 6H).

**Example 58**

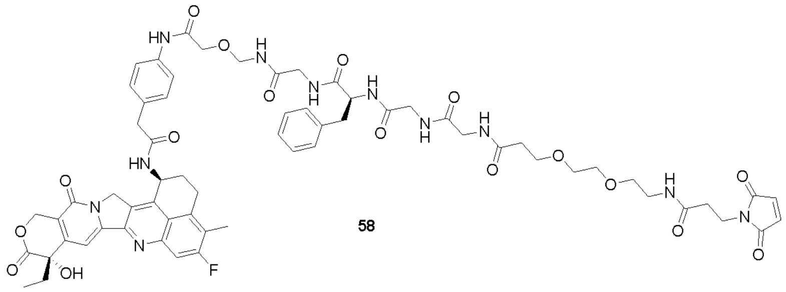

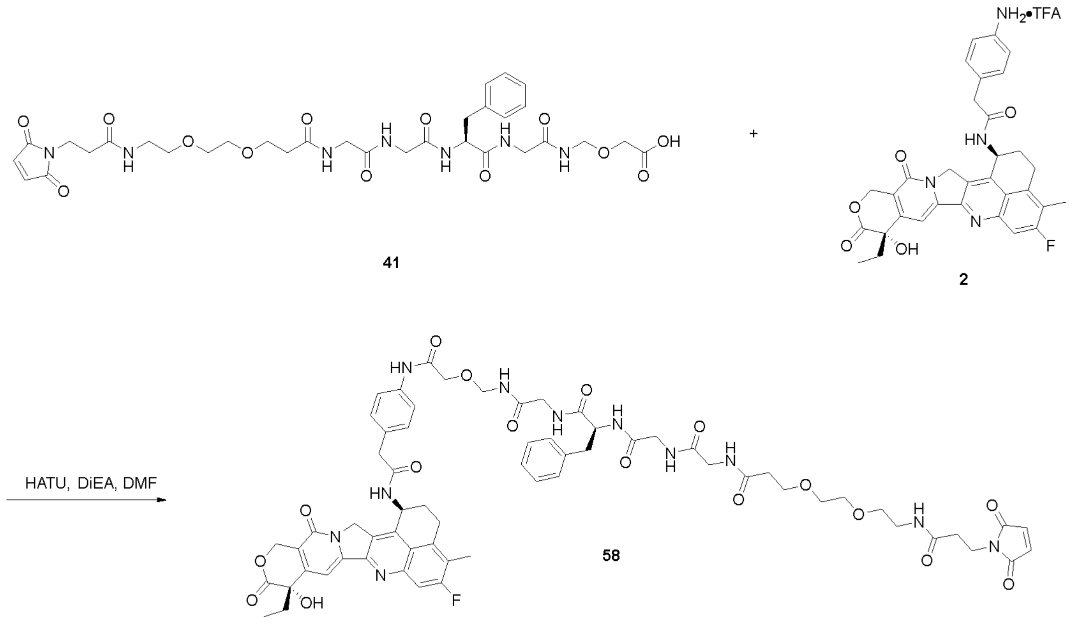


41

2

58

Synthesis of Compound 58

Compound 2 (30mg, 44μmol, 1.0eq) and compound 41 (32mg, 44μmol, 1.0eq) were dissolved in DMF (1mL), and HATU (20.6mg, 53μmol, 1.2eq) and DIEA (16μL, 97μmol, 2.2eq), and stirred at room temperature for 2 hours. Purified by HPLC to obtain a white solid (28 mg, 48%). MS(ESI):m/z found$[M/2+1]^+$ = 642.8.

$^1$H NMR (400 MHz, *DMSO-d$_6$*): $\delta$ 9.71 (s, 1H), 8.74 (t, *J* = 6.8 Hz, 1H), 8.66 (d, *J* = 8.6 Hz, 1H), 8.38 (t, *J* = 5.7 Hz, 1H), 8.16 (t, *J* = 9.1 Hz, 2H), 8.02 (q, *J* = 6.7 Hz, 2H), 7.80 (d, *J* = 10.9 Hz, 1H), 7.59 (d, *J* = 8.3 Hz, 2H), 7.30 (s, 1H), 7.25-7.22 (m, 5H), 7.20 (s, 1H), 7.18-7.13 (m, 1H), 6.99 (s, 2H), 6.53 (s, 1H), 5.53 (dt, *J* = 9.1, 4.8 Hz, 1H), 5.43 (s, 2H), 5.25 (d, *J* = 18.8 Hz, 1H), 5.16 (d, *J* = 18.9 Hz, 1H), 4.66 (d, *J* = 6.5 Hz, 2H), 4.52-4.47 (m, 1H), 4.03 (s, 2H), 3.80-3.66 (m, 6H), 3.59 (m, 5H), 3.55 (s, 1H), 3.45 (s, 7H), 3.19-3.15 (m, 2H), 3.12 (q, *J* = 5.7 Hz, 2H), 3.06 (dd, *J* = 13.6, 4.3 Hz, 1H), 2.81 (dd, *J* = 13.8, 9.6 Hz, 1H), 2.40 (s, 3H), 2.38 (t, *J* = 6.5 Hz, 2H), 2.31 (t, *J* = 7.2 Hz, 2H), 2.13-2.09 (m, 1H), 1.86 (tq, *J* = 14.1, 7.1 Hz, 2H), 0.87 (t, *J* = 7.3 Hz, 3H).

## Example 59

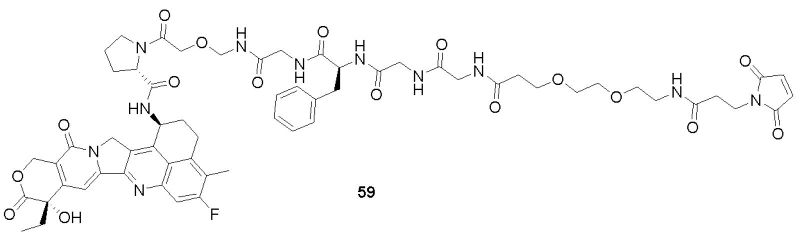

59

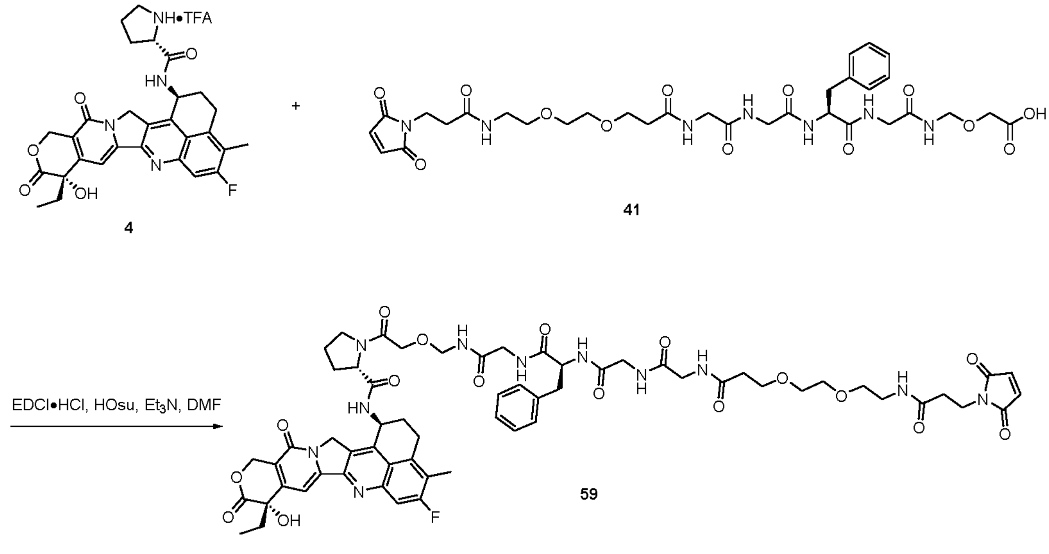


4 + 41 → 59

Synthesis of Compound 59

Compound 41 (23 mg, 31μmol, 1.0eq) was dissolved in DMF (1mL). EDCI·HCl (7 mg, 37μmol, 1.2eq) and HOSu (4.3 mg, 37μmol, 1.2eq) were added, and stirred at room temperature for 4 hours. Triethylamine (9μL, 68μmol, 2.2eq) and compound 4 (20mg, 31μmol, 1.0eq) were added to the reaction solution, and stirred at room temperature for 1.5 hours. Purified by reverse phase column chromatography (37% $CH_3CN$ in $H_2O$, 0.05% HCOOH) to obtain a white solid (23 mg, 58%). MS(ESI):m/z found$[M/2+1]^+$ = 643.0.

$^1H$ NMR (400 MHz, *DMSO-d*$_6$): $\delta$ 8.48 (m, 2H), 8.29 (t, *J* = 5.8 Hz, 1H), 8.21-8.11 (m, 2H), 8.06-7.98 (m, 2H), 7.78 (d, *J* = 11.0 Hz, 1H), 7.30 (s, 1H), 7.26-7.17 (m, 5H), 7.00 (s, 2H), 6.53 (s, 1H), 5.53 (dt, *J* = 9.3, 5.1 Hz, 1H), 5.43 (s, 2H), 5.28-5.17 (m, 2H), 4.48 (td, *J* = 8.9, 4.5 Hz, 1H), 4.30 (d, *J* = 6.5 Hz, 2H), 4.01 (s, 2H), 3.77-3.66 (m, 4H), 3.59 (m, 7H), 3.46 (s, 6H), 3.15 (m, 5H), 3.04 (dd, *J* = 13.8, 4.4 Hz, 1H), 2.78 (dd, *J* = 13.9, 9.7 Hz, 1H), 2.43-2.37 (m, 6H), 2.32 (t, *J* = 7.3 Hz, 2H), 2.19-2.05 (m, 3H), 1.88 (td, *J* = 14.2, 5.8 Hz, 5H), 0.88 (t, *J* = 7.3 Hz, 3H).

**Example 60**

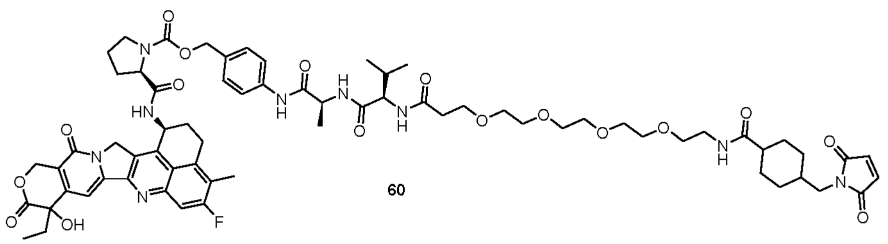

60

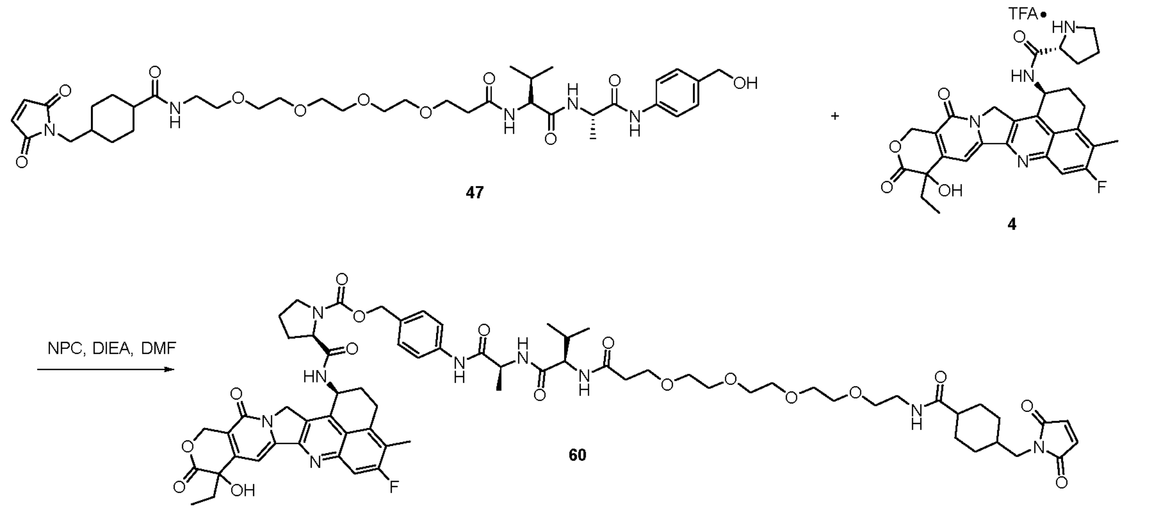


47

4

60

Synthesis of Compound 60

Compound 47 (30mg, 39μmol, 1.0eq) was dissolved in DMF (0.5mL). NPC (14.4mg, 47μmol, 1.2eq) and DIEA (13μL, 79μmol, 2.0eq) were added, and stirred at room temperature for 5 hours. Compound 4 (25.5 mg, 39μmol, 1.0eq) was added to the reaction solution, then stirring was continued at room temperature for 2 hours. The reaction solution was directly purified by reversed-phase column chromatography (45% $CH_3CN$ in $H_2O$, 0.05% HCOOH) to obtain a white solid (20 mg, 38%). MS (ESI): m/z found $[M/2+1]^+$=660.0.

**Example 61**

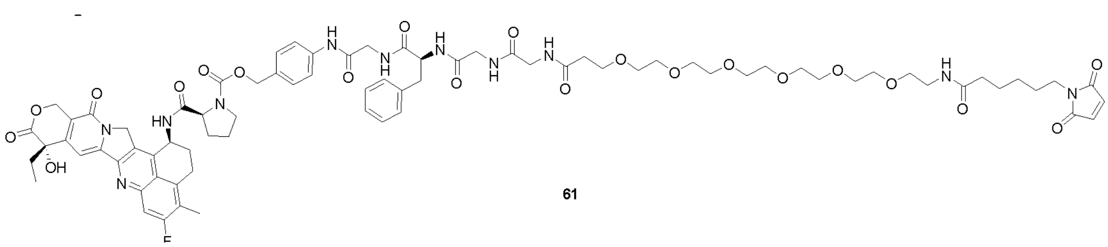

61

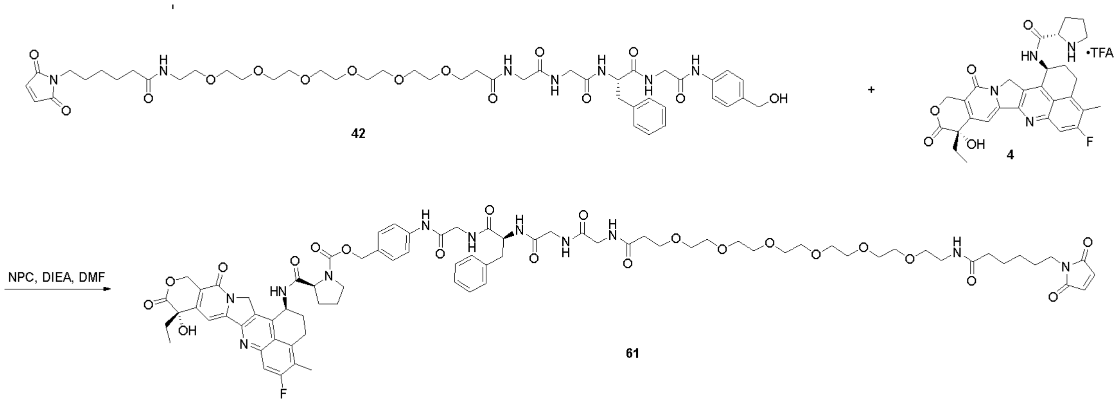


42

4

61

Synthesis of Compound 61

Compound 42 (50mg, 52μmol, 1.0eq) was dissolved in DMF (0.5mL). NPC (19mg, 62μmol, 1.2eq) and DIEA (17μL, 103μmol, 2.0eq) were added, and stirred at room temperature for 4 hours. Compound 4 (33 mg, 52μmol, 1.0eq) was added to the reaction solution, and stirring was continued at room temperature for 2 hours. The reaction solution was directly purified by reversed-phase column chromatography (41% $CH_3CN$ in $H_2O$, 0.05% HCOOH) to obtain a white solid

(25 mg, 32%). MS(ESI):m/z found$[M/2+1]^+$ = 765.0.

$^1H$ NMR (400 MHz, *DMSO-$d_6$*): $\delta$ 9.92 (m, 1H), 8.62-8.57 (m, 1H), 8.42-8.38 (m, 1H), 8.19-8.13 (m, 2H), 8.05-8.00 (m, 1H), 7.81-7.75 (m, 2H), 7.67-7.61 (m, 2H), 7.31-7.16 (m, 8H), 7.00 (s, 1H), 6.52 (s, 1H), 4.52-4.48 (m, 1H), 4.21-4.16 (m, 1H), 3.95-3.85 (m, 2H), 3.80-3.72 (m, 3H), 3.70-3.65 (m, 4H), 3.63-3.23 (m, 31H), 3.20-3.10 (m, 4H), 3.10-3.04 (m, 2H), 2.98-2.92 (m, 1H), 2.87-2.78 (m, 1H), 2.41-2.36 (m, 5H), 2.18-2.09 (m, 2H), 2.03 (t, *J* = 7.6 Hz, 2H), 2.00-1.82 (m, 7H), 1.84 (t, *J* = 6.4 Hz, 3H), 1.48-1.44 (m, 2H), 0.84 (t, *J* = 7.2 Hz, 3H).

**Example 62**

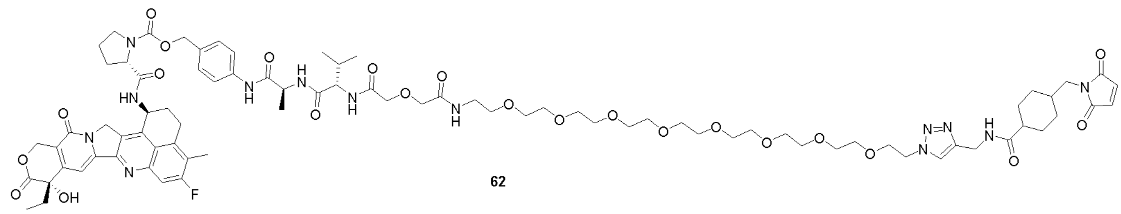

62

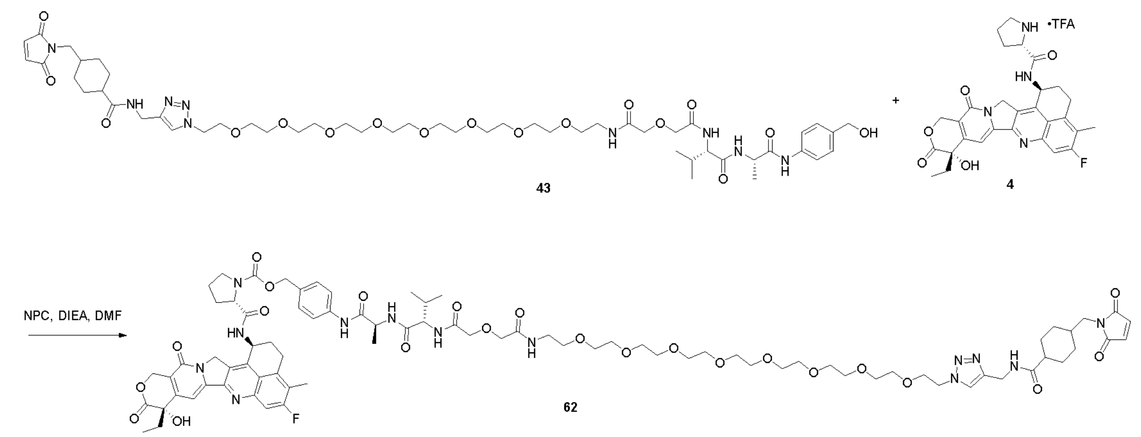


43

4

62

Synthesis of compound 62

Compound 43 (20mg, 18μmol, 1.0eq) was dissolved in DMF (0.5mL). NPC (7mg, 22μmol, 1.2eq) and DIEA (6μL, 36μmol, 2.0eq) were added, and stirred at room temperature for 5 hours. Compound 4 (12 mg, 18μmol, 1.0eq) was added to the reaction solution, and stirring was continued at room temperature for 2 hours. The reaction solution was directly purified by reversed-phase column chromatography (41% $CH_3CN$ in $H_2O$, 0.05% HCOOH) to obtain a white solid (9 mg, 30%). MS(ESI):m/z found$[M/2+1]^+$ = 832.0.

**Example 63**

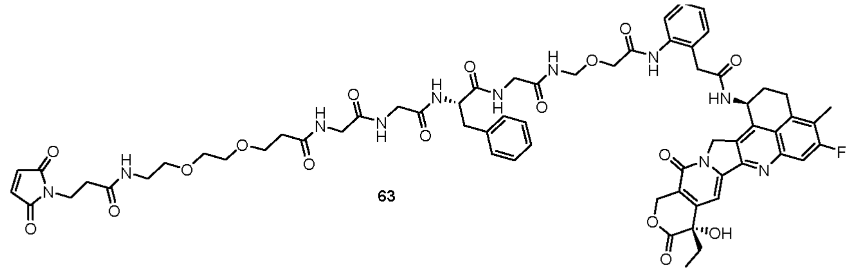

63

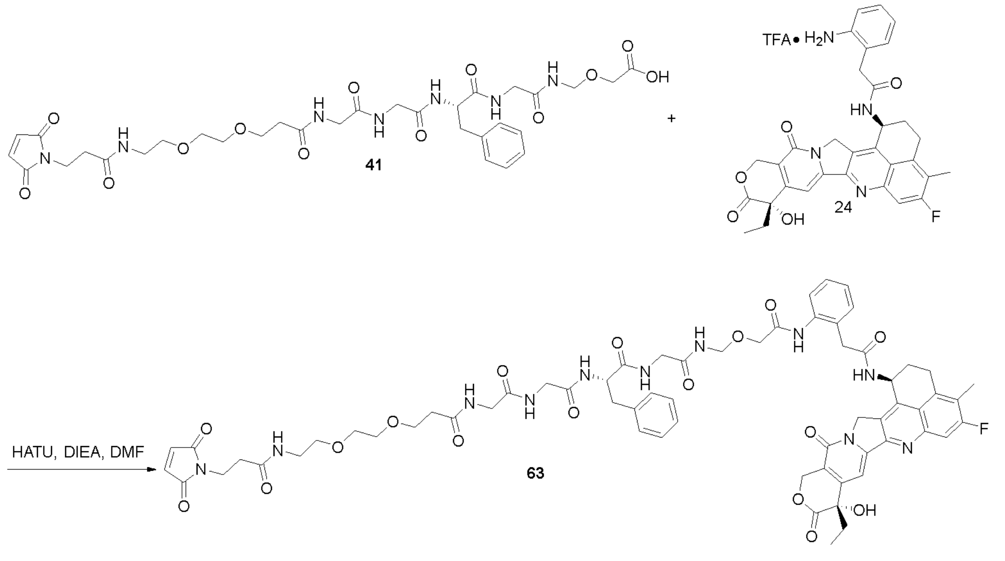


41

24

63

Synthesis of Compound 63:

Compound 24 (9 mg, 13μmol, 1.0eq) and compound 41 (9.6 mg, 13μmol, 1.0eq) were dissolved in DMF (0.5 mL). HATU (6.0 mg, 16μmol, 1.2eq) and DIEA (5μL, 33μmol, 2.5 eq) were added, and stirred for 3 hours at room temperature. The reaction solution was directly purified by reversed-phase column chromatography (41% $CH_3CN$ in $H_2O$, 0.05% HCOOH) to obtain a yellow solid (14 mg, 83%). MS(ESI): m/z found Fragment 1, Positive = 627.2, Fragment 2, Positive = 658.2

$^1$H NMR (400 MHz, *DMSO-d$_6$*): $\delta$ 10.14 (s, 1H), 8.86 (d, *J* = 8.7 Hz, 1H), 8.67 (t, *J* = 6.7 Hz, 1H), 8.35 (t, *J* = 5.9 Hz, 1H), 8.21-8.09 (m, 2H), 8.00 (t, *J* = 5.7 Hz, 2H), 7.80 (d, *J* = 10.9 Hz, 1H), 7.65 (d, *J* = 8.0 Hz, 1H), 7.30 (s, 1H), 7.27-7.18 (m, 6H), 7.15 (dt, *J* = 6.0, 2.8 Hz, 1H), 7.10 (t, *J* = 7.4 Hz, 1H), 6.97 (s, 2H), 6.52 (s, 1H), 5.53 (dt, *J* = 9.0, 4.6 Hz, 1H), 5.42 (s, 2H), 5.26 (d, *J* = 18.9 Hz, 1H), 5.12 (d, *J* = 19.0 Hz, 1H), 4.72 (d, *J* = 6.7 Hz, 2H), 4.48 (td, *J* = 8.6, 4.3 Hz, 1H), 4.07 (s, 2H), 3.75 (t, *J* = 5.6 Hz, 2H), 3.67 (d, *J* = 5.6 Hz, 2H), 3.60-3.53 (m, 6H), 3.43 (s, 4H), 3.31 (s, 2H), 3.18-3.09 (m, 4H), 3.03 (dd, *J* = 13.8, 4.4 Hz, 1H), 2.77 (dd, *J* = 13.8, 9.7 Hz, 1H), 2.40 (s, 3H), 2.36 (t, *J* = 6.5 Hz, 2H), 2.30 (t, *J* = 7.3 Hz, 2H), 2.19-2.03 (m, 2H), 1.90-1.79 (m, 2H), 0.85 (t, *J* = 7.3 Hz, 3H).

**Example 64**

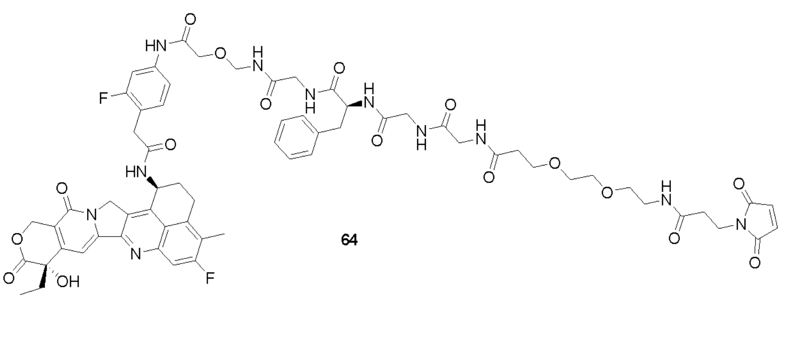

64

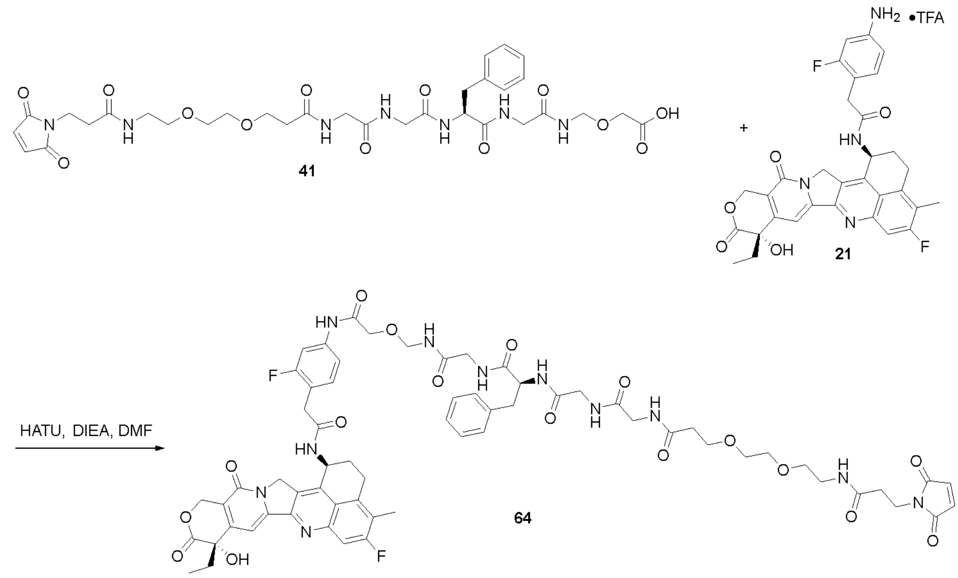


41

21

64

Synthesis of Compound 64:

Compound 21 (12mg, 17μmol, 1.0eq) and compound 41 (19mg, 26μmol, 1.5eq) were dissolved in DMF (0.5mL). HATU (9.8mg, 26μmol, 1.5eq) and DIEA (7μL, 43μmol, 2.5 eq) were added, and stirred at 50° C. for 3 hours. The reaction solution was directly purified by reverse phase column chromatography (40% $CH_3CN$ in $H_2O$, 0.05% HCOOH) to obtain a yellow solid (14 mg, 83%). MS(ESI):m/z found[M/2+1] = 615.8

**Example 65**

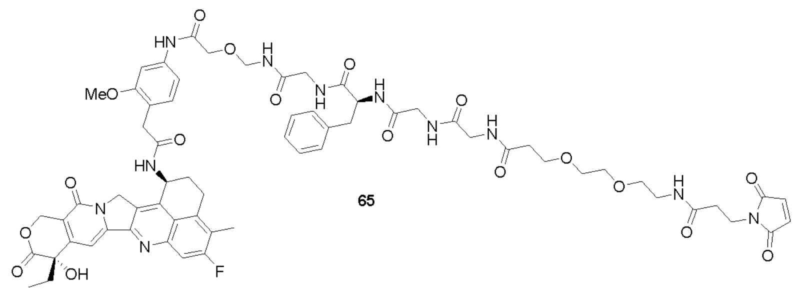

65

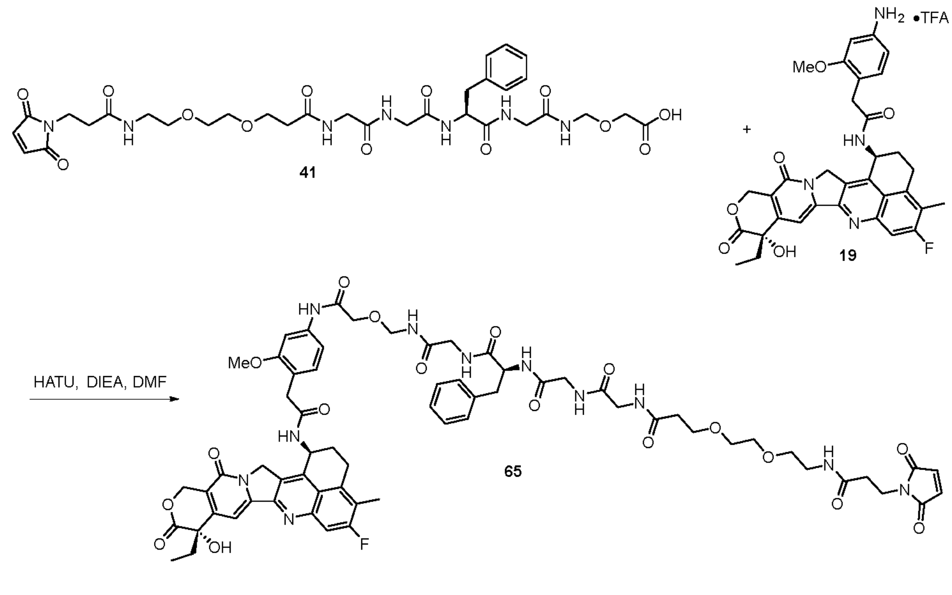


41

19

65

Synthesis of compound 65:

Compound 19 (11.5mg, 16μmol, 1.0eq) and compound 41 (18mg, 24μmol, 1.5eq) were dissolved in DMF (0.5mL). HATU (9.2mg, 24μmol, 1.5eq) and DIEA (7μL, 43μmol, 2.5eq) were added, and stirred at room temperature for 3 hours. The reaction solution was directly purified by reversed-phase column chromatography (40% $CH_3CN$ in $H_2O$, 0.05% HCOOH) to obtain a yellow solid (12 mg, 57%). MS(ESI):m/z found[M/2+1] = 657.2

$^1H$ NMR (400 MHz, *DMSO-d$_6$*): $\delta$ 9.73 (s, 1H), 8.80 (t, *J*=6.6 Hz, 1H), 8.56 (d, *J* = 8.7 Hz, 1H), 8.48 (s, 1H), 8.44 (t, *J* = 5.8 Hz, 1H), 8.27-8.18 (m, 2H), 8.08 (t, *J* = 5.7 Hz, 1H), 8.04 (t, *J* = 5.7 Hz, 1H), 7.81 (d, *J* = 11.0 Hz, 1H), 7.39 (d, *J* = 2.0 Hz, 1H), 7.33 (s, 1H), 7.27-7.21 (m, 5H), 7.18 (dq, *J* = 5.8, 2.9 Hz, 1H), 7.11 (d, *J* = 8.3 Hz, 1H), 7.00 (s, 1H), 6.56 (s, 1H), 5.55 (dt, *J* = 9.0, 4.8 Hz, 1H), 5.44 (s, 2H), 5.23 (q, *J* = 19.0 Hz, 2H), 4.68 (d, *J* = 6.7 Hz, 2H), 4.50 (dt, *J* = 8.7, 4.3 Hz, 1H), 4.05 (s, 2H), 3.80-3.73 (m, 2H), 3.70 (d, *J* = 5.5 Hz, 2H), 3.65 (s, 2H), 3.61-3.56 (m, 6H), 3.50-3.45 (m, 5H), 3.19 (t, *J* = 6.5 Hz, 2H), 3.14 (t, *J* = 5.7 Hz, 2H), 3.11-3.04 (m, 2H), 2.44-2.36 (m, 5H), 2.32 (t, *J* = 7.3 Hz, 2H), 2.15 (tt, *J* = 12.9, 7.5 Hz, 2H), 2.04-1.97 (m, 1H), 1.87 (dp, *J* = 21.4, 7.1 Hz, 3H), 1.24 (d, *J* = 3.2 Hz, 2H), 0.88 (t, *J* = 7.3 Hz, 3H).

## Example 66

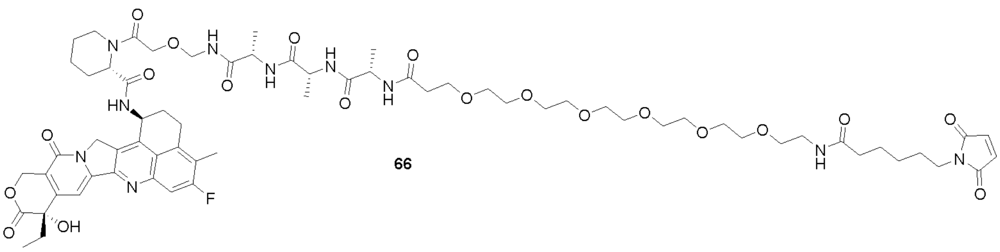

66

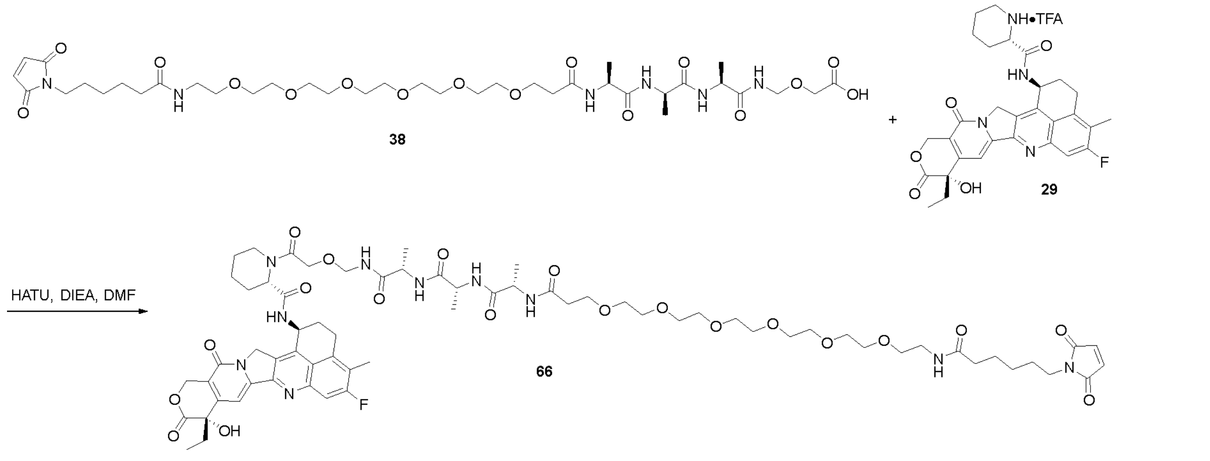


38 + 29 → 66

Synthesis of compound 66:

Compound 29 (18mg, 27μmol, 1.0eq) and compound 38 (23mg, 27μmol, 1.0eq) were dissolved in DMF (0.5mL). HATU (12mg, 33μmol, 1.2eq) and DIEA (11μL, 68μmol, 2.5eq ) were added, and stirred at room temperature for 2 hours. The reaction solution was directly purified by reverse phase column chromatography (40% $CH_3CN$ in $H_2O$, 0.05% HCOOH) to obtain a yellow solid (18 mg, 48%). MS(ESI): m/z found Fragment 1, Positive=605.2 Fragment 2, Positive=711.4

$^1$H NMR (400 MHz, *DMSO-d$_6$*): $\delta$ 8.60 (t, *J* = 6.6 Hz, 1H), 8.45 (s, 1H), 8.15 (dd, *J* = 15.4, 6.9 Hz, 2H), 8.03 (d, *J*= 7.3 Hz, 1H), 7.83 (t, *J* = 5.7 Hz, 1H), 7.79 (d, *J* = 10.6 Hz, 1H), 7.31 (d, *J* = 6.7 Hz, 1H), 7.01 (s, 2H), 6.54 (s, 1H), 5.61 (q, *J* = 6.5 Hz, 1H), 5.43 (s, 2H), 5.18-5.09 (m, 2H), 4.61-4.47 (m, 2H), 4.22-4.16 (m, 4H), 4.08 (td, *J* = 14.4, 7.0 Hz, 2H), 3.61-3.54 (m, 4H), 3.49 (dd, *J* = 8.5, 4.8 Hz, 23H), 3.23-3.11 (m, 6H), 2.40-2.36 (m, 5H), 2.33 (q, *J* = 8.1, 7.2 Hz, 1H), 2.21 (d, *J* = 13.4 Hz, 1H), 2.16 (q, *J* = 6.9 Hz, 2H), 2.03 (t, *J* = 7.4 Hz, 2H), 1.87 (dp, *J* = 21.3, 7.1 Hz, 2H), 1.63 (d, *J* = 12.2 Hz, 2H), 1.20-1.14 (m, 6H), 1.17 (m, 11H), 0.88 (t, *J* = 7.3 Hz, 3H).

## Example 67

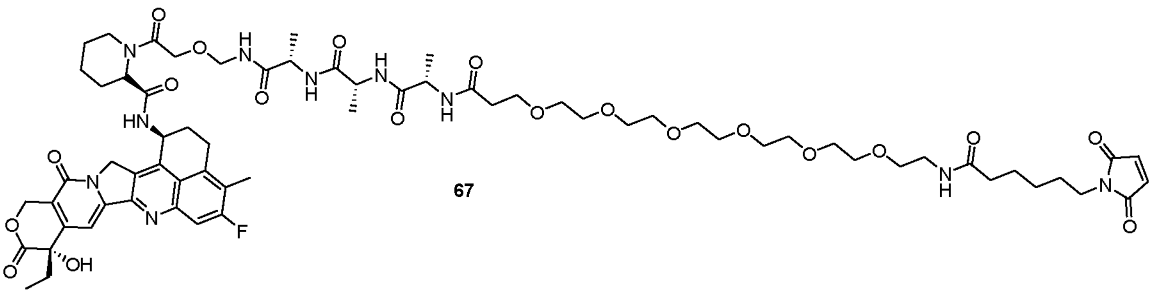

67

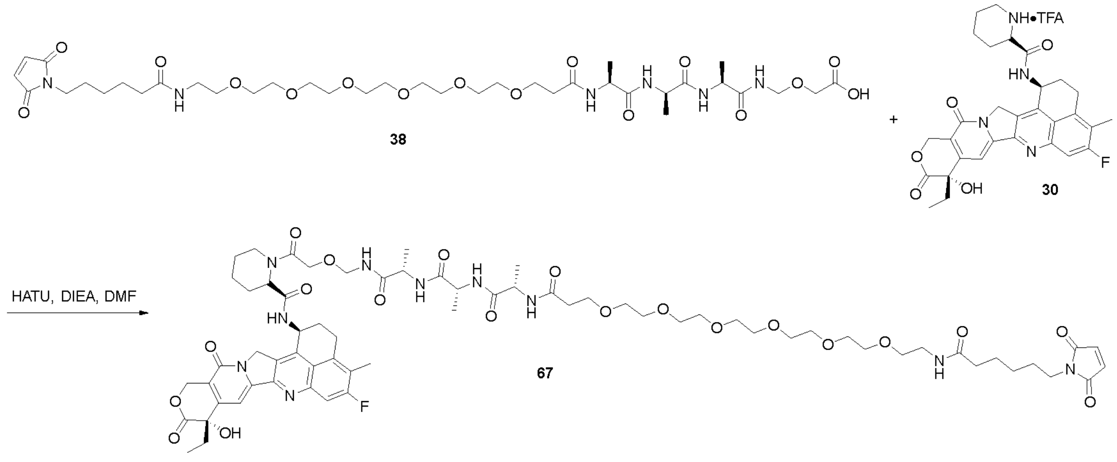

38 + 30 → 67

Synthesis of compound 67:

Compound 30 (22mg, 33μmol, 1.0eq) and compound 38 (28mg, 33μmol, 1.0eq) were dissolved in DMF (0.5mL). HATU (15mg, 40μmol, 1.2eq) and DIEA (14μL, 83μmol, 2.5eq ) were added, and stirred at room temperature for 2 hours. The reaction solution was directly purified by reversed-phase column chromatography (40% $CH_3CN$ in $H_2O$, 0.05% HCOOH) to obtain a yellow solid (19 mg, 42%). MS(ESI): m/z found Fragment 1, Positive=605.2 Fragment 2, Positive=711.4

## Example 68

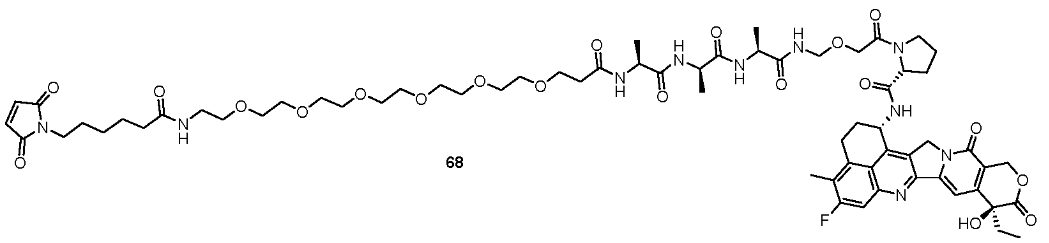
68

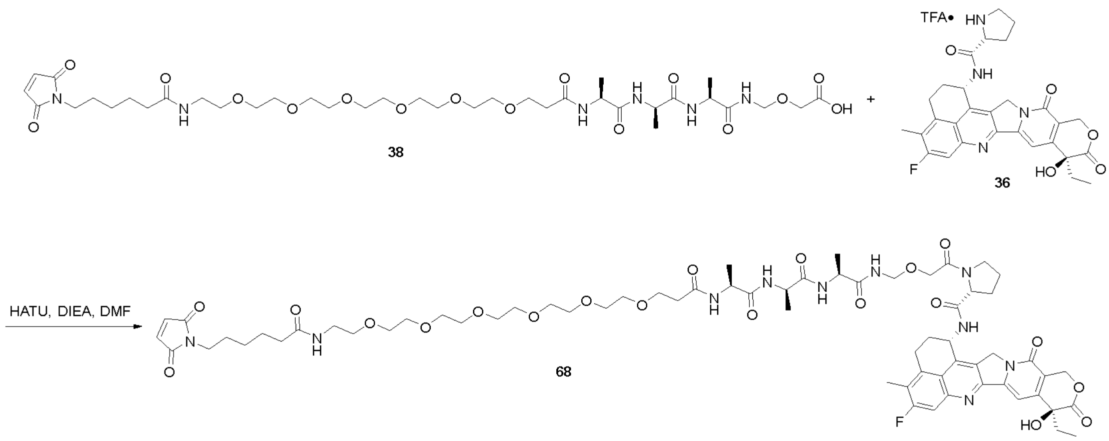

38 + 36 → 68

Synthesis of Compound 68:

Compound 36 (19mg, 29μmol, 1.0eq) and compound 38 (25mg, 29μmol, 1.0eq) were dissolved in DMF (0.5mL). HATU (13mg, 35μmol, 1.2eq) and DIEA (12μL, 73μmol, 2.5eq) were added, and stirred at room temperature for 2 hours. The reaction solution was directly purified by reverse phase column chromatography (38% $CH_3CN$ in $H_2O$, 0.05% HCOOH) to obtain a yellow solid (18 mg, 47%). MS(ESI): m/z found Fragment 1, Positive=519.2 Fragment 2, Posi-

tive=711.4

$^1H$ NMR (400 MHz, DMSO-$d_6$): $\delta$ 8.62 (t, *J* = 6.8 Hz, 1H), 8.48 (d, *J* = 8.9 Hz, 1H), 8.13 (dd, *J*= 7.0, 4.8 Hz, 2H), 8.00 (d, *J*= 7.3 Hz, 1H), 7.86-7.78 (m, 2H), 7.32 (s, 1H), 7.01 (s, 2H), 6.54 (s, 1H), 5.58 (dt, *J* = 9.0, 4.6 Hz, 1H), 5.43 (s, 2H), 5.22 (q, *J* = 18.8 Hz, 2H), 4.56-4.47 (m, 2H), 4.24 (dd, *J* = 8.4, 4.4 Hz, 1H), 4.19 (pd, *J* = 7.2, 2.8 Hz, 2H), 4.06 (qd, *J* = 14.7, 7.9 Hz, 3H), 3.60-3.54 (m, 2H), 3.52-3.44 (m, 22H), 3.39-3.36 (m, 6H), 3.17 (q, *J*= 5.9 Hz, 3H), 2.40-2.36 (m, 5H), 2.36-2.30 (m, 1H), 2.13-2.06 (m, 2H), 2.03 (t, *J* = 7.4 Hz, 2H), 1.93 (td, *J* = 11.0, 5.2 Hz, 1H), 1.87 (dq, *J* = 10.3, 6.8 Hz, 3H), 1.47 (q, *J* = 7.5 Hz, 4H), 1.17 (q, *J*= 6.8 Hz, 9H), 1.12 (d, *J*= 7.3 Hz, 3H), 0.91-0.86 (m, 3H).

**Example 69**

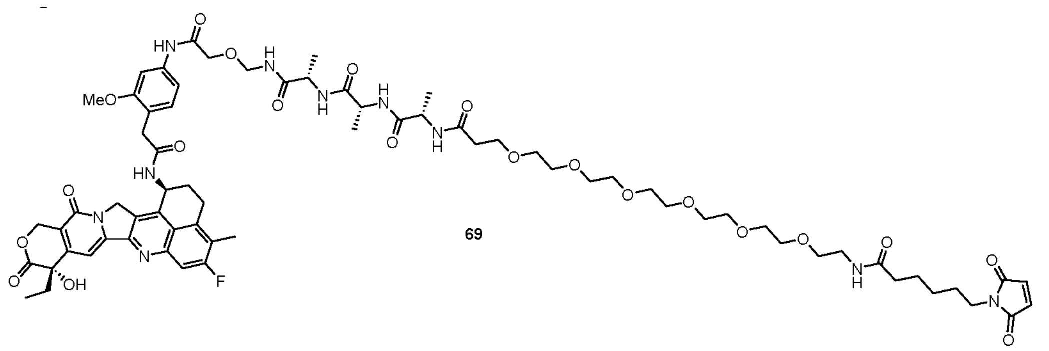

69

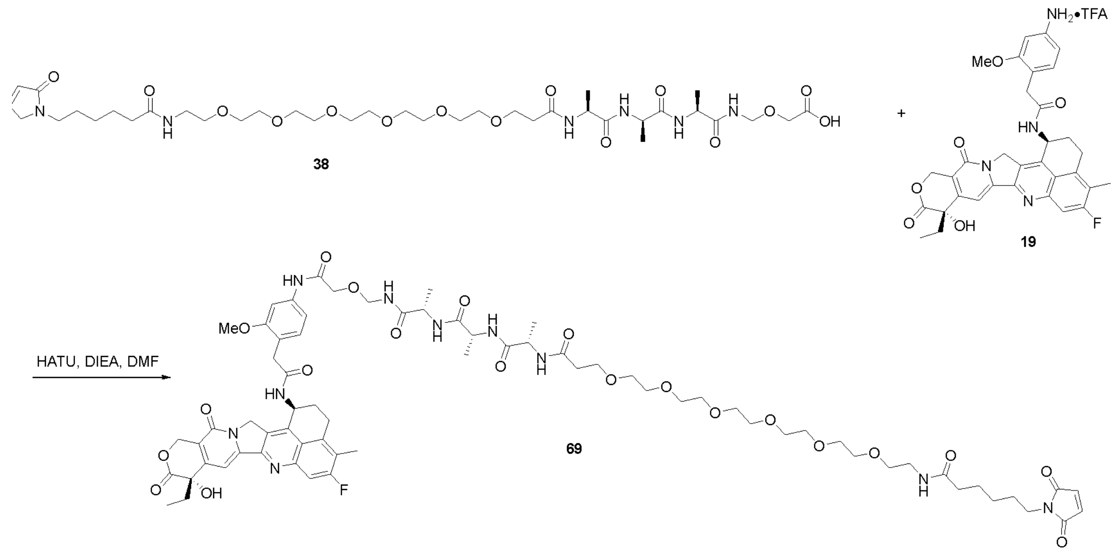


38

19

69

Synthesis of Compound 69:

Compound 19 (27mg, 38μmol, 1.0eq) and compound 38 (32mg, 38μmol, 1.0eq) were dissolved in DMF (0.5mL). HATU (24mg, 46μmol, 1.2eq) and DIEA (16μL, 95μmol, 2.5eq) were added, and stirred at room temperature for 3 hours. The reaction solution was directly purified by reversed-phase column chromatography (37% $CH_3CN$ in $H_2O$, 0.05% HCOOH) to obtain a yellow solid (18 mg, 33%). MS(ESI):m/z found[M/2+1] = 714.2

$^1H$ NMR (400 MHz, DMSO-$d_6$): $\delta$ 9.65 (s, 1H), 8.76 (t, *J*= 6.6 Hz, 1H), 8.52 (d, *J*= 8.6 Hz, 1H), 8.16-8.07 (m, 3H), 7.82-7.77 (m, 2H), 7.35 (d, *J*= 1.9 Hz, 1H), 7.30 (s, 1H), 7.19 (dd, *J* = 8.1, 1.9 Hz, 1H), 7.10 (d, *J* = 8.2 Hz, 1H), 6.98 (s, 2H), 6.53 (s, 1H), 5.54 (dt, *J*= 9.3, 4.8 Hz, 1H), 5.42 (s, 2H), 5.26 (d, *J* = 18.8 Hz, 1H), 5.17 (d, *J* = 18.8 Hz, 1H), 4.69-4.61 (m, 2H), 4.20 (ddd, *J* = 9.0, 4.9, 2.1 Hz, 3H), 4.01 (s, 2H), 3.64 (s, 3H), 3.60-3.53 (m, 2H), 3.50-3.43 (m, 20H), 3.40 (s, 2H), 3.36-3.31 (m, 4H), 3.15 (dt, *J* = 11.7, 6.9 Hz, 4H), 2.40 (d, *J* = 1.8 Hz, 3H), 2.38-2.29 (m, 2H), 2.19-2.08 (m, 2H), 2.01 (t, *J* = 7.4 Hz, 2H), 1.86 (ddd, *J*= 32.5, 14.1, 7.2 Hz, 2H), 1.44 (p, *J*= 7.4 Hz, 4H), 1.23 (d, *J*= 7.2 Hz, 3H), 1.20-1.12 (m, 8H), 0.86 (t, *J*= 7.3 Hz, 3H).

**Example 70**

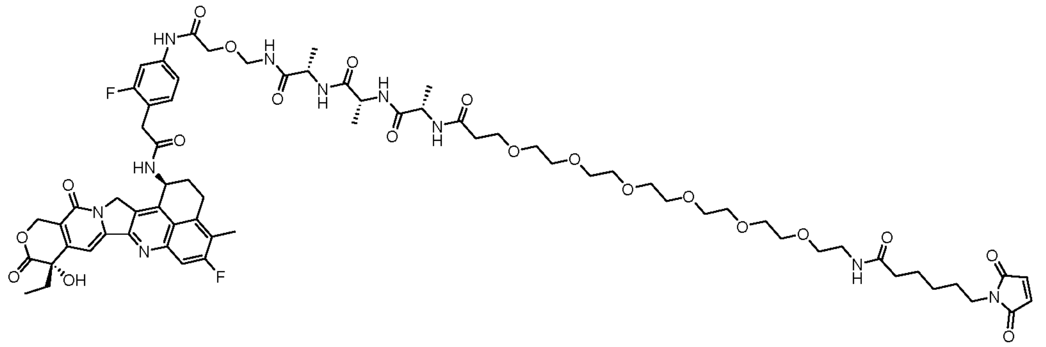

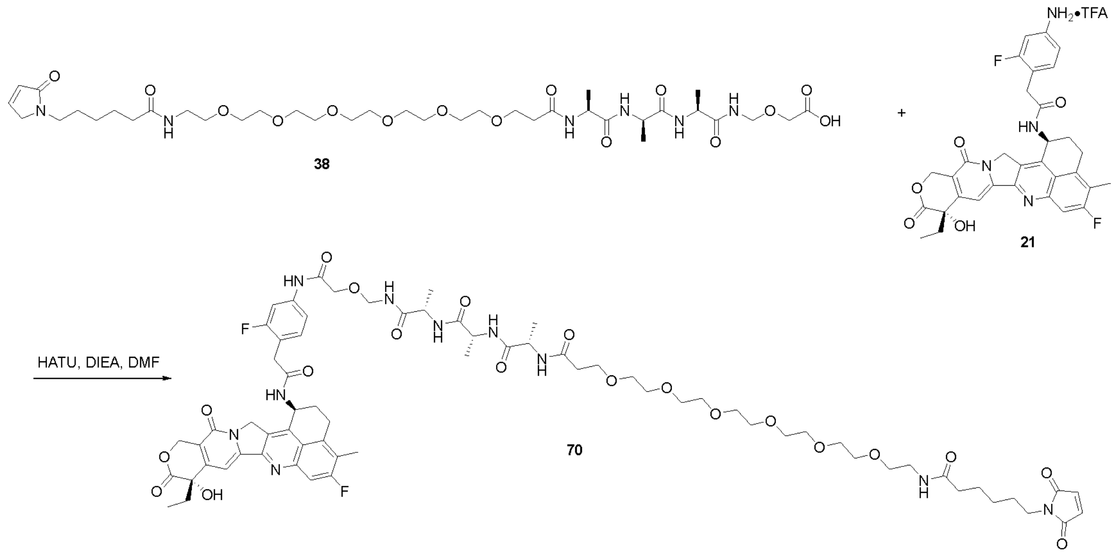

38

21

70

Synthesis of Compound 70:

Compound 21 (23mg, 33μmol, 1.0eq) and compound 38 (28mg, 33μmol, 1.0eq) were dissolved in DMF (0.5mL). HATU (15mg, 39μmol, 1.2eq) and DIEA (14μL, 83μmol, 2.5eq ) were added, and stirred at room temperature for 3 hours. The reaction solution was directly purified by reverse phase column chromatography (37% $CH_3CN$ in $H_2O$, 0.05% HCOOH) to obtain a white solid (24 mg, 52%). MS(ESI): m/z found Fragment 1, Positive=645.0 Fragment 2, Positive=771.2

$^1H$ NMR (400 MHz, DMSO-$d_6$): $\delta$ 9.91 (s, 1H), 8.80 (t, *J*= 6.6 Hz, 1H), 8.72 (d, *J*= 8.5 Hz, 1H), 8.21-8.10 (m, 3H), 7.85-7.81 (m, 2H), 7.61 (dd, *J*= 12.3, 2.0 Hz, 1H), 7.39 (dd, *J*= 8.4, 2.0 Hz, 1H), 7.32 (s, 1H), 7.29 (d, *J*= 8.5 Hz, 1H), 7.01 (s, 2H), 6.55 (s, 1H), 5.56 (dt, *J* = 8.8, 4.6 Hz, 1H), 5.44 (s, 2H), 5.29 (d, *J* = 19.0 Hz, 1H), 5.17 (d, *J* = 19.0 Hz, 1H), 4.72-4.62 (m, 2H), 4.22 (td, *J* = 7.1, 2.8 Hz, 3H), 4.05 (s, 2H), 3.60-3.54 (m, 3H), 3.52-3.44 (m, 21H), 3.37 (dd, *J*= 6.8, 2.5 Hz, 4H), 3.21-3.14 (m, 4H), 2.42 (d, *J*= 1.9 Hz, 4H), 2.42 (s, 3H), 2.39-2.31 (m, 2H), 2.23-2.08 (m, 2H), 2.03 (d, *J*= 7.4 Hz, 2H), 1.86 (dq, *J*= 14.1, 7.0 Hz, 2H), 1.46 (p, *J* = 7.4 Hz, 4H), 1.29-1.12 (m, 11H), 0.88 (t, *J* = 7.3 Hz, 3H).

**Example** 71:

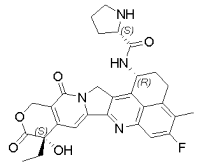

71

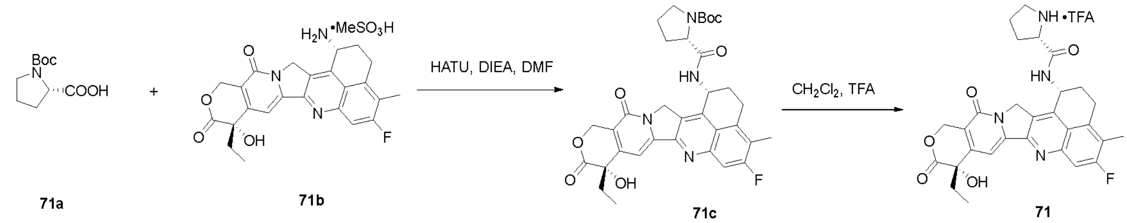


71a 71b 71c 71

Synthesis of compound 71c

R- Exatecan 71b (28.6mg, 54μmol, 1eq) and N-Boc-L-proline 71a (12mg, 56μmol, 1.05eq) were dissolved in DMF (1mL). HATU (25mg, 65μmol, 1.2eq) and DIEA (22uL, 134μmol, 2.5eq) were added, and stirred at room temperature for 1.5 hours. The reaction solution changed from cloudy to clear. Purified by reverse phase column chromatography (50% ACN in $H_2O$, HCOOH) obtain a white solid (34.5 mg, 100%). MS (ESI): m/z found $[M+1]^+$=633.0.

Synthesis of Compound 71

Compound 71c (34.5 mg, 54μmol) was dissolved in trifluoroacetic acid (0.4mL) and dichloromethane (2 mL), and stirred at room temperature for 2.5 hours. Concentrated and then purified by reverse phase column chromatography (34% $CH_3CN$ in $H_2O$, 0.1% TFA) obtain a yellow solid (18.2 mg, 52%). MS (ESI): m/z found $[M+1]^+$=533.0.

**$^1$H NMR (600 MHz,** DMSO-$d_6$): $\delta$ 9.11 (br, 1H), 9.00 (d, *J*= 8.4 Hz, 1H), 8.73 (br, 1H), 7.86 (d, *J*= 10.9 Hz, 1H), 7.34 (s, 1H), 6.56 (s, 1H), 5.63 (dt, *J*= 8.0, 3.7 Hz, 1H), 5.48 - 5.41 (m, 2H), 5.40 (d, *J* = 15.6 Hz, 1H), 5.27 (d, *J* = 18.9 Hz, 1H), 4.14 - 4.10 (m, 1H), 3.30 - 3.26 (m, 1H), 3.25 - 3.19 (m, 2H), 3.12 (dt, *J*= 17.3, 8.5 Hz, 1H), 2.43 (s, 4H), 2.29 - 2.21 (m, 2H), 2.12 (tt, *J*= 13.1, 4.6 Hz, 1H), 1.90 (m, 5H), 0.88 (t, *J*= 7.3 Hz, 3H).

**Example** 72:

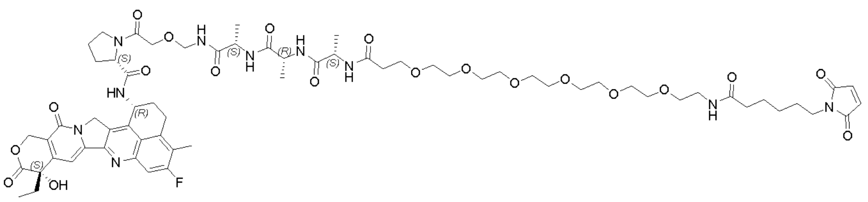

72

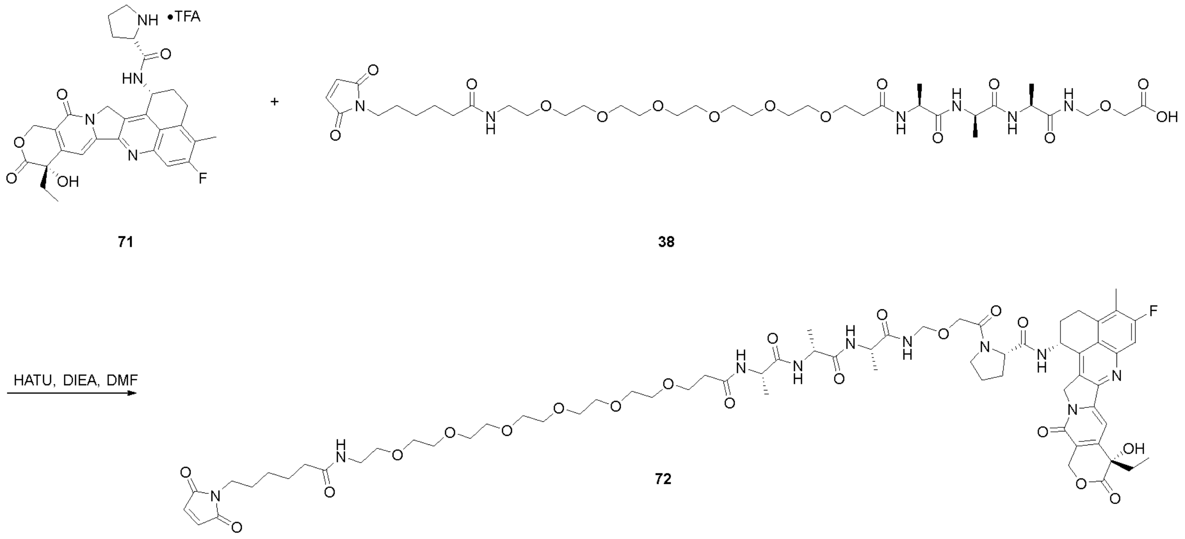


71

38

72

Synthesis of Compound 72:

Compound 71 (16mg, 25μmol, 1.0eq) and compound 38 (24mg, 28μmol, 1.0eq) were dissolved in DMF (0.5mL). HATU (14.5mg, 38μmol, 1.5eq) and DIEA (10μL, 64μmol, 2.5 eq) were added, and stirred at room temperature for 2 hours. The reaction solution was directly purified by reverse phase column chromatography (42% $CH_3CN$ in $H_2O$, 0.05% HCOOH) to obtain a white solid (18 mg, 52%). MS (ESI): m/z found Fragment 1, Positive = 591.0, Fragment 2, Positive = 771.2.

**$^1$H NMR (400 MHz, DMSO-*d*$_6$)** $\delta$: 8.60 (t, *J* = 6.8 Hz, 1H), 8.51 (d, *J* = 8.8 Hz, 1H), 8.15 (d, *J* = 7.3 Hz, 1H), 8.12 (d, *J* = 6.5 Hz, 1H), 8.01 (d, *J* = 7.1 Hz, 1H), 7.85 - 7.78 (m, 2H), 7.32 (s, 1H), 7.01 (s, 2H), 6.53 (s, 1H), 5.58 (dt, *J* = 9.3, 4.7 Hz, 1H), 5.46 - 5.40 (m, 2H), 5.26 - 5.16 (m, 2H), 4.58 - 4.53 (m, 2H), 4.24 (dd, *J* = 5.5, 2.9 Hz, 1H), 4.20 (tt, *J* = 7.0, 3.1 Hz, 2H), 4.09 (q, *J* = 7.1 Hz, 1H), 4.05 (s, 2H), 3.60 - 3.54 (m, 3H), 3.53 - 3.44 (m, 21H), 3.39 - 3.35 (m, 6H), 3.17 (q, *J* = 5.9 Hz, 3H), 2.42 - 2.36 (m, 4H), 2.33 (dt, *J* = 14.6, 6.2 Hz, 1H), 2.13 - 2.06 (m, 3H), 2.02 (t, *J* = 4.9, 2H), 1.97 - 1.79 (m, 4H), 1.46 (p, *J* = 7.4 Hz, 4H), 1.23 - 1.13 (m, 9H), 1.11 (d, *J* = 7.2 Hz, 2H), 0.87 (t, *J* = 7.3 Hz, 3H).

**Example** 73:

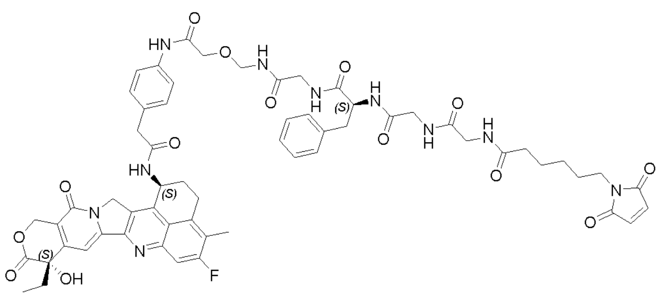

73

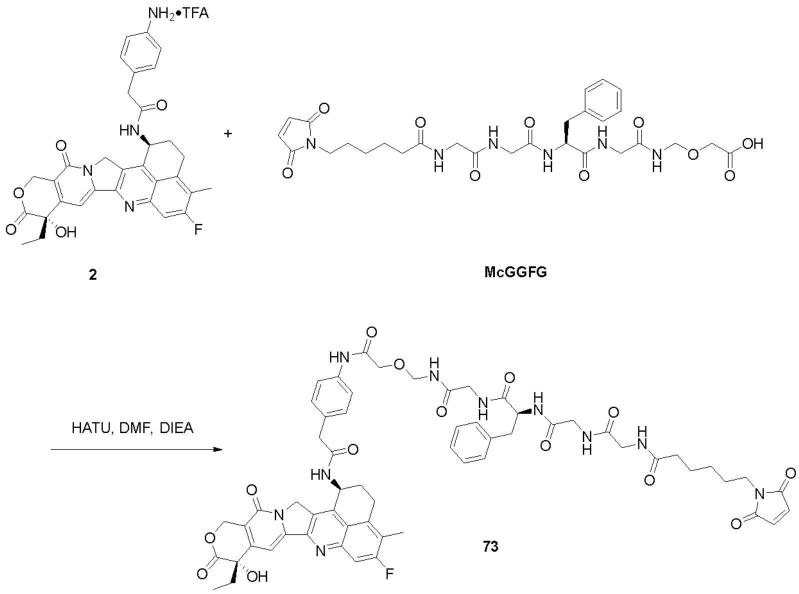


2

McGGFG

73

Synthesis of compound 73:

Compound 2 (20mg, 29μmol, 1.0eq) and compound McGGFG (20mg, 32μmol, 1.1eq) were dissolved in DMF (0.5mL). HATU (11mg, 35μmol, 1.2eq) and DIEA (12μL, 11μmol, 2.2eq) were added, and stirred at room temperature for 3 hours. The reaction solution was directly purified by reversed-phase column chromatography (42% $CH_3CN$ in $H_2O$, 0.05% HCOOH) to obtain a white solid (9.8 mg, 29%). MS (ESI): m/z found Fragment 1, Positive=541.0 Fragment 2, Positive=627.0.

**$^1$H NMR (400 MHz, DMSO-*d*$_6$):** δ 9.71 (d, *J* = 4.0 Hz, 1H), 8.74 (t, *J* = 6.7 Hz, 1H), 8.70 - 8.65 (m, 1H), 8.37 (t, *J*= 5.8 Hz, 1H), 8.17 (d, *J*= 8.2 Hz, 1H), 8.08 (t, *J*= 5.8 Hz, 1H), 8.03 (t, *J* = 5.9 Hz, 1H), 7.82 (d, *J* = 10.9 Hz, 1H), 7.60 (d, *J* = 8.7 Hz, 2H), 7.32 (d, *J*= 2.9 Hz, 1H), 7.28 - 7.21 (m, 6H), 7.18 (ddt, *J*= 7.3, 5.5, 2.4 Hz, 1H), 6.99 (s, 2H), 6.54 (d, *J*= 2.3 Hz, 1H), 5.54 (dd, *J*= 9.1, 4.5 Hz, 1H), 5.44 (d, *J*= 5.5 Hz, 2H), 5.26 (dd, *J* = 18.7, 4.9 Hz, 1H), 5.16 (dd, *J*= 18.6, 5.0 Hz, 1H), 4.67 (d, *J*= 6.7 Hz, 2H), 4.51 (td, *J* = 8.8, 4.4 Hz, 1H), 4.05 (s, 2H), 3.81 - 3.72 (m, 3H), 3.67 (d, *J*= 5.5 Hz, 2H), 3.60 (dd, *J* = 16.6, 5.4 Hz, 1H), 3.46 (t, *J* = 4.4 Hz, 2H), 3.37 (t, *J* = 7.1 Hz, 2H), 3.17 (d, *J* = 6.4 Hz, 2H), 3.07 (dd, *J* = 13.9, 4.6 Hz, 1H), 2.82 (dd, *J* = 13.9, 9.6 Hz, 1H), 2.41 (s, 3H), 2.17 - 2.07 (m, 4H), 1.87 (ddp, *J* = 21.4, 14.4, 7.3 Hz, 2H), 1.46 (ddd, *J* = 19.0, 11.2, 6.3 Hz, 4H), 1.19 (tt, *J* = 9.8, 6.5 Hz, 2H), 0.88 (td, *J* = 7.4, 3.2 Hz, 3H).

**Example** 74:

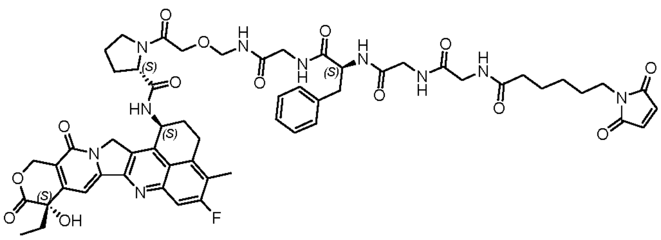

74

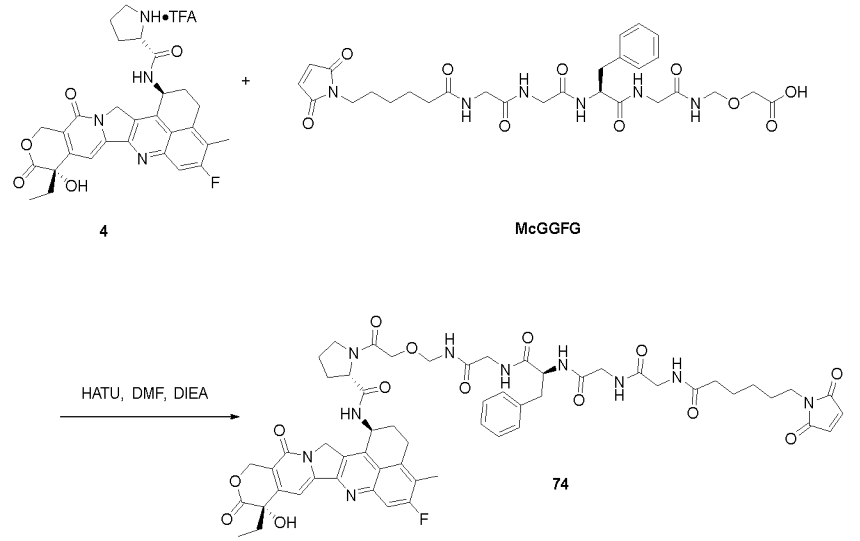


4

McGGFG

74

Synthesis of Compound 74:

Compound 4 (25 mg, 39μmol, 1.0eq) and compound McGGFG (26mg, 43μmol, 1.1eq) were dissolved in DMF (0.5 mL), and HATU (18 mg, 46μmol, 1.2eq) and DIEA (14μL, 85μmol, 2.2eq), and stirred at room temperature for 3 hours. The reaction solution was directly purified by reverse phase column chromatography (38% $CH_3CN$ in $H_2O$, 0.05% HCOOH) to obtain a white solid (10.3 mg, 23%). MS (ESI): m/z found Fragment 1, Positive=541.0 Fragment 2, Positive=591.0.

1H NMR (600 MHz, DMSO-d6) δ 8.50 - 8.44 (m, 2H), 8.27 (t, J = 5.9 Hz, 1H), 8.14 (d, J = 8.1 Hz, 1H), 8.08 (t, J = 5.8 Hz, 1H), 8.01 (t, J = 5.8 Hz, 1H),7.78 (d, J = 10.9 Hz, 1H), 7.31 (s, 1H), 7.24 (dt, J = 13.4, 7.4 Hz, 4H), 7.19 (q, J = 7.5, 6.7 Hz, 1H), 7.00 (s, 2H), 6.53 (d, J = 6.9 Hz, 1H), 5.53 (dt, J = 9.4, 5.1 Hz, 1H), 5.43 (d, J = 5.3 Hz, 2H), 5.29 - 5.17 (m, 2H).4.48 (td, J = 8.9, 4.5 Hz, 1H), 4.31 (td, J = 7.6, 6.0, 3.3 Hz, 2H), 4.01 (s, 2H), 3.73 (dd, J = 16.6, 5.9 Hz, 1H), 3.66 (d, J = 5.7 Hz, 2H), 3.59 (dd, J = 16.4, 5.6 Hz, 2H), 3.53 (dd, J = 16.5, 5.7 Hz, 1H), 3.44 (dt, J = 12.7, 5.7 Hz, 1H), 3.36 (t, J = 7.2 Hz, 3H), 3.17 (t, J = 7.4 Hz, 2H), 3.04 (dd, J = 13.9, 4.6 Hz, 1H), 2.79 (dd, J = 13.8, 9.6 Hz, 1H), 2.40 (d, J = 6.7 Hz, 3H), 2.19 - 2.06 (m, 5H), 1.97 - 1.81 (m, 5H), 1.47 (m, 4H), 1.22 - 1.16 (m, 2H), 0.88 (t, J = 7.3 Hz, 3H).

**Example** 75:

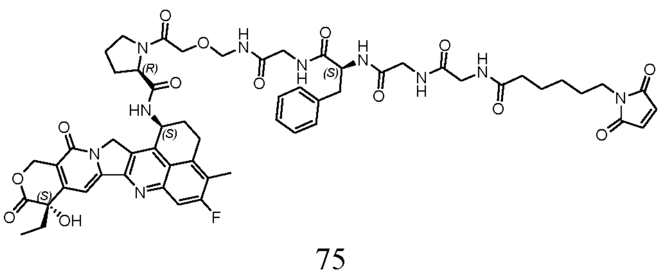

75

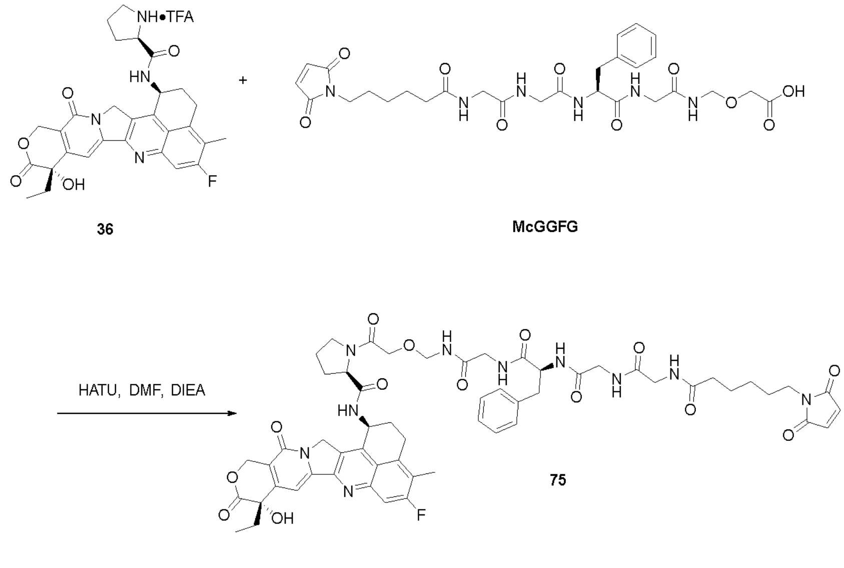


36

McGGFG

75

Synthesis of compound 75:

Compound 36 (17 mg, 26μmol, 1.0 eq) and compound McGGFG (18 mg, 29μmol, 1.1 eq) were dissolved in DMF (0.5 mL), and HATU (11 mg, 29μmol, 1.1eq) and DIEA (10μL, 58μmol, 2.2eq) were added, and stirred at room temperature for 3 hours. The reaction solution was directly purified by reversed-phase column chromatography (40% $CH_3CN$ in $H_2O$, 0.05% HCOOH) to obtain a white solid (6 mg, 20%). MS (ESI): m/z found Fragment 1, Positive=541.0 Fragment 2, Positive=591.0.

$^1H$ NMR (600 MHz, DMSO-d6) δ 8.60 - 8.53 (m, 2H), 8.28 (t, J = 5.5 Hz, 1H), 8.14 (d, J = 7.9 Hz, 1H), 8.06 (t, J = 6.8 Hz, 1H), 8.00 (t, J = 5.7 Hz, 1H), 7.82 (d, J = 10.9 Hz, 1H), 7.33 (s, 1H), 7.24 (hept, J = 5.6, 4.9 Hz, 4H), 7.18 (q, J = 7.6 Hz, 1H), 7.00 (s, 2H), 6.53 (s, 1H), 5.58 (dt, J = 9.0, 4.5 Hz, 1H), 5.46 - 5.39 (m, 2H), 5.26 - 5.18 (m, 2H), 4.58 (dd, J = 10.3, 6.8 Hz, 1H), 4.51 (dt, J = 23.5, 8.2 Hz, 2H), 4.22 (dd, J = 8.2, 4.5 Hz, 1H), 4.08 (s, 2H), 3.75 (q, J = 4.3, 3.7 Hz, 1H), 3.71 (d, J = 5.0 Hz, 1H), 3.69 (d, J = 5.6 Hz, 1H), 3.65 (d, J = 5.7 Hz, 2H), 3.58 (dd, J = 16.7, 5.4 Hz, 1H), 3.50 (d, J = 7.6 Hz, 1H), 3.41 (q, J = 7.6 Hz, 1H), 3.37 (d, J = 7.1 Hz, 2H), 3.17 (q, J = 14.3, 11.5 Hz, 2H), 3.07 - 3.01 (m, 1H), 2.79 (dd, J = 13.9, 9.7 Hz, 1H), 2.41 (s, 3H), 2.14 - 1.99 (m, 6H), 1.88 (ddt, J = 17.5, 14.3, 6.6 Hz, 4H), 1.46 (h, J = 7.3 Hz, 4H), 1.18 (p, J = 7.9 Hz, 2H), 0.88 (q, J = 7.0 Hz, 3H).

**Example** 76: **Preparation of anti-human Trop-2 monoclonal antibody**

The anti-human TROP-2 monoclonal antibody used in the present invention is an anti-hRS7 antibody with an amino acid sequence as follows:

Complementarity Determining Region of Heavy Chain Variable Region (HCDR1) of the anti-human TROP-2 Antibody (SEQ ID NO: 1):
NYGMN
Complementarity Determining Region of Heavy Chain Variable Region (HCDR2) of the anti-human TROP-2 Antibody (SEQ ID NO:2):
WINTYTGEPTYTDDFKG
Complementarity Determining Region of Heavy Chain Variable Region (HCDR3) of the anti-human TROP-2 Antibody (SEQ ID NO: 3):
GGFGSSYWYFDV
The complementarity determining region (LCDR1) of Light Chain Variable Region of the anti-human TROP-2 Antibody (SEQ ID NO: 4):
KASQDVSIAVA
Complementarity Determining Region of Light Chain Variable Region (LCDR2) of the anti-human TROP-2 Antibody (SEQ ID NO:5):
SASYRYT;
The Complementarity Determining Region (LCDR3) of Light Chain Variable Region of the anti-human TROP-2 antibody (SEQ ID NO: 6):
QQHYITPLT

VH amino acid sequence of the anti-human TROP-2 antibody (VH-TROP2) (SEQ ID NO: 7:

QVQLQQSGSELKKPGASVKVSCKASGYTFTNYGMNWVKQAPGQGLKWMGW INTYTGEPTYTDDFKGRFAFSLDTSVSTAYLQISSLKADDTAVYFCARGGFGSSY WYFDVWGQGSLVTVSS

VL amino acid sequence of the anti-human TRO-P2 antibody (VL-TROP2) (SEQ ID NO: 8):

DIQLTQSPSSLSASVGDRVSITCKASQDVSIAVAWYQQKPGKAPKLLIYSASYR YTGVPDRFSGSGSGTDFTLTISSLQPEDFAVYYCQQHYITPLTFGAGTKVEIKR

Heavy chain of the anti-human TROP-2 antibody: (SEQ ID NO:9)

QVQLQQSGSELKKPGASVKVSCKASGYTFTNYGMNWVKQAPGQGLKWMGW INTYTGEPTYTDDFKGRFAFSLDTSVSTAYLQISSLKADDTAVYFCARGGFGSSY WYFDVWGQGSLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSREEMTKNQVSLTCLVKGFY PSDIAVEWESNGQPENNYKTTPPVLDSDGSFFLYSKLTVDKSRWQQGNVFSCSV MHEALHNHYTQKSLSLSPGK;

Light chain of the anti-human TROP-2 antibody: (SEQ ID NO: 10)

DIQLTQSPSSLSASVGDRVSITCKASQDVSIAVAWYQQKPGKAPKLLIYSASYR YTGVPDRFSGSGSGTDFTLTISSLQPEDFAVYYCQQHYITPLTFGAGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC;

The anti-human TROP-2 monoclonal antibody was prepared according to the following method: the heavy chain and light chain genes synthesized by Nanjing GenScript Biotechnology Co., Ltd. were constructed into the pCGS3 expression vector, the endotoxin-free plasmid extraction kit (Tiangen Biochemical Technology Co., Ltd., item number: DP117) was used to extract the plasmid, and the specific operation was carried out according to the instructions. HEK293f cells were cultured using KOP293 cell culture medium (Zhuhai Kairui, item number: K03252), placed in a 37°C, 5% $CO_2$ shaker, with a rotation speed of 100-130rpm, and a humidity control of more than 75%. The day before transfection, HEK 293f cells in logarithmic growth phase and in good growth state were passaged to $2\times10^6$/mL, cultured overnight on a shaker (110rpm, 37°C, 5% $CO_2$), and transfected the next day. Before transfection, preheat TA-293 (293 cell suspension chemical transfection reagent) and KPM (serum-free cell transfection buffer solution) at room temperature, and measure the cell density and viability. The density is $4\times10^6$/mL, and the viability is greater than 97% can be used. Transfection was performed according to the KOP293 Transient Transfection Protein Expression System User Guide, and the expression supernatant was harvested by centrifugation 5 days after transfection.

The expression supernatant was filtered with a 0.45 $\mu$M filter membrane, and an antibody with an Fc domain was obtained from the expression supernatant using an affinity chromatography column. Equilibrium buffer is 9.5mM sodium dihydrogen phosphate plus 40.5mM disodium hydrogen phosphate, pH 7.4; elution buffer is 0.1M glycine, pH 3.0. The eluted antibody was replaced with PBS buffer at pH=7.2.

**Example** 77: **TROP-2 ADC conjugation**

The interchain disulfide bonds were reduced by adding 1 mM EDTA (invitrogen, item number: AM9260G) and 8-fold molar equivalent of TECP (Thermo, item number: 77720) to the 10 mg/mL Trop-2 monoclonal antibody solution, and the mixture was stirred at 37°C for 2 hours. The mixture was cooled to the target temperature of 4 °C and 10 drug equivalents per mole of compounds 48, 49, 50, 51, 52, 53, 54, 55, 56, 57, 58, 59, 60, 61, 62, 63, 64, 65, 66, 67, 68, 69 or 70 were added as a 10% (v/v) solution in DMSO (Sigma, Cat. No.: D2660). After 2 hours, the antibody-drug conjugate was

obtained by ultrafiltration with PBS, named TROP-2-ADC-1, TROP-2-ADC-2, TROP-2-ADC-3, TROP-2-ADC-4, TROP-2-ADC-5, TROP-2-ADC-6, TROP-2-ADC-7, TROP-2-ADC-8, TROP-2-ADC-9, TROP-2-ADC-10, TROP-2-ADC-11, TROP-2-ADC-12, TROP- 2-ADC-13, TROP-2-ADC-14, TROP-2-ADC-15, TROP-2-ADC-16, TROP-2-ADC-17, TROP-2-ADC-18, TROP-2- ADC-19, TROP-2-ADC-20, TROP-2-ADC-21, TROP-2-ADC-22, TROP-2-ADC-23 in order. After the ultrafiltration was completed, DAR (Drug-to-Antibody Ratio) was detected by using the absorbance method at 280nm and 365nm and HIC-HPLC, and the results are shown in Table 3.

Table 3 ADC drug conjugation results of Trop-2 antibody

| ADC number | DAR | ADC number | DAR |
|---|---|---|---|
| TROP-2-ADC-1 | 7.63 | TROP-2-ADC-12 | 4.57 |
| TROP-2-ADC-2 | 4.13 | TROP-2-ADC-14 | 8.20 |
| TROP-2-ADC-3 | 14.74 | TROP-2-ADC-15 | 1.65 |
| TROP-2-ADC-4 | 8.49 | TROP-2-ADC-16 | 5.48 |
| TROP-2-ADC-5 | 5.93 | TROP-2-ADC-18 | 10.07 |
| TROP-2-ADC-6 | 5.02 | TROP-2-ADC-19 | 4.65 |
| TROP-2-ADC-7 | 2.80 | TROP-2-ADC-20 | 4.81 |
| TROP-2-ADC-8 | 5.96 | TROP-2-ADC-21 | 4.78 |
| TROP-2-ADC-9 | 4.55 | TROP-2-ADC-22 | 4.85 |
| TROP-2-ADC-10 | 2.67 | TROP-2-ADC-23 | 6.42 |
| TROP-2-ADC-11 | 4.96 | -- | -- |

**Example 78: Preparation of an anti-human HER3 monoclonal antibody**

The anti-human HER3 monoclonal antibody sequence in the present invention is as follows:

The complementarity determining region H'CDR1 (SEQ ID NO: 11) of the heavy chain variable region of the anti-human HER3 antibody:
GGSFSGYYWS
The complementarity determining region H'CDR2 (SEQ ID NO: 12) of the heavy chain variable region of the anti-human HER3 antibody:
EINHSGSTNYNPSLKS
The complementarity determining region H'CDR3 (SEQ ID NO: 13) of the heavy chain variable region of the anti-human HER3 antibody:
DKWTWYFDL
The complementarity determining region L'CDR1 (SEQ ID NO: 14) of the light chain variable region of the anti-human HER3 antibody:
RSSQSVLYSSSNRNYLA
The complementarity determining region L'CDR2 (SEQ ID NO: 15) of the light chain variable region of the anti-human HER3 antibody:
WASTRES
The complementarity determining region L'CDR3 (SEQ ID NO: 16) of the light chain variable region of the anti-human HER3 antibody:
QQYYSTPRT
Heavy chain variable region of the anti-HER3 antibody (SEQ ID NO: 17):

QVQLQQWGAGLLKPSETLSLTCAVYGGSFSGYYWSWIRQPPGKGLEWIGEIN
HSGSTNYNPSLKSRVTISVETSKNQFSLKLSSVTAADTAVYYCARDKWTWYFDL
WGRGTLVTVSS

Light chain variable region of the anti-HER3 antibody (SEQ ID NO: 18):

DIEMTQSPDSLAVSLGERATINCRSSQSVLYSSSNRNYLAWYQQNPGQPPKLLIYWASTRESGVPDRFSGSGSGTDFTLTISSLQAEDVAVYYCQQYYSTPRTFGQGTKVEIK

Heavy chain of the anti-HER3 antibody: SEQ ID NO: 19

QVQLQQWGAGLLKPSETLSLTCAVYGGSFSGYYWSWIRQPPGKGLEWIGEINHSGSTNYNPSLKSRVTISVETSKNQFSLKLSSVTAADTAVYYCARDKWTWYFDLWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

Light chain of the anti-HER3 antibody SEQ ID NO:20

DIEMTQSPDSLAVSLGERATINCRSSQSVLYSSSNRNYLAWYQQNPGQPPKLLIYWASTRESGVPDRFSGSGSGTDFTLTISSLQAEDVAVYYCQQYYSTPRTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

The anti-human HER3 monoclonal antibody was prepared according to the following method: the heavy chain and light chain genes synthesized by Nanjing GenScript Biotechnology Co., Ltd. were constructed into the pCGS3 expression vector. The plasmid was extracted using an endotoxin-free plasmid extraction kit (Tiangen Biochemical Technology Co., Ltd., catalog number: DP117), and the specific operation was performed according to the instructions. HEK293f cells were cultured using KOP293 cell culture medium (Zhuhai Kairui, product number: K03252), placed in a 37°C, 5% $CO_2$ shaker, with a rotation speed of 100-130rpm, and a humidity control of more than 75%. The day before transfection, HEK 293f cells in logarithmic growth phase and in good growth state were passaged to $2\times10^6$/mL, cultured overnight on a shaker (110rpm, 37°C, 5% $CO_2$), and transfected the next day. Before transfection, preheat TA-293 (293 cell suspension chemical transfection reagent) and KPM (serum-free cell transfection buffer solution) at room temperature, and measure the cell density and viability. The density is $4\times10^6$/mL, and the viability is greater than 98% can be used. Transfection was performed according to the KOP293 Transient Transfection Protein Expression System User Guide, and the expression supernatant was harvested by centrifugation 6 days after transfection.

The expression supernatant was filtered with a 0.45μM filter membrane, and an antibody with an Fc domain was obtained from the expression supernatant using an affinity chromatography column. Equilibrium buffer is 9.5mM sodium dihydrogen phosphate plus 40.5mM disodium hydrogen phosphate, pH 7.4; elution buffer is 0.1M glycine, pH 3.0. The eluted antibody was replaced with PBS buffer at pH=7.2.

## Example 79: HER3 ADC conjugation

By adding 1mM EDTA (invitrogen, item number: AM9260G) and 8-fold molar equivalent of TECP (Thermo,item number: 77720) to the 10 mg/mL HER3 monoclonal antibody solution to reduce the interchain disulfide bonds, the mixture was stirred at 37 ° C and 250 rpm for 2.5 hours. The mixture was cooled to the target temperature of 4 °C and 10 drug equivalents per mole of compounds 48, 49, 50, 51, 52, 53, 54, 55, 56, 57, 58, 59, 60, 61, 62, 63, 64, 65, 66, 67, 68, 69, 70, 72, 73, 74, and 75 were added as a 10% (v/v) solution in DMSO (Sigma, Cat. No.: D2660). After 3 hours, replace antibody-drug conjugates with PBS ultrafiltration, named HER3-ADC-1, HER3-ADC-2, HER3-ADC-3, HER3-ADC-4, HER3-ADC-5, HER3-ADC-6, HER3-ADC-7 , HER3-ADC-8, HER3-ADC-9, HER3-ADC-10, HER3-ADC-11, HER3-ADC-12, HER3-ADC-13, HER3-ADC-14, HER3-ADC-15, HER3 -ADC-16, HER3-ADC-17, HER3-ADC-18, HER3-ADC-19, HER3-ADC-20, HER3-ADC-21, HER3-ADC-22, HER3-ADC-23, HER3-ADC -24, HER3-ADC-25, HER3-ADC-26, HER3-ADC-27. The specific structure of the antibody conjugates are shown in Table 4.

Table 4 structural formulas of the HER3-ADCs

| ADC number | ADC structure |
|---|---|
| HER3 -ADC-1 | 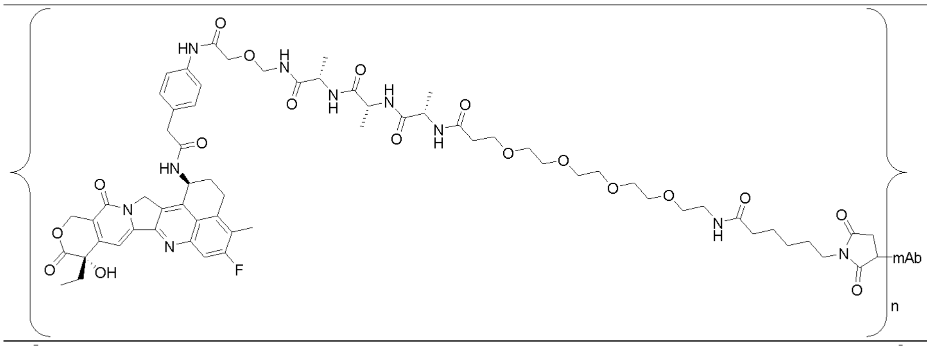 |
| HER3 -ADC-2 | 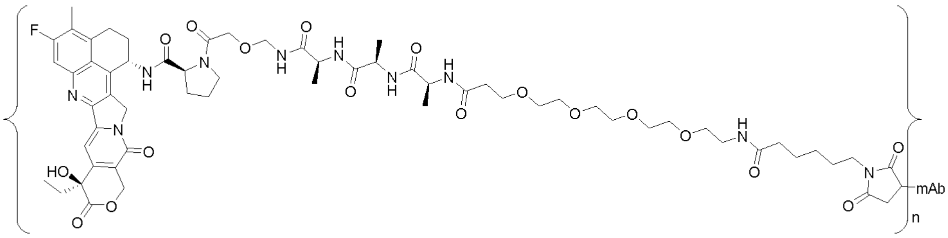 |
| HER3 -ADC-3 | 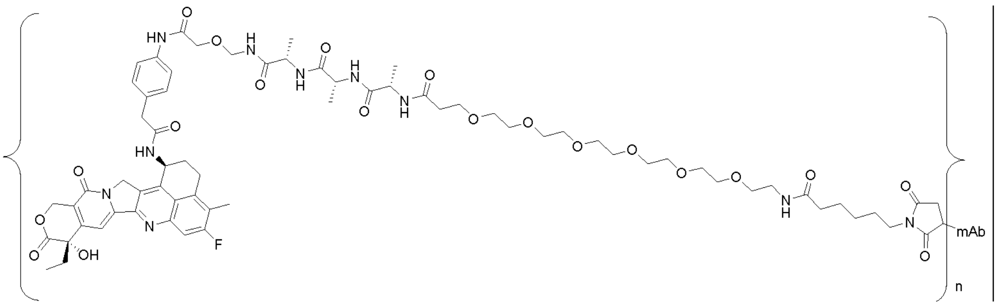 |
| HER3 -ADC-4 | 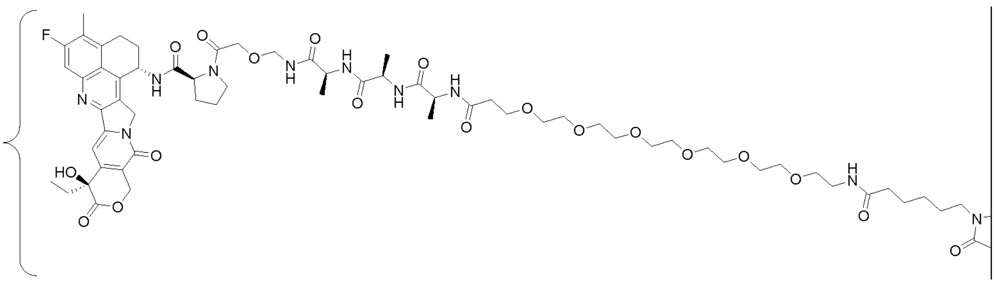 |
| HER3 -ADC-5 | 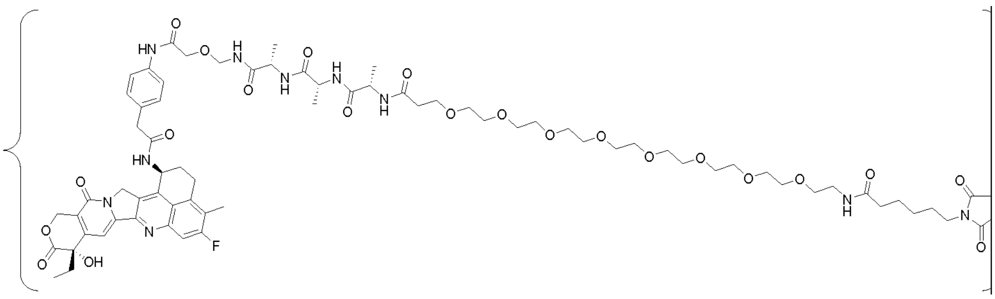 |

(continued)

| ADC number | ADC structure |
|---|---|
| HER3 -ADC-6 | 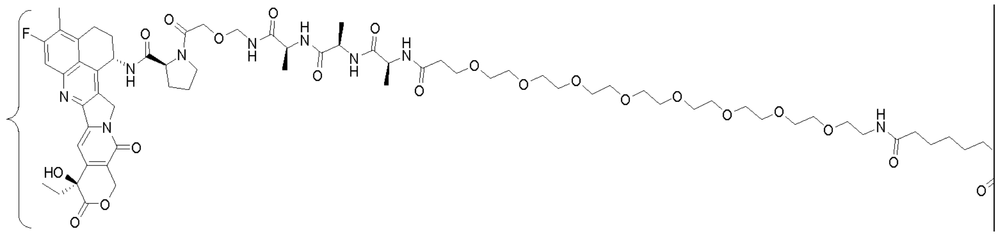 |
| HER3 -ADC-7 | 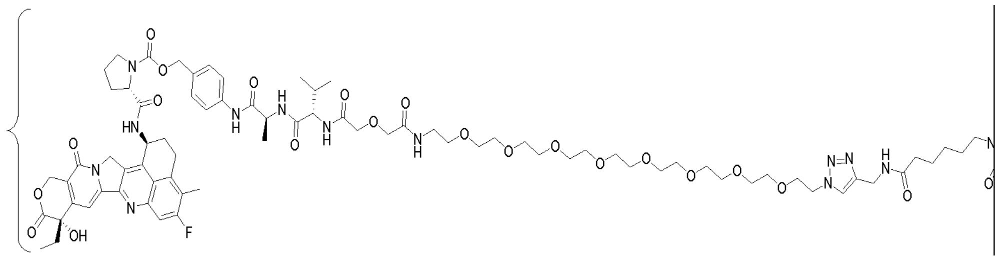 |
| HER3 -ADC-8 | 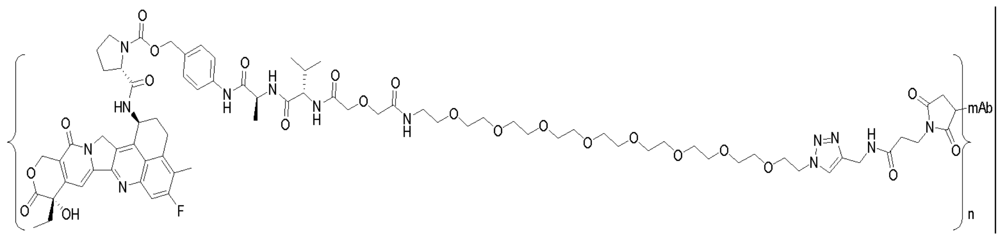 |
| HER3 -ADC-9 | 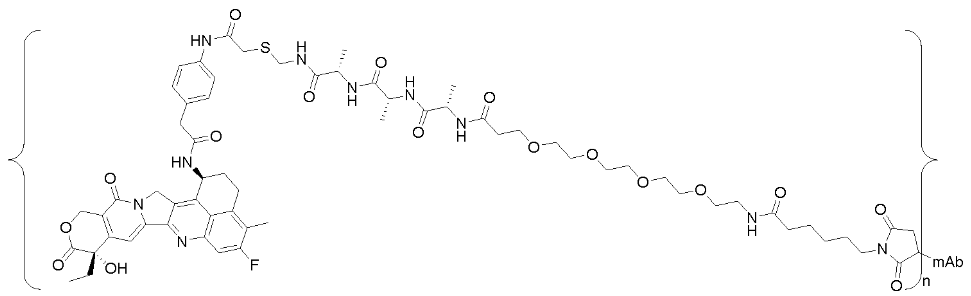 |
| HER3 -ADC-10 | 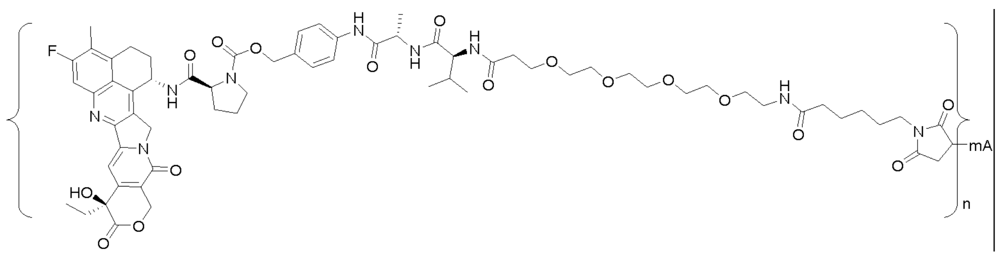 |
| HER3 -ADC-11 | 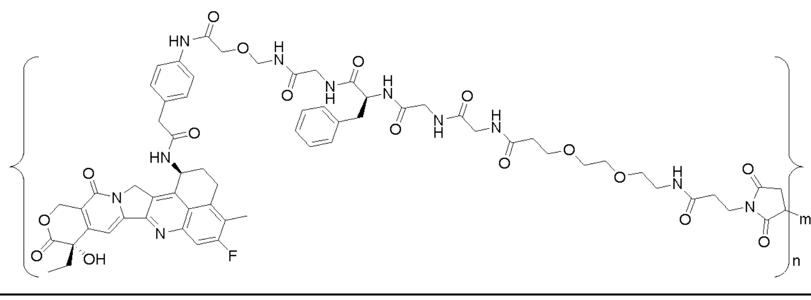 |

(continued)

| ADC number | ADC structure |
|---|---|
| HER3 -ADC-12 | 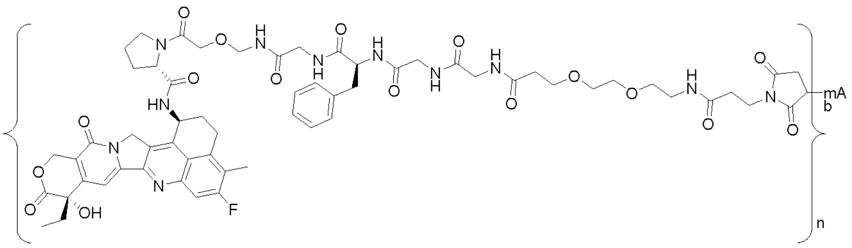 |
| HER3 -ADC-13 | 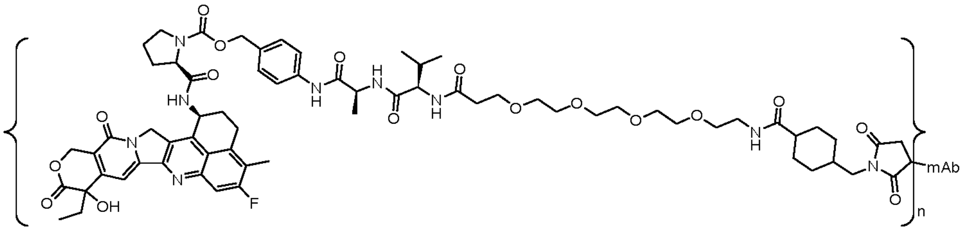 |
| HER3 -ADC-14 | 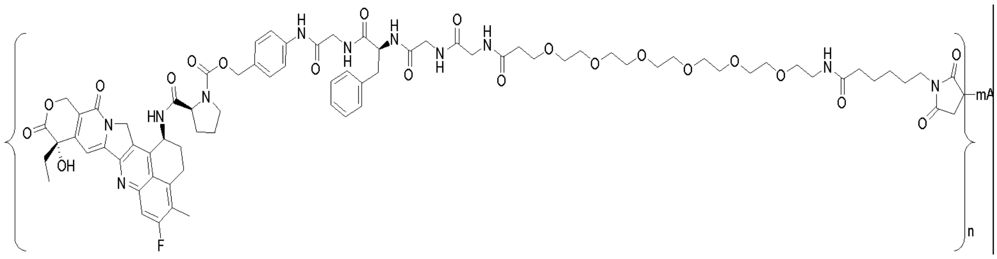 |
| HER3 -ADC-15 | 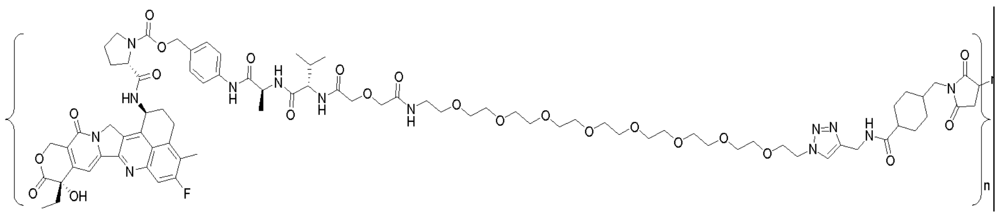 |
| HER3 -ADC-16 | 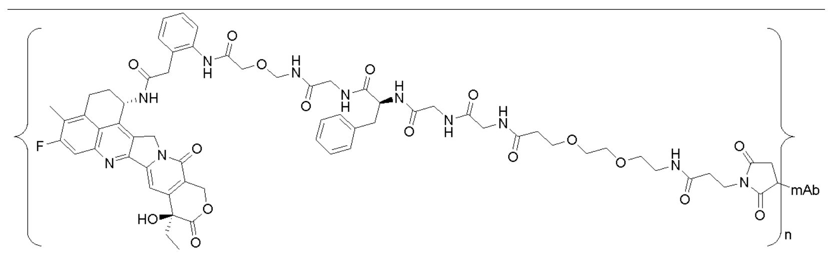 |
| HER3 -ADC-17 | 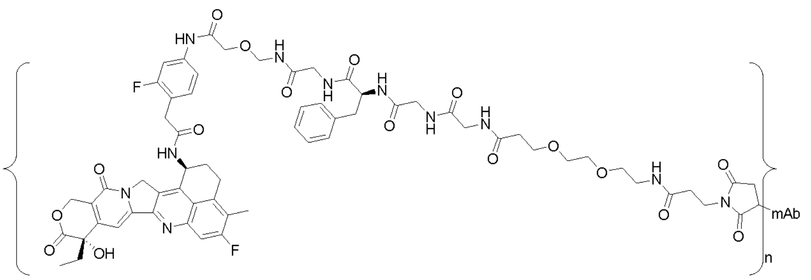 |

(continued)

| ADC number | ADC structure |
|---|---|
| HER3 -ADC-18 | 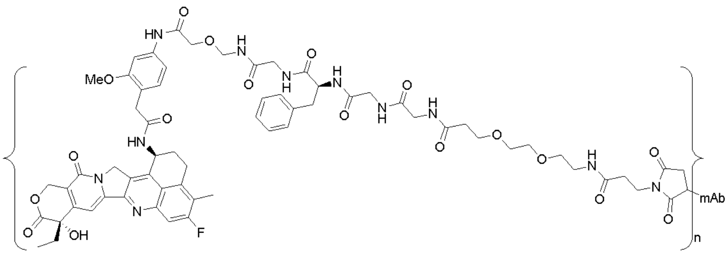 |
| HER3 -ADC-19 | 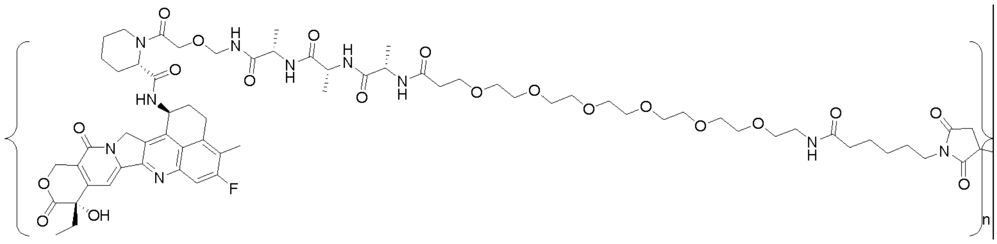 |
| HER3 -ADC-20 | 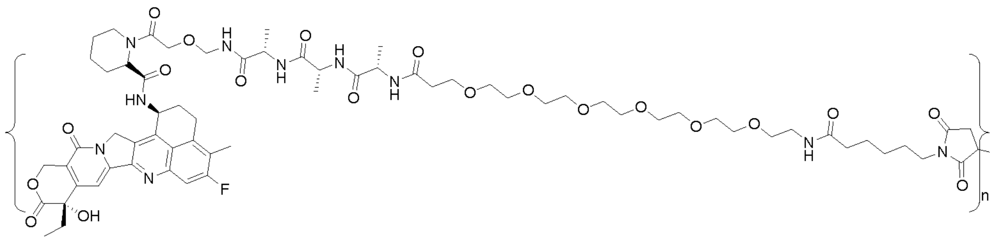 |
| HER3 -ADC-21 | 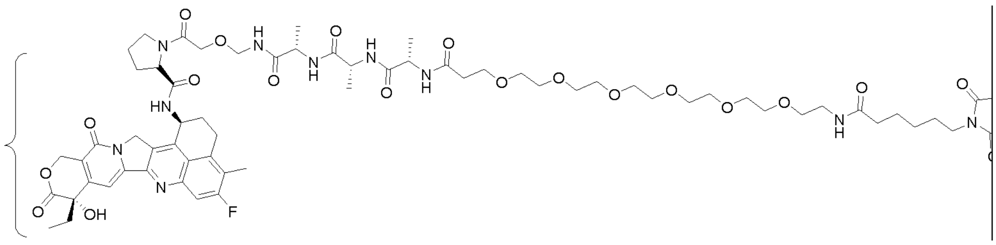 |
| HER3 -ADC-22 | 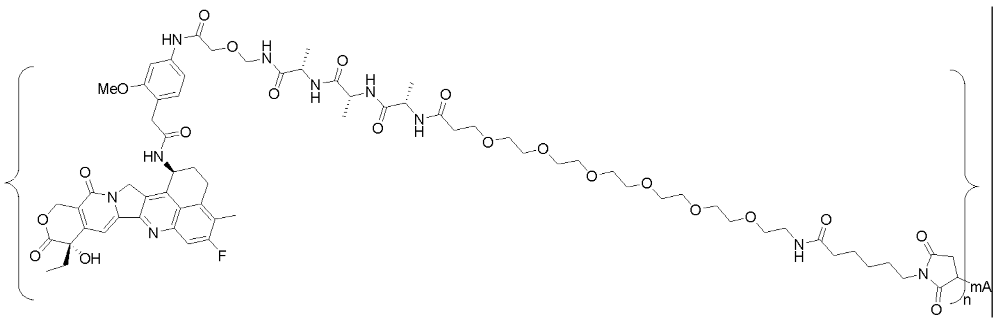 |
| HER3 -ADC-23 | 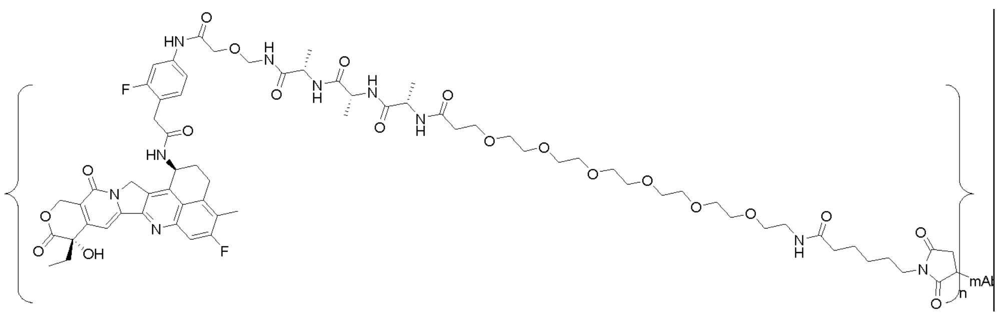 |

(continued)

| ADC number | ADC structure |
|---|---|
| HER3 -ADC-24 | 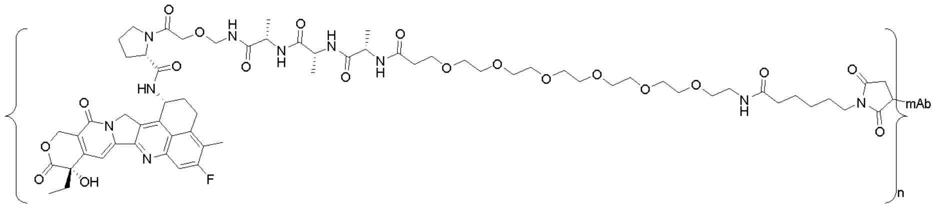 |
| HER3 -ADC-25 | 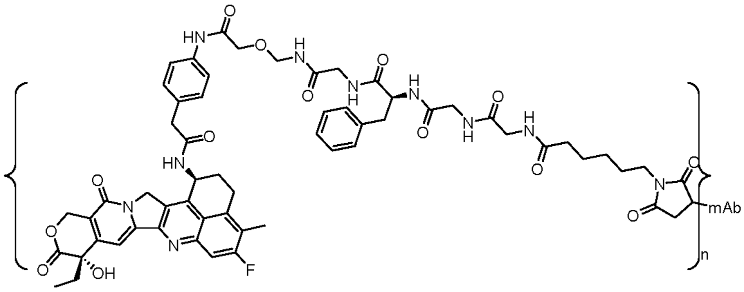 |
| HER3 -ADC-26 | 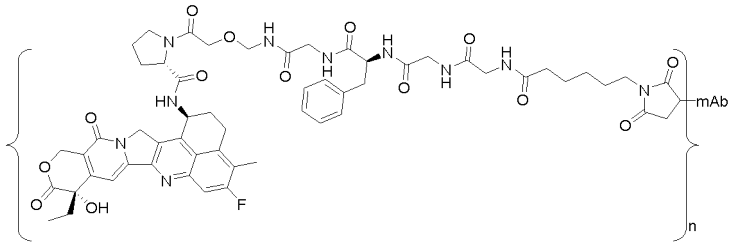 |
| HER3 -ADC-27 | 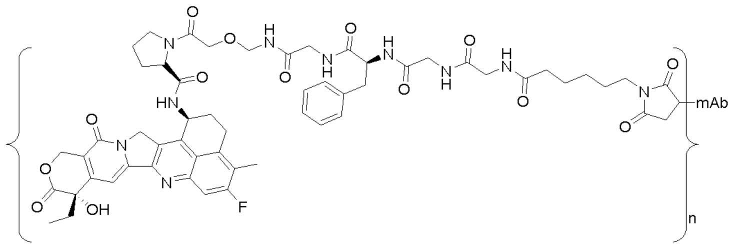 |
| Note: The mAb in Table 4 in this example refers to the HER3 monoclonal antibody in the above examples and test examples, and n in each occurrence is an integer or decimal with a value of 1-15, indicating the average number of units coupled to each antibody for each of the selected drug-linker compounds, and n can also be expressed as DAR. | |

After the ultrafiltration is completed, the concentration of the ADC coupling product is determined by absorbance at 280nm and 365nm; whether the coupling is successful is determined by HPLC-HIC analysis; the purity of the antibody is detected by SEC-HPLC; the DAR value is detected by LC-MS. The results are shown in Table 5.

Table 5 ADC drug conjugation results of HER3 antibody

| ADC number | DAR | ADC number | DAR |
|---|---|---|---|
| HER3-ADC-3 | 7.78 | HER3-ADC-21 | 8.00 |
| HER3-ADC-4 | 8.16 | HER3-ADC-22 | 4.90 |
| HER3-ADC-8 | 6.38 | HER3-ADC-23 | 4.69 |
| HER3-ADC-9 | 4.26 | HER3-ADC-24 | 6.55 |
| HER3-ADC-16 | 5.36 | HER3-ADC-25 | 5.20 |
| HER3-ADC-18 | 12.83 | HER3-ADC-26 | 6.71 |

(continued)

| ADC number | DAR | ADC number | DAR |
|---|---|---|---|
| HER3-ADC-19 | 7.63 | HER3-ADC-27 | 6.45 |
| HER3-ADC-20 | 7.54 | | |

**Comparative example 1:**

The preparation method of the positive reference drug U3-1402 (PATRITUMAB DERUXTECAN) used in the test example of the present invention is prepared by referring to the preparation method of the antibody-drug conjugate (16a) in patent CN106163559B.

**Test Example 1: Inhibition Test of Exatecan Derivatives or Analogues (Payload) of the Present Invention on In Vitro Proliferation of Tumor Cells**

The cell line used in this experiment is the MDA-MB-468 cell line with high TROP-2 expression (Nanjing Cobioer Biosciences Co., Ltd., catalog number: CBP60387). Prepare cell suspension with fresh cell culture medium containing 10% FBS at a density of $3\times10^3$ cells/mL, add 90μL per well into an all-white 96-well cell culture plate (Beyotime, item number: FCP968-80pcs), 5 % carbon dioxide 37 °C for 24h. Compounds 1-36 were formulated with DMSO to 1 mM, respectively. Add 20μL of different samples to be tested in the first column of the 96-well U-bottom dispensing plate, and the sample concentration is 1000uM; add 20μL DMSO to each well of the second to tenth columns. Take 5μL of the sample from the first column to 20μL DMSO in the second column, mix well, take 5μL to the third column, and so on to the ninth column. Take 5μL of the medicine from each well of the dispensing plate to 95μL of DMEM medium, and mix well. Add 10μL of prepared samples to be tested at different concentrations to the culture plate, and duplicate wells for each sample. 5% carbon dioxide and cultured at 37°C for 3 days. Add 50μL of CTG (CellTiter-Glo® Luminescent Cell Viability Assay, Promega, item number: G7573) to each well, mix on a shaker at room temperature for 30 minutes, incubate for 10 minutes, and read chemiluminescence on a microplate reader (TECAN, Spark). Graphpad Prism 8 software used for data analysis. The experimental results are shown in Table 6.

Table 6 The $IC_{50}$ value of Exatecan derivatives of the present invention to MDA-MB-468 cell proliferation inhibition in vitro

| Compound number | $IC_{50}$ (nM) | Compound number | $IC_{50}$ (nM) |
|---|---|---|---|
| 1 | 135.20 | 19 | 7.52 |
| 2 | 3.38 | 20 | 8.60 |
| 3 | 43.59 | 21 | 3.05 |
| 4 | 4.32 | 22 | 2.60 |
| 5 | 19.34 | 23 | 2.64 |
| 6 | 9.81 | 24 | 1.65 |
| 7 | 9.45 | 25 | 8.63 |
| 8 | 2.72 | 26 | 3.28 |
| 9 | 4.77 | 27 | 43.21 |
| 10 | 50.73 | 28 | 35.37 |
| 11 | 96.27 | 29 | 6.00 |
| 12 | 56.25 | 30 | 6.85 |
| 13 | 76.51 | 31 | 135.71 |
| 14 | 47.99 | 32 | 28.14 |
| 15 | 20.68 | 33 | 32.28 |
| 16 | 3.29 | 34 | 56.58 |
| 17 | 2.01 | 35 | 101.96 |

(continued)

| Compound number | $IC_{50}$ (nM) | Compound number | $IC_{50}$ (nM) |
|---|---|---|---|
| 18 | 10.22 | 36 | 6.34 |
| Conclusion: the Exatecan derivatives provided by the present invention have obvious proliferation inhibitory activity on MDA-MB-468 cells. | | | |

**Test Experiment 2: Antibody and ADC endocytosis experiment**

The cell line used in this test example was purchased from Nanjing Cobioer Biosciences Co., Ltd. MDA-MB-468 cells, item number: CBP60387, TROP-2 high expression cell line.

The cell suspension was prepared with FACS (2% FBS+PBS) staining buffer, and the density was $2\times10^6$ cells/mL, the cell suspension was spread on a 96-well U-bottom cell plate (UWP043096) at 50μL/well, and each sample was set up a set of parallel experiments. Dilute TROP-2 antibody (naked antibody) or TROP-2-ADC compound (TROP2-ADC-3, TROP2-ADC-4, TROP2-ADC-19, TROP2-ADC-21 or TROP2-ADC-23) to a concentration of 20ug/ml. Take 50 μl of TROP-2 antibody or TROP-2-ADC compound dilutions and add them to 96-well U-bottom cell plates, mix gently and then ice-bath for 60 minutes. Add 200μL of FACS staining buffer to wash the cells, centrifuge at 300 g at 4°C for 5mins, discard the supernatant, and repeat the washing twice. Add 30μL of FACS to each well to resuspend the cells, and incubate at 37°C for 15 min, 30 min, 60 min, and 90 min to internalize the antibodies bound to the cell surface. At each time point, another sample incubated for the same time at 4°C was used as a negative control for no internalization. At each time point, transfer the corresponding 37°C and 4°C incubated samples to ice and add 170μL of ice FACS to terminate internalization. Cells were precipitated by centrifugation at 300g for 5 min at 4°C. Add secondary antibody at a ratio of 0.5:100, 100μL/well. Incubate at 4°C for 30 min in the dark. Add 200μL FACS staining buffer to wash the cells and remove unbound antibody. Centrifuge at 300g at 4°C for 5 min to precipitate the cells, and discard the supernatant. Repeat the wash 2 times. Resuspend the stained cells in 100μL ice-cold PBS. After washing the cells, they were analyzed by flow cytometry.

The reduction in mean fluorescence intensity (MFI) of samples incubated at 37°C relative to samples incubated at 4°C can be obtained by the following formula:

(a) %MFI at $t_x$ time point=MFI of samples incubated at 37°C×100/MFI of samples incubated at 4°C;
(b) Percentage of internalization at $t_x$ time point = 100-% MFI at tx time point

According to the calculated percentage of internalization, a line graph of the percentage of internalization was drawn, as shown in Figure 1.

**Test Example 3 TROP-2 Antibody and TROP-2-ADC Affinity Detection**

In this test example, the affinity of the TROP-2 antibody and the TROP-2-ADC compound obtained according to the above examples to TROP-2 is detected by means of capturing antibody.

Use the Sensor Chip Protein A biosensor chip (Cytiva) to capture the antibody (TROP-2 antibody or TROP-2-ADC compound), then flow through the antigen TROP-2-his on the surface of the chip, and use the Biacore K8 instrument (Cytiva) Real-time detection of reaction signals to obtain association and dissociation curves. After the dissociation of each experiment cycle, the chip was washed and regenerated with regeneration buffer. After the experiment, GE Biacore K8 Evaluation version 3.0 software was used to fit the data with the (1:1) Langmuir model to obtain the affinity value. The affinity between antibody and protein is shown in Table 7:

Table 7 Affinity test results of TROP-2 antibody and TROP-2-ADC compound and protein

| ADC name | $K_D$(M) |
|---|---|
| Naked antibody (anti-TROP-2 monoclonal antibody) | 3.18E-10 |
| TROP-2-ADC-3 | 2.76E-10 |
| TROP-2-ADC-4 | 2.93E-10 |
| TROP-2-ADC-19 | 3.19E-10 |
| TROP-2-ADC-21 | 2.75E-10 |

(continued)

| ADC name | $K_D$(M) |
|---|---|
| TROP-2-ADC-23 | 2.62E-10 |

As shown in Table 7, the $K_D$ values of the naked antibody and the TROP-2-ADC compounds of the present invention are both in the picomolar range, and there is no significant difference between the two.

**Test Example 4: In vitro Proliferation Inhibition Test of Antibody Drug Conjugates of the Present Invention on TROP-2 Target Tumor Cells**

The cell line used in this experiment is the MDA-MB-468 cell line with high TROP-2 expression (Nanjing Cobioer Biosciences Co., Ltd., catalog number: CBP60387). Prepare cell suspension with fresh cell culture medium containing 10% FBS at a density of $2\times10^{5}$cells/mL, add 100μL per well into an all-white 96-well cell culture plate (Beyotime, product number: FCP968-80pcs), and culture with 5 % carbon dioxide at 37 °C for 24h. ADC samples were made up to 10μM in PBS. Take this as the first concentration, and use PBS for five-fold serial dilution, a total of 9 concentrations. Aspirate 50μL of culture solution from each well, and then add 50μL of the above-mentioned ADC solution, so the ADC concentration in the first well is 5μM, and the final volume is 100μL per well. 5% carbon dioxide and cultured at 37°C for 3 days. Add 100μL of CTG (CellTiter-Glo® Luminescent Cell Viability Assay, Promega, item number: G7573) to each well, mix on a decolorizing shaker at room temperature for 30 min, incubate for 10 minutes, read chemiluminescence on a microplate reader (TECAN, Spark), and use Graphpad Prism 5 software for data analysis. The experimental results are shown in Table 8.

Table 8 The $IC_{50}$ value of ADCs of the present invention on the in vitro proliferation inhibition of TROP-2 target tumor cells

| **ADC number** | **$IC_{50}$(nM)** | **ADC number** | **$IC_{50}$(nM)** |
|---|---|---|---|
| TROP-2-ADC-1 | 21.39 | TROP-2-ADC-14 | 33.98 |
| TROP-2-ADC-3 | 31.83 | TROP-2-ADC-16 | 17.99 |
| TROP-2-ADC-4 | 17.75 | TROP-2-ADC-18 | 6.598 |
| TROP-2-ADC-5 | 20.86 | TROP-2-ADC-19 | 37.52 |
| TROP-2-ADC-6 | 15.12 | TROP-2-ADC-20 | 421.5 |
| TROP-2-ADC-8 | 34.59 | TROP-2-ADC-21 | 57.22 |
| TROP-2-ADC-9 | 51.1 | TROP-2-ADC-22 | 519 |
| TROP-2-ADC-11 | 290.4 | TROP-2-ADC-23 | 20.39 |
| TROP-2-ADC-12 | 312.50 | -- | -- |
| Conclusion: the antibody-drug conjugates against TROP-2 target of the present invention has obvious proliferation inhibitory activity on TROP-2 positive cell MDA-MB-468 cells. | | | |

**Test Example 5 In vivo drug efficacy evaluation of MDA-MB-468 cell CDX mouse model**

Human triple-negative breast cancer cell MDA-MB-468 (Nanjing Cobioer Biosciences Co., Ltd., item number: CBP60387) was inoculated subcutaneously in the right flank of BALB/c-Nude nude mice ($5\times10^{6}$/200μL/mouse, with 50% low growth factor artificial basement membrane). After the cells were inoculated, the tumor grew for 10 days, the tumor volume grew to about 130 $mm^3$, and the animals were randomly divided into groups (D0), 5 animals in each group, 12 groups in total.

Tail vein injection was used, the control group was given PBS, and the experimental group was given the TROP-2-ADC compounds TROP2-ADC-3, TROP2-ADC-19, TROP2-ADC-21 or TROP2-ADC-23 provided by the present invention. The multiple doses are 5mg/kg, 2mg/kg, 1mg/kg, twice a week, for 2 weeks. Tumor volume and body weight were measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V): V=1/2L $_{long\ diameter}$ L $_{short\ diameter}$$^2$

Relative tumor volume (RTV): RTV=$V_T/V_0$

$$\text{Relative tumor proliferation rate T/C (\%)=TRTV/CRTV}\times 100\%$$

$$\text{Tumor inhibition rate (\%)=(CRTV-TRTV)/CRTV(\%)}$$

Wherein, $V_0$ and $V_T$ are the tumor volumes at the beginning of the experiment and at the end of the experiment, respectively. CRTV and TRTV are the relative tumor volumes of the blank control group (PBS) and the experimental group at the end of the experiment, respectively. The results are shown in Figure 2, the TROP-2-ADC compounds provided by the present invention can effectively inhibit the growth of MDA-MB-468 transplanted tumors in tumor-bearing nude mice, and in a dose-dependent manner, especially TROP-2-ADC19, TROP-2-ADC-21 have a more significant inhibitory effect.

**Test Example 6: In vivo drug efficacy evaluation of Bxpc-3 cell CDX mouse model**

Human orthotopic pancreatic cancer cells Bxpc-3 (Cobioer) ($4\times10^6$/200μL/mouse, with 50% low growth factor artificial basement membrane) were inoculated subcutaneously in the right flank of BALB/c-Nude nude mice. After the cells were inoculated, the tumor grew for 6 days, the tumor volume grew to about 130 $mm^3$, and the animals were randomly divided into groups ($D_0$), 5 animals in each group, 4 groups in total.

Adopt tail vein injection administration, the control group applies PBS, the experimental group applies the TROP-2-ADC-21 compound provided by the present invention, and the administration dosage is 3mg/kg, 6mg/kg, 12mg/kg, 2 times a week, 5 times. Tumor volume and body weight were measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V): V=1/2L$_{long\ diameter}$ L $_{short\ diameter}$$^2$
Relative tumor volume (RTV): RTV=$V_T/V_0$

$$\text{Relative tumor proliferation rate T/C (\%)=TRTV/CRTV}\times 100\%$$

$$\text{Tumor inhibition rate (\%)=(CRTV-TRTV)/ CRTV(\%)}$$

Wherein, $V_0$ and $V_T$ are the tumor volumes at the beginning of the experiment and at the end of the experiment, respectively. CRTV and TRTV are the relative tumor volumes of the blank control group (PBS) and the experimental group at the end of the experiment, respectively. The results are shown in Figure 3, and TROP-2-ADC-21 provided by the present invention can effectively inhibit the growth of Bxpc-3 transplanted tumors in tumor-bearing nude mice in a dose-dependent manner.

**Test Example 7 In Vivo Drug Efficacy Evaluation of NCI-N87 Cell CDX Mouse Model**

Human gastric cancer cells NCI-N87 (Cobioer) ($5\times10^6$/200 μL/mouse, with 50% low growth factor artificial basement membrane) were inoculated subcutaneously in the right flank of BALB/c-Nude nude mice. After the cells were inoculated, the tumor grew for 3 days, and when the tumor volume grew to about 130 $mm^3$, the animals were randomly divided into groups ($D_0$), 5 animals in each group, 4 groups in total.

Adopt tail vein injection administration, the control group applies PBS, and the experimental group applies the TROP-2-ADC-21 compound provided by the present invention. The administration dosage is 3mg/kg, 6mg/kg, 12mg/kg, 2 times a week for 2 weeks. Tumor volume and body weight were measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V): V=1/2L $_{long\ diameter}$ L $_{short\ diameter}$$^2$
Relative tumor volume (RTV): RTV=$V_T/V_0$

$$\text{Relative tumor proliferation rate T/C (\%)=TRTV/CRTV}\times 100\%$$

$$\text{Tumor inhibition rate (\%)=(CRTV-TRTV)/CRTV(\%)}$$

Wherein, $V_0$ and $V_T$ are the tumor volumes at the beginning of the experiment and at the end of the experiment, respectively. CRTV and TRTV are the relative tumor volumes of the blank control group (PBS) and the experimental group at the end of the experiment, respectively. The results are shown in Figure 4, and the results show that TROP-2-ADC-21 provided by the present invention can effectively inhibit the growth of NCI-N87 transplanted tumors in tumor-bearing nude mice in a dose-dependent manner.

**Test Example 8: In vitro proliferation inhibition test of the antibody drug conjugates of the present invention on tumor cells targeting HER3**

The cell lines used in this experiment were human breast cancer cell line HCC1569, human colorectal adenocarcinoma cell line SW620, human malignant melanoma cell line A375, and human non-small cell lung cancer cell line NCI-H358 with high expression of HER3. The above cell lines were inoculated into a 96-well plate at a density of $10^3$ cells/100μL/well, and 200μL PBS was added to the outermost circle to reduce the volatilization of the medium. Incubate at 37°C, 5% $CO_2$ for 48h and remove the 96-well plate. 100μL of diluted ADC samples were added to the wells, and the ADC samples were prepared to 10μM with PBS, which was used as the first concentration, and five-fold gradient dilutions were made with PBS, a total of 9 gradients, and the concentration at the last point was 0. Incubate at 37°C, 5% $CO_2$ for 120h. Take out the 96-well plate, remove the culture solution, and add 100μL of detection solution (CCK8:culture solution=1:9) to each well; place it in a 5% $CO_2$ incubator at 37°C for 4 hours; and use a microplate reader (TECAN, Cat. No.: CEY0017) to read the value at 450nm, record it with Excel, and use Graphpad Prism 5 software for data analysis. The experimental results are shown in Table 9-Table 12.

Table 9 $IC_{50}$ value of HER3-ADCs of the present invention on SW620 cell proliferation inhibition in vitro

| ADC number | $IC_{50}$ (nM) | ADC number | $IC_{50}$ (nM) |
|---|---|---|---|
| HER3-ADC-3 | 1635 | HER3-ADC-19 | 687.3 |
| HER3-ADC-4 | 950.9 | HER3-ADC-20 | 274.3 |
| HER3-ADC-8 | 2611 | HER3-ADC-21 | 579.9 |
| HER3-ADC-9 | 2212 | HER3-ADC-22 | 2664 |
| HER3-ADC-16 | 697.2 | HER3-ADC-23 | 1026 |
| HER3-ADC-18 | 2377 | | |

Table 10 The $IC_{50}$ value of HER3-ADCs of the present invention on HCC1569 cell proliferation inhibition in vitro

| ADC number | $IC_{50}$ (nM) | ADC number | $IC_{50}$ (nM) |
|---|---|---|---|
| HER3-ADC-19 | 193.8 | HER3-ADC-25 | 131.8 |
| HER3-ADC-20 | 25.38 | HER3-ADC-26 | 80.72 |
| HER3-ADC-21 | 54.57 | HER3-ADC-27 | 88.07 |
| HER3-ADC-24 | 94.82 | | |

Table 11 $IC_{50}$ value of HER3-ADCs of the present invention on A375 cell proliferation inhibition in vitro

| ADC number | $IC_{50}$ (nM) | ADC number | $IC_{50}$ (nM) |
|---|---|---|---|
| HER3-ADC-19 | 4472 | HER3-ADC-21 | 225.9 |
| HER3-ADC-20 | 4143 | | |

Table 12 The $IC_{50}$ value of the ADCs of the present invention on the in vitro proliferation inhibition of NCI-H358 cells

| ADC number | $IC_{50}$ (nM) | ADC number | $IC_{50}$ (nM) |
|---|---|---|---|
| HER3-ADC-19 | 613.2 | HER3-ADC-21 | 42.43 |
| HER3-ADC-20 | 888.6 | | |
| Conclusion: The antibody-drug conjugates against HER3 target of the present invention have obvious proliferation inhibitory activity on HER3 positive cells SW620 cells, HCC1569 cells, A375 cells and NCI-H358 cells. | | | |

**Test Example 9: In vivo drug efficacy evaluation of the antibody-drug conjugates of the present invention on the CDX mouse model of tumor cell MDA-MB-453**

Human breast cancer cells MDA-MB-453 (Cobioer) ($5\times10^6$/200μL/mouse, with 50% low growth factor artificial basement membrane) were inoculated subcutaneously in the right flank of BALB/c-Nude nude mice. After the cells were inoculated, the tumor grew for 10 days, the tumor volume grew to about 130 $mm^3$, and the animals were randomly divided into groups (denoted as day 0, ie., D0), with 5 animals in each group, 10 groups in total. It is administered by tail vein injection, with multiple doses of 5mg/kg and 10 mg/kg administered twice a week for 2 weeks. Tumor volume and body weight were measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V):

$$V=1/2*L_{\text{long diameter}}*L_{\text{short diameter}}2$$

Relative tumor volume (RTV): $RTV=V_T/V_0$

$$\text{Relative tumor proliferation rate T/C (\%)}=TRTV/CRTV\times100\%$$

$$\text{Tumor inhibition rate (\%)}=(CRTV-TRTV)/\ CRTV(\%)$$

Wherein, $V_0$ and $V_T$ are the tumor volumes at the beginning of the experiment and at the end of the experiment, respectively. CRTV and TRTV are the relative tumor volumes of the blank control group (PBS) and the experimental group at the end of the experiment, respectively. The tumor growth curve is shown in Figure 5. On the whole, the inhibitory effect of the HER3-ADC compound provided by the present invention on tumors in animals is positively correlated with the dosage. The HER3-ADC-24, HER3-ADC- 25. HER3-ADC-26, HER3-ADC-27, HER3-ADC-19, HER3-ADC-20 and HER3-ADC-21 provided by the present invention can all have a very significant inhibitory effect on MDA-MB-453 tumors in mice.

**Test Example 10: In vivo drug efficacy evaluation of the antibody-drug conjugates of the present invention on tumor cell SW620 cell CDX mouse model**

Human colorectal adenocarcinoma cells SW620 ($5\times10^6$/200μL/mouse) were inoculated subcutaneously in the right flank of BALB/c-Nude nude mice. After the tumor grew for 8 days, the tumor volume grew to about 130$mm^3$, and the animals were randomly divided into groups (denoted as day 0, ie., $D_0$), with 5 animals in each group, 14 groups in total. Tail vein injection was used, and multiple doses of 3mg/kg, 6mg/kg and 12 mg/kg were administered, twice a week for 2 weeks. Tumor volume and body weight were measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V): $V=1/2*L_{\text{long diameter}}*L_{\text{short diameter}}^2$
Relative tumor volume (RTV): $RTV=V_T/V_0$

$$\text{Relative tumor proliferation rate T/C (\%)}=TRTV/CRTV\times100\%$$

$$\text{Tumor inhibition rate (\%)}=(CRTV-TRTV)/CRTV(\%)$$

Wherein, $V_0$ and $V_T$ are the tumor volumes at the beginning of the experiment and at the end of the experiment, respectively. CRTV and TRTV are the relative tumor volumes of the blank control group (PBS or vehicle) and the experimental group at the end of the experiment, respectively. The inhibitory effect of the HER3-ADC compound provided by the present invention on tumors in animals shows a positive correlation with the dose, and the tumor growth curve is shown in Figure 6. HER3-ADC-19, HER3-ADC-20 and HER3-ADC-21 provided by the present invention can have a very significant inhibitory effect on tumors, and HER3-ADC-21 still has an obvious inhibitory effect on tumors at a low dose of 3mg/kg.

**Test Example 11 Detection of Antibodies and ADC Endocytosis by Flow Cytometry**

1. Instruments and reagents

| Name | Model and level | Manufacturer |
|---|---|---|
| 96 Well EIA/RIA Plate | 3590 | Corning Incorporated Costor |
| Flow cytometry | Novocyte3130 | ACEA Biosciences,Inc |
| Goat anti-Human IgG Fc Secondary Antibody, PE, eBioscience™ | 12-4998-82 | Thermo Fisher Scientific |
| NowFlow Sheath Fluid | 875B601C | Agilent Biosciences |
| DMEM(1X) | 11885084 | gibco |
| Pancreatic enzyme | 8049-47-6 | J&K Scientific |
| PBS | 20012027 | GIBCO |

2. Experimental steps

a. Harvest cells: MAD-MB-453, HCC1569, and SW620 are adherent cells. Trypsinization is used to detach the cells from the culture dish, and the cell suspension is placed at the bottom of the centrifuge tube. The cell viability was measured by trypan blue staining, the cells were precipitated by centrifugation at 300 g at 4°C for 5 min, and the cells were washed once with FACS staining buffer. Centrifuge the cells again and resuspend the cells with an appropriate volume of FACS staining buffer to make the cell concentration $2\times10^6$ cells/ml after resuspension.
b. Prepare cells for staining: spread the cell suspension in 96-well U-shaped bottom cell plate at 50 μL/well ($1\times10^5$ cells/well), and set up a group of parallel experiments for each sample.
c. Antibody binding to cells: the HER3 monoclonal antibody (naked antibody) prepared according to the example of the present invention, or the drug-coupled ADC compound was used as the primary antibody. Dilute the primary antibody to a concentration of 20μg/ml with FACS staining buffer. Take 50μL of the primary antibody dilution, add it to a 96-well U-shaped bottom cell plate, mix gently and then ice-bath for 60 min.
d. Wash unbound antibody: Add 200μL FACS staining buffer to wash the cells, centrifuge at 300 g at 4°C for 5 min, discard the supernatant, and repeat the washing twice.
e. Incubate at 37°C to internalize the bound antibody: Add 30μL FACS to resuspend the cells in each well, and incubate at 37°C for 15mins, 30mins, 45 min, and 60mins to internalize the antibody bound to the cell surface. At each time point, another sample without adding antibody incubated at 4°C for the same time was used as a negative control for no internalization. At each time point, transfer the corresponding 37°C and 4°C incubated samples to ice and add 170μL of ice FACS to terminate internalization. Cells were precipitated by centrifugation at 300 g for 5mins at 4°C.
f. Use a fluorescently labeled secondary antibody (Goat anti-Human IgG Fc Secondary Antibody) to label the antibody bound to the cell surface: add the secondary antibody at a ratio of 0.5:100, 100μL/well. Incubate at 4°C for 30mins in the dark.
g. Wash unbound secondary antibody: add 200μL FACS staining buffer to wash the cells and remove unbound antibody. Centrifuge at 300 g at 4°C for 5 min to precipitate the cells, and discard the supernatant. Repeat the wash 2 times. Resuspend the stained cells in 100μL ice-cold PBS.
h. Fluorescence intensity on the cell surface detected by flow cytometry: After the cells are washed, analyzed by flow cytometry as soon as possible.
i. Calculation of the internalization level of the cell surface-bound antibody: It is obtained by the reduction level of the mean fluorescence intensity (MFI) of the sample incubated at 37°C relative to the sample incubated at 4°C, and the formula is as follows: %MFI at $t_x$ time point = MFI of samples incubated at 37°C × 100/MFI of samples incubated at 4°C;

Percentage of internalization at $t_x$ time point = 100 - %MFI at $t_x$ time point

Draw a line graph of the percentage of internalization based on the percentage of internalization calculated. The endocytosis of the HER3-ADC compound provided by the present invention is the key to the efficacy of the ADC. In this experiment, flow cytometry was used to detect the internalization level of the naked antibody and the HER3-ADC compound in cells HCC1569, SW620, and MDA-MB-453. The results are shown in Figure 7, with the prolongation of time, the internalization rate gradually increases; at 60 min, the internalization rates of the naked antibody and the HER3-ADC compounds of the present invention in the three kinds of cells all reach more than 50%, and the conjugation does not affect antibody endocytosis.

**Test example 12 Biacore detects the affinity of ADC compounds**

1. Instruments and reagents

| Name | Model and level | Manufacturer |
|---|---|---|
| Protein Analysis System | Biacore 8K | Cytiva |
| Biacore chip | Sensor Chip Protein A | Cytiva |
| Mobile phase | HBS-P+buffer | Cytiva |
| Regenerative liquid | Regeneration buffer | Cytiva |
| Ultrapure water | ≥ 18.2MΩ | Milli-Q |

2. Experimental steps

Taking the HER3 monoclonal antibody (naked antibody) and the ADC compounds after coupling the drug provided by the present invention as the detection target

a. Capture antibody: capture antibody at a flow rate of 10μl/min for 60 seconds.
b. Analysis: antigen binding at a flow rate of 30μl/min for 120 s, followed by dissociation at a flow rate of 30μl/min for 400 s.
c. Regeneration: Regenerate for 30 seconds at a flow rate of 30μl/min.
d. Process the results: wait for the end of the experiment, and open the analysis software to process the results.

The binding ability of naked antibody, HER3-ADC-19, HER3-ADC-20, HER3-ADC-21 to human HER3-His antigen was determined at 7 different concentrations (0.625-40nM) using protein A biosensor chip. As shown in Table 13, the $K_D$ values of both naked antibody and HER3-ADC compounds were in the nanomolar range, and there was no significant difference between them.

Table 13 Affinity between ADC and antigen

| ADC Name | Antigen | ka (1/Ms) | kd (1/s) | $K_D$(M) |
|---|---|---|---|---|
| Naked antibody | HER3-His | 2.57e+5 | 3.63e-4 | 1.41e-9 |
| HER3-ADC-19 | HER3-His | 2.18e+5 | 4.28e-4 | 1.96e-9 |
| HER3-ADC-20 | HER3-His | 2.99e+5 | 4.06e-4 | 1.36e-9 |
| HER3-ADC-21 | HER3-His | 2.17e+5 | 4.51e-4 | 2.08e-9 |

**Test Example 13 Evaluation of the Drug Efficacy of the Antibody-Drug Conjugate of the Present Invention on the Melanoma Cell A375 Mouse Model in Vivo**

Melanoma cell A375 (derived from ATCC, $5\times10^6$/200μL/mouse, with 50% low growth factor artificial basement membrane) was inoculated subcutaneously in the right flank of BALB/c-Nude nude mice. After the cells were inoculated, the tumor grew for 8 days, the tumor volume grew to about 130 $mm^3$, and the animals were randomly divided into groups (denoted as D0, that is, day 0), with 5 animals in each group, 4 groups in total. Tail vein injection was used, and the tested

drugs were HER3-ADC-21 (5 mg/kg), positive control drug U3-1402 (5 mg/kg), HER3 monoclonal antibody (10 mg/kg) and blank control group (PBS), 2 times a week for 2 weeks. Tumor volume was measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V): $V=1/2L_{\text{long diameter}} L_{\text{short diameter}}^2$
Relative tumor volume (RTV): $RTV=V_T/V_0$

$$\text{Relative tumor proliferation rate T/C (\%)=TRTV/CRTV}\times 100\%$$

$$\text{Tumor inhibition rate (\%)=(CRTV-TRTV)/ CRTV (\%)}$$

Wherein, $V_0$ and $V_T$ are the tumor volumes at the beginning of the experiment and at the end of the experiment, respectively. CRTV and TRTV are the relative tumor volumes of the blank control group (Vehicle) and the experimental group at the end of the experiment, respectively. The results were shown in Figure 8.

According to the results in FIG. 8, the HER3-ADC-21 provided by the present invention can effectively inhibit the growth of A375 transplanted tumors in tumor-bearing nude mice. Moreover, the HER3-ADC-21 provided by the present invention has a better tumor inhibitory effect than the positive reference drug U3-1402 at a dose of 5mg/kg.

**Test Example 14: In vivo drug efficacy evaluation of the antibody-drug conjugates of the present invention on human non-small cell lung cancer cell NCI-H358 mouse model**

Human non-small cell lung cancer cells NCI-H358 (derived from ATCC, $3\times10^6$/200 μL/mouse, with 50% low growth factor artificial basement membrane) were inoculated subcutaneously in the right flank of BALB/c-Nude nude mice. After the cells were inoculated, the tumor grew for 5 days, the tumor volume grew to about 130 $mm^3$, and the animals were randomly divided into groups (denoted as D0, ie., day 0), with 5 animals in each group, 4 groups in total. Tail vein injection was used, and the tested drugs were HER3-ADC-21 (10 mg/kg), positive control drug U3-1402 (10 mg/kg), HER3 monoclonal antibody (10mg/kg) and blank control group (PBS), 2 times a week for 2 weeks. Tumor volume was measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V): $V=1/2L_{\text{long diameter}} L_{\text{short diameter}}^2$
Relative tumor volume (RTV): $RTV=V_T/V_0$

$$\text{Relative tumor proliferation rate T/C (\%)=TRTV/CRTV}\times 100\%$$

$$\text{Tumor inhibition rate (\%)=(CRTV-TRTV)/ CRTV (\%)}$$

Wherein, $V_0$ and $V_T$ are the tumor volumes at the beginning of the experiment and at the end of the experiment, respectively. CRTV and TRTV are the relative tumor volumes of the blank control group (Vehicle) and the experimental group at the end of the experiment, respectively. The result is shown in Figure 9.

The results shown in Figure 9 show that the HER3-ADC-21 provided by the present invention can effectively inhibit the growth of NCI-H358 xenografted tumors in tumor-bearing nude mice, and has a better tumor inhibitory level than the positive reference drug U3-1402.

**Test Example 15 Plasma Stability Detection**

1. Instruments and Reagents

Table 14 Instruments and Reasents

| Name | Model and level | Manufacturer |
|---|---|---|
| One-component TMB color development solution | PR1200-1000ml | Solarbio |

(continued)

| Name | Model and level | Manufacturer |
|---|---|---|
| Tissue Culture Plate 96 well | TCP-012-096 | JETBIOFIL |
| Mouse Anti-Human IgG Fc Antibody [HRP],mAb | A01854-200 | GenScript |
| Biochemical Incubator | CRH-250 | Shanghai Yiheng Instrument Co., Ltd. |
| PBS | 20012027 | GIBCO |
| ELISA Terminator | C1058 | Solarbio |

2. Experimental steps

a. Dilute HER3-ADC-21 and U3-1402 (positive drugs) to 1 mg/ml with PBS, add the above ADC samples to mouse, rat, monkey, and human plasma to obtain a final concentration of 100μg/ml ADC plasma solution.
b. Dispense the ADC drugs, culture them in a 37°C incubator, take out samples at 0h, 24h (1 day), 48h (2 days), 96h (4 days), 168h (7 days), and 336h (14 days), and put them in freeze at -80°C. The last one was incubated and all the samples were tested uniformly.
c. Antigen package: HER3 antigen detects total antibody content, and small molecule antibody (payload partial antibody) detects ADC content.
d. HER3-ADC-21 and the positive reference drug U3-1402 were used as standard curves, with three gradients: the concentration of the mother solution was 100ng/ml, 40ng/ml, and 10ng/ml, and the plasma solution of each antibody was used as the primary antibody. Each sample was diluted 1000, 3000, 9000, 27000 times. Incubate at 37°C for 1 h, and wash the plate 3 times with PBST.
e. Use Mouse Anti-Human IgG Fc Antibody [HRP] secondary antibody, diluted 1: 10000 times, incubate with 100μL per well for 1 h, and wash the plate 4 times with PBST.
f. Add color development solution to develop color for 5 minutes, add the terminator solution to terminate the reaction, and read at 450nm wavelength. The results are shown in Figure 10.

According to the results shown in Figure 10, the drug HER3-ADC-21 has higher stability in the four plasmas, and is more stable than the positive reference substance U3-1402.

**Test Example 16 Rat PK Experiment**

1. Instruments and Reagents

Table 15 Instruments and Reagents

| Name | Model and level | Manufacturer |
|---|---|---|
| SD rats | males/9 weeks/200g | Beijing Vital River Laboratory Animal Technology Co., Ltd. |
| 0.5ml insulin syringe | 0.5ml/0.33mm*12.7mm | Beijing Likang Shengda Technology Development Co. |
| Single-use anticoagulation tubes | 20210101 | Jiangsu Kangjian Medical Apparatus Co. , Ltd |
| One-component TMB color development solution | PR1200-1000ml | Solarbio |
| Tissue Culture Plate 96 well | TCP-012-096 | JETBIOFIL |
| Mouse Anti-Human IgG Fc Antibody [HRP],mAb | A01854-200 | GenScript |
| PBS | 20012027 | GIBCO |
| ELISA Terminator | C1058 | Solarbio |
| Biochemical Incubator | CRH-250 | Shanghai Yiheng Scientific Instrument Co. |

2. Experimental steps

a, 8 rats with a body weight about 200g were screened including in the experiment, and were randomly divided into 2 groups according to body weight, with 4 rats in each group. The dose of HER3-ADC compound was 5mg/kg, and the volume of administration was 1 ml/kg. The method of administration was single tail vein administration.
b, Sample collection time points: before administration (0h) and after administration 5min, 1h, 4h, 8h, 24h, 48h, 96h (Day4), 168h (Day7), 336h (Day14), 504h (Day21), 672h (Day 28); Whole blood was collected from the jugular vein in each treatment group. Blood collection volume: 0.3mL/time point, EDTA anticoagulated whole blood. After the whole blood was collected, it was placed on wet ice and centrifuged within 1 hour to obtain the supernatant. The centrifugation speed was 4000 rpm and the centrifugation time was 10 minutes. Plasma samples were frozen and stored in a -80°C refrigerator after collection.
c, Antigen-coated plate: HER3 antigen was used to detect the total antibody content, and the small molecule antibody was used to detect the ADC content.
d, ADC-21 and U3-1402 antibodies were used for standard curve, rat plasma solution was used as the primary antibody, and each sample was diluted 1000, 3000, 9000, 27000 times. Incubate at 37°C for 1 h, and wash the plate 3 times with PBST.
e, Use Mouse Anti-Human IgG Fc Antibody [HRP] secondary antibody, diluted 1:10000 times, incubate with 100μL per well for 1 h, and wash the plate 4 times with PBST.
f, add color development solution to develop color for 5 minutes, add terminator solution to terminate the reaction, and read at 450nm wavelength.
g, The payload (Exatecan derivative) dropped in rat plasma was detected by LC-MS. The experimental results are shown in Figures 11-14.

According to the experimental results shown in Figures 11-14: when the administration concentration is 5 mg/ml, the half-life of HER3-ADC-21 is longer than the positive reference drug (U3-1402) (HER3-ADC-21: U3-1402= 91.378h:83.014h). Using LC-MS to detect the small molecule content of HER3-ADC-21 and U3-1402 in plasma showed that the small molecule content of HER3-ADC-21 in rat plasma was lower than the detection limit (0.5ng/ml); the small molecules of the positive reference drug U3-1402 were shed to some extent in rats.

**Test Example 17 Bystander Effect of HER3-ADC Drugs**

In this experiment, HCC1569 cells (HER3 positive cell line) and Nalm6-GFP (HER3 negative cell line) cells were co-cultured to detect the bystander killing ability of HER3-ADC-21.
1. Instruments and Reagents

Table 16 Instruments and Reagents

| Name | Model and level | Manufacturer |
|---|---|---|
| Zombie NIR™ Fixable Viability Kit | 3590 | Corning Incorporated Costor |
| flow cytometer | Novocyte3130 | ACEA Biosciences,Inc |
| 6-hole plate | 140675 | Thermo Fisher Scientific |
| RPMI+GlutaMAX(1X)RPMI medium 1640 | 61870036 | gibco |
| pancreatic enzyme | 8049-47-6 | Beijing J&K Scientific Ltd. |
| PBS | 20012027 | GIBCO |
| Tissue Culture Plate 96 well | TCP-012-096 | JETBIOFIL |

2. Experimental steps

1. The process of dispensing drug:

a. Collect HCC1569 cells for counting, and the cell viability is guaranteed to be above 91%. HCC1569 cells (HER3-positive cell line) were plated in 6-well plates at $3\times10^5$ cells/well and incubated for 24 hours. Another set of only HCC1569 was used for control experiments.
b. After culturing for 24 hours, collect Nalm6-GFP cells (HER3-negative cell line) for counting, and the cell viability is guaranteed to be above 96%. Nalm6 cells were plated in 6-well plates at $1.5\times10^{\wedge5}$ cells/well. In addition, a

group of only Nalm6-GFP cells was prepared for the control experiment.
c. After incubation for 24 hours, HER3-ADC-21 compounds were added at concentrations of 5nM, 10nM, 40nM, and 80nM.
d. Continue to incubate for 5 days for detection.

2. Detection steps:

a. After collecting the cells, wash them three times with PBS.
b. 100uL NIR staining solution (diluted 1: 1000) was stained in the dark for 15 minutes, washed once with PBS, and then performed flow cytometric detection.
c. Flow excitation light selection: APC-cy7, FITC. The sample volume is 70uL, and the sample loading speed is high speed.

Calculation formula: Killing rate = (cell viability rate without drug application * cell positive rate - cell viability rate with drug application* cell positive rate) / cell viability rate without drug application * cell positive rate, wherein the cell positive rate refers to the percentage of Nalm6 cells Ratio in co-cultured cells (HCC1569 cells and Nalm6-GFP cells). The results are shown in Table 17.

According to the results shown in Table 17, when cultured alone, HER3-ADC-21 has no killing effect on Nalm6-GFP (HER3-negative cell line) cells, but has a killing effect on HCC1569 (HER3-positive cell line) cells. After HCC1569 (HER3-positive cell line) cells and Nalm6-GFP (HER3-negative cell line) cells were co-cultured, HER3-ADC-21 had a killing effect on Nalm6-GFP (HER3-negative cell line) cells. In summary, HER3-ADC-21 has a bystander effect.

Table 17 HER3-ADC bystander effect test results

| | kill rate | | |
|---|---|---|---|
| ADC compound concentration (nM, based on payload(exatecan derivatives) ) | Nalm6-GFP cultured separately. | HCC1569 cultured separately. | Killing rate of Nalm6-GFP (HER3-negative cell line) by co-culture of Nalm6-GFP cells+ HCC1569 cells |
| 80 | 2.80% | 96.66% | 88.46% |
| 40 | -7.29% | 71.73% | 61.92% |
| 10 | 7.43% | 71.12% | 38.22% |
| 5 | 4.82% | 19.64% | 25.62% |
| 0 | 0.00% | 0.00% | 0.00% |

**Test Example 18: TROP-2-ADC drug bystander effect**

MDA-MB-468 (human breast cancer cells, Nanjing Cobioer, CBP60387, TROP-2 positive cells) and Nalm6-GFP cells (human B lymphoid leukemia cells, purchased from Creative Biogene, CSC-RR0360, TROP-2 negative cells) were culture using DMEM/low glucose + 10% FBS and RPMI1640 + 10% FBS respectively, trypsinize the cells, neutralize with fresh medium, centrifuge at 300g for 5 minutes, discard the supernatant, and resuspend the cells in RPMI1640 + 10% FBS. After cell counting, 200,000 MDA-MB-468 cells/well and 100,000 Nalm6-GFP cells/well were spread in a 6-well plate according to the ratio of MDA-MB-468:Nalm6-GFP=2:1. In addition, two groups of cells containing only MDA-MB-468 and Nalm6-GFP were used for control experiments. After 24 hours of incubation, ADC compounds were added at drug concentrations of 5nM, 10nM, 20nM, 40nM, 80nM. After continuing to incubate for 5 days, detection was performed. For detection, cells were collected and washed three times with PBS. 100uL NIR (Corning Incorporated Costor, 3590) staining solution (diluted 1: 1000) was stained in the dark for 15min, washed once with PBS, and flow cytometric detection was performed.

According to the streaming results, calculate the killing rate:
Killing rate= (cell viability rate without drug application*cell positive rate- cell viability rate with drug application *cell positive rate)/ cell viability rate without drug application *cell positive rate; the cell positive rate refers to the percentage of Nalm6-GFP cells in the total ratio of cultured cells (MDA-MB-468 cells and Nalm6-GFP cells). The results are shown in Table 18 and Table 19.

According to the results in Table 18, the TROP-2-ADC molecule of the present invention has obvious killing effect on TROP-2 positive cells, but has no obvious inhibitory effect on TROP-2 negative cells. In cells co-cultured with TROP-2 positive cells and TROP-2 negative cells, the ADC molecule of the present application can significantly inhibit both

TROP-2 positive and TROP-2 negative cells, showing obvious bystander killing effect. TROP-2-ADC drugs also have a bystander effect. The results in Table 19 show that other TROP-2-ADC molecules of the present invention also have obvious bystander killing effects.

Table 18 The killing rate of TROP-2-ADC-21 on MDA-MB-468 cells and Nalm6-GFP cells when cultured alone and co-cultured respectively

| | killing rate(%) | | |
|---|---|---|---|
| ADC compound concentration (nM, Based on payload (exatecan derivatives) | Nalm6-GFP cells | MDA-MB-468 cells | Killing rate of Nalm6-GFP by co-culture of Nalm6-GFP cells+ MD-MB-468 cells |
| 80 | -4.85% | 73.68% | 80.13% |
| 40 | -2.82% | 61.58% | 78.97% |
| 20 | -1.21% | 67.63% | 62.16% |
| 10 | -5.80% | 56.21% | 77.79% |
| 5 | -6.36% | 20.49% | 63.98% |
| 0 | 0.00% | 0.00% | 0.00% |

Table 19 The killing rate of TROP-2-ADC to Nalm6-GFP cells in the co-culture of MDA-MB-468 cells and Nalm6-GFP cells

| ADC name | 80nM | 40nM | 20nM | 10nM | 5nM |
|---|---|---|---|---|---|
| TROP-2-ADC-3 | 56.71% | 52.78% | 36.59% | 45.66% | 31.56% |
| TROP-2-ADC-4 | 74.08% | 59.29% | 49.92% | 45.44% | 47.42% |
| TROP-2-ADC-19 | 75.06% | 64.13% | 61.27% | 61.19% | 49.02% |
| TROP-2-ADC-23 | 78.74% | 76.22% | 74.73% | 71.85% | 63.76% |

**Test Example 19: In vivo drug efficacy evaluation of the antibody-drug conjugate of the present invention on tumor cell SW620 cell CDX mouse model**

Human colorectal adenocarcinoma cells SW620 ($5\times10^6$/200 μL/mouse) were inoculated subcutaneously in the right flank of BALB/c-Nude nude mice. The tumor grew for 8 days, the tumor volume grew to about 130 $mm^3$, and the animals were randomly divided into groups (denoted as D0, ie., day 0), with 5 animals in each group, 11 groups in total. It is administered by tail vein injection, with a single dose of 10 mg/kg, twice a week, for 2 weeks. Tumor volume and body weight were measured twice a week, and the data were recorded. Excel 2016 statistical software is used for data statistics: average value is calculated by average; SD value is calculated by STDEV; SEM value is calculated by STDEV/SQRT. Tumor growth curves were made using GraphPad Prism 8.0.2.2.263 software.

Tumor volume (V): $V=1/2*L_{\text{long diameter}}*L_{\text{short diameter}}^2$
Relative tumor volume (RTV): $RTV=V_T/V_0$

$$\text{Relative tumor proliferation rate T/C (\%)}=\text{TRTV/CRTV}\times100\%$$

$$\text{Tumor inhibition rate (\%)}=(\text{CRTV-TRTV})/\text{CRTV}(\%)$$

Wherein, $V_0$ and $V_T$ are the tumor volumes at the beginning of the experiment and at the end of the experiment, respectively. CRTV and TRTV are the relative tumor volumes of the blank control group (Vehicle) and the experimental group at the end of the experiment, respectively. The results are shown in Figure 15, and the HER3-ADC compounds provided by the present invention have a significant inhibitory effect on tumors in animals.

The present invention has been described through the above-mentioned examples, but it should be understood that the above-mentioned examples are only for the purpose of illustration and description, and are not intended to limit the present invention to the scope of the described embodiments. In addition, those skilled in the art can understand that the

present invention is not limited to the above-mentioned examples, and more variations and modifications can be made according to the teachings of the present invention, and these variations and modifications all fall within the claimed scope of the present invention, within the range. The protection scope of the present invention is defined by the appended claims and their equivalent scope.

## Claims

**1.** A ligand-drug conjugate or a pharmaceutically acceptable salt thereof, wherein the ligand-drug conjugate comprises a structure shown by formula ($-D_0$ ):

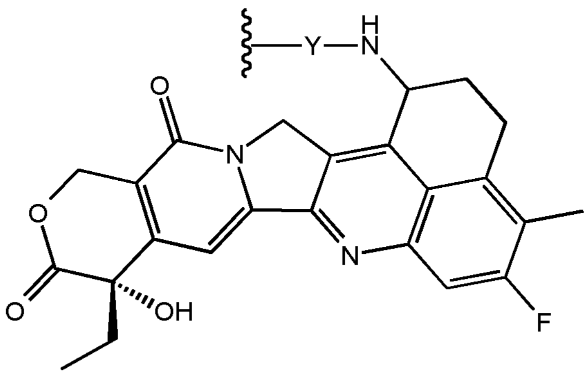

**($-D_0$)**

wherein , Y is

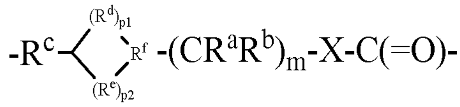

,

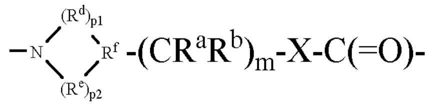

,

$-R^c-Ar^1-(Cy)_m-(CR^aR^b)_m-X-C(=O)-$, $-N((CR^aR^b)m\ CF3)-C(=O)-$, $-R^c-(CR^aR^b)m-C(=O)-R^c-Ar^1-(Cy)m-(CR^aR^b)m-X-C(=O)-$,

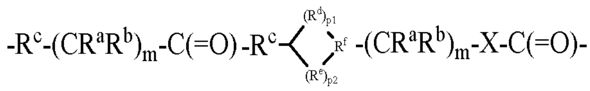

,

or

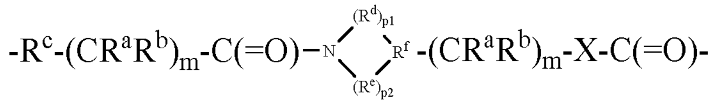

,

wherein the C=O- end of Y is connected to -NH- in the structure of formula ($-D_0$); preferably, Y is

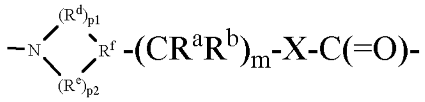

,

-O-$Ar^1$-$(Cy)_m$-$(CR^aR^b)_m$-X-C(=O)-, -NH-$Ar^1$-$(Cy)_m$-$(CR^aR^b)_m$-X-C(=O)-, -N($(CR^aR^b)_m CF_3$)-C(=O)-, -O-$(CR^aR^b)_m$-C(=O)-NH-$Ar^1$-$(Cy)_m$-$(CR^aR^b)_m$-X-C(=O)-, or

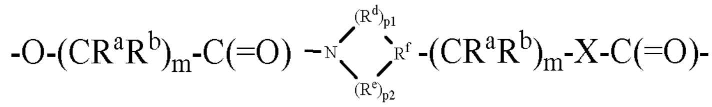

,

wherein the C(=O)- end of Y is connected to -NH- in the structure of formula (-$D_0$ );

$R^c$ at each occurrence is each independently NH, O, or S; $R^d$ and $R^e$ at each occurrence are each independently $C(R^m)_2$, $NR^m$, O, or S; $R^f$ at each occurrence is each independently $CR^m$ or N; preferably, when the ring formed by $R^d$, $R^e$, and $R^f$ is heterocyclyl , the heterocyclyl contains 1, 2, 3, or 4 heteroatoms, and the heteroatoms are each independently selected from N, O, and S;

$R^m$ at each occurrence is each independently H, halogen, haloalkyl , -OH, - CN, -$NO_2$, alkyl, alkoxy, -$NH_2$, -NH-alkyl, or -$N(alkyl)_2$, deuterium, deuterated alkyl, aminoalkylene, hydroxyalkylene, nitroalkylene, or cyanoalkylene; preferably, $R^m$ at each occurrence is each independently H, halogen, haloalkyl, -OH, -CN, -$NO_2$, alkyl, alkoxy, -$NH_2$, -NH-alkyl, or - $N(alkyl)_2$;

p1 is an integer from 0 to 17, p2 is an integer from 1 to 18, and $p1+p2\leq18$; preferably, p1 is an integer of 0, 1, 2, 3, 4, 5, 6, or 7, p2 is an integer of 1, 2, 3, 4, 5, 6, 7, or 8, and $p1+p2\leq8$;

Cy at each occurrence is each independently selected from cycloalkylene, heterocyclylene, arylene,or heteroarylene; preferably, Cy at each occurrence is each independently selected from 3-10 membered cycloalkylene, 3-10 membered heterocyclylene, 6-10 membered arylene, or 5-10 membered heteroarylene; preferably, the heterocyclylene and heteroarylene contain 1, 2, 3, or 4 heteroatoms, wherein the heteroatoms are independently selected from N, O, and S; the cycloalkylene, heterocyclylene, arylene, and heteroarylene are unsubstituted or optionally substituted with a substituent selected from halogen, -OH, -CN, haloalkyl, -$NO_2$, alkyl, alkoxy, -$NH_2$, - NH-alkyl, and -$N(alkyl)_2$, preferably unsubstituted or optionally substituted with a substituent selected from halogen, -OH, -CN, -$CF_3$, -$NO_2$, $C_{1-6}$ alkyl, - $OC_{1-6}$ alkyl, -$NH_2$, -NH($C_{1-6}$ alkyl), and -N($C_{1-6}$ alkyl$)_2$;

$Ar^1$ at each occurrence is each independently selected from arylene or heteroarylene; preferably, $Ar^1$ at each occurrence is each independently selected from 6-10 membered arylene or 5-10 membered heteroarylene; preferably, the heteroarylene contains 1, 2, 3, or 4 heteroatoms, wherein the heteroatoms are independently selected from N, O, and S; the arylene and heteroarylene are unsubstituted or optionally substituted with a substituent selected from halogen, -OH, -CN, haloalkyl, -$NO_2$, alkyl, alkoxy, -$NH_2$, - NH-alkyl, and -$N(alkyl)_2$, preferably, unsubstituted or optionally substituted with a substituent selected from halogen, -OH, -CN, -$CF_3$, -$NO_2$, $C_{1-6}$ alkyl, - $OC_{1-6}$ alkyl, -$NH_2$, -NH($C_{1-6}$ alkyl), and -N($C_{1-6}$ alkyl$)_2$;

$R^a$ and $R^b$ are identical or different, and at each occurrence each independently selected from hydrogen, halogen, haloalkyl , -OH, -CN, -$NO_2$, alkyl, alkoxy, -$NH_2$, -NH-alkyl, or -$N(alkyl)_2$, deuterium, deuterated alkyl, aminoalkylene, hydroxyalkylene, nitroalkylene and cyanoalkylene; preferably, at each occurrence each independently selected from hydrogen, deuterium, halogen, alkyl, haloalkyl , deuterated alkyl, alkoxy, -OH, -$NH_2$, - CN, nitro, and hydroxyalkylene; preferably, at each occurrence each independently selected from hydrogen, deuterium, halogen, $C_{1-6}$ alkyl, halogenated $C_{1-6}$ alkyl, deuterated $C_{1-6}$ alkyl, -$OC_{1-6}$ alkyl, -OH, -$NH_2$, -CN, - $CF_3$, nitro, -$C_{1-6}$ alkylene, and -OH;

X at each occurrence is independently a single bond, -NH-, O, or S;

the wavy line in formula (-$D_0$) represents a covalent attachment to a linker unit, an antibody or its antigen-binding fragment or a polypeptide binding to an antigen expressed by a target cell; preferably, the wavy line in formula (-$D_0$) represents a covalent attachment through a linker unit to an antibody or its antigen-binding fragment or a polypeptide binding to an antigen expressed by a target cell; and

m at each occurrence is each independently an integer of 0, 1, 2, 3, or 4.

**2.** A ligand-drug conjugateor a pharmaceutically acceptable salt thereof, wherein the ligand-drug conjugate comprises a structure shown by formula (-D):

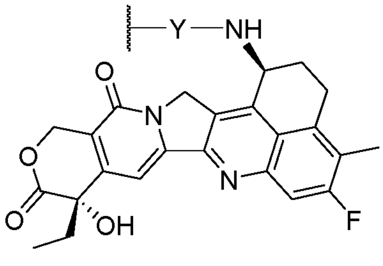

**(-D)**

wherein Y is

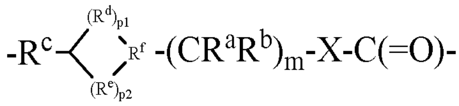 , 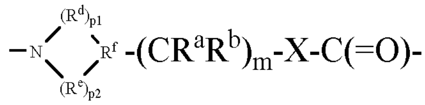 ,

-$R^c$-$Ar^1$-$(Cy)_m$-$(CR^aR^b)_m$-X-C(=O)-, -N($(CR^aR^b)_m CF_3$)-C(=O)-, -$R^c$-$(CR^aR^b)_m$-C(=O)-$R^c$-$Ar^1$-$(Cy)_m$-$(CR^aCR^b)_m$-X-C(=O)-,

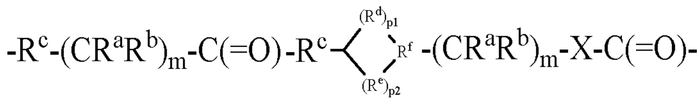 ,

or

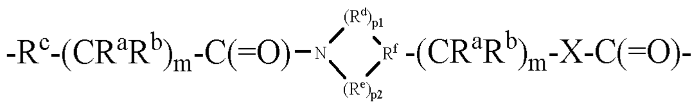 ,

wherein the C=O- end of Y is connected to -NH- in the structure of formula (-D); preferably, Y is

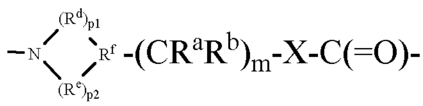 ,

-O-$Ar^1$-$(Cy)_m$-$(CR^aR^b)_m$-X-C(=O)-, -NH-$Ar^1$-$(Cy)_m$-$(CR^aR^b)_m$-X-C(=O)-, -N($(CR^aR^b)_m CF_3$)-C(=O)-, -O-$(CR^aR^b)_m$-C(=O)-NH-$Ar^1$-$(Cy)_m$-$(CR^aR^b)_m$-X-C(=O)-, or

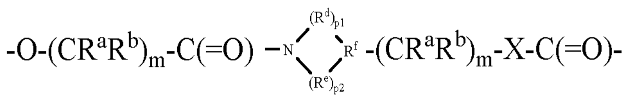 ,

wherein the C(=O)- end of Y is connected to -NH- in the structure of Formula (-D);

$R^c$ at each occurrence is each independently NH, O, or S; $R^d$ and $R^e$ at each occurrence are each independently $C(R)^m{}_2$, $NR^m$, O, or S; $R^f$ at each occurrence is independently $CR^m$ or N; preferably, when the ring formed by $R^d$, $R^e$ and $R^f$ is heterocyclyl, the heterocyclyl contains 1, 2, 3, or 4 heteroatoms, and the heteroatoms are each independently selected from N, O, and S;

$R^m$ at each occurrence is each independently H, halogen, haloalkyl, -OH, -CN, $-NO_2$, alkyl, alkoxy, $-NH_2$, -NH-alkyl, or -N$(alkyl)_2$, deuterium atom, deuterated alkyl, aminoalkylene, hydroxyalkylene, nitroalkylene, or cyanoalkylene, preferably, $R^m$ at each occurrence is each independently H, halogen, haloalkylene, -OH,

-CN, $-NO_2$, alkyl, alkoxy, $-NH_2$, -NH-alkyl, or - $N(alkyl)_2$;
p1 is an integer from 0 to 17, p2 is an integer from 1 to 18, and $p1 + p2 \leq 18$; preferably, p1 is an integer of 0, 1, 2, 3, 4, 5, 6, or 7, and p2 is an integer of 1, 2, 3, 4, 5, 6, 7, or 8, and $p1 + p2 \leq 8$;
the Cy at each occurrence is each independently selected from cycloalkylene, heterocyclylene, arylene,or heteroarylene, preferably the Cy at each occurrence is each independently selected from 3-10 membered cycloalkylene, 3-10 membered heterocyclylene, 6-10 membered arylene, or 5-10 membered heteroarylene; preferably, the heterocyclylene and heteroarylene contain 1, 2, 3, or 4 heteroatoms, and the heteroatoms are independently selected from N, O and S; the cycloalkylene, heterocyclylene, arylene, andheteroarylene are unsubstituted or optionally substituted with a substituent selected from halogen, -OH, -CN, haloalkylene, $-NO_2$, alkyl, alkoxy, $-NH_2$, -NH-alkyl, and $-N(alkyl)_2$, preferably unsubstituted or optionally substituted with a substituent selected from halogen, -OH, -CN, $-CF_3$, $-NO_2$ , $C_{1-6}$ alkyl, $-OC_{1-6}$ alkyl, $-NH_2$ , $-NH(C_{1-6}$ alkyl), and $-N(C_{1-6}$ alkyl$)_2$;
the $Ar^1$ at each occurrence is each independently selected from arylene or heteroarylene, preferably, the $Ar^1$ at each occurrence is each independently selected from 6-10 membered arylene or 5-10 membered heteroarylene; preferably the heteroarylene comprises 1, 2, 3, or 4 heteroatoms, and the heteroatoms are independently selected from N, O, and S; the arylene and heteroarylene are unsubstituted or optionally substituted with a substituent selected from halogen, -OH, -CN, haloalkyl, $-NO_2$, alkyl, alkoxy, $-NH_2$, -NH-alkyl, and $-N(alkyl)_2$, preferably, unsubstituted or optionally substituted with a substituent selected from halogen, -OH, -CN, $-CF_3$, $-NO_2$ , $C_{1-6}$ alkyl, $-OC_{1-6}$ alkyl, $-NH_2$, $-NH(C_{1-6}$ alkyl), and $-N(C_{1-6}$ alkyl$)_2$;
$R^a$ and $R^b$ are identical or different, and at each occurrence is each independently selected from hydrogen atom, halogen, haloalkyl, -OH, -CN, - $NO_2$, alkyl, alkoxyl, $-NH_2$, -NH-alkyl, or $-N(alkyl)_2$, deuterium atom, deuterated alkyl, aminoalkylene, hydroxyalkylene, nitroalkylene, and cyanoalkylene, preferably, at each occurrence each independently selected from hydrogen atom, deuterium atom, halogen, alkyl, haloalkyl, deuterated alkyl, alkoxy, -OH, $-NH_2$, -CN, nitro, and hydroxyalkylene, preferably, at each occurrence each independently selected from hydrogen atom, deuterium atom, halogen, $C_{1-6}$ alkyl, $C_{1-6}$ haloalkyl, deuterated $C_{1-6}$ alkyl, $-OC_{1-6}$ alkyl, -OH, - $NH_2$, -CN, $-CF_3$, nitro, $-C_{1-6}$ alkylene and -OH-;

X at each occurrence is each independently a single bond, -NH-, O, or S; the wavy line in formula (-D) represents a covalent attachment to a linker unit, an antibody or an antigen-binding fragment thereof, or a polypeptide binding to an antigen expressed by a target cell; preferably, the wavy line in formula ($-D_0$) represents a covalent attachment through a linker unit to an antibody or an antigen-binding fragment thereof, or a polypeptide binding to an antigen expressed by a target cell; and
m at each occurrence is each independently an integer of 0, 1, 2, 3, or 4.

**3.** The ligand-drug conjugate or a pharmaceutically acceptable salt thereof according to claim 1 or 2, which is a ligand-drug conjugate shown by the general formula ($Pc-L-D_0$) or the general formula (Pc-L-D), or a pharmaceutically acceptable salt thereof:

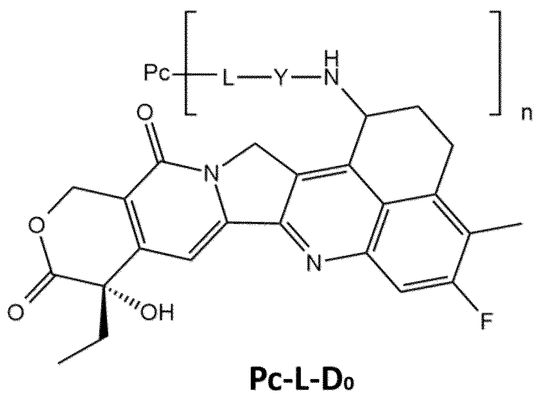

**Pc-L-D₀**

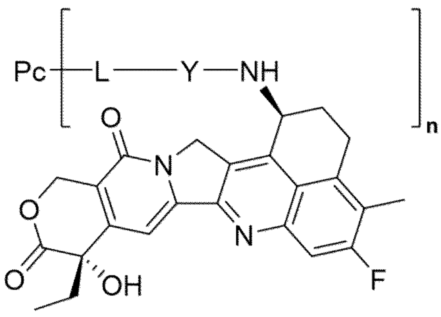

Pc-L-D

wherein n is an integer or a decimal from 1 to 15; preferably, n is an integer or a decimal from 1 to 13; preferably, n is an integer or a decimal from 1 to 10; more preferably, n is an integer or a decimal from 3 to 8;
Pc is a ligand; and L is a linker.

**4.** The ligand-drug conjugate or the pharmaceutically acceptable salt thereof according to any one of claims 1-3, wherein $R^c$ at each occurrence is each independently NH, or O; $R^d$ and $R^e$ at each occurrence are each independently $C(R^m)_2$ or $NR^m$; $R^f$ at each occurrence is $CR^m$; and when the ring formed by $R^d$, $R^e$, and $R^f$ is heterocyclyl, the heterocyclyl contains 1, 2, or 3 heteroatoms, and the heteroatoms are each independently selected from N and O; and/or

$R^m$ at each occurrence is each independently H, halogen, -OH, -CN, $-NO_2$, $-CF_3$, $-C_{1-6}$ alkyl, $-OC_{1-6}$ alkyl, $-NH_2$, $-NH(C_{1-6}$ alkyl), or $-N(C_{1-6}$ alkyl$)_2$ , preferably H, halogen, -OH, -CN, $-CF_3$, $-NO_2$ , $C_{1-6}$ alkyl, or $-OC_{1-6}$ alkyl, preferably H, halogen, -OH, -CN, $-CF_3$, $-NO_2$ , $C_{1-3}$ alkyl, or $-OC_{1-3}$ alkyl, preferably -Cl, -Br, -F, -OH, -CN, $-CF_3$, $-NO_2$ , $-CH_3$, or $-OCH_3$; and/or
p1 is an integer of 0, 1, 2, or 3, p2 is an integer of 1, 2, 3, or 4, and $2 \leq p1 + p2 \leq 4$; and/or
the Cy at each occurrence is each independently selected from 6-10 membered arylene, or 5-10 membered heteroarylene, preferably selected from phenylene, or 5-6 membered heteroarylene; preferably, the heteroarylene contains 1, 2, or 3 heteroatoms, and the heteroatoms are independently selected from N, O, and S; the heteroarylene is unsubstituted or optionally substituted with a substituent selected from halogen, -OH, -CN, $-CF_3$, $-NO_2$ , $C_{1-6}$ alkyl, $-OC_{1-6}$ alkyl, $-NH_2$, $-NH(C_{1-6}$ alkyl), and $-N(C_{1-6}$ alkyl$)_2$, preferably unsubstituted or optionally substituted with a substituent selected from halogen, -OH, -CN, $-CF_3$, $-NO_2$, $C_{1-6}$ alkyl, and $-OC_{1-6}$alkyl, preferably unsubstituted or optionally substituted with a substituent selected from halogen, -OH, -CN, $-CF_3$, $-NO_2$, $C_{1-3}$ alkyl, and $-OC_{1-3}$ alkyl, preferably unsubstituted or optionally substituted with a substituent selected from -Cl, - Br, -F, -OH, -CN, $-CH_3$, and $-OCH_3$;
$Ar^1$ at each occurrence is each independently selected from 6-10 membered arylene, preferably selected from phenylene; the arylene or phenylene is unsubstituted or optionally substituted with a substituent selected from halogen, -OH, -CN, $-CF_3$, $-NO_2$, $C_{1-6}$ alkyl, $-OC_{1-6}$ alkyl, $-NH_2$ , $-NH(C_{1-6}$ alkyl), and $-N(C_{1-6}$ alkyl$)_2$ , preferably unsubstituted or optionally substituted with a substituent selected from halogen, -OH, -CN, $-CF_3$, $-NO_2$, $C_{1-6}$ alkyl, and $-OC_{1-6}$ alkyl, preferably unsubstituted or optionally substituted with a substituent selected from halogen, -OH, -CN, $-CF_3$, $-NO_2$, $C_{1-3}$ alkyl, and - $OC_{1-3}$ alkyl, preferably unsubstituted or optionally substituted with a substituent selected from -Cl, -Br, -F, -OH, -CN, $-CF_3$, $-NO_2$, $-CH_3$, and $-OCH_3$; and/or
$R^a$ and $R^b$ are identicalor different and are each independently selected from hydrogen atom, deuterium atom, halogen, $C_{1-3}$ alkyl, $C_{1-3}$ haloalkyl, deuterated $C_{1-3}$alkyl, $-OC_{1-3}$ alkyl, -OH, $-NH_2$, -CN, $-CF_3$, $-NO_2$, and $C_{1-3}$ alkylene-OH; preferably, $R^a$ and $R^b$ are identical or different and are each independently selected from hydrogen atom, deuterium atom, and halogen; and/or
X is independently selected from single bond, -NH-, or O; and/or
m at each occurrence is each independently an integer of 0, 1, 2, or 3.

**5.** The ligand-drug conjugate or the pharmaceutically acceptable salt thereof according to any one of claims 1-4, wherein Y is selected from:

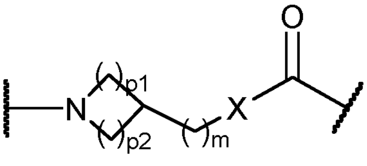 or 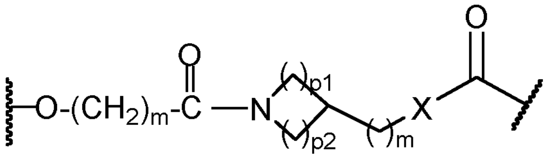 ,

wherein p1 is an integer of 0, 1, 2, or 3, p2 is an integer of 1, 2, 3, or 4, and $2 \leq p1 + p2 \leq 4$; X is independently selected from single bond, -NH-, or O, and m is an integer of 0, 1, 2, or 3;

or Y is selected from:

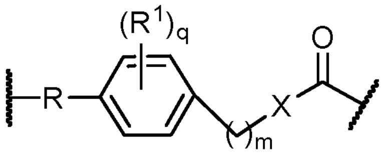 , 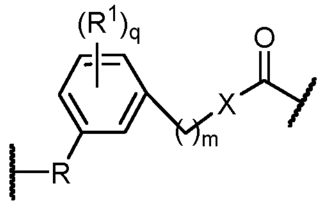 ,

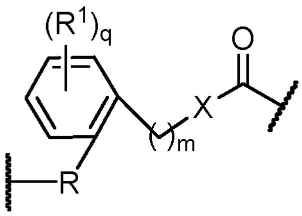 , or 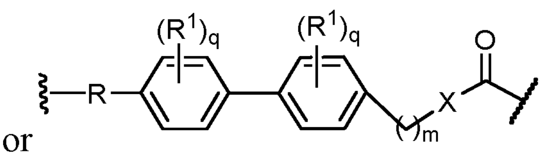 ,

wherein R is - NH-, O, or $-O-(CH_2)_m-C(=O)-NH-$, and the -NH- in $-O-(CH_2)_m-C(=O)-NH-$is "connected to phenylene or biphenylene in Y; X is independently selected from a single bond, -NH-, or O, m is an integer of 0, 1, 2, or 3, $R^1$ at each occurrence is each independently selected from halogen, -OH, -CN , $-CF_3$ , - $NO_2$ , $C_{1-6}$ alkyl, $-OC_{1-6}$ alkyl, $-NH_2$ , $-NH(C_{1-6}$ alkyl), and $-N(C_{1-6}$ alkyl$)_2$, and q is an integer of 0, 1, 2, 3, or 4; preferably, X is independently selected from a single bond or -NH-, m is an integer of 0 or 1, and $R^1$ at each occurrence is each independently selected from halogen, -OH, -CN, $-CF_3$, $-NO_2$ , $C_{1-3}$ alkyl, and $-OC_{1-3}$ alkyl, preferably, independently selected from -Cl, -Br, -F, -OH, - CN, $-CF_3$, $-NO_2$ , $-CH_3$, and $-OCH_3$ , and q is an integer of 0, 1, or 2; or Y is selected from:

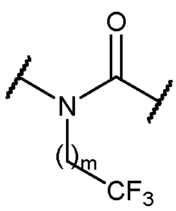 ,

wherein m is an integer of 0, 1, 2, or 3; preferably, m is 1 or 2; and the C(=O)- end of the Y structure is connected to -NH- in the structure of formula (-D) or formula ($-D_0$ ).

**6.** The ligand-drug conjugate or the pharmaceutically acceptable salt thereof according to any one of claims 1-5, wherein Y is selected from:

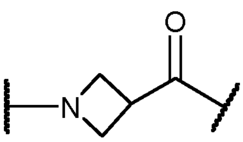 , 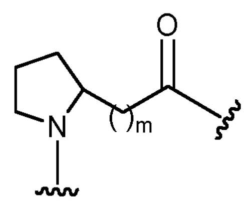 , 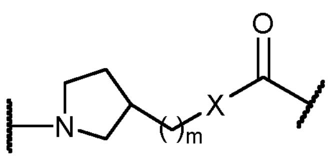 ,

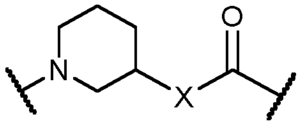,
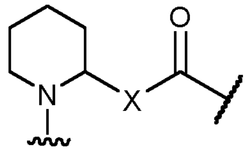,
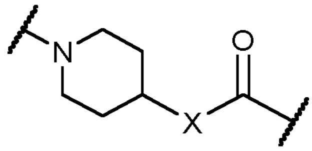, ,

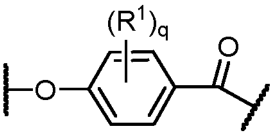
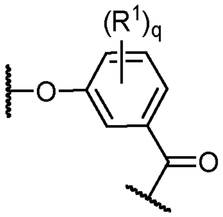,
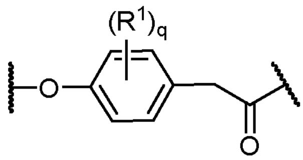,
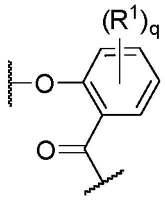,

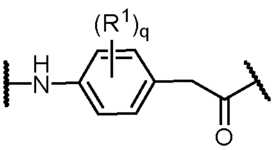,
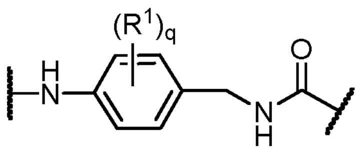,
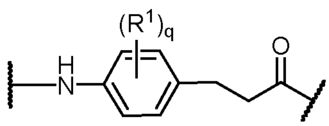,

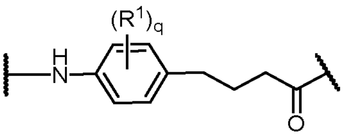,
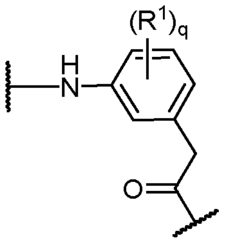,
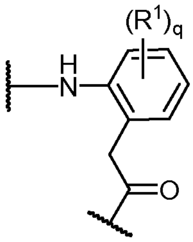,

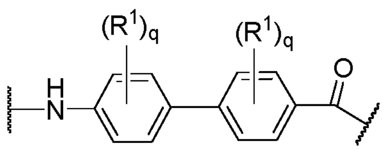,
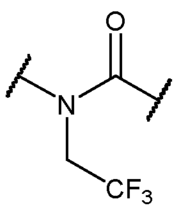,
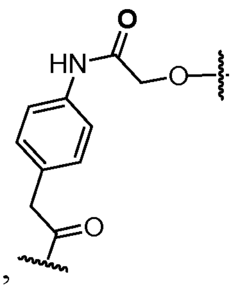 and
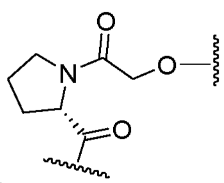,

wherein, X is independently selected from single bond or -NH-, m is an integer of 0 or 1;
$R^1$ is selected from halogen, -OH, -CN, $-CF_3$, $-NO_2$ , $-CH_3$, $C_{1-6}$ alkyl, $-OC_{1-6}$ alkyl, $-NH_2$, $-NH(C_{1-6}$ alkyl), and $-N(C_{1-6}$ alkyl$)_2$, preferably $R^1$ is selected from halogen, -OH, -CN, $C_{1-3}$ alkyl, and $-OC_{1-3}$ alkyl, preferably $R^1$ is selected from -Cl, -Br, -F, -OH, -CN, $-CF_3$, $-NO_2$ , $-CH_3$, and $-OCH_3$;
q is an integer of 0, 1, 2, 3 or 4; preferably, q is an integer of 0, 1 or 2; and the C(=O)- end of the Y structure is connected to -NH- in the structure of formula (-D) or formula $(-D_0)$.

**7.** The ligand-drug conjugate or the pharmaceutically acceptable salt thereof according to any one of claims 3-6, wherein the linker unit -L- is $-L^1$ $-L^2$ $-L^3$ - L4 -.

$L^1$ is selected from -(succinimidyl-3-yl-N)-W-C(=O)-, wherein W is $C_{1-10}$ alkylene, $C_{1-10}$ alkylene-cycloalkylene, $C_{1-10}$ heteroalkylene, $C_{1-10}$ alkylene-heterocyclylene, or $C_{1-10}$ heteroalkylene-cycloalkylene, preferably W is $C_{1-8}$ alkylene, $C_{1-8}$ alkylene-cycloalkylene, or $C_{1-8}$ heteroalkylene, the heteroalkyl contains 1-3 heteroatoms each independently selected from N, O or S, wherein the alkylene, cycloalkylene and heteroalkylene are unsubstituted or each independently optionally further substituted with one or more substituents selected from halogen, hydroxyl, -CN, amino, C alkyl, haloalkyl, deuterated alkyl, alkoxy and cycloalkyl, preferably unsubstituted or each independently optionally further substituted with one or more substituents selected from halogen, hydroxyl, -CN, amino, $C_{1-6}$ alkyl, $C_{1-6}$ haloalkyl, deuterated $C_{1-6}$ alkyl, $C_{1-6}$ alkoxy and $C_{5-8}$ cycloalkyl, preferably unsubstituted or optionally substituted with one or more substituents selected from halogen, -OH, -CN, $C_{1-3}$ alkyl, and -$OC_{1-3}$ alkyl, preferably unsubstituted or optionally substituted with a substituent selected from Cl, Br, F, -OH, -CN, methyl, and -$OCH_3$ ;
$L^2$ is selected from -$NR^4(CH_2CH_2O)_rCH_2CH_2C(=O)$-,
-$NR^4(CH_2CH_2O)_rCH_2$ C(=O)-,
-NR4$CH_2$-$Ar^2$-$(CH_2CH_2O)_rCH_2CH_2NR^4C(=O)CH_2OCH_2C(=O)$-,-$S(CH_2)_rC(=O)$-, or a single bond, wherein r is an integer of from 1 to 20, preferably r is an integer of 1, 2, 3, 4, 5, 6, 7, or 8;
$Ar^2$ is selected from

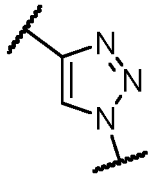 , 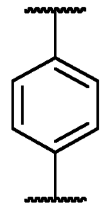 , 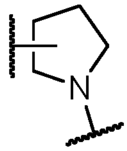 , 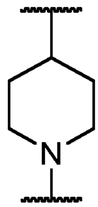 , 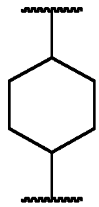 ,

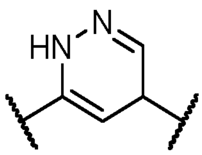 , 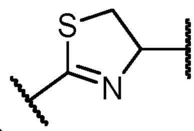 , 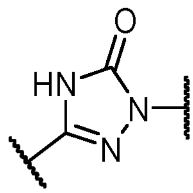 , 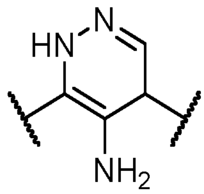 and 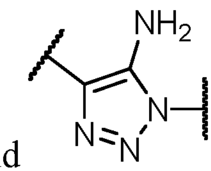 ;

$L^3$ is a peptide residue consisting of from 2 to 7 amino acids, preferably $L^3$ is a peptide residue consisting of 2, 3, 4, 5 or 6 amino acids, wherein the amino acids are unsubstituted or optionally further substituted with one or more substituents selected from halogen, hydroxyl, -CN, amino, alkyl, haloalkyl, deuterated alkyl, alkoxy, and cycloalkyl, preferably, optionally further substituted with one or more substituents selected from halogen, hydroxyl, - CN, amino, $C_{1-6}$ alkyl, halo$C_{1-6}$alkyl, deuterated $C_{1-6}$alkyl, $C_{1-6}$ alkoxy and $C_{5-8}$ cycloalkyl;
$L^4$ is selected from -$NR^5(CR^6R^7)_t$-Z-$(CR^6R^7)_t$-C(=O)-, -$NR^5$-$Ar^3$-$(CR^6R^7)_t$-Z-C(=O)-, or a single bond, wherein t at each occurrence is each independently an integer of 0, 1, 2, 3, 4, 5, or 6; Z at each occurrence is each independently a single bond, O, S, or -NH-; $Ar^3$ is a arylene or heteroarylene, preferably selected from 6-8 membered arylene or 5-8 membered heteroarylene, the heteroarylene contains 1, 2 or 3 heteroatoms, and the heteroatoms are independently selected from N, O, and S; the arylene or heteroarylene is unsubstituted or optionally substituted with a substituent selected from H, halogen, -OH, -CN, $C_{1-6}$ alkyl, -$OC_{1-6}$ alkyl, -$NH_2$ , -NH($C_{1-6}$ alkyl), and -N($C_{1-6}$ alkyl)$_2$;

$R^4$ and $R^5$ are identical or different and at each occurrence are each independently selected from hydrogen atom, halogen, $C_{1-6}$ alkyl, $C_{1-6}$ haloalkyl, deuterated $C_{1-6}$ alkyl, and -$C_{1-6}$ alkylene-OH;
$R^6$ and $R^7$ are identical or different and at each occurrence are each independently selected from hydrogen atom, halogen, $C_{1-6}$ alkyl, $C_{1-6}$ haloalkyl, deuterated $C_{1-6}$ alkyl, and -$C_{1-6}$ alkylene-OH; and
the $L^1$ end is connected to the ligand, and the $L^4$ end is connected to Y

**8.** The ligand-drug conjugate or the pharmaceutically acceptable salt thereof according to claim 7, wherein $L^1$ is selected from -(succinimidyl-3-yl-N)-$(CH_2)_s$-C(=O)- and -(succinimidyl-3-yl-N)-$CH_2$-cyclohexyl-C(=O)-, wherein s is an integer of 2, 3, 4, 5, 6, 7, or 8.

**9.** The ligand-drug conjugate or the pharmaceutically acceptable salt thereof according to claim 7 or 8, wherein $L^2$ is selected from
-$NR^4(CH_2CH_2O)_rCH_2CH_2C(=O)$-, and -$NR^4CH_2$-$Ar^2$-$(CH_2CH_2O)_rCH_2CH_2NR^4C(=O)CH_2OCH_2C(=O)$-, wherein r is an integer from 1 to 20; preferably r is an integer of 1, 2, 3, 4, 5, 6, 7, or 8, and $Ar^2$ is

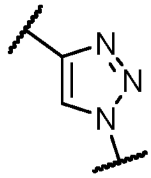
.

**10.** The ligand-drug conjugate or the pharmaceutically acceptable salt thereof according to any one of claims 7-9, wherein $L^3$ is a peptide residue consisting of 2 to 7 amino acids selected from alanine, phenylalanine, glycine, valine, lysine, citrulline, serine, glutamic acid, and aspartic acid; preferably $L^3$ is a dipeptide residue, tripeptide residue, or tetrapeptide residue consisting of amino acids selected from alanine, phenylalanine, glycine, valine, lysine, citrulline, serine, glutamic acid, and aspartic acid; more preferably $L^3$ is selected form dipeptide residue of valine-alanine, tripeptide residue of alanine-alanine-alanine, or tetrapeptide residue of glycine-glycine-phenylalanine-glycine.

**11.** The ligand-drug conjugate or the pharmaceutically acceptable salt thereof according to any one of claims 7-10, wherein $L^4$ is $-NR^5(CR^6R^7)_t-Z-(CR^6R^7)_t-C(=O)-$ or $-NR^5-Ar^3-(CR^6R^7)_t-Z-C(=O)-$, t at each occurrence is independently an integer of 1, 2 or 3;

Z is selected from a single bond, O, S or -NH-;

$Ar^3$ is selected from 6-8 membered arylene, preferably is phenylene, the arylene or phenylene is unsubstituted or optionally substituted with a substituent selected from H, halogen, -OH, -CN, and $C_{1-6}$ alkyl;

$R^5$ at each occurrence is each independently selected from hydrogen atom, $C_{1-3}$ alkyl, halo$C_{1-3}$ alkyl, and deuterated$C_{1-3}$ alkyl;

$R^6$ and $R^7$ are identical or different and at each occurrence are each independently selected from hydrogen atom, halogen, $C_{1-3}$ alkyl, $C_{1-3}$ haloalkyl, and deuterated $C_{1-3}$ alkyl.

**12.** The ligand-drug conjugate or the pharmaceutically acceptable salt thereof according to any one of claims 3-12, wherein the linker unit -L- is:

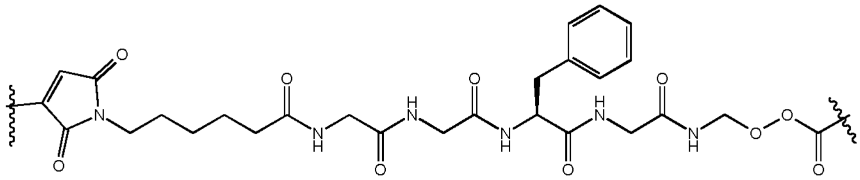

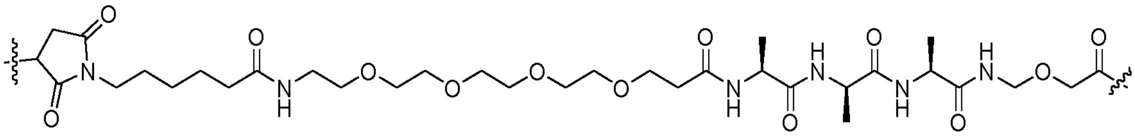

,

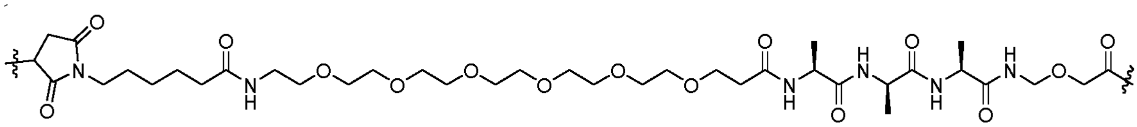

,

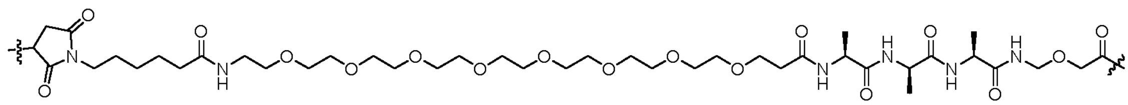

,

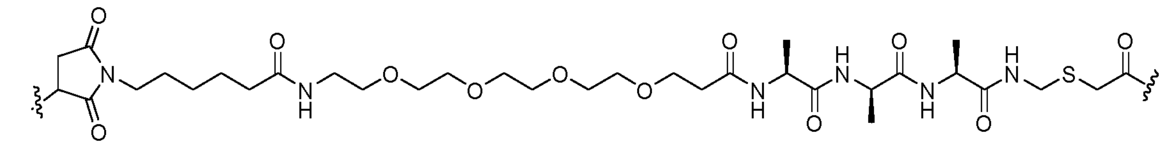

,

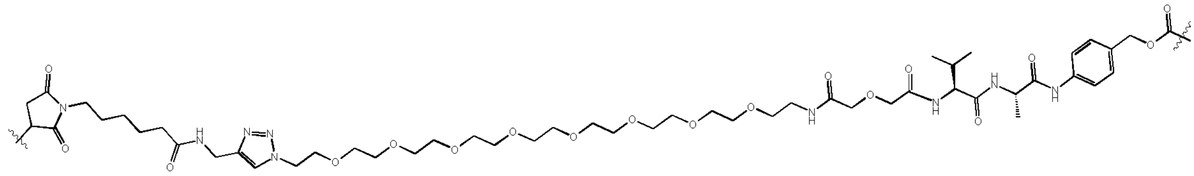

,

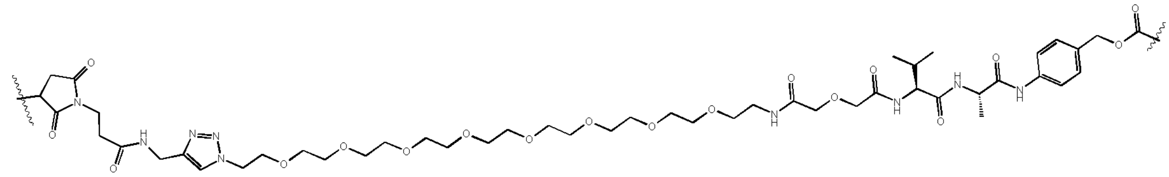

,

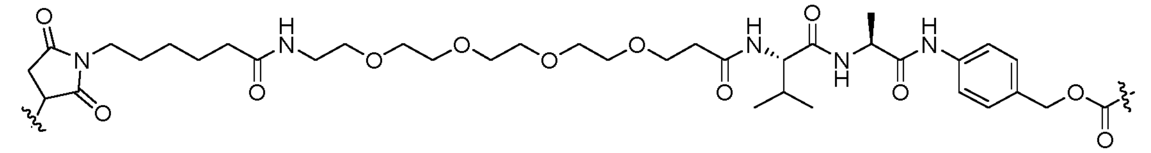

,

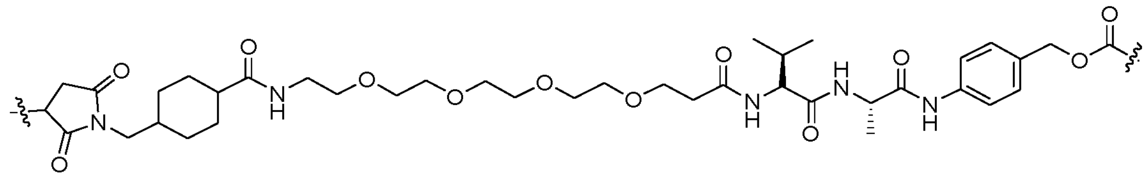

,

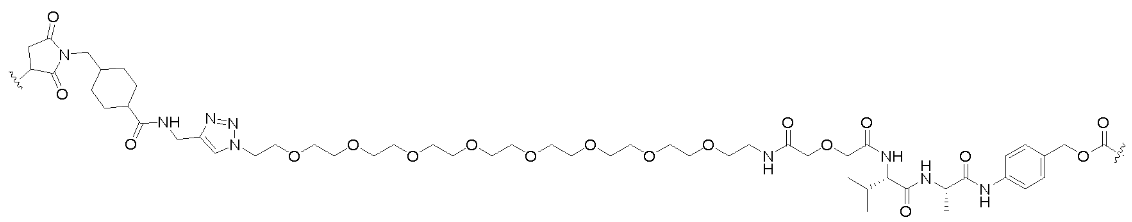

,

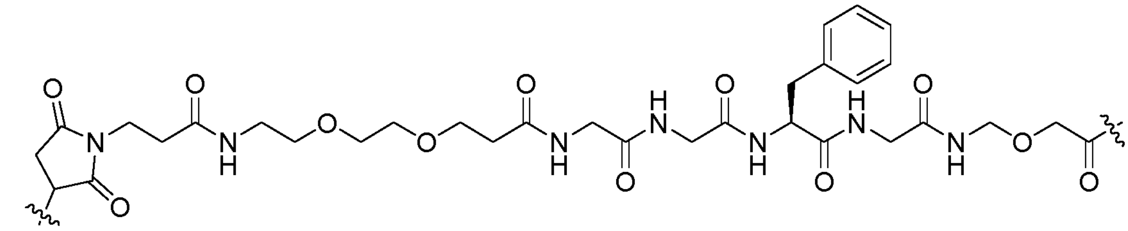

or

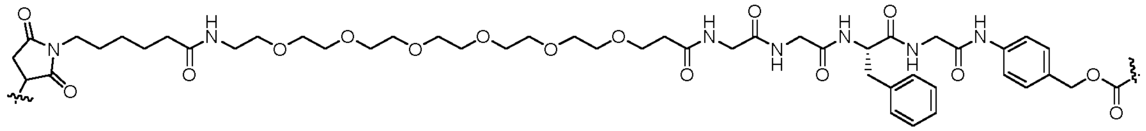

.

13. The ligand-drug conjugate or the pharmaceutically acceptable salt thereof according to any one of claims 1-12, selected from the following structural formulas:

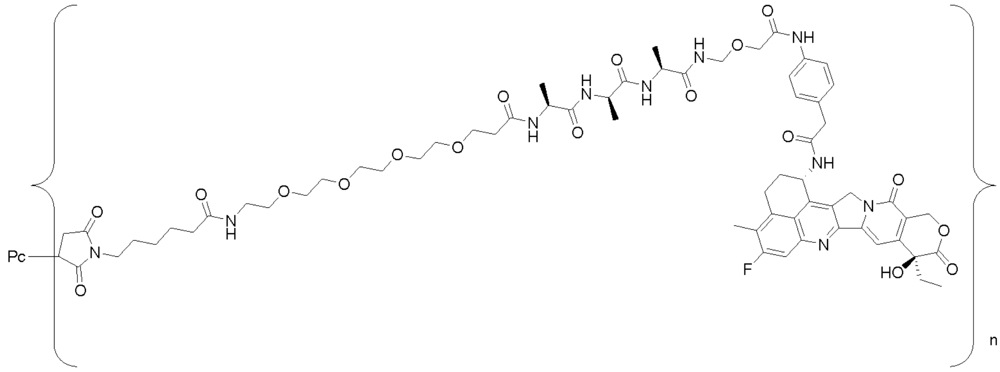

,

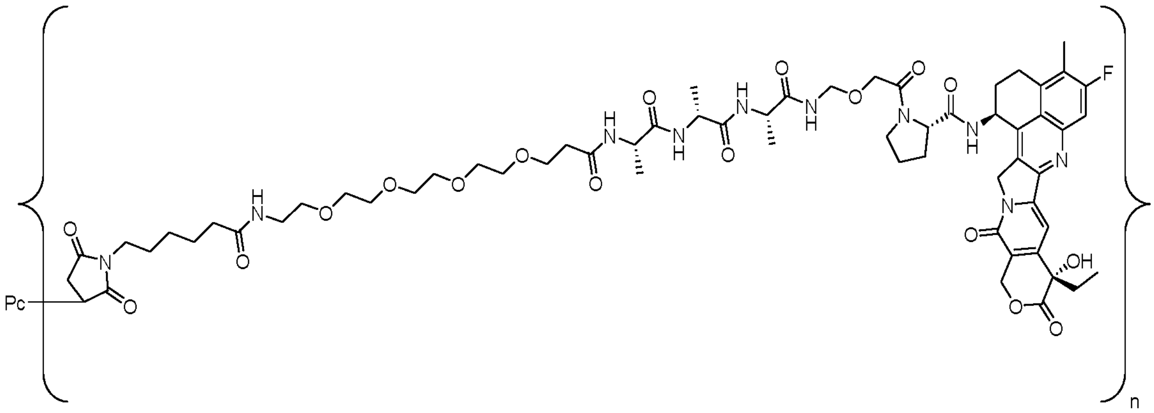

,

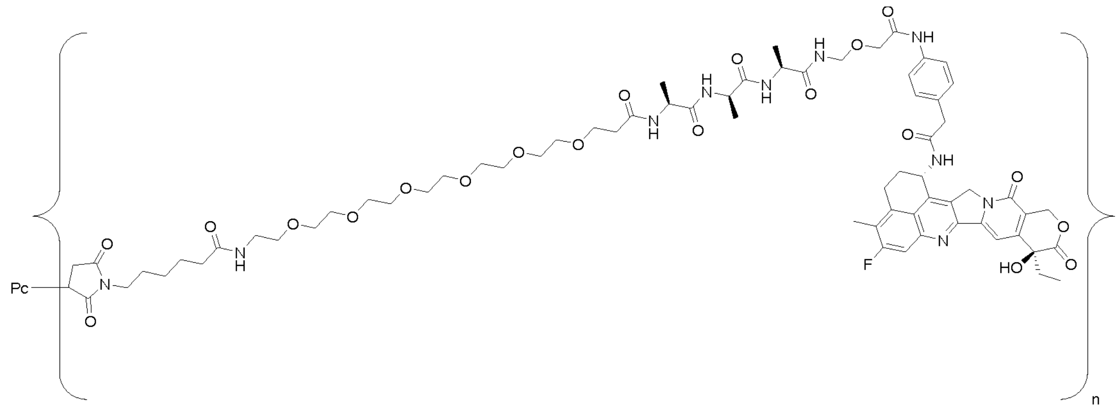

,

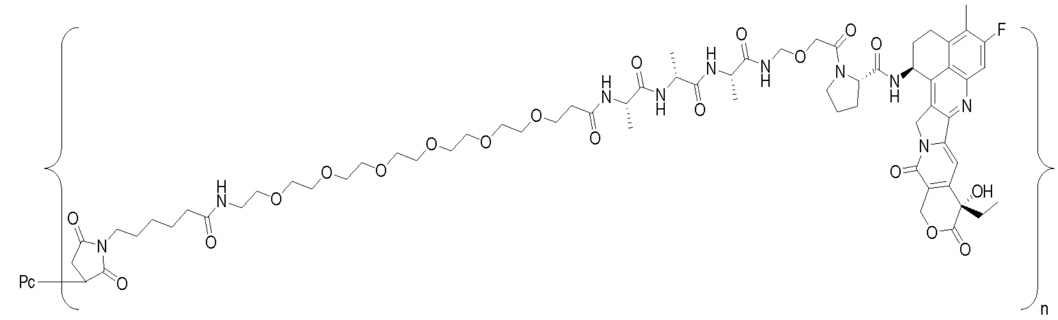
,

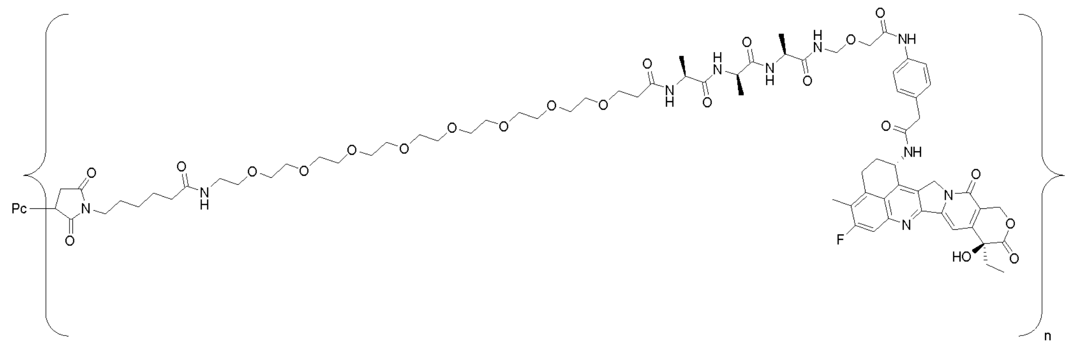
,

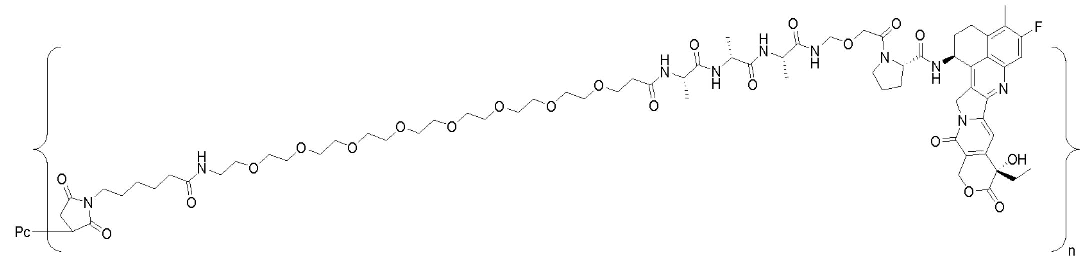
,

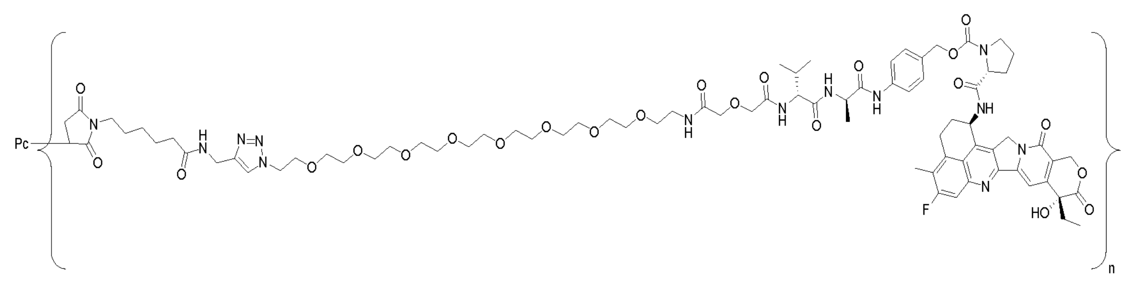
,

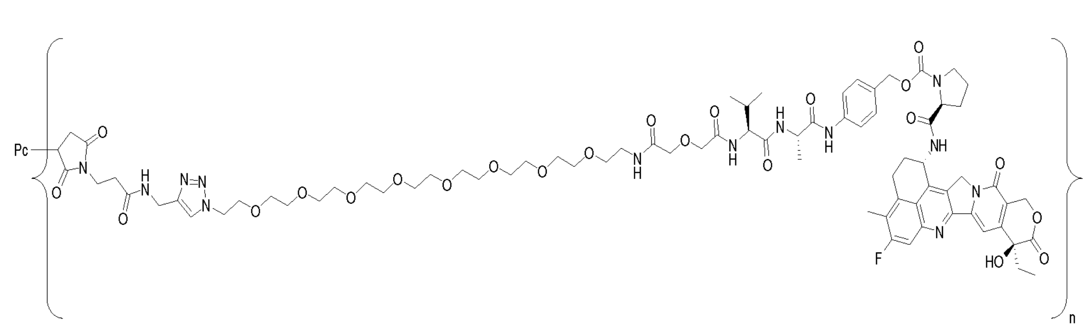
,

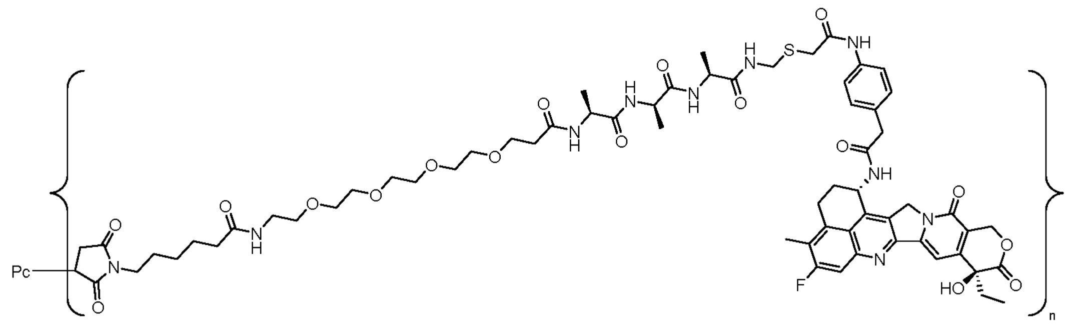

,

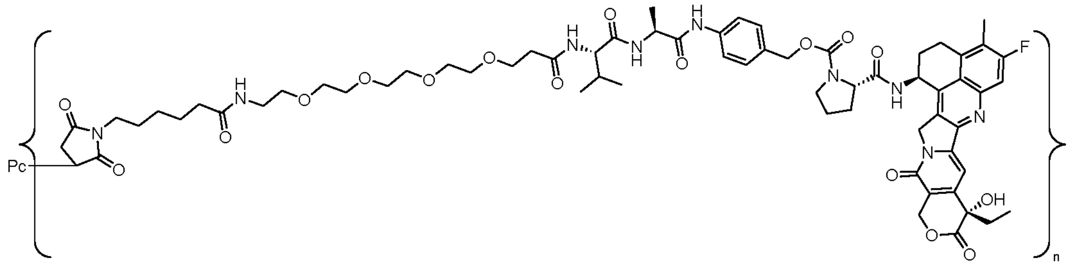

,

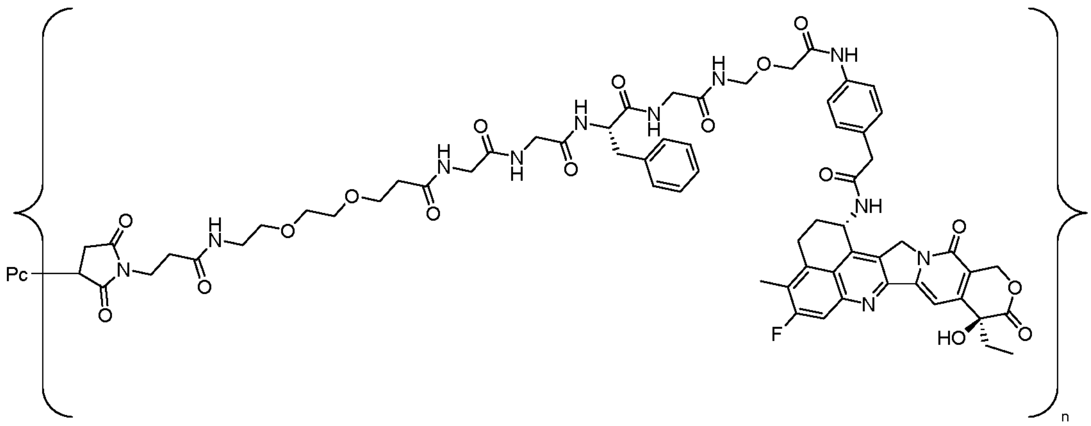

,

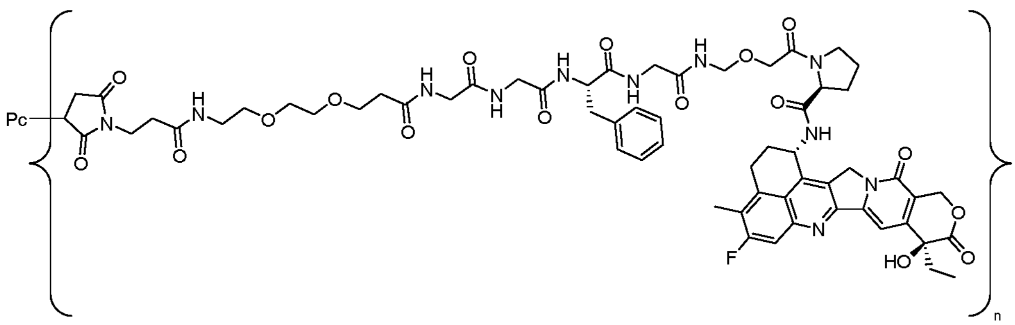

,

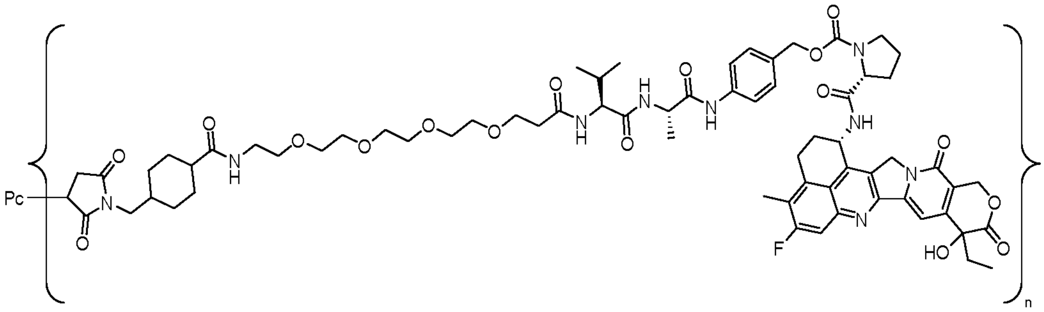
,

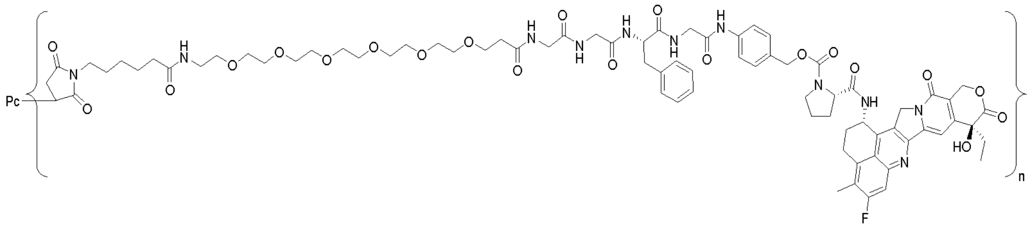
,

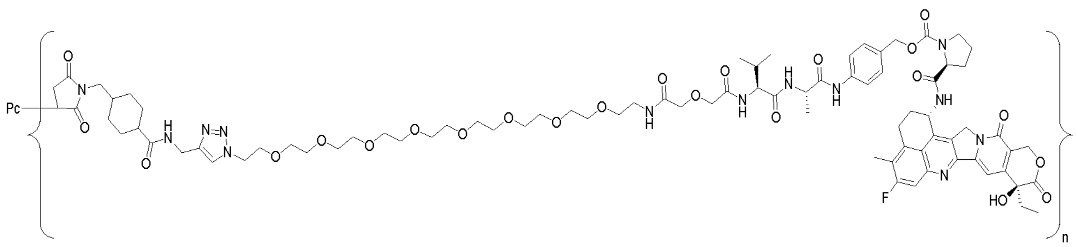
,

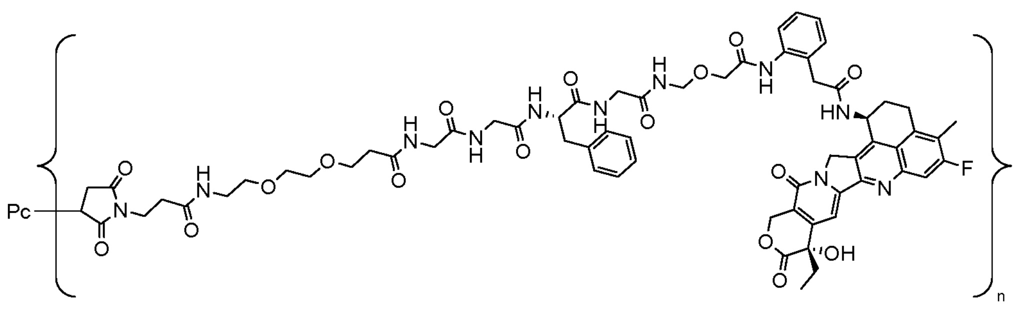
,

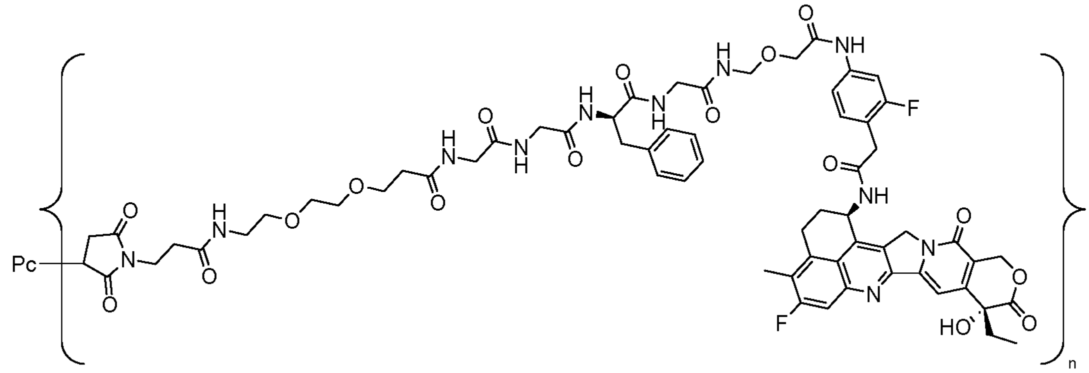
,

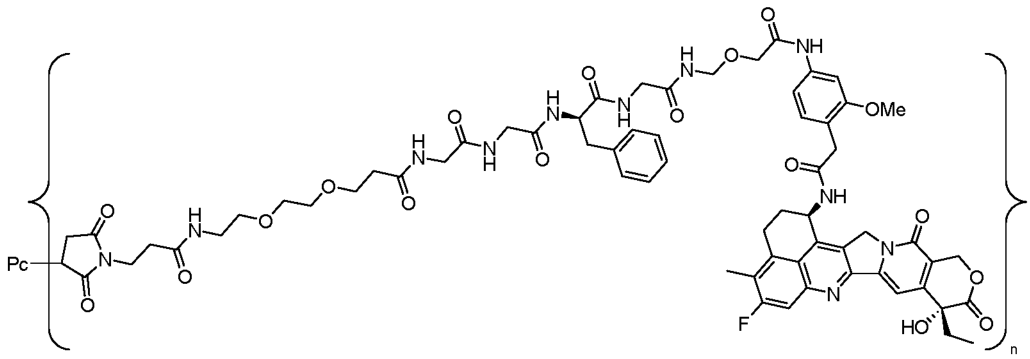

,

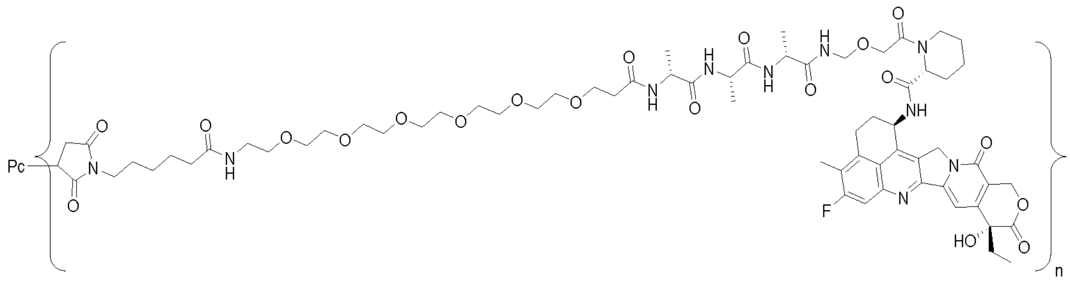

,

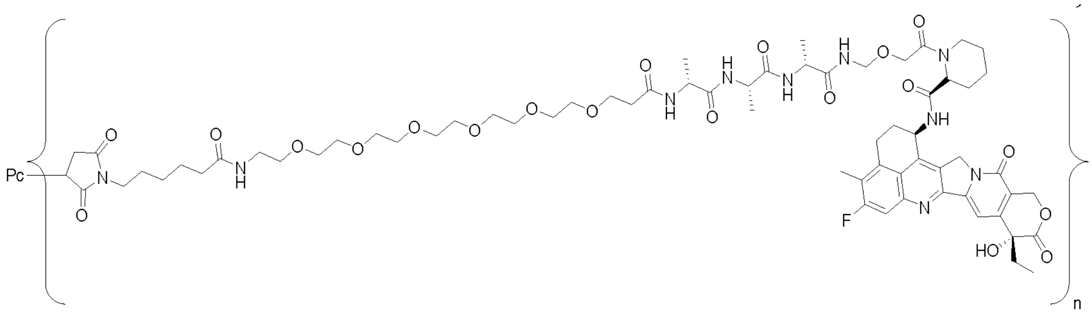

,

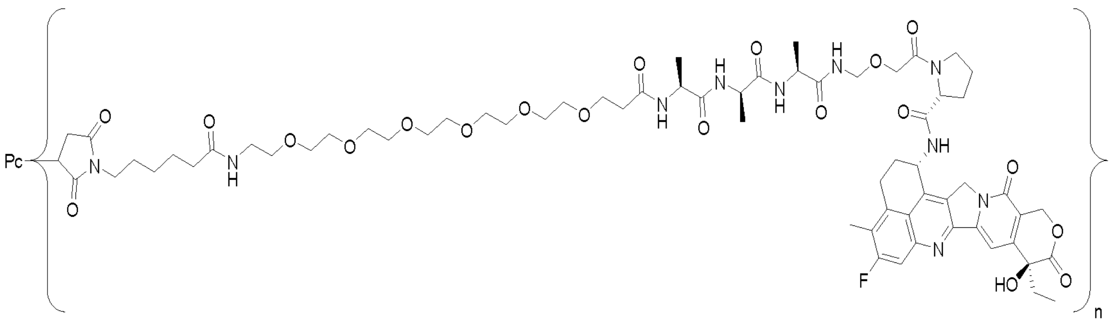

,

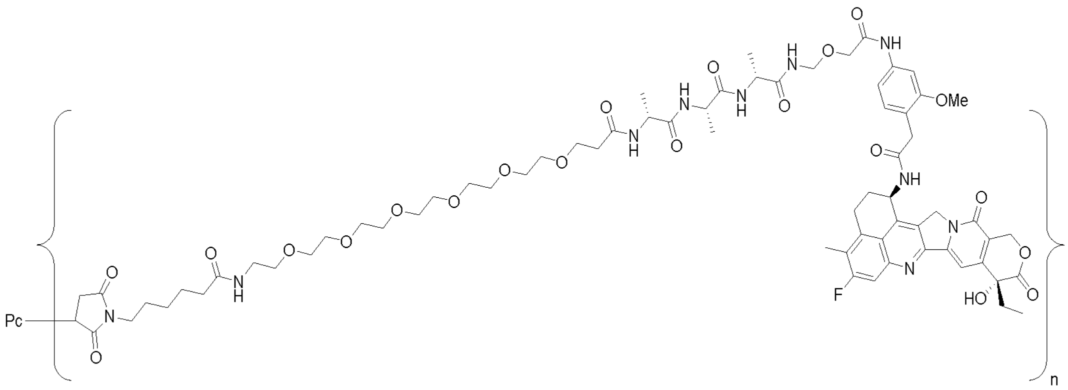,

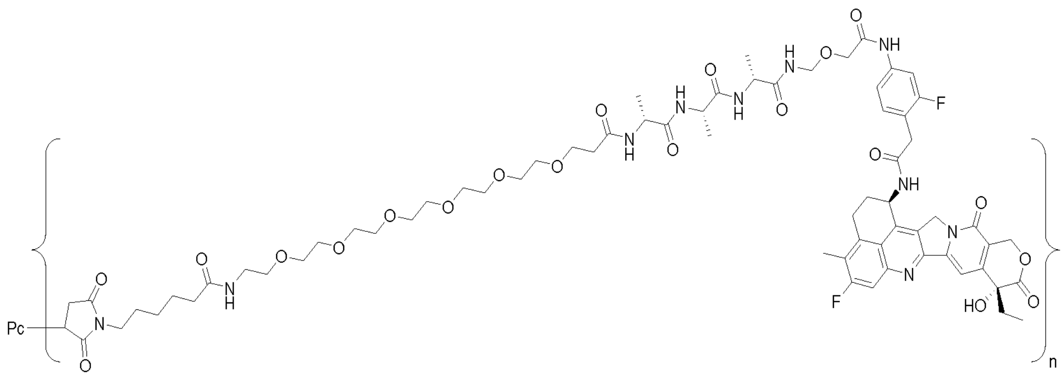,

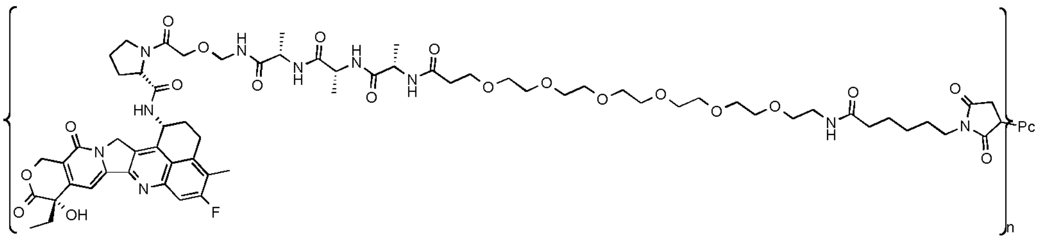,

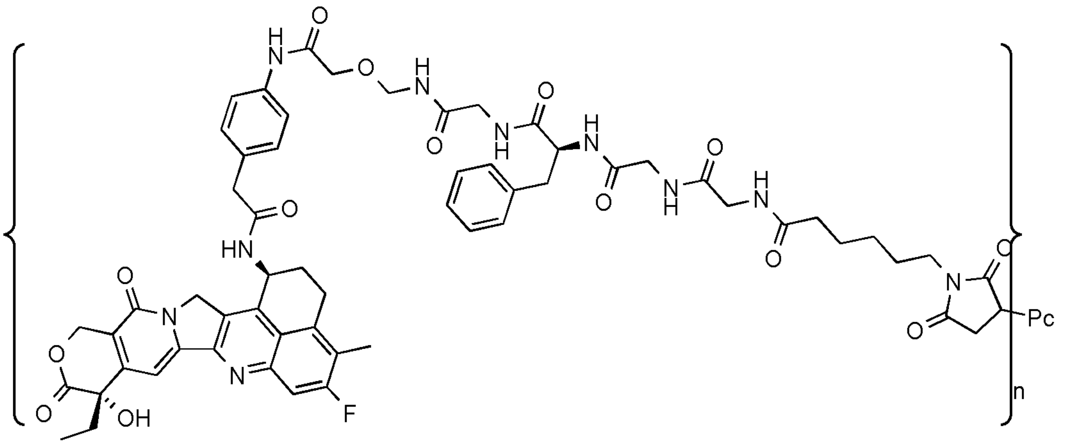,

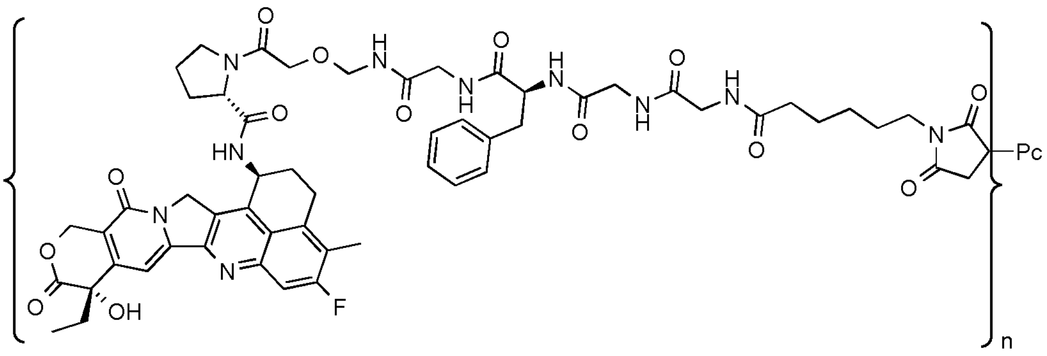

,

and

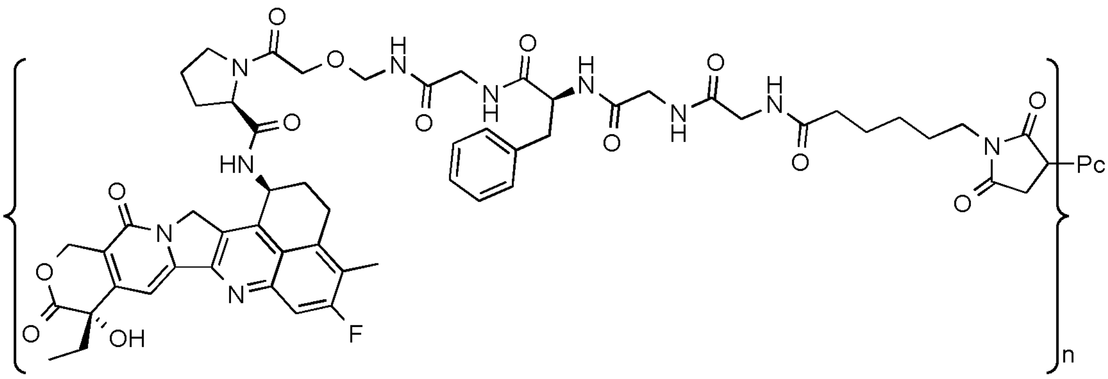

;

wherein n is an integer or a decimal from 1 to 15; preferably, n is an integer or a decimal from 1 to 13; preferably, n is an integer or a decimal from 1 to 10; more preferably, n is an integer or a decimal from 3 to 8; and Pc is a ligand.

**14.** The ligand-drug conjugate or the pharmaceutically acceptable salt thereof according to any one of claims 3 to 13, wherein the Pc is an antibody or an antigen-binding fragment thereof or a polypeptide, wherein the antibody is selected from a chimeric antibody, a humanized antibody and a fully human antibody;

preferably, the antibody or antigen-binding fragment thereof is selected from an anti-TROP-2 antibody, an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-LIV-1 antibody, an anti-ROR1 antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-Mesothelin antibody, or an antigen-binding fragment thereof;
preferably, the antibody or antigen-binding fragment thereof is an anti-TROP-2 antibody, an anti-HER2(ErbB2) antibody, an anti-HER3(ErbB3) antibody, an anti-LIV-1 antibody, an anti-ROR1 antibody, or an antigen-binding fragment thereof;
preferably, the antibody or antigen-binding fragment thereof is an anti-TROP-2 antibody or an anti-HER3 (ErbB3) antibody, or an antigen-binding fragment thereof;
preferably, the heavy chain of the anti-TROP-2 antibody or antigen-binding fragment thereof comprises: an HCDR1 consisting of the amino acid sequence of SEQ ID No: 1, an HCDR2 consisting of the amino acid sequence of SEQ ID No:2 and an HCDR3 consisting of the amino acid sequence of SEQ ID No:3 and/or, the light chain comprises: an LCDR1 consisting of the amino acid sequence of SEQ ID No:4, an LCDR2 consisting of the amino acid sequence of SEQ ID No:5, an LCDR3 consisting of the amino acid sequence of SEQ ID No:6; more preferably, the heavy chain of the anti-TROP-2 antibody or antigen-binding fragment thereof comprises, the heavy chain variable region shown in SEQ ID No:7, and/or, the light chain of the anti-TROP-2 antibody or antigen-binding fragment thereof comprises the light chain variable region shown in SEQ ID No:8; more preferably, the

anti-TROP-2 antibody or antigen-binding fragment thereof comprises a heavy chain of the amino acid sequence shown in SEQ ID NO:9, and/or, a light chain of the amino acid sequence shown in SEQ ID NO:10;
preferably, the heavy chain of the anti-HER3 antibody or antigen-binding fragment thereof comprises, an H'CDR1 consisting of the amino acid sequence of SEQ ID No:11, an H'CDR2 consisting of the amino acid sequence of SEQ ID No: 12 and an H'CDR3 consisting of the amino acid sequence of SEQ ID No:13, and/or, the light chain of the anti-HER3 antibody or antigen-binding fragment thereof comprises an L'CDR1 consisting of the amino acid sequence of SEQ ID No: 14, an L'CDR2 consisting of the amino acid sequence of SEQ ID No:15 and an L'CDR3 consisting of the amino acid sequence of SEQ ID No:16; more preferably, the anti-HER3 antibody or antigen-binding fragment thereof comprises a heavy chain variable region as shown in SEQ ID No: 17 in the heavy chain and/or a light chain variable region as shown in SEQ ID No:18 in the light chain; more preferably, the anti-HER3 antibody or antigen-binding fragment thereof comprises a heavy chain of the amino acid sequence shown by SEQ ID NO :19, and/or, a light chain of the amino acid sequence shown by SEQ ID NO:20; further preferably, the antibody or antigen-binding fragment thereof is sacituzumab, trastuzumab, patritumab, or pertuzumab.

**15.** A compound shown by the general formula ($E_0$) or a pharmaceutically acceptable salt thereof:

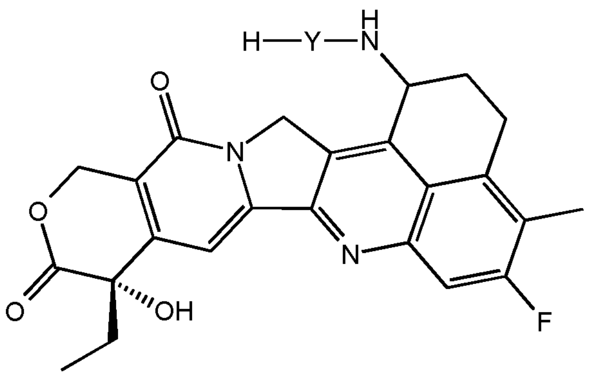

**($E_0$)**

wherein, Y is

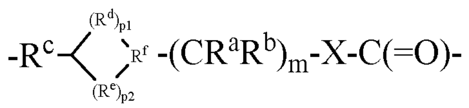

,

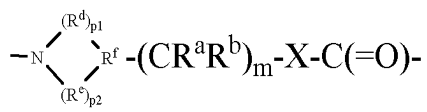

,

$-R^c-Ar^1-(Cy)_m-(CR^aR^b)_m-X-C(=O)-$, $-N((CR^aR^b)_mCF_3)-C(=O)-$, $-R^c-(CR^aR^b)_m-C(=O)-R^c-Ar^1-(Cy)_m-(CR^aR^b)_m-X-C(=O)-$,

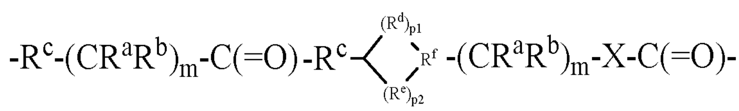

,

or

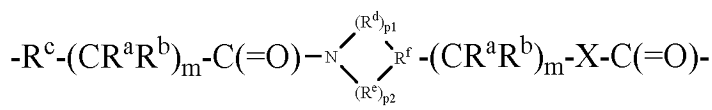

,

wherein the C(=O)-end of Y is connected to -NH- in the structure of formula ($E_0$); preferably, Y is

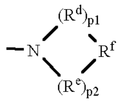 $-(CR^aR^b)_m$-X-C(=O)-

,

-O-$Ar^1$-$(Cy)_m$-$(CR^aR^b)_m$-X-C(=O)-, -NH-$Ar^1$-$(Cy)_m$-$(CR^aR^b)_m$-X-C(=O)-, -N($(CR^aR^b)_mCF_3$)-C(=O)-, -O-$(CR^aR^b)_m$-C(=O)-NH-$Ar^1$-$(Cy)_m$-$(CR^aR^b)_m$-X-C(=O)- or

-O-$(CR^aR^b)_m$-C(=O) 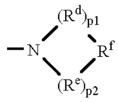 $-(CR^aR^b)_m$-X-C(=O)-

,

wherein the C=O-end of Y is connected to the -NH- in the structure of formula ($E_0$ );

$R^c$ at each occurrence is each independently NH, O, or S; $R^d$ and $R^e$ at each occurrence are each independently $C(R^m)_2$, $NR^m$, O, or S; $R^f$ at each occurrence is each independently $CR^m$ or N; preferably, when the ring formed by $R^d$, $R^e$, and $R^f$ is heterocyclyl, the heterocyclyl comprises 1, 2, 3, or 4 heteroatoms, and the heteroatoms are each independently selected from N, O, and S;

$R^m$ at each occurrence is each independently H, halogen, haloalkyl, -OH, - CN, $-NO_2$, alkyl, alkoxy, $-NH_2$, -NH-alkyl, or $-N(alkyl)_2$, deuterium atom, deuterated alkyl, aminoalkylene, hydroxyalkylene, nitroalkylene, or cyanoalkylene, preferably, $R^m$ at each occurrence is each independently H, halogen, haloalkyl, -OH, -CN, $-NO_2$, alkyl, alkoxy, $-NH_2$, -NH-alkyl, or - $N(alkyl)_2$;

p1 is an integer from 0 to 17, p2 is an integer from 1 to 18, and $p1 + p2 \leq 18$; preferably, p1 is an integer of 0, 1, 2, 3, 4, 5, 6, or 7, p2 is an integer of 1, 2, 3, 4, 5, 6, 7, or 8, and $p1 + p2 \leq 8$;

the Cy at each occurrence is each independently selected from cycloalkylene, heterocycloalkylene, arylene, or heteroarylene, preferably the Cy at each occurrence is each independently selected from 3-10 membered cycloalkylene, 3-10 membered heterocycloalkylene, 6-10 membered arylene, or 5-10 membered heteroarylene; preferably, the heterocycloalkylene and heteroarylene contain 1, 2, 3, or 4 heteroatoms, and the heteroatoms are independently selected from N, O and S; the cycloalkylene, heterocyclylene, arylene, and heteroarylene are unsubstituted or optionally substituted with a substituent selected from halogen, -OH, -CN, haloalkyl, $-NO_2$ , alkyl, alkoxy, $-NH_2$, -NH-alkyl, and $-N(alkyl)_2$, preferably unsubstituted or optionally substituted with a substituent selected from halogen, -OH, -CN, $-CF_3$ , $-NO_2$ , $C_{1-6}$ alkyl, $-OC_{1-6}$ alkyl, $-NH_2$ , $-NH(C_{1-6}$ alkyl), and $-N(C_{1-6}$ $alkyl)_2$ ;

the $Ar^1$ at each occurrence is each independently selected from arylene or heteroarylene, preferably, the $Ar^1$ at each occurrence is each independently selected from 6-10 membered arylene or 5-10 membered heteroarylene; preferably the heteroarylene comprises 1, 2, 3, or 4 heteroatoms, the heteroatoms are each independently selected from N, O, and S; the arylene and heteroarylene are unsubstituted or optionally substituted with a substituent selected from halogen, -OH , -CN, haloalkyl, $-NO_2$ , alkyl, alkoxy, $-NH_2$ , - NH-alkyl, and $-N(alkyl)_2$, preferably, unsubstituted or optionally substituted with a substituent selected from halogen, -OH, -CN, $-CF_3$ , $-NO_2$ , $C_{1-6}$ alkyl, $-OC_{1-6}$ alkyl, $-NH_2$ , $-NH(C_{1-6}$ alkyl), and $-N(C_{1-6}$ $alkyl)_2$ ;

$R^a$ and $R^b$ are identical or different and at each occurrence are each independently selected from hydrogen atom, halogen, haloalkyl, -OH, -CN, $-NO_2$, alkyl, alkoxy, $-NH_2$, -NH-alkyl, or $-N(alkyl)_2$, deuterium atom, deuterated alkyl, aminoalkylene, hydroxylalkylene, nitroalkylene, and cyanoalkylene, preferably, $R^a$ and $R^b$ at each occurrence are each independently selected from hydrogen atom, deuterium atom, halogen, alkyl, haloalkyl, deuterated alkyl, alkoxy, -OH, $-NH_2$, -CN, nitro, hydroxyalkylene, preferably, $R^a$ and $R^b$ at each occurrence are each independently selected from hydrogen atom, deuterium atom, halogen, $C_{1-6}$ alkyl, halo $C_{1-6}$ alkyl, deuterated $C_{1-6}$ alkyl, $-OC_{1-6}$ alkyl, -OH, $-NH_2$, -CN, - $CF_3$, nitro, $-C_{1-6}$ alkylene, and -OH;

X at each occurrence is each independently a single bond, -NH-, O or S; and

m at each occurrence is each independently an integer of 0, 1, 2, 3, or 4;

preferably, the compound of formula ($E_0$ ) is not

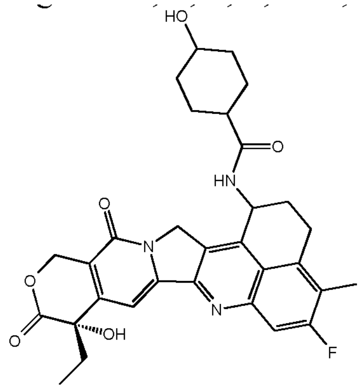

,

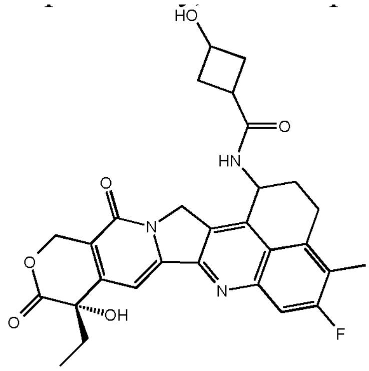

,

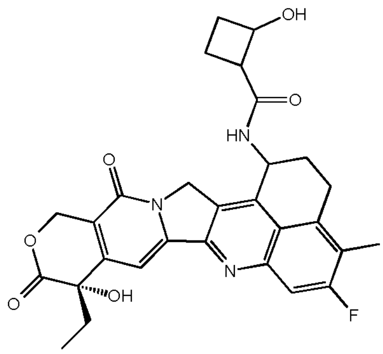

,

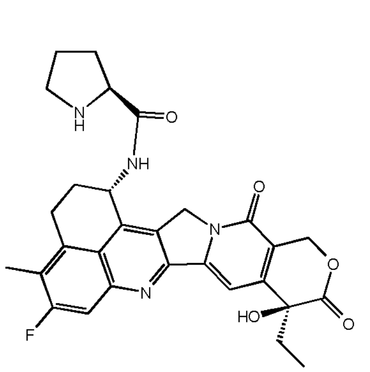

,

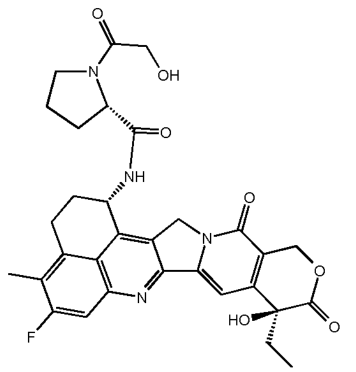

,

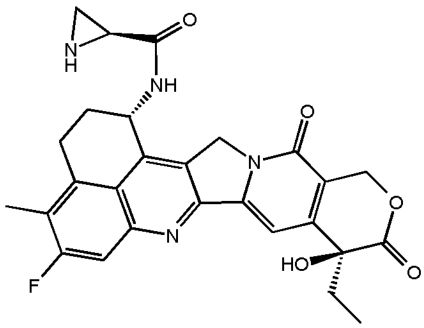

,

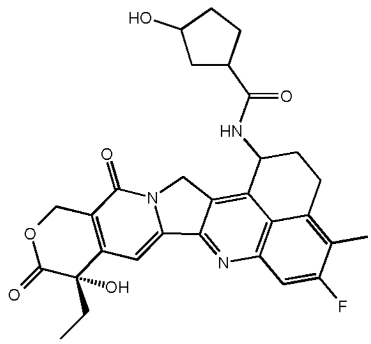

,

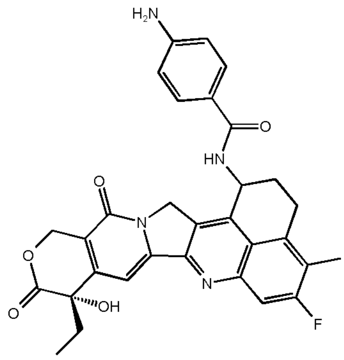

,

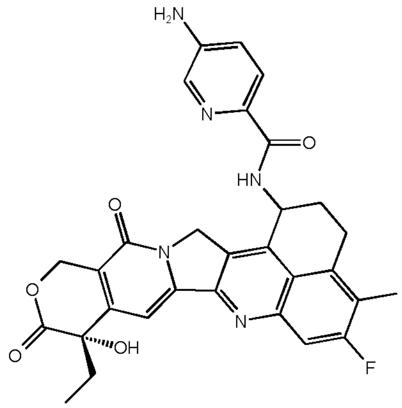

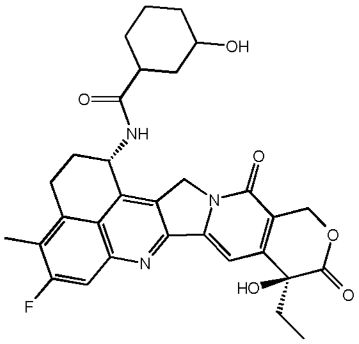

,

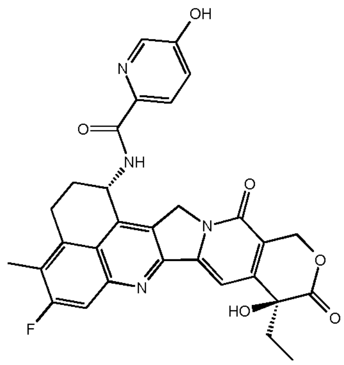

or

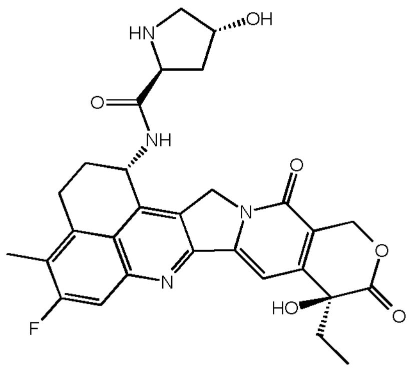

.

**16.** A compound shown by general formula (E) or a pharmaceutically acceptable salt thereof:

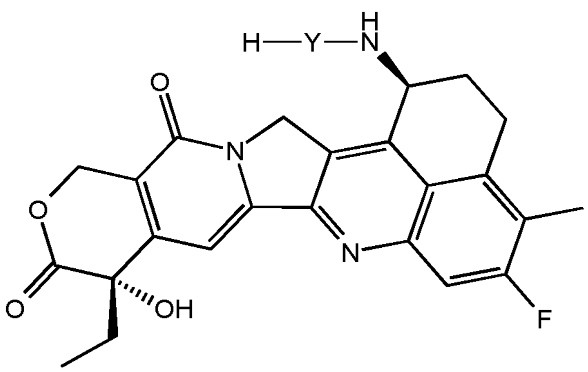

**(E)**

wherein, Y is

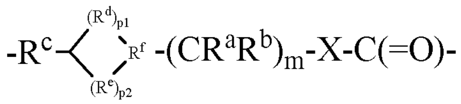, 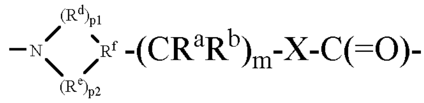,

$-R^c-Ar^1-(Cy)_m-(CR^aR^b)_m-X-C(=O)-$, $-N((CR^aR^b)_mCF_3)-C(=O)-$, $-R^c-(CR^aR^b)_m-C(=O)-R^c-Ar^1-(Cy)_m-(CR^aR^b)_m-X-C(=O)-$,

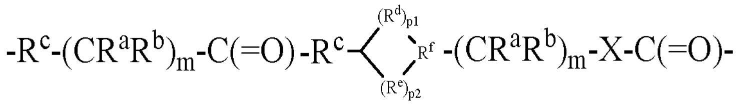,

or

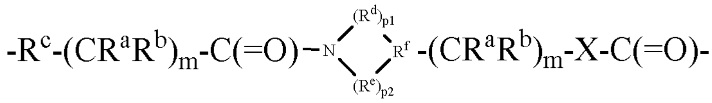,

wherein the C(=O)-end of Y is connected to -NH- in the structure of formula (D); preferably, Y is

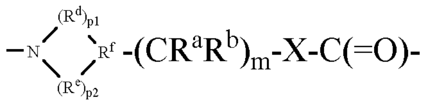,

$-O-Ar^1-(Cy)_m-(CR^aR^b)_m-X-C(=O)-$, $-NH-Ar^1-(Cy)_m-(CR^aR^b)_m-X-C(=O)-$, $-N((CR^aR^b)_mCF_3)-C(=O)-$, $-O-(CR^aR^b)_m-C(=O-$– $NH-Ar^1-(Cy)_m-(CR^aR^b)_m-X-C(=O)-$, or

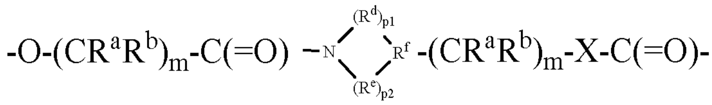,

wherein the C(=O)-end of Y is connected to -NH- in the structure of formula (D);

$R^c$ at each occurrence is each independently NH, O, or S; $R^d$ and $R^e$ at each occurrence are each independently $C(R^m)_2$, $NR^m$, O, or S; $R^f$ at each occurrence is each independently $CR^m$ or N; preferably, when the ring formed by $R^d$, $R^e$, and $R^f$ is heterocyclyl, the heterocyclyl comprises 1, 2, 3, or 4 heteroatoms, and the heteroatoms each independently selected from N, O, and S;

$R^m$ at each occurrence is each independently H, halogen, haloalkyl, -OH, - CN, $-NO_2$, alkyl, alkoxy, $-NH_2$, -NH-alkyl, or $-N(alkyl)_2$, deuterium atom, deuterated alkyl, aminoalkylene, hydroxyalkylene, nitroalkylene, or cyanoalkylene, and preferably $R^m$ at each occurrence is each independently H, halogen, haloalkylene, -OH, -CN, $-NO_2$, alkyl, alkoxy, $-NH_2$ , -NH-alkyl, or - $N(alkyl)_2$ ;

p1 is an integer from 0 to 17, p2 is an integer from 1 to 18, and $p1+p2 \leq 18$; preferably, p1 is an integer of 0, 1, 2, 3, 4, 5, 6, or 7, and p2 is an integer of 1, 2, 3, 4, 5, 6, 7, or 8, and $p1 + p2 \leq 8$;

the Cy at each occurrence is each independently selected from cycloalkylene, heterocyclylene, arylene or heteroarylene, preferably the Cy at each occurrence is each independently selected from 3-10 membered cycloalkylene, 3-10 membered heterocyclylene, 6-10 membered arylene, or 5-10 membered heteroarylene; preferably, the heterocyclylene and heteroarylene contain 1, 2, 3, or 4 heteroatoms, and the heteroatoms are independently selected from N, O and S; the cycloalkylene, heterocyclylene, arylene, and heteroarylene are unsubstituted or optionally substituted with a substituent selected from halogen, -OH, -CN, haloalkyl, $-NO_2$ , alkyl, alkoxy, $-NH_2$ , -NH-alkyl, and $-N(alkyl)_2$, preferably unsubstituted or optionally substituted with a substituent selected from halogen, -OH, -CN, $-CF_3$ , $-NO_2$ , $C_{1-6}$ alkyl, -O $C_{1-6}$ alkyl, $-NH_2$ , $-NH(C_{1-6}$ alkyl), and $-N(C_{1-6}$ alkyl$)_2$;

$Ar^1$ at each occurrence is each independently selected from aryl or heteroaryl, preferably, the $Ar^1$ at each

occurrence is each independently selected from a 6-10 membered aryl or a 5-10 membered heteroaryl; preferably the heteroaryl contains 1, 2, 3, or 4 heteroatoms, and the heteroatoms are independently selected from N, O, and S; the aryl and heteroaryl are unsubstituted or optionally substituted with substituents selected from halogen, -OH, -CN, haloalkyl, $-NO_2$, alkyl, alkoxy, $-NH_2$ , -NH-alkyl, and $-N(alkyl)_2$, preferably, unsubstituted or optionally substituted with substituents selected from halogen, -OH, -CN, $-CF_3$, $-NO_2$ , $C_{1-6}$ alkyl, $-OC_{1-6}$ alkyl, $-NH_2$ , $-NH(C_{1-6}$ alkyl), and $-N(C_{1-6}$ alkyl$)_2$ ;

$R^a$ and $R^b$ are identical or different and at each occurrence are each independently selected from hydrogen atom, halogen, haloalkyl, -OH, -CN, - $NO_2$, alkyl, alkoxyl, $-NH_2$ , -NH-alkyl, or $-N(alkyl)_2$, deuterium atom, deuterated alkyl, aminoalkylene, hydroxyalkylene, nitroalkylene, and cyanoalkylene, and preferably, at each occurrence $R^a$ and $R^b$ are each independently selected from hydrogen atom, deuterium atom, halogen, alkyl, haloalkyl, deuterated alkyl, alkoxy, -OH, $-NH_2$, -CN, nitro, and hydroxyalkylene, preferably, at each occurrence $R^a$ and $R^b$ are each independently selected from hydrogen atom, deuterium atom, halogen, $C_{1-6}$ alkyl, $C_{1-6}$ haloalkyl, deuterated $C_{1-6}$ alkyl, $-OC_{1-6}$ alkyl, -OH, $-NH_2$ , -CN, - $CF_3$, nitro, $-C_{1-6}$ alkylene, and -OH;

X at each occurrence is each independently a single bond, -NH-, O or S; and m at each occurrence is each independently an integer of 0, 1, 2, 3, or 4;

preferably, the compound shown by formula (E) is not

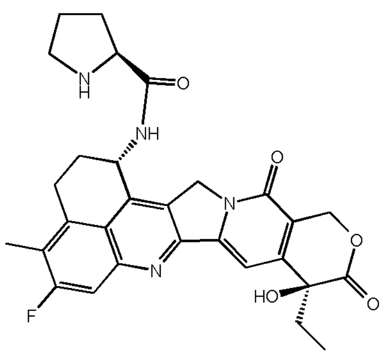
,
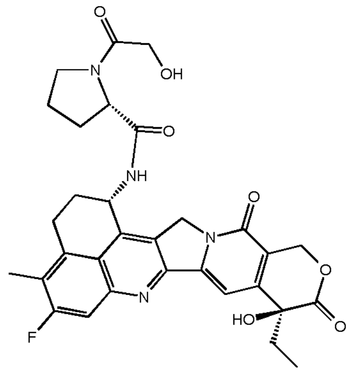
,
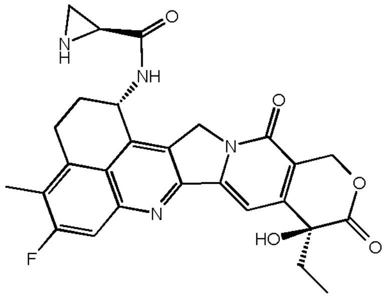
,
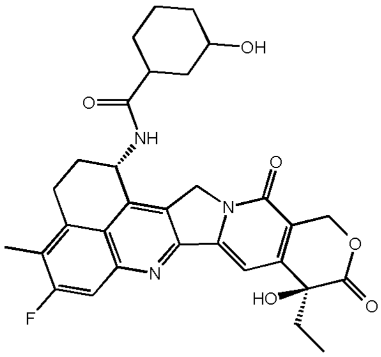
,
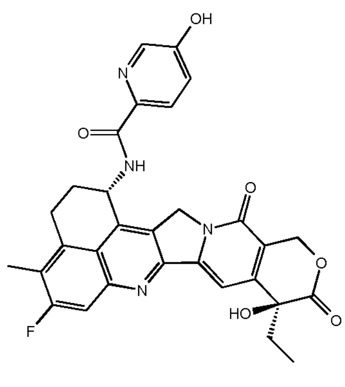
or
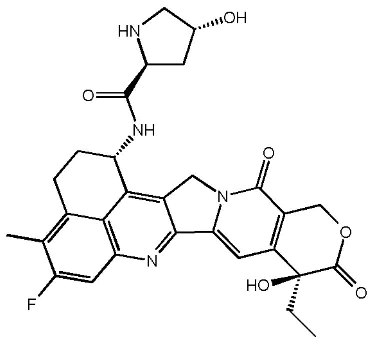
.

**17.** The compound according to claims 15 or 16 or a pharmaceutically acceptable salt thereof, wherein,

$R^c$ at each occurrence is each independently NH, or O ; $R^d$ and $R^e$ at each occurrence are each independently $C(R)^m{}_2$ or $NR^m$ ; $R^f$ at each occurrence is $CR^m$; when the ring formed by $R^d$, $R^e$, and $R^f$ is heterocyclyl, the heterocyclyl comprises 1, 2, or 3 heteroatoms, and the heteroatoms are each independently selected from N and O; and/or

$R^m$ at each occurrence is each independently H, halogen, -OH, -CN, $-NO_2$, $-CF_3$, $-C_{1-6}$ alkyl, $-OC_{1-6}$ alkyl, $-NH_2$, $-NH(C_{1-6}$ alkyl), or $-N(C_{1-6}$ alkyl$)_2$, preferably $R^m$ at each occurrence is each independently H, halogen, -OH, -CN, $-CF_3$, $-NO_2$, $C_{1-6}$ alkyl, or $-OC_{1-6}$ alkyl, preferably $R^m$ at each occurrence is each independently H, halogen, -OH, -CN, $-CF_3$ , $-NO_2$ , $C_{1-3}$ alkyl, or $-OC_{1-3}$ alkyl, preferably $R^m$ at each occurrence is each independently -Cl, -Br, -F, -OH, -CN, $-CF_3$, $-NO_2$ , $-CH_3$, or $-OCH_3$; and/or

p1 is an integer of 0, 1, 2, or 3, p2 is an integer of 1, 2, 3, or 4, and $2 \leq p1 + p2 \leq 4$; and/or

the Cy at each occurrence is each independently selected from 6-10 membered arylene, or 5-10 membered heteroarylene, preferably Cy at each occurrence is each independently selected from phenylene, or 5-6 membered heteroarylene; preferably, the heteroarylene comprises 1, 2, or 3 heteroatoms, and the heteroatoms are independently selected from N, O, and S; and the heteroarylene is unsubstituted or optionally substituted with a substituent selected from halogen, -OH, -CN, $-CF_3$, $-NO_2$, $C_{1-6}$ alkyl, $-OC_{1-6}$ alkyl, $-NH_2$, - $NH(C_{1-6}$ alkyl), and $-N(C_{1-6}$ alkyl$)_2$; preferably the heteroarylene is unsubstituted or optionally substituted with a substituent selected from halogen, -OH, -CN, $-CF_3$, $-NO_2$ , $C_{1-6}$ alkyl, and $-OC_{1-6}$ alkyl; preferably the heteroarylene is unsubstituted or optionally substituted with a substituent selected from halogen, -OH, -CN, $-CF_3$ , $-NO_2$ , $C_{1-3}$ alkyl, and - $OC_{1-3}$alkyl, preferably the heteroarylene is unsubstituted or optionally substituted with a substituent selected from -Cl, -Br, -F, -OH, -CN, $-CH_3$, and $-OCH_3$ ;

$Ar^1$ at each occurrence is each independently selected from a 6-10 membered arylene; preferably, $Ar^1$ is selected from phenylene; the arylene or phenylene is unsubstituted or optionally substituted with a substituent selected from halogen, -OH, -CN, $-CF_3$, $-NO_2$, $C_{1-6}$ alkyl, $-OC_{1-6}$ alkyl, $-NH_2$, $-NH(C_{1-6}$ alkyl), and $-N(C_{1-6}$ alkyl$)_2$, preferably, the arylene or phenylene is unsubstituted or optionally substituted with a substituent selected from halogen, -OH, CN, $-CF_3$, $-NO_2$ , $C_{1-6}$ alkyl, and $-OC_{1-6}$ alkyl; preferably, the arylene or phenylene is unsubstituted or optionally substituted with a substituent selected from halogen, -OH, -CN, $-CF_3$ , $-NO_2$ , $C_{1-3}$ alkyl, and - $OC_{1-3}$ alkyl; preferably, the arylene or phenylene is unsubstituted or optionally substituted with a substituent selected from -Cl, -Br, -F, -OH, -CN, $-CF_3$, $-NO_2$ , $-CH_3$, and $-OCH_3$; and/or

$R^a$ and $R^b$ are identical or different and are each independently selected from hydrogen atom, deuterium atom, halogen, $C_{1-3}$ alkyl, $C_{1-3}$ haloalkyl, deuterated $C_{1-3}$ alkyl, $-OC_{1-3}$alkyl, -OH, $-NH_2$, -CN, $-CF_3$, $-NO_2$, and $C_{1-3}$ alkylene-OH; preferably, $R^a$ and $R^b$ are identical or different, and are each independently selected from hydrogen atom, deuterium atom, and halogen; and/or

X is independently selected from a single bond, -NH-, or O; and/or

m at each occurrence is each independently an integer of 0, 1, 2, or 3.

**18.** The compound according to any one of claims 15-17 or the pharmaceutically acceptable salt thereof, wherein Y is selected from:

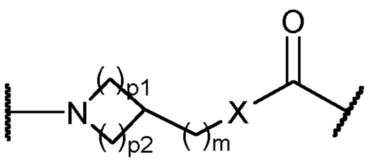
or
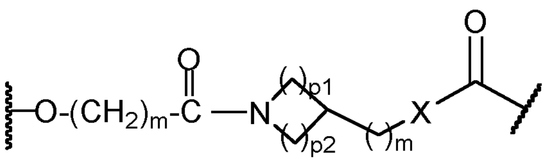
,

wherein p1 is an integer of 0, 1, 2, or 3, p2 is an integer of 1, 2, 3, or 4, and $2 \leq p1 + p2 \leq 4$; X is independently selected from single bond, -NH- or O, and m is an integer of 0, 1, 2, or 3;

or Y is selected from:

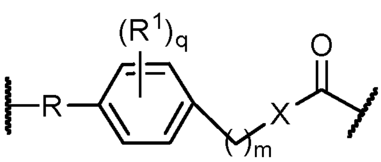
,
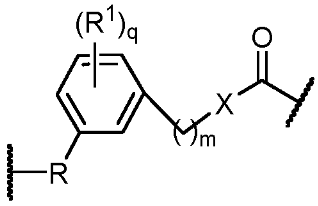
,

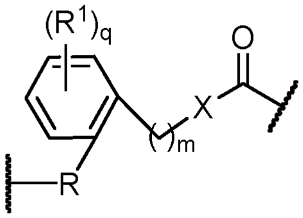

, or

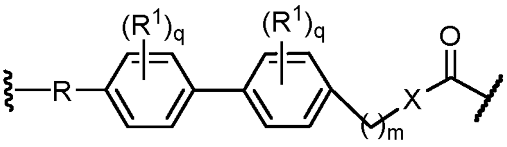

,

wherein R is - NH-, O, or -O-$(CH_2)_m$-C(=O)-NH- , and the -NH- in -O-$(CH_2)_m$-C(=O)-NH- is connected to the phenyl or biphenyl group in Y; X is independently selected from a single bond, -NH-, or O, m is an integer of 0, 1, 2, or 3, and $R^1$ at each occurrence is each independently selected from halogen, -OH, -CN, -$CF_3$, - $NO_2$, $C_{1-6}$ alkyl, -$OC_{1-6}$ alkyl, -$NH_2$, -NH($C_{1-6}$ alkyl), and -N ($C_{1-6}$ alkyl)$_2$, q is an integer of 0, 1, 2, 3, or 4; preferably, X is independently selected from a single bond or -NH-, m is an integer of 0 or 1, and $R^1$ at each occurrence is each independently selected from halogen, -OH, -CN, -$CF_3$, -$NO_2$, $C_{1-3}$ alkyl, and -$OC_{1-3}$ alkyl, preferably $R^1$ at each occurrence is each independently selected from -Cl, -Br, -F, -OH, -CN, -$CF_3$, -$NO_2$ , methyl, and -$OCH_3$ , and q is an integer of 0, 1, or 2;

or Y is selected from:

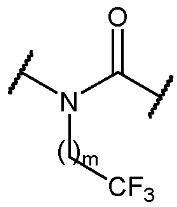

,

wherein m is an integer of 0, 1, 2 or 3; preferably, m is 1 or 2; and the C(=O)-end of the structure Y is connected to - NH- in the structure of formula (-E) or formula (-$E_0$).

**19.** The compound according to any one of claims 15-18 or the pharmaceutically acceptable salt thereof, wherein Y is selected from:

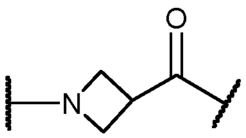

,

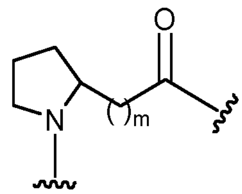

,

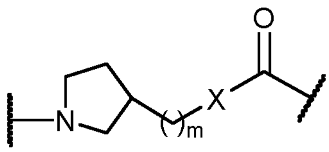

,

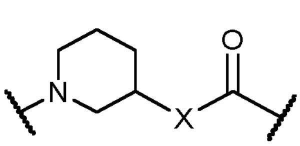

,

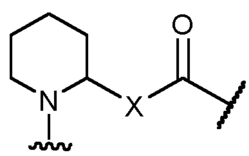

,

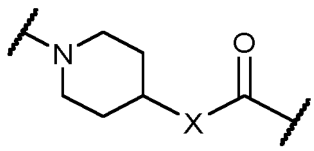

, ,

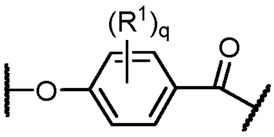

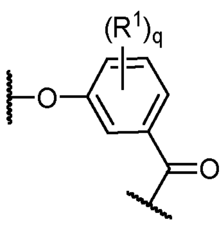

,

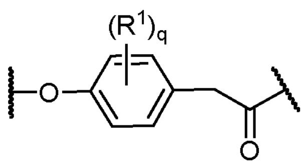

,

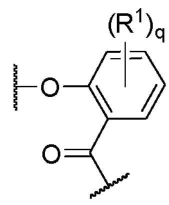

,

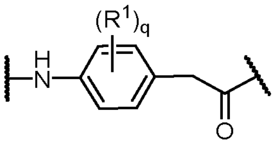, 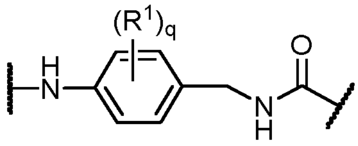, 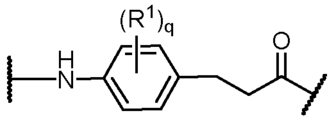,

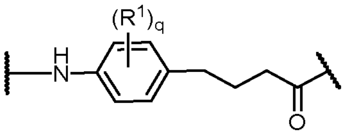, 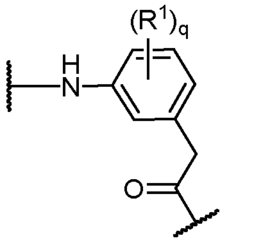, 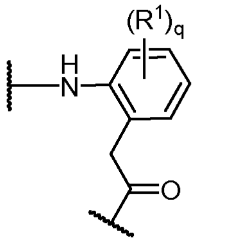,

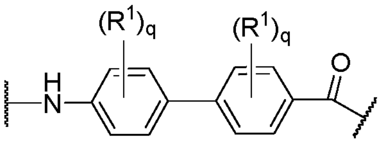, 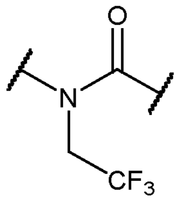, 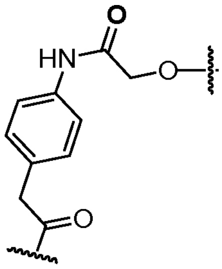

and

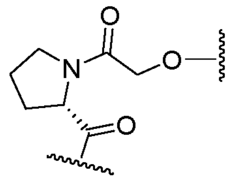.

wherein, X is independently selected from a single bond or -NH-, m is an integer of 0 or 1;
$R^1$ is selected from halogen, -OH, -CN, $-CF_3$, $-NO_2$, $C_{1-6}$ alkyl, $-OC_{1-6}$ alkyl, $-NH_2$, $-NH(C_{1-6}$ alkyl), and $-N(C_{1-6}$ alkyl$)_2$, preferably $R^1$ is selected from halogen, -OH, -CN, $C_{1-3}$ alkyl, $-OC_{1-3}$ alkyl, preferably $R^1$ is selected from -Cl, -Br, -F, -OH, -CN, $-CF_3$, $-NO_2$, methyl, and $-OCH_3$;
q is an integer of 0, 1, 2, 3 or 4; preferably, q is an integer of 0, 1 or 2; and
the C(=O)-end of the Y structure is connected to -NH- in the structure of formula (E) or formula $(E_0)$.

**20.** The compound according to any one of claims 15-19 or the pharmaceutically acceptable salt thereof, the compound is selected from:

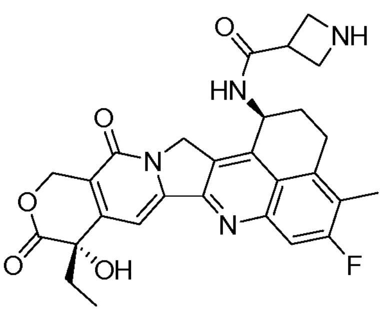, 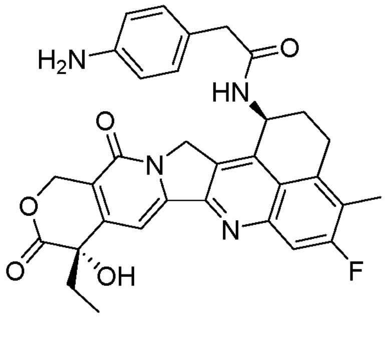,

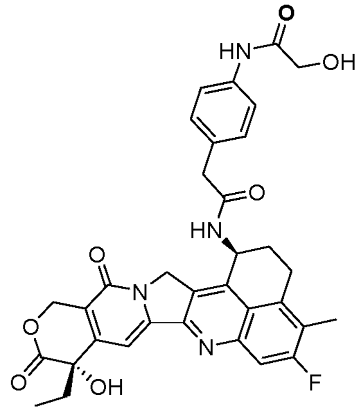

,

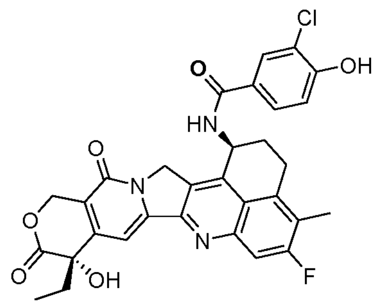

,

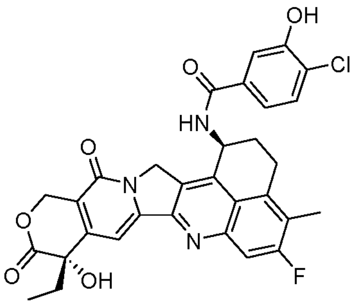

,

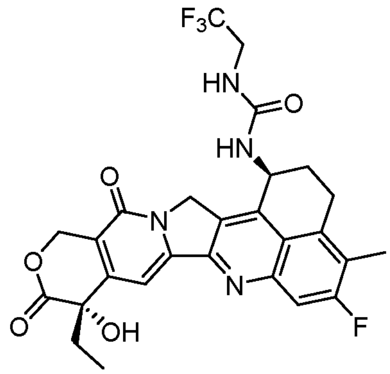

,

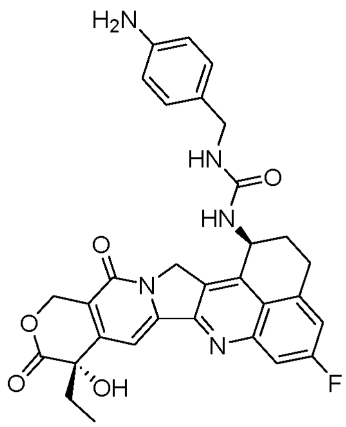

,

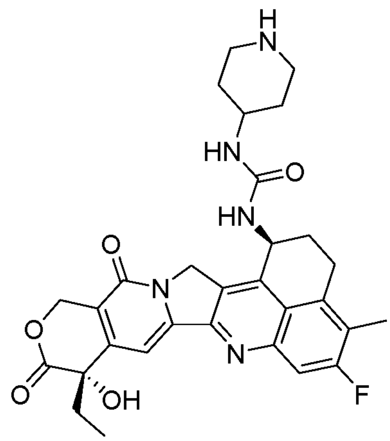

,

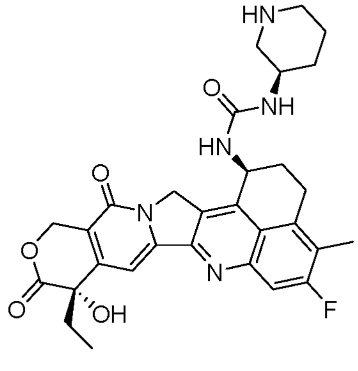

, ,

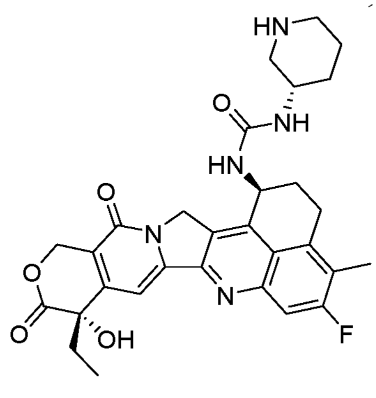

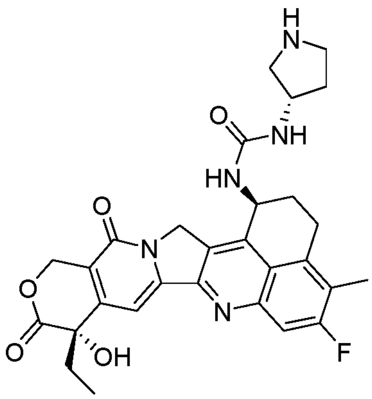

,

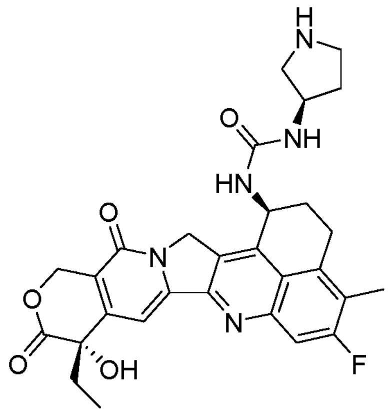

,

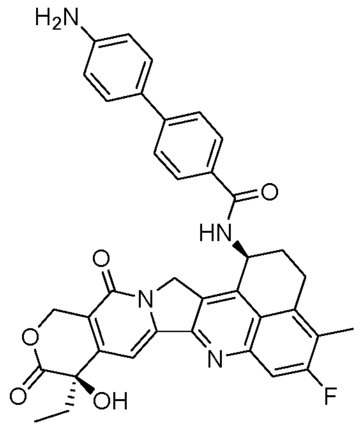

,

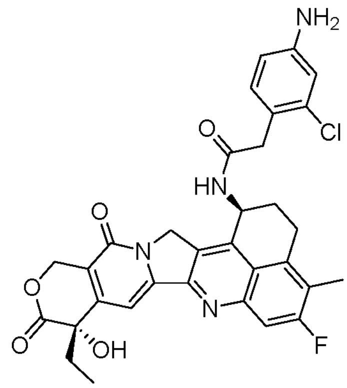

,

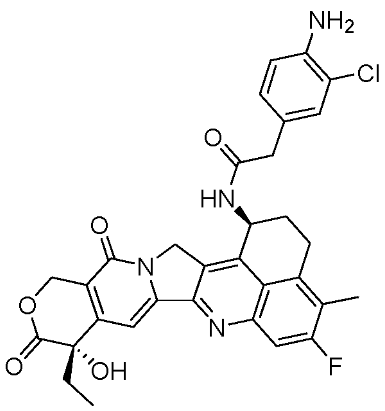

,

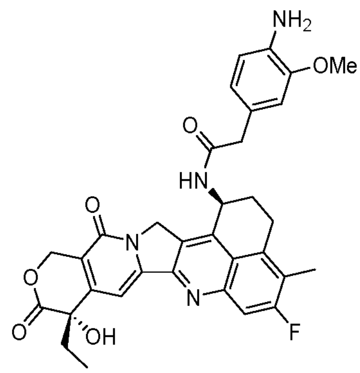

,

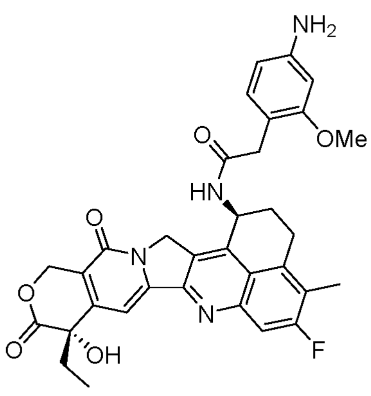

,

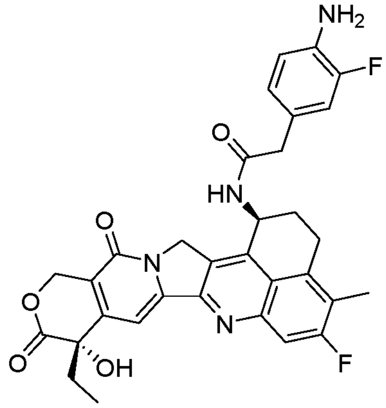

,

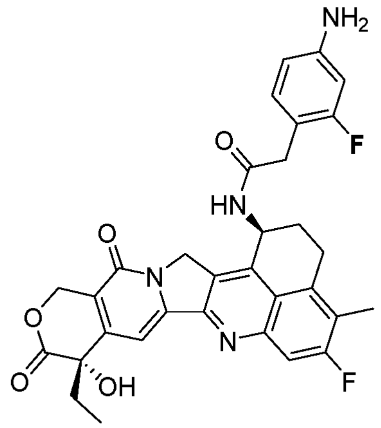

,

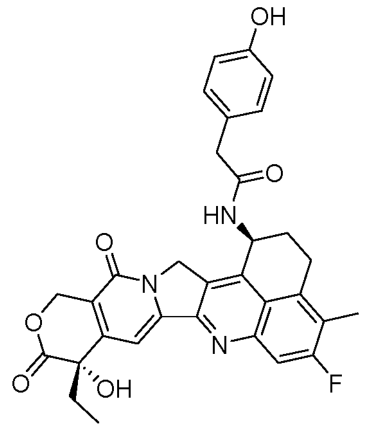

,

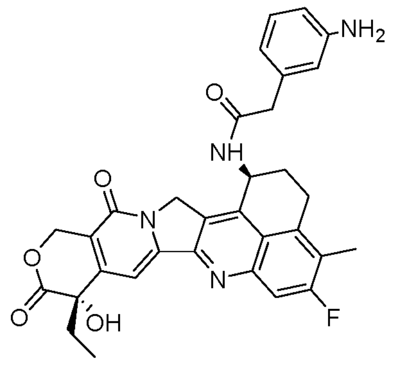

,

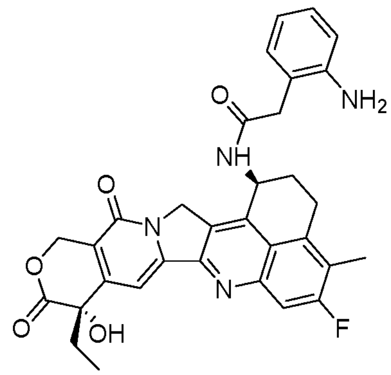

,

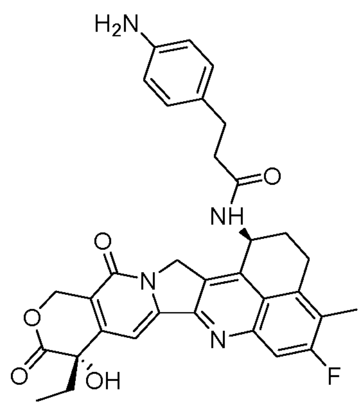

,

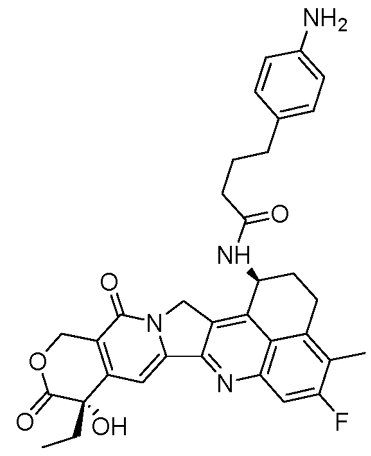

,

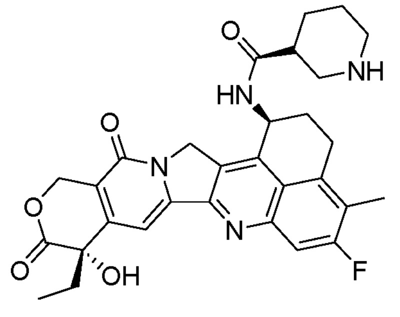

,

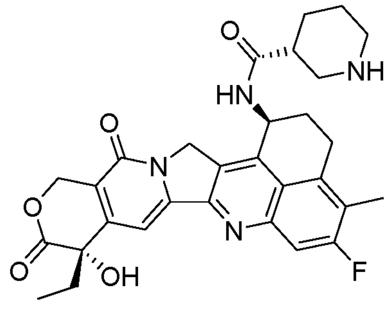

,

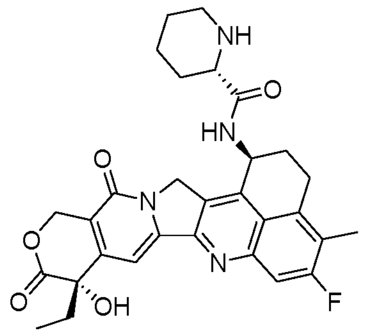

,

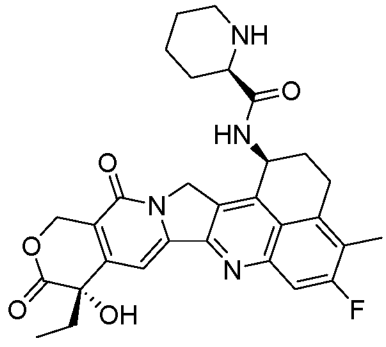

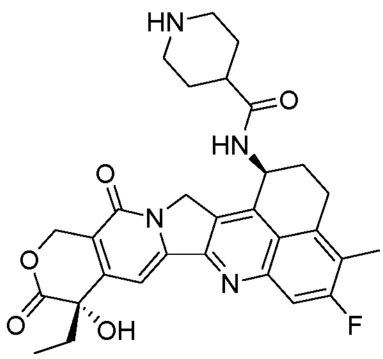

,

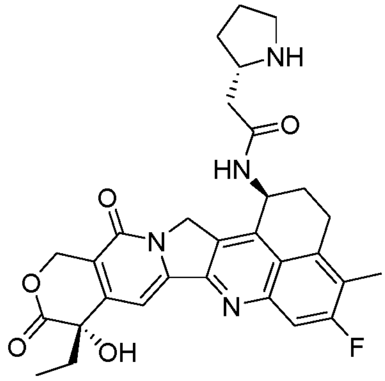

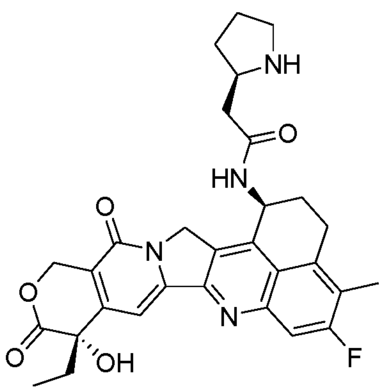

,

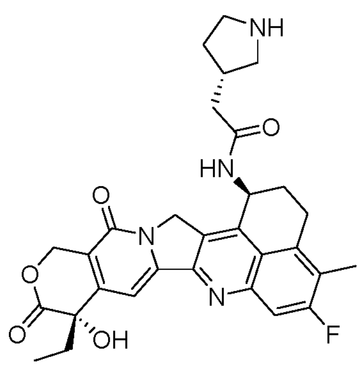

,

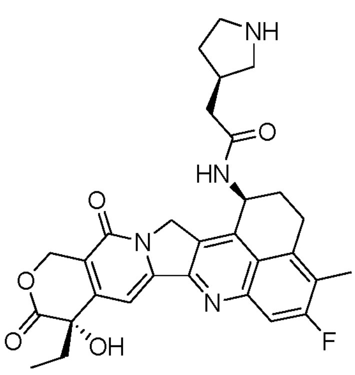

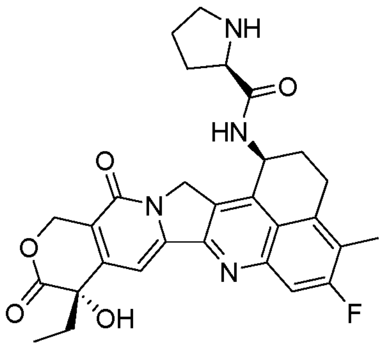

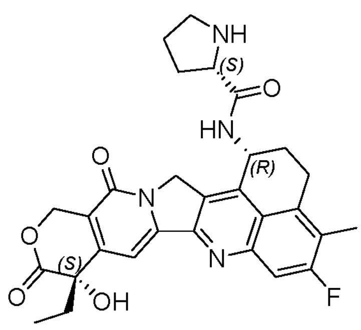

and

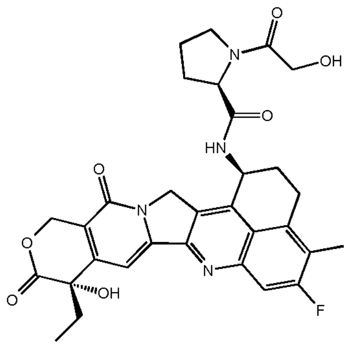

.

**21.** A compound having the structure shown in the following formula (I):

$L^1$-$L^2$-$L^3$-$L^4$ (I)

$L^1$ is selected from maleimidyl-N-$(CH_2)_s$-C(=O)-, maleimidyl-N-$CH_2$-cyclohexyl-C(=O)-, wherein s is an integer from 2 to 8;

$L^2$ is selected from -$NR^4(CH_2CH_2O)_rCH_2CH_2C$(=O)- and -$NR^4CH_2$-$Ar^2$-$(CH_2CH_2O)_rCH_2CH_2NR^4C$(=O)$CH_2OCH_2C$(=O)-, wherein r is an integer from 1 to 20, and $Ar^2$ is

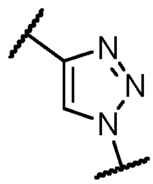

;

$L^3$ is a dipeptide residue of valine-alanine, a tripeptide residue of alanine-alanine-alanine, or a tetrapeptide residue of glycine-glycine-phenylalanine-glycine;

$L^4$ is selected from -$NR^5(CR^6R^7)_t$-Z-$(CR^6R^7)_t$-COOH or -$NR^5$-$Ar^3$-$(CR^6R^7)_t$-OH, wherein, t is an integer from 1 to 3;

Z is selected from a single bond, O, S or -NH-;

$Ar^3$ is selected from phenyl, the phenyl is unsubstituted or optionally substituted with a substituent selected from H, halogen, -OH, -CN, -$CF_3$, -$NO_2$, $C_{1-6}$ alkyl;

$R^4$ and $R^5$ are identical or different, and at each occurrence are each independently selected from hydrogen atom, halogen, $C_{1-3}$alkyl, $C_{1-3}$haloalkyl and deuterated $C_{1-3}$ alkyl; and

$R^6$ and $R^7$ are identical or different, and at each occurrence are each independently selected from hydrogen atom, halogen, $C_{1-3}$ alkyl, $C_{1-3}$ haloalkyl and deuterated $C_{1-3}$ alkyl.

**22.** The compound of formula (I) according to claim 21, the compound is selected from:

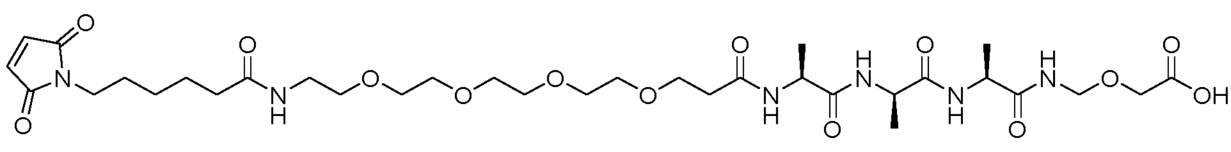

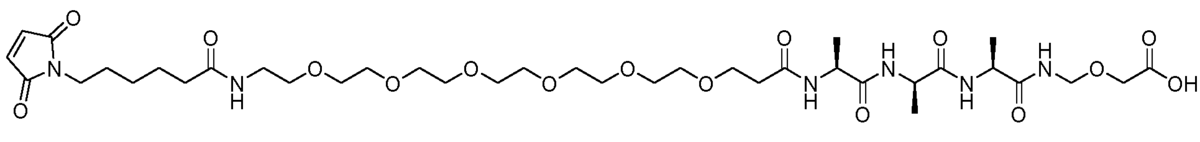

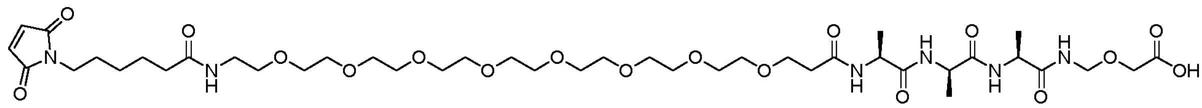

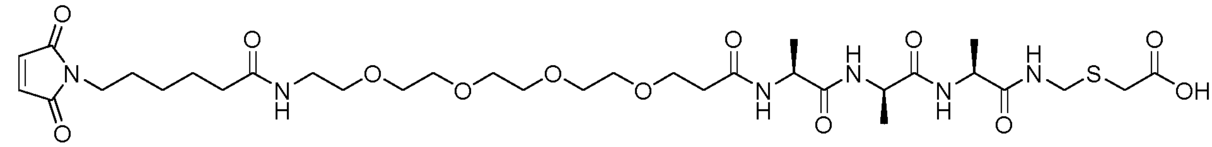

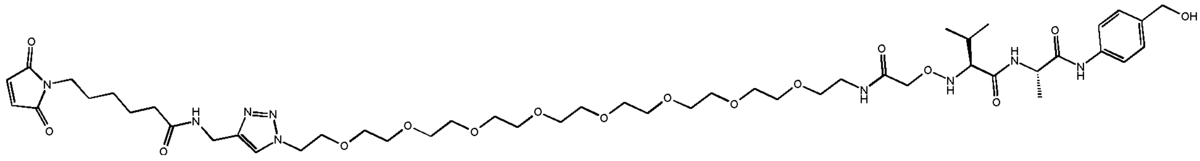

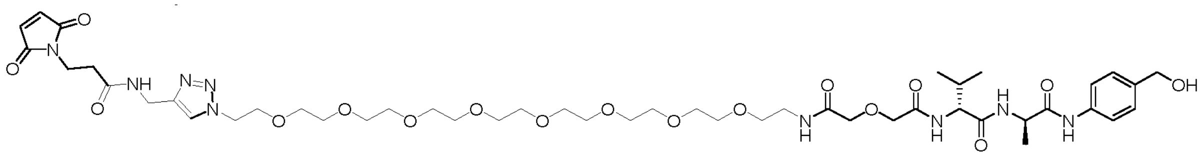

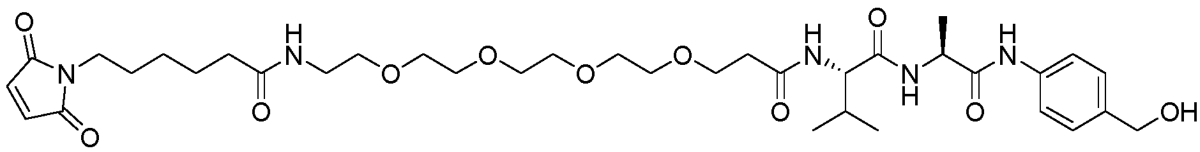

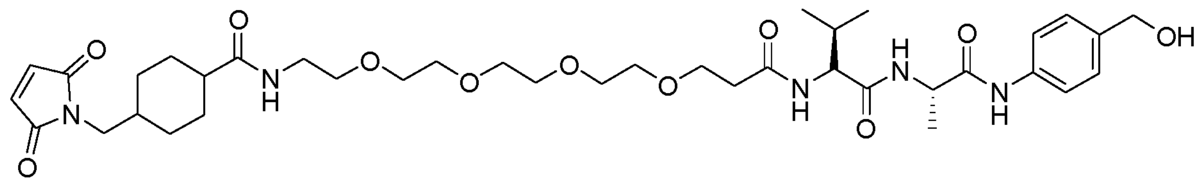

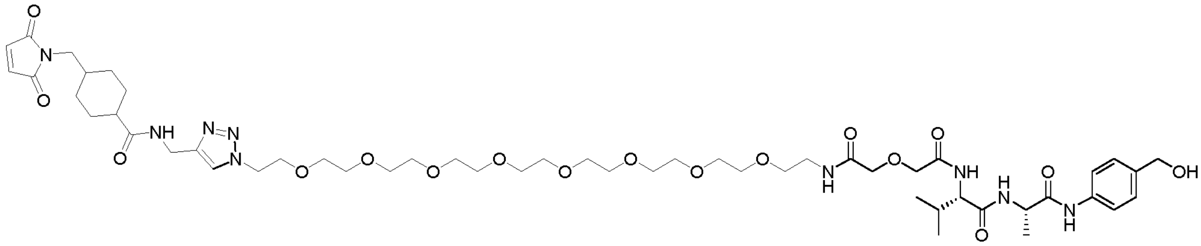

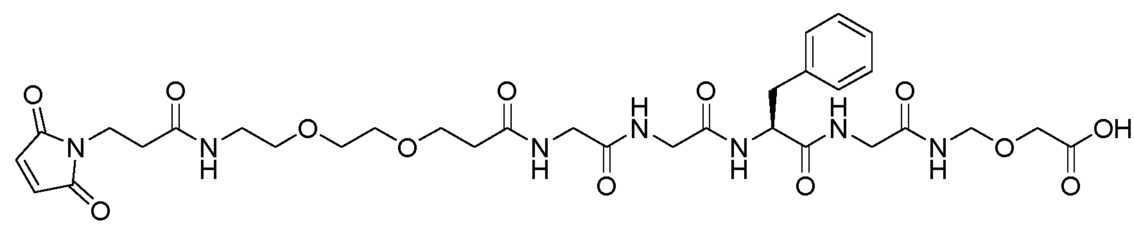

and

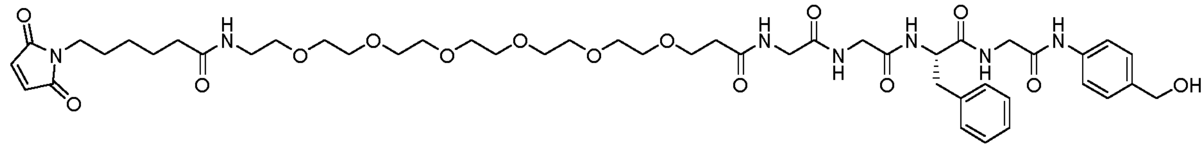

**23.** A pharmaceutical composition, comprising a therapeutically effective amount of the ligand-drug conjugate according to any one of claims 1 to 14, or a pharmaceutically acceptable salt thereof, or the compound according to any one of claims 15 to 20 or a pharmaceutically acceptable salt thereof, and a pharmaceutically acceptable carrier.

**24.** Use of the ligand-drug conjugate or pharmaceutically acceptable salt thereof according to any one of claims 1 to 14, or the compound or pharmaceutically acceptable salt thereof according to any one of claims 15 to 20, or the pharmaceutical composition according to claim 23 in the preparation of a medicament for the treatment or prevention of a tumor;

preferably, wherein the tumor is a cancer associated with TROP-2 expression, or the tumor is a cancer associated with HER3 expression;
preferably, the HER3 expression-associated cancer is selected from non-small cell lung cancer, melanoma, breast cancer and colorectal adenocarcinoma;
preferably, the TROP-2 expression-associated cancer are selected from breast cancer, triple negative breast cancer, gastric cancer and pancreatic cancer.

**25.** A method of preventing or treating a tumor, including administering a therapeutically effective amount of the ligand-drug conjugate or the pharmaceutically acceptable salt thereof according to any one of claims 1 to 14 , or the compound or the pharmaceutically acceptable salt thereof according to any one of claims 15 to 20, or the pharmaceutical composition according to claim 23 to a subject in need thereof;

preferably, wherein the tumor is a cancer associated with TROP-2 expression, or the tumor is a cancer associated with HER3 expression;
preferably, the HER3 expression-associated cancer is selected from non-small cell lung cancer, melanoma, breast cancer and colorectal adenocarcinoma;
preferably, the TROP-2 expression-associated cancers are selected from breast cancer, triple negative breast cancer, gastric cancer and pancreatic cancer.

**26.** A ligand-drug conjugate or a pharmaceutically acceptable salt thereof according to any one of claims 1 to 13, or a compound or a pharmaceutically acceptable salt thereof according to any one of claims 15 to 20, or a pharmaceutical composition according to claim 23, for use in the prevention or treatment of a tumor;
preferably, wherein the tumor is a cancer associated with TROP-2 expression, or the tumor is a cancer associated with HER3 expression.

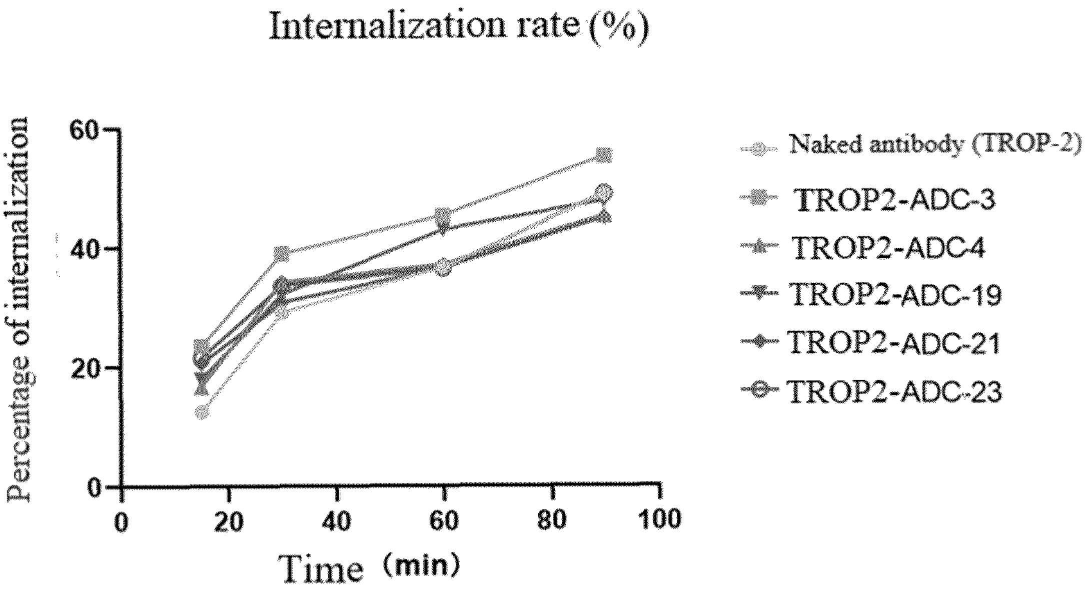


Figure 1

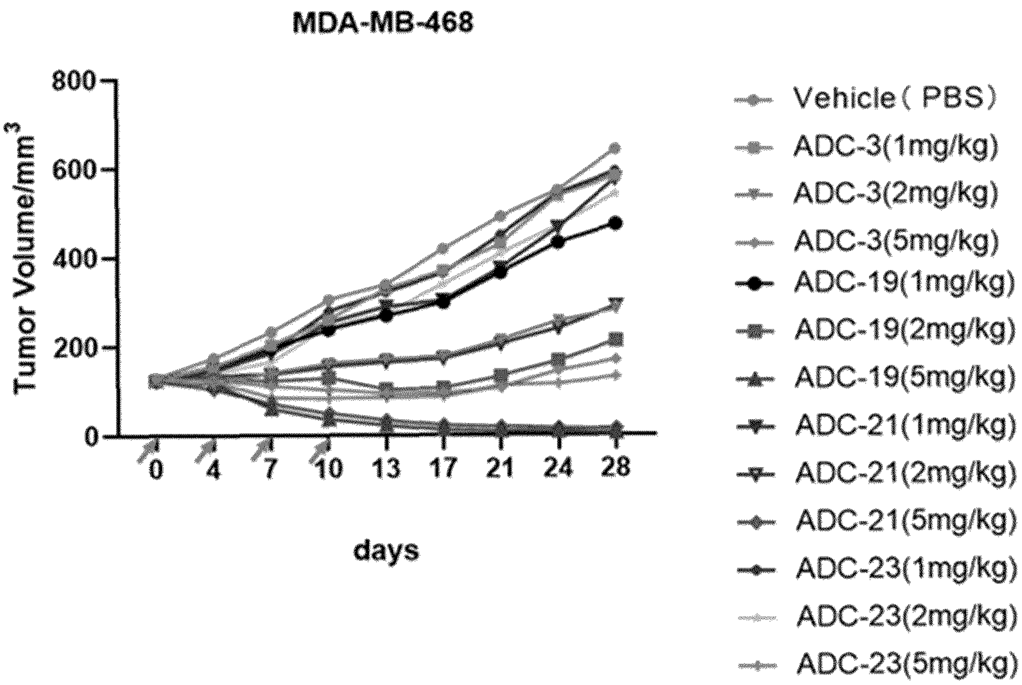


Figure 2

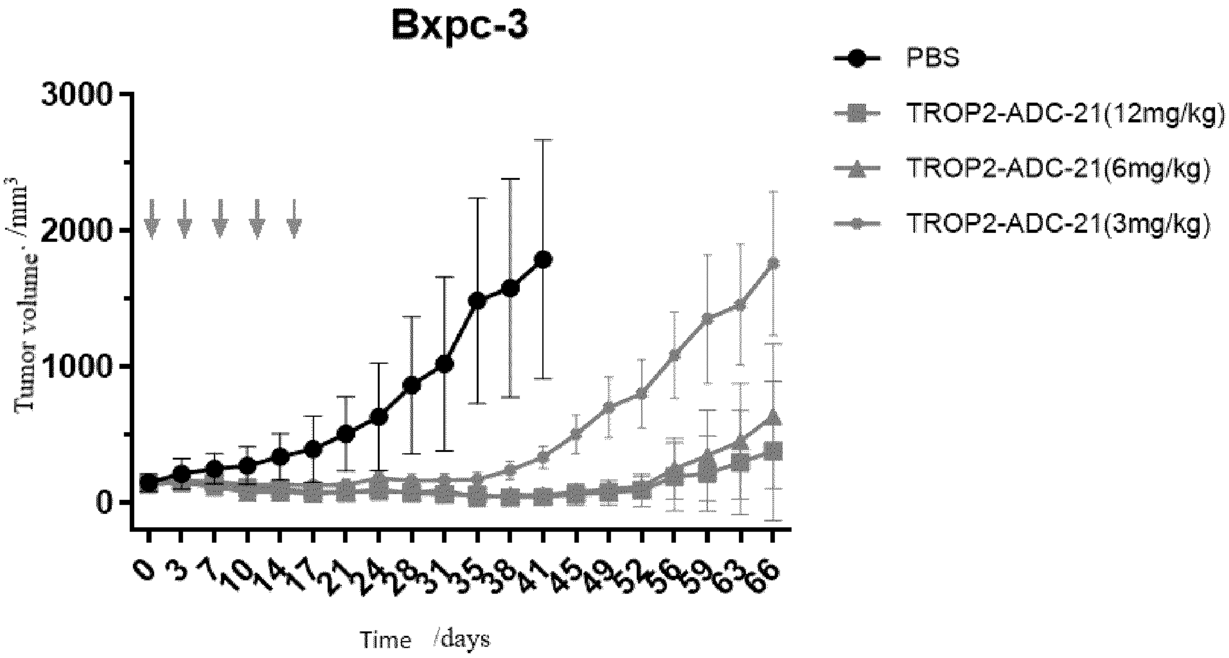


Figure 3

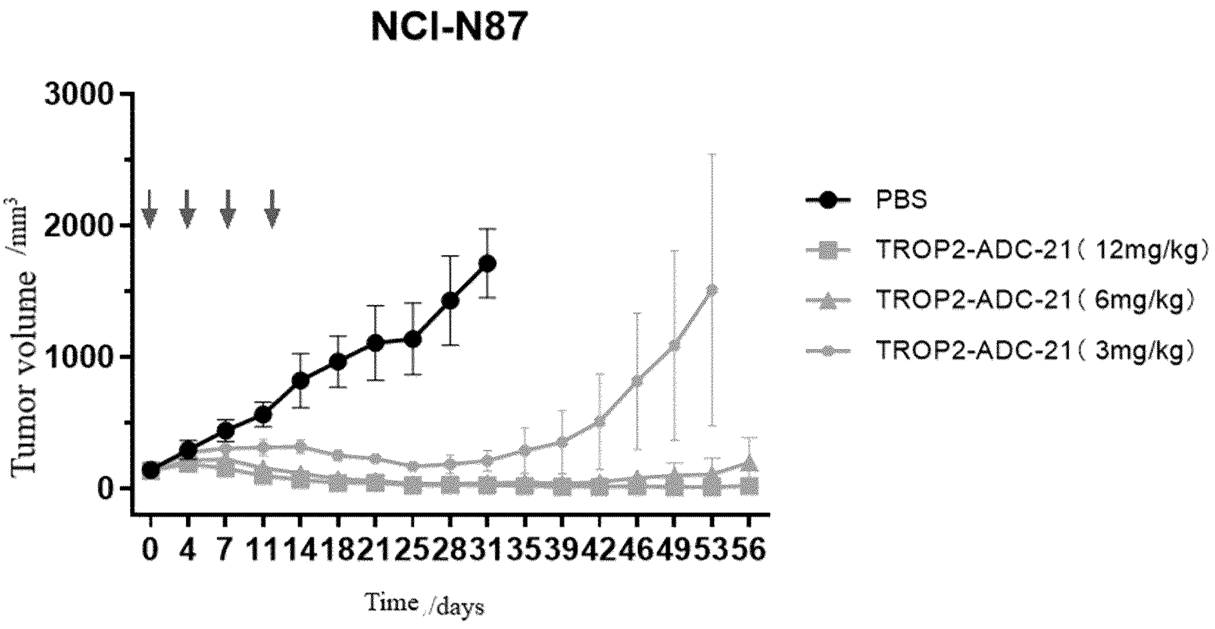


Figure 4

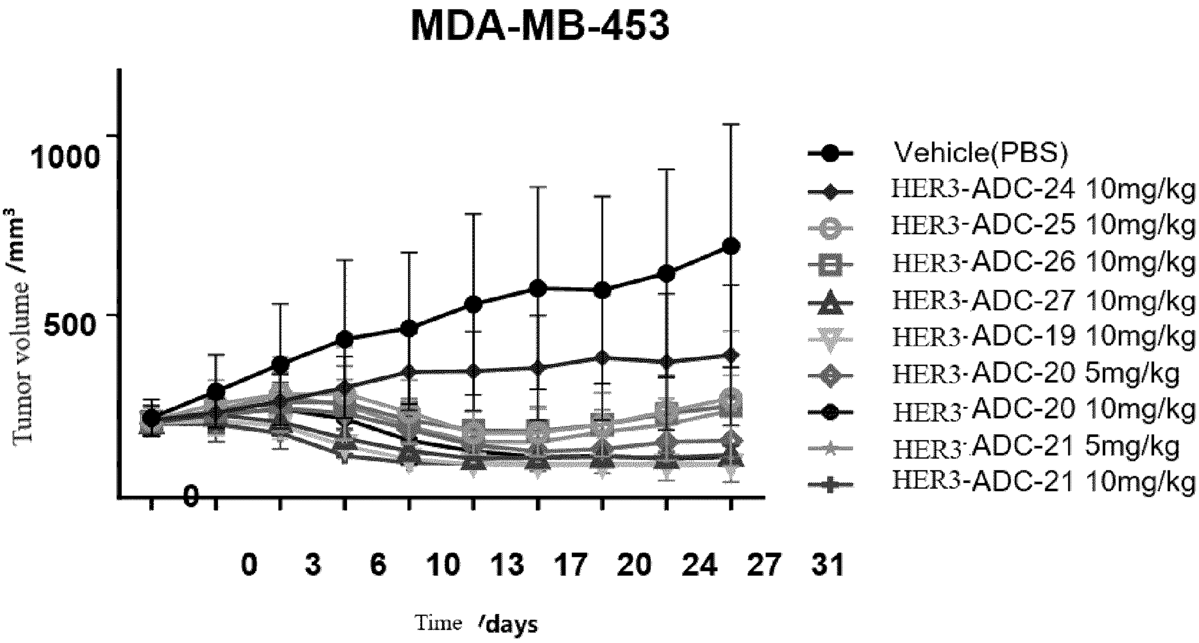


Figure 5

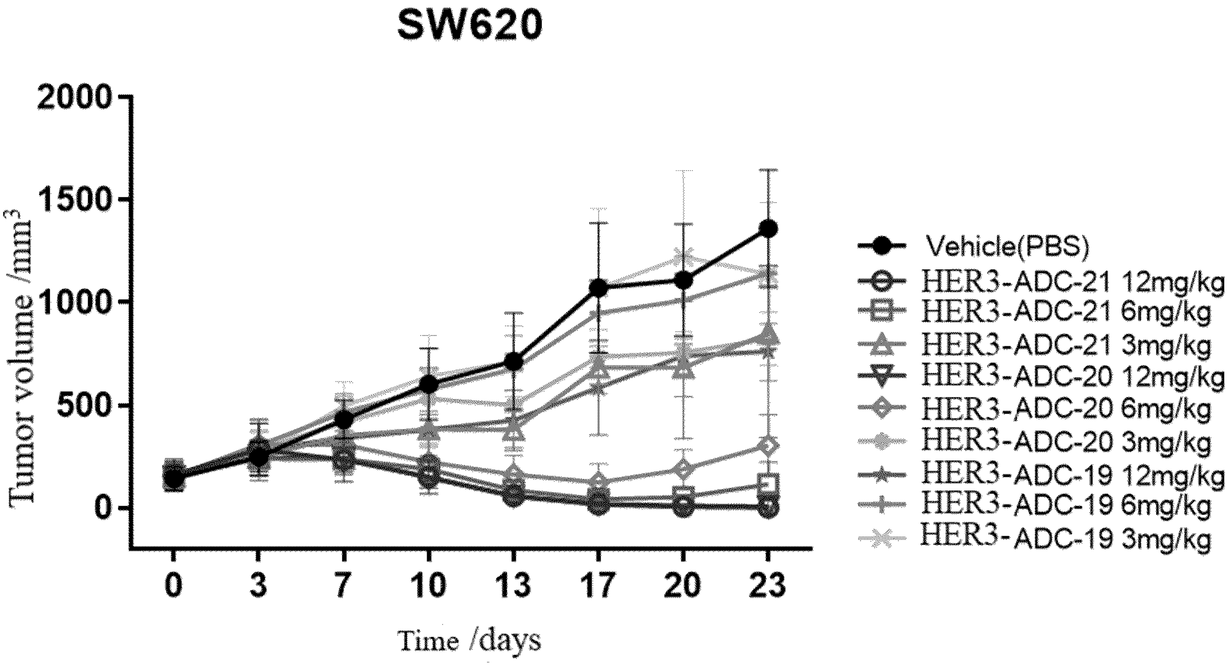


Figure 6

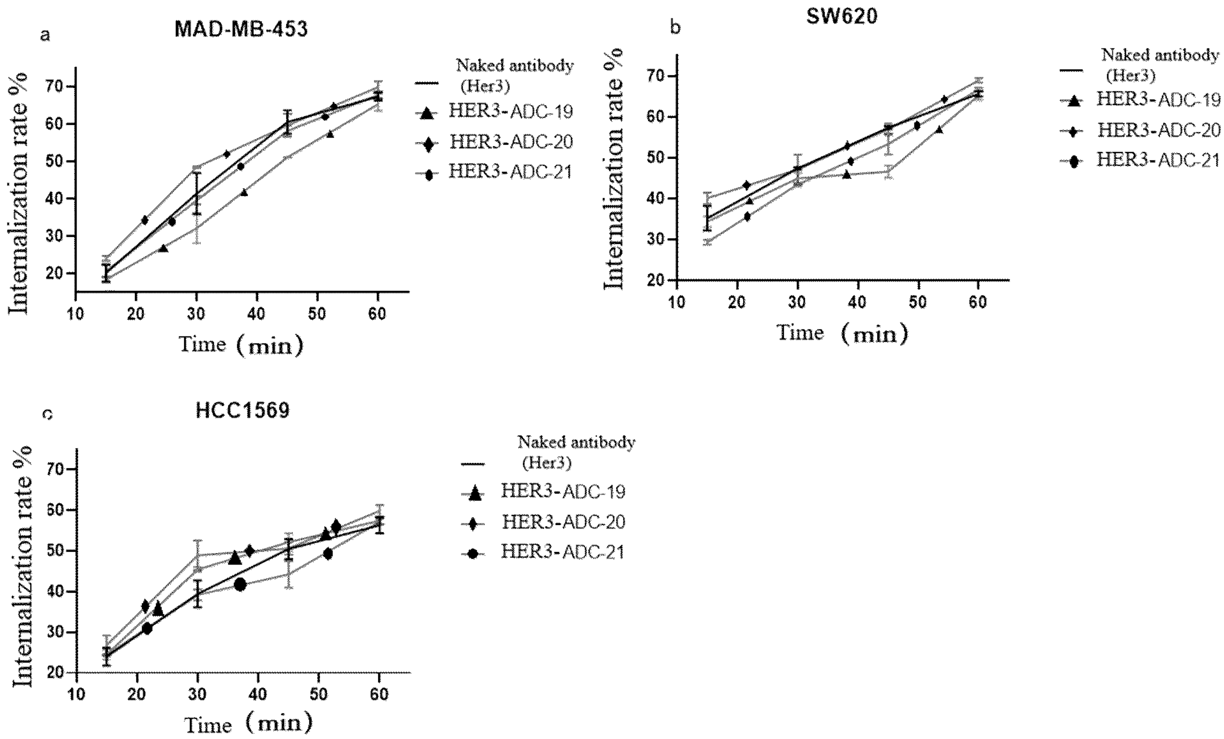


Figure 7

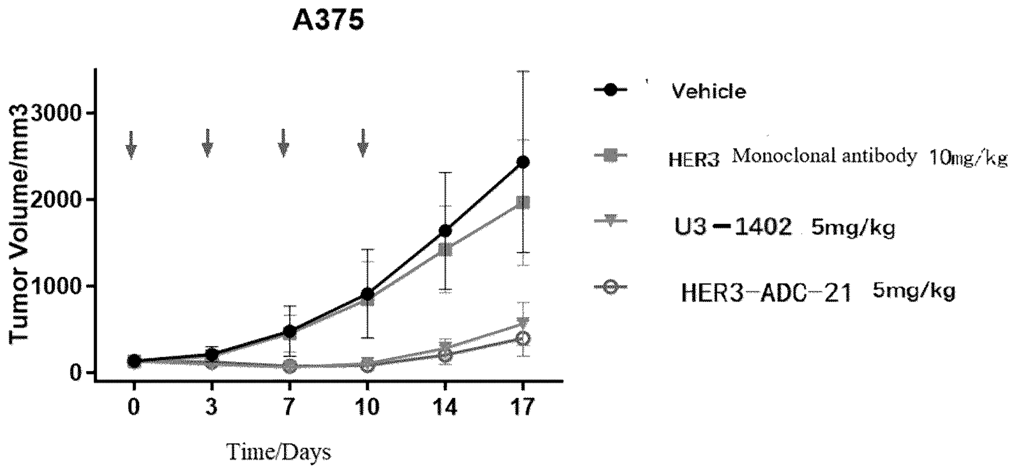


Figure 8

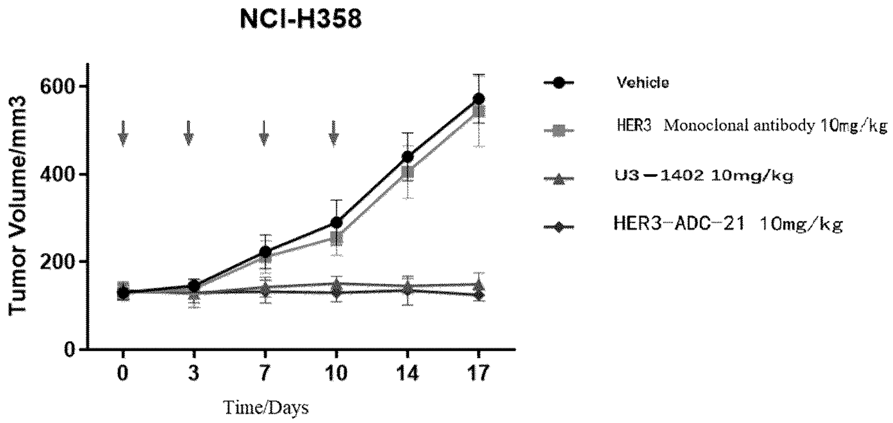


Figure 9

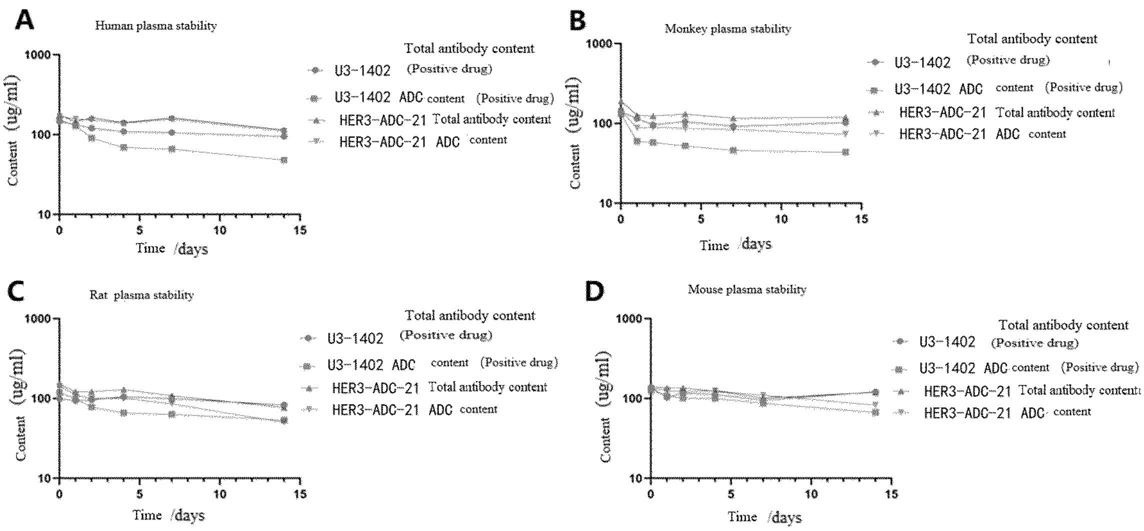


Figure 10

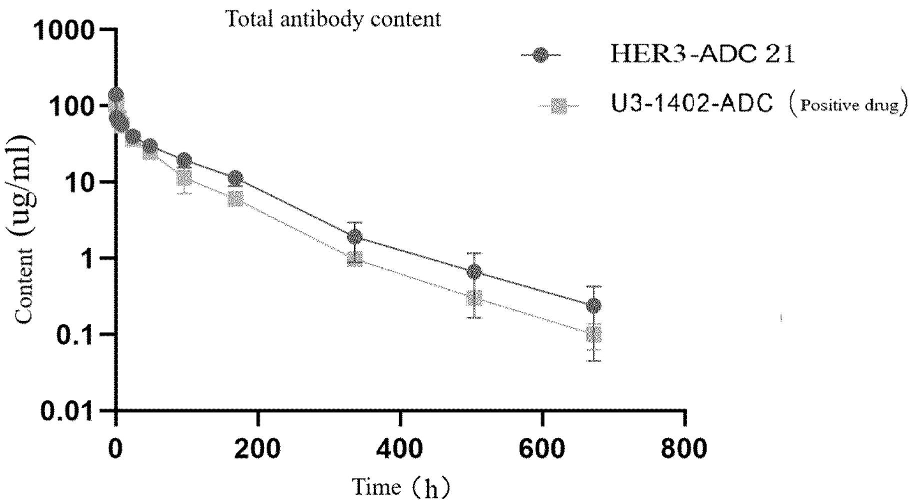


Figure 11

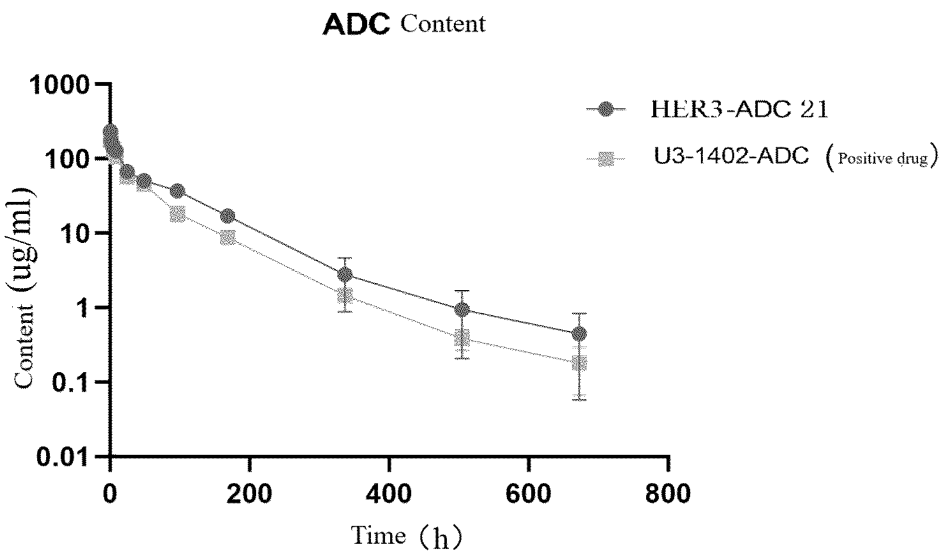


Figure 12

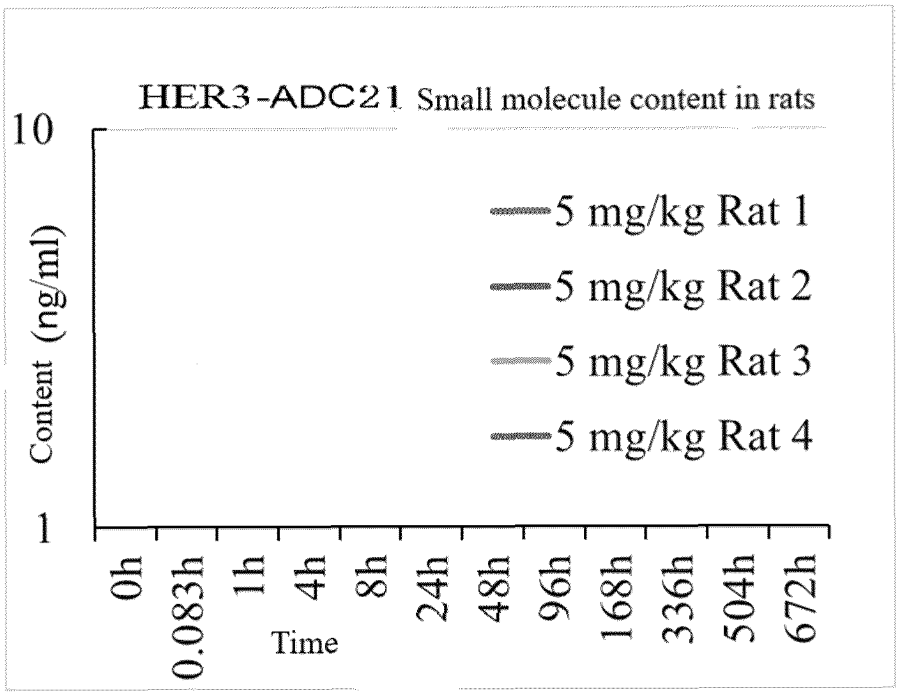


Figure 13

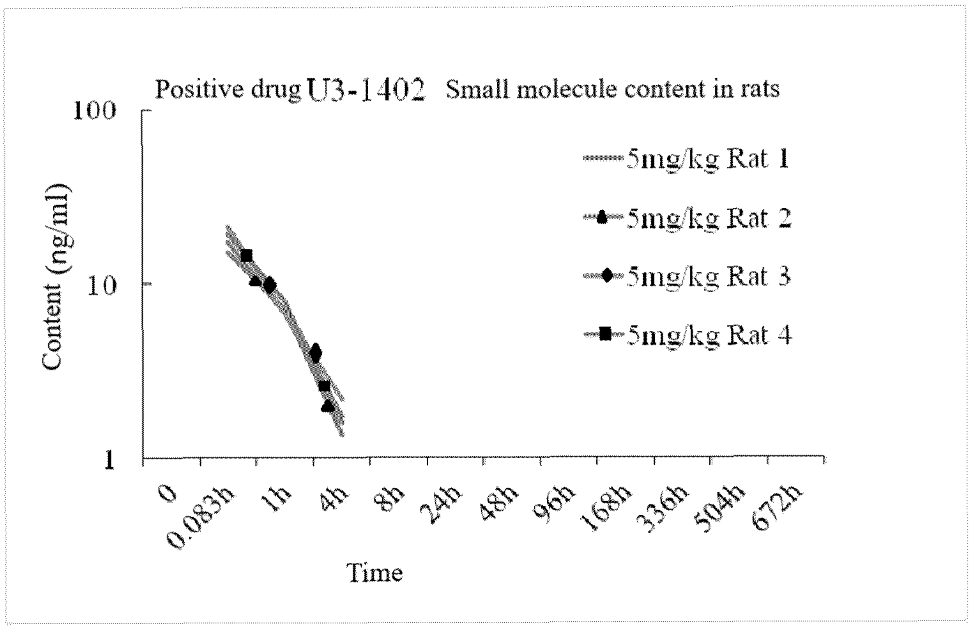


Figure 14

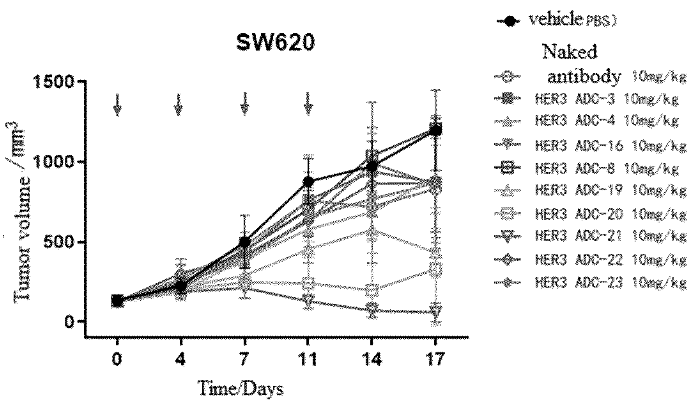
SW620
Tumor volume /mm3
1500
1000
500
0
0
4
7
11
14
17
Time/Days
vehicle PBS)
Naked antibody 10mg/kg
HER3 ADC-3 10mg/kg
HER3 ADC-4 10mg/kg
HER3 ADC-16 10mg/kg
HER3 ADC-8 10mg/kg
HER3 ADC-19 10mg/kg
HER3 ADC-20 10mg/kg
HER3 ADC-21 10mg/kg
HER3 ADC-22 10mg/kg
HER3 ADC-23 10mg/kg

Figure 15

## INTERNATIONAL SEARCH REPORT

International application No.
**PCT/CN2023/073014**

### A. CLASSIFICATION OF SUBJECT MATTER

A61K47/68(2017.01)i; A61K31/48(2006.01)i; A61K31/4745(2006.01)i; C07K16/30(2006.01)i; C07K16/28(2006.01)i; A61P35/00(2006.01)i

According to International Patent Classification (IPC) or to both national classification and IPC

### B. FIELDS SEARCHED

Minimum documentation searched (classification system followed by classification symbols)

A61K C07K A61P

Documentation searched other than minimum documentation to the extent that such documents are included in the fields searched

Electronic data base consulted during the international search (name of data base and, where practicable, search terms used)

CNABS, CNTXT, VEN, WOTXT, EPTXT, USTXT, CNKI, 万方, WANFANG, 读秀, DUXIU, PUBMED, ISI_Web of Science, Science Direct, STNext: 甘李药业, 秦文芳, 依喜替康, 抗体, 偶联物, 肿瘤, 癌症, 结构检索, exatecan, antibody, conjugates, tumor, cancer, structure search

### C. DOCUMENTS CONSIDERED TO BE RELEVANT

| Category* | Citation of document, with indication, where appropriate, of the relevant passages | Relevant to claim No. |
|---|---|---|
| PX | WO 2022068878 A1 (DUALITY BIOLOGICS (SUZHOU) CO., LTD.) 07 April 2022 (2022-04-07)<br>claims 1-103 | 1-26 |
| X | CN 112533958 A (DAIICHI SANKYO COMPANY, LIMITED) 19 March 2021 (2021-03-19)<br>claims 1-9, 45, description paragraphs 0007, 0137-0148 | 1-26 |
| X | CN 109081871 A (DAIICHI SANKYO COMPANY, LIMITED) 25 December 2018 (2018-12-25)<br>description paragraphs 0546-0767, claims 1-20 | 1-26 |
| X | CN 113939318 A (DAIICHI SANKYO COMPANY, LIMITED) 14 January 2022 (2022-01-14)<br>claims 1-66, figure 1, embodiment 1 | 1-26 |
| X | EP 2910573 A1 (DAIICHI SANKYO COMPANY, LIMITED) 26 August 2015 (2015-08-26)<br>description paragraphs 0003-0005, 0012-0014, embodiments 1-41 | 1-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 June 2023** | **02 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No. **PCT/CN2023/073014**

### C. DOCUMENTS CONSIDERED TO BE RELEVANT

| Category* | Citation of document, with indication, where appropriate, of the relevant passages | Relevant to claim No. |
|---|---|---|
| X | EP 3130608 A1 (DAIICHI SANKYO CO., LTD.) 15 February 2017 (2017-02-15) description, paragraphs 0016-0017 and 0150-0299, and embodiments 1-158 | 1-26 |
| X | WO 2022011075 A1 (VELOSBIO INC.) 13 January 2022 (2022-01-13) description paragraphs 0012-0013, 0071-0079, claims 1-31 | 1-26 |

Form PCT/ISA/210 (second sheet) (July 2022)

INTERNATIONAL SEARCH REPORT

International application No.
PCT/CN2023/073014

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/073014**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **25**
because they relate to subject matter not required to be searched by this Authority, namely:

Claim 25 sets forth a method for preventing or treating tumors, which comprises a treatment solution for the human or animal body, and therefore does not comply with PCT Rule 39.1(iv). The search for claim 25 is performed on the basis of the following amendment: the use of the ligand-drug conjugate of any one of claims 1 to 14 or a pharmaceutically acceptable salt thereof, or the compound of any one of claims 15 to 20 or a pharmaceutically acceptable salt thereof, or the pharmaceutical composition of claim 23 in the preparation of a drug for preventing or treating tumors.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/073014**

| Patent document cited in search report | Publication date (day/month/year) | Patent family member(s) | Publication date (day/month/year) |
|---|---|---|---|
| WO 2022068878 A1 | 07 April 2022 | US 2023086097 A1 | 23 March 2023 |
| | | AU 2021354823 A1 | 30 March 2023 |
| | | US 11607459 B1 | 21 March 2023 |
| | | TW 202214230 A | 16 April 2022 |
| | | CA 3195515 A1 | 07 April 2022 |
| CN 112533958 A | 19 March 2021 | CA 3107732 A1 | 30 January 2020 |
| | | JPWO 2020022475 A1 | 12 August 2021 |
| | | US 2021169852 A1 | 10 June 2021 |
| | | WO 2020022475 A1 | 30 January 2020 |
| | | AU 2019311596 A1 | 28 January 2021 |
| | | IL 280296 A | 25 March 2021 |
| | | EP 3831853 A1 | 09 June 2021 |
| | | EP 3831853 A4 | 01 June 2022 |
| | | TW 202019973 A | 01 June 2020 |
| | | KR 20210040059 A | 12 April 2021 |
| CN 109081871 A | 25 December 2018 | LT 2907824 T | 11 June 2018 |
| | | US 2016279259 A1 | 29 September 2016 |
| | | US 9808537 B2 | 07 November 2017 |
| | | JP 2022008581 A | 13 January 2022 |
| | | JP 7170812 B2 | 14 November 2022 |
| | | JP 2023011799 A | 24 January 2023 |
| | | CA 3021435 A1 | 17 April 2014 |
| | | CA 3021435 C | 12 October 2021 |
| | | JP 6030267 B1 | 24 November 2016 |
| | | JP 2017036274 A | 16 February 2017 |
| | | BR 122021014396 B1 | 05 July 2022 |
| | | SI 2907824 T1 | 29 June 2018 |
| | | BR 112015006521 A2 | 05 September 2017 |
| | | BR 112015006521 B1 | 03 March 2022 |
| | | US 2020282073 A1 | 10 September 2020 |
| | | US 11633493 B2 | 25 April 2023 |
| | | KR 20220100727 A | 15 July 2022 |
| | | KR 102456726 B1 | 20 October 2022 |
| | | PT 3342785 T | 25 February 2020 |
| | | SG 10201804788 XA | 30 July 2018 |
| | | PT 2907824 T | 07 June 2018 |
| | | NZ 746440 A | 29 November 2019 |
| | | JP 5953378 B2 | 20 July 2016 |
| | | JPWO 2014057687 A1 | 05 September 2016 |
| | | ES 2773710 T3 | 14 July 2020 |
| | | WO 2014057687 A1 | 17 April 2014 |
| | | CA 2885800 A1 | 17 April 2014 |
| | | CA 2885800 C | 04 December 2018 |
| | | US 2015297748 A1 | 22 October 2015 |
| | | US 10195288 B2 | 05 February 2019 |
| | | AU 2020200548 A1 | 13 February 2020 |
| | | AU 2020200548 B2 | 25 August 2022 |
| | | TR 201809636 T4 | 23 July 2018 |
| | | KR 20180030734 A | 23 March 2018 |
| | | KR 101901558 B1 | 21 September 2018 |

Form PCT/ISA/210 (patent family annex) (July 2022)

**INTERNATIONAL SEARCH REPORT**
**Information on patent family members**

International application No.
**PCT/CN2023/073014**

| Patent document cited in search report | Publication date (day/month/year) | Patent family member(s) | | | Publication date (day/month/year) |
|---|---|---|---|---|---|
| | | HRP | 20180870 | T1 | 13 July 2018 |
| | | KR | 20150067149 | A | 17 June 2015 |
| | | KR | 101841818 | B1 | 26 March 2018 |
| | | TW | 202233186 | A | 01 September 2022 |
| | | MX | 2020010964 | A | 09 November 2020 |
| | | KR | 20190135559 | A | 06 December 2019 |
| | | KR | 102087017 | B1 | 10 March 2020 |
| | | KR | 20180105271 | A | 27 September 2018 |
| | | KR | 102052319 | B1 | 05 December 2019 |
| | | SG | 11201502887 | WA | 28 May 2015 |
| | | CY | 1120363 | T1 | 10 July 2019 |
| | | TW | 201811746 | A | 01 April 2018 |
| | | TWI | 650312 | B | 11 February 2019 |
| | | TW | 201420117 | A | 01 June 2014 |
| | | TWI | 615152 | B | 21 February 2018 |
| | | RS | 57278 | B1 | 31 August 2018 |
| | | TW | 202033499 | A | 16 September 2020 |
| | | TWI | 757740 | B | 11 March 2022 |
| | | KR | 20220029776 | A | 08 March 2022 |
| | | KR | 102417310 | B1 | 05 July 2022 |
| | | DK | 3342785 | T3 | 23 March 2020 |
| | | EP | 3632471 | A1 | 08 April 2020 |
| | | PL | 3342785 | T3 | 07 September 2020 |
| | | PH | 12018501433 | A1 | 27 February 2019 |
| | | DK | 2907824 | T3 | 23 July 2018 |
| | | PL | 2907824 | T3 | 31 August 2018 |
| | | ZA | 201501825 | B | 25 May 2022 |
| | | KR | 20210132240 | A | 03 November 2021 |
| | | KR | 102369419 | B1 | 02 March 2022 |
| | | IL | 238143 | A0 | 31 May 2015 |
| | | IL | 238143 | B | 30 January 2020 |
| | | KR | 20210038728 | A | 07 April 2021 |
| | | KR | 102320907 | B1 | 02 November 2021 |
| | | HK | 1256631 | A1 | 27 September 2019 |
| | | KR | 20200026323 | A | 10 March 2020 |
| | | KR | 102237639 | B1 | 07 April 2021 |
| | | JP | 2016196484 | A | 24 November 2016 |
| | | JP | 6186045 | B2 | 23 August 2017 |
| | | SI | 3342785 | T1 | 28 February 2020 |
| | | NZ | 705394 | A | 26 October 2018 |
| | | EP | 2907824 | A1 | 19 August 2015 |
| | | EP | 2907824 | A4 | 08 June 2016 |
| | | EP | 2907824 | B1 | 11 April 2018 |
| | | RU | 2015113767 | A | 10 December 2016 |
| | | RU | 2664465 | C2 | 17 August 2018 |
| | | AU | 2013328111 | A1 | 12 March 2015 |
| | | AU | 2013328111 | B2 | 02 November 2017 |
| | | RS | 60000 | B1 | 30 April 2020 |
| | | IL | 271760 | A | 27 February 2020 |
| | | IL | 271760 | B | 01 August 2022 |

Form PCT/ISA/210 (patent family annex) (July 2022)

**INTERNATIONAL SEARCH REPORT**
**Information on patent family members**

International application No.
**PCT/CN2023/073014**

| Patent document cited in search report | Publication date (day/month/year) | Patent family member(s) | Publication date (day/month/year) |
|---|---|---|---|
| | | MX 2015003903 A | 17 July 2015 |
| | | MX 364484 B | 29 April 2019 |
| | | ES 2671644 T3 | 07 June 2018 |
| | | TW 201920098 A | 01 June 2019 |
| | | TWI 696611 B | 21 June 2020 |
| | | HK 1210477 A1 | 22 April 2016 |
| | | NZ 746439 A | 29 November 2019 |
| | | EP 3342785 A1 | 04 July 2018 |
| | | EP 3342785 B1 | 25 December 2019 |
| | | JP 2020180124 A | 05 November 2020 |
| | | JP 6952835 B2 | 27 October 2021 |
| | | NZ 740948 A | 29 November 2019 |
| | | BR 122021014365 B1 | 05 July 2022 |
| | | JP 2018188455 A | 29 November 2018 |
| | | JP 6715888 B2 | 01 July 2020 |
| | | HUE 039000 T2 | 28 December 2018 |
| | | KR 20220146669 A | 01 November 2022 |
| | | KR 102498405 B1 | 09 February 2023 |
| | | PH 12015500667 A1 | 18 May 2015 |
| | | MX 2019004502 A | 21 August 2019 |
| | | ZA 201900820 B | 31 August 2022 |
| | | LT 3342785 T | 10 February 2020 |
| | | US 2019008981 A1 | 10 January 2019 |
| | | US 10973924 B2 | 13 April 2021 |
| | | AU 2022203560 A1 | 16 June 2022 |
| | | JP 2018008982 A | 18 January 2018 |
| | | JP 6371452 B2 | 08 August 2018 |
| | | HRP 20200353 T1 | 12 June 2020 |
| | | RU 2018128384 A | 02 October 2018 |
| | | RU 2018128384 A3 | 20 December 2021 |
| | | HUE 048748 T2 | 28 August 2020 |
| | | KR 20230024433 A | 20 February 2023 |
| | | IL 294622 A | 01 September 2022 |
| | | AU 2018200308 A1 | 01 February 2018 |
| | | AU 2018200308 B2 | 07 November 2019 |
| CN 113939318 A | 14 January 2022 | KR 20220015445 A | 08 February 2022 |
| | | BR 112021023901 A2 | 18 January 2022 |
| | | TW 202108180 A | 01 March 2021 |
| | | EP 3976113 A1 | 06 April 2022 |
| | | SG 11202112429 PA | 30 December 2021 |
| | | CA 3142119 A1 | 03 December 2020 |
| | | WO 2020240467 A1 | 03 December 2020 |
| | | JP 2022534725 A | 03 August 2022 |
| | | IL 288485 A | 01 January 2022 |
| | | AU 2020285681 A1 | 27 January 2022 |
| EP 2910573 A1 | 26 August 2015 | TW 201420118 A | 01 June 2014 |
| | | US 2015352224 A1 | 10 December 2015 |
| | | US 9872924 B2 | 23 January 2018 |
| | | WO 2014061277 A1 | 24 April 2014 |
| | | JPWO 2014061277 A1 | 05 September 2016 |

Form PCT/ISA/210 (patent family annex) (July 2022)

**INTERNATIONAL SEARCH REPORT**
**Information on patent family members**

International application No.
**PCT/CN2023/073014**

| Patent document cited in search report | Publication date (day/month/year) | Patent family member(s) | Publication date (day/month/year) |
|---|---|---|---|
| | | JP 6272230 B2 | 31 January 2018 |
| | | EP 2910573 A4 | 15 June 2016 |
| | | EP 2910573 B1 | 19 February 2020 |
| | | ES 2782248 T3 | 11 September 2020 |
| | | US 2018071403 A1 | 15 March 2018 |
| | | US 10729782 B2 | 04 August 2020 |
| EP 3130608 A1 | 15 February 2017 | ES 2754348 T3 | 17 April 2020 |
| | | US 2017035906 A1 | 09 February 2017 |
| | | US 11185594 B2 | 30 November 2021 |
| | | WO 2015155976 A1 | 15 October 2015 |
| | | JP 2020079237 A | 28 May 2020 |
| | | JP 6861777 B2 | 21 April 2021 |
| | | JPWO 2015155976 A1 | 13 April 2017 |
| | | JP 6612738 B2 | 27 November 2019 |
| | | TW 201620554 A | 16 June 2016 |
| | | EP 3130608 A4 | 13 September 2017 |
| | | EP 3130608 B1 | 04 September 2019 |
| WO 2022011075 A1 | 13 January 2022 | WO 2022011075 A9 | 10 March 2022 |
| | | EP 4178559 A1 | 17 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 106163559 B


### Non-patent literature cited in the description


- **H. BUNDGAARD**. Design of Prodrugs. Elsevier, 1985
- *J. biol. chem*, 1968, vol. 243, 3558
- **WARD et al.** *Nature*, 1989, vol. 341, 544-546
- **BIRD et al.** *Science*, 1988, vol. 242, 423-426
- **HUSTON et al.** *Proc. Natl. Acad. Sci USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immuno l.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol.*, 2001
- **KABAT E.A. et al.** *Sequences of proteins of immunological interest*, 1991